# EUROPEAN PATENT SPECIFICATION

(11) **EP 1 664 029 B1**
(45) Date of publication and mention of the grant of the patent: **02.12.2009**
(21) Application number: 04768482.4
(22) Date of filing: 14.09.2004
(51) Int. Cl.: C07D 401/14, C07D 401/12, C07D 413/12, C07D 417/14, C07D 403/14, A61K 31/517, A61P 35/00

(54) **QUINAZOLINE DERIVATIVES AS ANTITUMOR AGENTS**
CHINAZOLINDERIVATE ALS ANTITUMORMITTEL
DERIVES DE QUINAZOLINE UTILISES COMME AGENTS ANTI-TUMEUR

(30) Priority: 16.09.2003 GB 0321648
(43) Date of publication of application: 07.06.2006
(73) Proprietor: AstraZeneca AB, 151 85 Södertälje (SE)
(72) Inventor: BRADBURY, Robert, H., c/o AstraZeneca R & D, Macclesfield, Cheshire SK10 4TG (GB); HENNEQUIN, Laurent, F.A., c/o AstraZeneca Pharma, F-51689 Reims (FR); KETTLE, Jason, G., c/o AstraZeneca R & D, Macclesfield, Cheshire SK10 4TG (GB)
(86) International application number: PCT/GB2004/003936
(87) International publication number: WO 2005/026152

(56) References cited:
- WO-A-03/040108
- WO-A-03/040109
- WO-A-2004/096226

## Description

The invention concerns certain novel quinazoline derivatives, or pharmaceutically-acceptable salts thereof, which possess anti-tumour activity and are accordingly useful in methods of treatment of the human or animal body. The invention also concerns processes for the manufacture of said quinazoline derivatives, to pharmaceutical compositions containing them and to their use in therapeutic methods, for example in the manufacture of medicaments for use in the prevention or treatment of solid tumour disease in a warm-blooded animal such as man.

Many of the current treatment regimes for diseases resulting from the abnormal regulation of cellular proliferation such as psoriasis and cancer, utilise compounds that inhibit DNA synthesis and cellular proliferation. To date, compounds used in such treatments are generally toxic to cells however their enhanced effects on rapidly dividing cells such as tumour cells can be beneficial. Alternative approaches to these cytotoxic anti-tumour agents are currently being developed, for example selective inhibitors of cell signalling pathways. These types of inhibitors are likely to have the potential to display an enhanced selectivity of action against tumour cells and so are likely to reduce the probability of the therapy possessing unwanted side effects.

Eukaryotic cells are continually responding to many diverse extracellular signals that enable communication between cells within an organism. These signals regulate a wide variety of physical responses in the cell including proliferation, differentiation, apoptosis and motility. The extracellular signals take the form of a diverse variety of soluble factors including growth factors as well as paracrine and endocrine factors. By binding to specific transmembrane receptors, these ligands integrate the extracellular signal to the intracellular signalling pathways, therefore transducing the signal across the plasma membrane and allowing the individual cell to respond to its extracellular signals. Many of these signal transduction processes utilise the reversible process of the phosphorylation of proteins that are involved in the promotion of these diverse cellular responses. The phosphorylation status of target proteins is regulated by specific kinases and phosphatases that are responsible for the regulation of about one third of all proteins encoded by the mammalian genome. As phosphorylation is such an important regulatory mechanism in the signal transduction process, it is therefore not surprising that aberrations in these intracellular pathways result in abnormal cell growth and differentiation and so promote cellular transformation (reviewed in Cohen et al, Curr Opin Chem Biol, 1999, 3, 459-465).

It has been widely shown that a number of these tyrosine kinases are mutated to constitutively active forms and/or when over-expressed result in the transformation of a variety of human cells. These mutated and over-expressed forms of the kinase are present in a large proportion of human tumours (reviewed in Kolibaba et al, Biochimica et Biophysica Acta, 1997, 133, F217-F248). As tyrosine kinases play fundamental roles in the proliferation and differentiation of a variety of tissues, much focus has centred on these enzymes in the development of novel anti-cancer therapies. This family of enzymes is divided into two groups - receptor and non-receptor tyrosine kinases e.g. EGF Receptors and the SRC family respectively. From the results of a large number of studies including the Human Genome Project, about 90 tyrosine kinase have been identified in the human genome, of this 58 are of the receptor type and 32 are of the non-receptor type. These can be compartmentalised in to 20 receptor tyrosine kinase and 10 non-receptor tyrosine kinase sub-families (Robinson et al, Oncogene, 2000, 19, 5548-5557).

The receptor tyrosine kinases are of particular importance in the transmission of mitogenic signals that initiate cellular replication. These large glycoproteins, which span the plasma membrane of the cell possess an extracellular binding domain for their specific ligands (such as Epidermal Growth Factor (EGF) for the EGF Receptor). Binding of ligand results in the activation of the receptor's kinase enzymatic activity that is encoded by the intracellular portion of the receptor. This activity phosphorylates key tyrosine amino acids in target proteins, resulting in the transduction of proliferative signals across the plasma membrane of the cell.

It is known that the erbB family of receptor tyrosine kinases, which include EGFR, erbB2, erbB3 and erbB4, are frequently involved in driving the proliferation and survival of tumour cells (reviewed in Olayioye et al., EMBO J., 2000, 19, 3159). One mechanism in which this can be accomplished is by overexpression of the receptor at the protein level, generally as a result of gene amplification. This has been observed in many common human cancers (reviewed in Klapper et al., Adv. Cancer Res., 2000, 77, 25) such as breast cancer (Sainsbury et al., Brit. J. Cancer, 1988, 58, 458; Guerin et al., Oncogene Res., 1988, 3, 21; Slamon et al., Science, 1989, 244, 707; Klijn et al., Breast Cancer Res. Treat., 1994, 29, 73 and reviewed in Salomon et al., Crit. Rev. Oncol. Hematol., 1995, 19, 183), non-small cell lung cancers (NSCLCs) including adenocarcinomas (Cerny et al., Brit. J. Cancer, 1986, 54, 265; Reubi et al., Int. J. Cancer, 1990, 45, 269; Rusch et al., Cancer Research, 1993, 53, 2379; Brabender et al, Clin. Cancer Res., 2001, 7, 1850) as well as other cancers of the lung (Hendler et al., Cancer Cells, 1989, 7, 347; Ohsaki et al., Oncol. Rep., 2000, 7, 603), bladder cancer (Neal et al., Lancet, 1985, 366; Chow et al., Clin. Cancer Res., 2001, 7, 1957, Zhau et al., Mol Carcinog., 3, 254), oesophageal cancer (Mukaida et al., Cancer, 1991, 68, 142), gastrointestinal cancer such as colon, rectal or stomach cancer (Bolen et al., Oncogene Res., 1987, 1, 149; Kapitanovic et al., Gastroenterology, 2000, 112, 1103; Ross et al., Cancer Invest., 2001, 19, 554), cancer of the prostate (Visakorpi et al., Histochem. J., 1992, 24, 481; Kumar et al., 2000, 32, 73; Scher et al., J. Natl. Cancer Inst., 2000, 92, 1866), leukaemia (Konaka et al., Cell, 1984, 37, 1035, Martin-Subero et al., Cancer Genet Cytogenet., 2001, 127, 174), ovarian (Hellstrom et al., Cancer Res., 2001, 61, 2420), head and neck (Shiga et al., Head Neck, 2000, 22, 599) or pancreatic cancer (Ovotny et al., Neoplasma, 2001, 48, 188). As more human tumour tissues are tested for expression of the erbB family of receptor tyrosine kinases it is expected that their widespread prevalence and importance will be further enhanced in the future.

As a consequence of the mis-regulation of one or more of these receptors (in particular erbB2), it is widely believed that many tumours become clinically more aggressive and so correlate with a poorer prognosis for the patient (Brabender et al, Clin. Cancer Res., 2001, 7, 1850; Ross et al, Cancer Investigation, 2001, 19, 554, Yu et al., Bioessays, 2000, 22.7, 673). In addition to these clinical findings, a wealth of pre-clinical information suggests that the erbB family of receptor tyrosine kinases are involved in cellular transformation. This includes the observations that many tumour cell lines overexpress one or more of the erbB receptors and that EGFR or erbB2 when transfected into non-tumour cells have the ability to transform these cells. This tumourigenic potential has been further verified as transgenic mice that overexpress erbB2 spontaneously develop tumours in the mammary gland. In addition to this, a number of pre-clinical studies have demonstrated that anti-proliferative effects can be induced by knocking out one or more erbB activities by small molecule inhibitors, dominant negatives or inhibitory antibodies (reviewed in Mendelsohn et al., Oncogene, 2000, 19, 6550). Thus it has been recognised that inhibitors of these receptor tyrosine kinases should be of value as a selective inhibitor of the proliferation of mammalian cancer cells (Yaish et al. Science, 1988, 242, 933, Kolibaba et al, Biochimica et Biophysica Acta, 1997, 133, F217-F248; Al-Obeidi et al, 2000, Oncogene, 19, 5690-5701; Mendelsohn et al, 2000, Oncogene, 19, 6550-6565). In addition to this pre-clinical data, findings using inhibitory antibodies against EGFR and erbB2 (c-225 and trastuzumab respectively) have proven to be beneficial in the clinic for the treatment of selected solid tumours (reviewed in Mendelsohn et al, 2000, Oncogene, 19, 6550-6565).

Amplification and/or activity of members of the ErbB type receptor tyrosine kinases have been detected and so have been implicated to play a role in a number of non-malignant proliferative disorders such as psoriasis (Ben-Bassat, Curr. Pharm. Des., 2000, 6, 933; Elder et al., Science, 1989, 243, 811), benign prostatic hyperplasia (BPH) (Kumar et al., Int. Urol. Nephrol., 2000, 32,73), atherosclerosis and restenosis (Bokemeyer et al., Kidney Int., 2000, 58, 549). It is therefore expected that inhibitors of erbB type receptor tyrosine kinases will be useful in the treatment of these and other non-malignant disorders of excessive cellular proliferation.

International Patent Applications WO 96/09294, WO 96/15118, WO 96/16960, WO 96/30347, WO 96/33977, WO 96/33978, WO 96/33979, WO 96/33980, WO 96/33981, WO 97/03069, WO 97/13771, WO 97/30034, WO 97/30035, WO 97/38983, WO 98/02437, WO 98/02434, WO 98/02438, WO 98/13354, WO 99/35132, WO 99/35146, WO01/21596, WO 01/55141 and WO 02/18372 disclose that certain quinazoline derivatives which bear an anilino substituent at the 4-position possess receptor tyrosine kinase inhibitory activity.

International Patent Applications WO01/94341 discloses that certain quinazoline derivatives which carry a 5-substituent are inhibitors of the Src family of non-receptor tyrosine kinases, such as c-Src, c-Yes and c-Fyn.

International Patent applications WO03/040108 and WO03/040109 disclose that certain quinazoline derivatives which carry a 5-substituent are inhibitors of the erbB family of tyrosine kinase inhibitors, particularly EGFR and erb-B2 receptor tyrosine kinases.

We have now found that surprisingly certain quinazoline derivatives substituted at the 5-position with a substituent containing certain substituted alkanoyl groups possess potent anti-tumour activity. Without wishing to imply that the compounds disclosed in the present invention possess pharmacological activity only by virtue of an effect on a single biological process, it is believed that the compounds provide an anti-tumour effect by way of inhibition of one or more of the erbB family of receptor tyrosine kinases that are involved in the signal transduction steps which lead to the proliferation of tumour cells. In particular, it is believed that the compounds of the present invention provide an anti-tumour effect by way of inhibition of EGFR and/or erbB2 receptor tyrosine kinases.

Generally the compounds of the present invention possess potent inhibitory activity against the erbB receptor tyrosine kinase family, for example by inhibition of EGFR and/or erbB2 and/or erbB4 receptor tyrosine kinases, whilst possessing less potent inhibitory activity against other kinases. Furthermore, generally the compounds of the present invention possess substantially better potency against the erbB2 over that of the EGFR tyrosine kinase, thus potentially providing effective treatment for erbB2 driven tumours. Accordingly, it may be possible to administer a compound according to the present invention at a dose that is sufficient to inhibit erbB2 tyrosine kinase whilst having no significant effect upon EGFR (or other) tyrosine kinases. The selective inhibition provided by the compounds according to the present invention may provide treatments for conditions mediated by erbB2 tyrosine kinase, whilst reducing undesirable side effects that may be associated with the inhibition of other tyrosine kinases.

Generally the compounds according to the invention exhibit favourable DMPK properties, for example high bioavailability and/or high free-plasma levels.

According to a first aspect of the invention there is provided a quinazoline derivative of the Formula Ia: 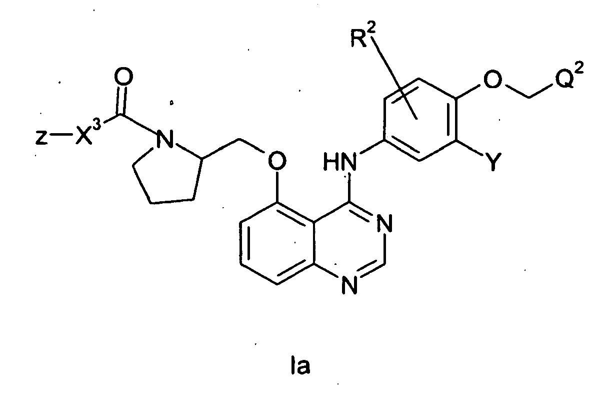 wherein:
**R²** is selected from hydrogen and halogeno;
**Y** is selected from halogeno, (1-4C)alkyl, (1-4C)alkoxy, (2-4C)alkenyl and (2-4C)alkynyl;
**Q²** is selected from phenyl, pyridyl, pyrazinyl, 1,3-thiazolyl and isoxazolyl
   and wherein Q² optionally bears one or more substituents, which may be the same or different, selected from halogeno, hydroxy, cyano, carboxy, nitro, amino, (1-4C)alkyl, (1-4C)alkoxy, (2-4C)alkenyl, (2-4C)alkynyl, (1-4C)alkylthio, (1-4C)alkylsulfinyl, (1-4C)alkylsulfonyl, (2-4C)alkanoyl, N-(1-4C)alkylamino, N, N-di-[(1-4C)alkyl]amino, (1-4C)alkoxycarbonyl, carbamoyl, N-(1-4C)alkylcarbamoyl, N, N-di-[(1-4C)alkyl]carbamoyl, (2-4C)alkanoyloxy, (2-4C)alkanoylamino, N-(1-4C)alkyl-(2-4C)alkanoylamino, halogeno-(1-4C)alkyl, hydroxy-(1-4C)alkyl, (1-4C)alkoxy-(1-4C)alkyl, cyano-(1-4C)alkyl, carboy-(1-4C)alkyl, amino-(1-4C)alkyl, N-(1-4C)alkylamino-(1-4C)alkyl and N, N-di-[(1-4C)alkyl]amino-(1-4C)alkyl;
   **Z** is selected from hydroxy, di-[(1-3C)alkyl]amino and (1-6C)alkoxy;
   **X³** is selected from -CH₂-, -CH₂CH₂-, -(CR⁸R⁹)-, -(CR⁸R⁹CH₂)-, -(CH₂CR⁸R⁹)- and (3-6C)cycloalkylene, wherein each of R⁸ and R⁹, which may be the same or different, is selected from hydrogen and methyl;
   or a pharmaceutically acceptable salt thereof.

In this specification the generic term "alkyl" includes both straight-chain and branched-chain alkyl groups such as propyl, isopropyl and tert-butyl, and (3-7C)cycloalkyl groups such as cyclopropyl, cyclobutyl, cyclopentyl, cyclohexyl and cycloheptyl. However references to individual alkyl groups such as "propyl" are specific for the straight-chain version only, references to individual branched-chain alkyl groups such as "isopropyl" are specific for the branched-chain version only and references to individual cycloalkyl groups such as "cyclopentyl" are specific for that 5-membered ring only. An analogous convention applies to other generic terms, for example (1-6C)alkoxy includes methoxy, ethoxy, cyclopropyloxy and cyclopentyloxy, (1-6C)alkylamino includes methylamino, ethylamino, cyclobutylamino and cyclohexylamino, and di-[(1-6Calkyl]amino includes dimethylamino, diethylamino, N-cyclobutyl-N-methylamino and N-cyclohexyl-N-ethylamino.

It is to be understood that, insofar as certain of the compounds of Formula Ia defined above may exist in optically active or racemic forms by virtue of one or more asymmetric carbon atoms, the invention includes in its definition any such optically active or racemic form which possesses the above-mentioned activity. It is further to be understood that in the names of chiral compounds (*R*,*S*) denotes any scalemic or racemic mixture while (*R*) and (*S*) denote the enantiomers. In the absence of (*R*,*S*), (*R*) or (*S*) in the name it is to be understood that the name refers to any scalemic or racemic mixture, wherein a scalemic mixture contains *R* and *S* enantiomers in any relative proportions and a racemic mixture contains R and S enantiomers in the ratio 50:50. The synthesis of optically active forms may be carried out by standard techniques of organic chemistry well known in the art, for example by synthesis from optically active starting materials or by resolution of a racemic form. Similarly, the above-mentioned activity may be evaluated using the standard laboratory techniques referred to hereinafter.

Suitable values for the generic radicals referred to above include those set out below.

It is to be understood that reference to (3-7C)cycloalkylene used herein refers to a divalent (3-7C)cycloalkane linking group, which group may be linked via different carbon atoms in the (3-7C)cycloalkylene ring, or which may be linked via a single carbon atom in the (3-7C)cycloalkylene ring. Accordingly, reference to, for example, a "cyclopropylene" group includes cycloprop-1,2-ylene and a cyclopropylidene group of the formula: 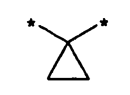 wherein * represent the bonds from the divalent cyclopropylidene group.

However references to an individual (3-7C)cycloalkylene group such as cyclopropylidene are specific for that group only. A similar convention is adopted for the (3-7C)cycloalkenylene groups.

For the avoidance of any doubt the nitrogen atom in the pyrrolidin-2-yl group to which the group ZX³C(O) is attached is not quatemised; namely the group ZX³C(O) is attached to the nitrogen atom in the pyrrolidin-2-yl group via substitution of an NH group in the heterocyclyl ring, for example the ZX³C(O)group is attached to the pyrrolidin-2-yl ring at the 1-position.

Suitable values for any of the 'R' groups (R², R⁸, R⁹), Y, or for various groups within a Q², X³ or Z group include:-

| | |
|---|---|
| for halogeno | fluoro, chloro, bromo and iodo; |
| for (1-6C)alkyl: | methyl, ethyl, propyl, isopropyl and tert-butyl; |
| for (2-8C)alkenyl: | vinyl, isopropenyl, allyl and but-2-enyl; |
| for (2-8C)alkynyl: | ethynyl, 2-propynyl and but-2-ynyl; |
| for (1-6C)alkoxy: | methoxy, ethoxy, propoxy, isopropoxy and butoxy; |
| for (2-6C)alkenyloxy: | vinyloxy and allyloxy; |
| for (2-6C)alkynyloxy: | ethynyloxy and 2-propynyloxy; |
| for (1-6C)alkylthio: | methylthio, ethylthio and propylthio; |
| for (1-6C)alkylsulfinyl: | methylsulfinyl and ethylsulfinyl; |
| for (1-6C)alkylsulfonyl: | methylsulfonyl and ethylsulfonyl; |
| for (1-6C)alkylamino: | methylamino, ethylamino, propylamino, isopropylamino and butylamino; |
| for di-[(1-6C)alkyl]amino: | dimethylamino, diethylamino, N-ethyl-N-methylamino and diisopropylamino; |
| for (1-6C)alkoxycarbonyl: | methoxycarbonyl, ethoxycarbonyl, propoxycarbonyl and tert-butoxycarbonyl; |
| for N-(1-6C)alkylcarbamoyl: | N-methylcarbamoyl, N-ethylcarbamoyl and N-propylcarbamoyl; |
| for N,N-di-[(1-6C)alkyl]carbamoyl: | N,N-dimethylcarbamoyl, N-ethyl-N-methylcarbamoyl and N,N-diethylcarbamoyl; |
| for (2-6C)alkanoyl: | acetyl, propionyl, butyryl and isobuyryl; |
| for (2-6C)alkanoyloxy: | acetoxy and propionyloxy; |
| for (2-6C)alkanoylamino: | acetamido and propionamido; |
| for N-(1-6C)alkyl-(2-6C)alkanoylamino: | N-methylacetamido and N-methylpropionamido; |
| for N-(1-6C)alkylsulfamoyl: | N-methylsulfamoyl and N-ethylsulfamoyl; |
| for N,N-di-[(1-6C)akyl]sulfamoyl: | N,N-dimethylsulfamoyl; |
| for (1-6C)alkanesulfonylamino: | methanesulfonylamino and ethanesulfonylamino; |
| for N-(1-6C)alkyl-(1-6C)alkanesulfonylamino: | N-methylmethanesulfonylamino and N-methylethanesulfonylamino; |
| for (3-6C)alkenoylamino: | acrylamido, methacrylamido and crotonamido; |
| for N-(1-6C)alkyl-(3-6C)alkenoylamino: | N-methylacrylamido and N-methylcrotonamido; |
| for (3-6C)alkynoylamino: | propiolamido; |
| for N-(1-6C)alkyl-(3-6C)alkynoylamino: | N-methylpropiolamido; |
| for amino-(1-6C)alkyl: | aminomethyl, 2-aminoethyl, 1-aminoethyl and 3-aminopropyl; |
| for (1-6C)alkylamino-(1-6C)alkryl: | methylaminomethyl, ethylaminomethyl, 1-methylaminoethyl, 2-methylaminoethyl, 2-ethylaminoethyl and 3-methylaminopropyl; |
| for di-[(1-6C)alkyl]amino-(1-6C)alkyl: | dimethylaminomethyl, diethylaminomethyl, 1-dimethylaminoethyl, 2-dimethylaminoethyl and 3-dimethylaminopropyl; |
| for halogeno-(1-6C)alkyl: | chloromethyl, 2-chloroethyl, 1-chloroethyl and 3-chloropropyl; |
| for hydroxy-(1-6C)alkyl: | hydroxymethyl, 2-hydroxyethyl, 1-hydroxyethyl and 3-hydroxypropyl; |
| for (1-6C)alkoxy-(1-6C)alkyl: | methoxymethyl, ethoxymethyl, 1-methoxyethyl, 2-methoxyethyl, 2-ethoxyethyl and 3-methoxypropyl; |
| for cyano-(1-6C)alkyl: | cyanomethyl, 2-cyanoethyl, 1-cyanoethyl and 3-cyanopropyl; |
| for (1-6C)alkylthio-(1-6C)alkyl: | methylthiomethyl, ethylthiomethyl, 2-methylthioethyl, 1-methylthioethyl and 3-methylthiopropyl; |
| for (1-6C)alkylsulfinyl-(1-6C)alkyl: | methylsulfinylmethyl, ethylsulfinylmethyl, 2-methylsulfinylethyl, 1-methylsulfinylethyl and 3-methylsulfinylpropyl; |
| for (1-6C)alkylsulfonyl-(1-6C)alkyl: | methylsulfonylmethyl, ethylsulfonylmethyl, 2-methylsulfonylethyl, 1-methylsulfonylethyl and 3-methylsulfonylpropyl; |
| for (2-6C)alkanoylamino-(1-6C)alkyl: | acetamidomethyl, propionamidomethyl and 2-acetamidoethyl; |
| for N-(1-6C)alkyl-(2-6C)alkanoylamino-(1-6C)alkyl: | N-methylacetamidomethyl, 2-(N-methylacetamido)ethyl and 2-(N-methylpropionamido)ethyl; |
| for (1-6C)alkoxycarbonylamino-(1-6C)alkyl: | methoxycarbonylaminomethyl, ethoxycarbonylaminomethyl, tert-butoxycarbonylaminomethyl and 2-methoxycarbonylaminoethyl; |
| for (2-6C)alkanoyloxy-(1-6C)alkyl: | acetoxymethyl, 2-acetoxyethyl and 2-propionyloxyethyl; |
| for carbamoyl-(1-6C)alkyl: | carbamoylmethyl, 1-carbamoylethyl, 2-carbamoylethyl and 3-carbamoylpropyl; |
| for (2-6C)alkanoyl-(1-6C)alkyl: | acetylmethyl and 2-acetylethyl; |
| for N-(1-6C)alkylcarbamoyl-(1-6C)alkyl: | N-methylcarbamoylmethyl, |
| | N-ethylcarbamoylmethyl, |
| | N-propylcarbamoylinethyl, |
| | 1-(N-methylcarbamoyl)ethyl, |
| | 1-(N-ethylcarbamoyl)ethyl, |
| | 2-(N-methylcarbamoyl)ethyl, |
| | 2-(N-ethylcarbamoyl)ethyl and |
| | 3-(N-methylcarbamoyl)propyl; |
| for N,N-di[(1-6C)alkyl]carbamoyl-(1-6C)akyl: | N,N-dimethylcarbamoylmethyl, N,N-diethylcarbamoylmethyl, |
| | 2-(N,N-dimethylcarbwnoyl)ethyl, and 3-(N,N-dimethylcarbamoyl)propyl; |
| for sulfamoyl(1-6C)alkyl: | sulfamoylmethyl, 1-sulfamoylethyl, 2-sulfamoylethyl and 3-sulfamoylpropyl; |
| for N-(1-6C)alkylsulfamoyl(1-6C)alkyl: | N-methylsulfamoylmethyl, N-ethylsulfamoylmethyl, N-propylsulfamoylmethyl, 1-(N-methylsulfamoyl)ethyl, 2-(N-methylsulfamoyl)ethyl and 3-(N-methylsulfamoyl)propyl; |
| for N,N di-(1-6C)alkylsulfamoyl(1-6C)alkyl: | N,N-dimethylsulfamoylmethyl, N,N-diethylsulfamoylmethyl, N-methyl,N-ethylsulfamoylmethyl, 1-( N,N-dimethylsulfamoyl)ethyl, 1-(N,N-diethylsulfamoyl)ethyl, 2-(N,N-dimethylsulfamoyl)ethyl, 2-(N,N-diethylsulfamoyl)ethyl and 3-(N,N-dimethylsulfamoyl)propyl; |
| for carboxy-(1-6C)alkyl: | carboxymethyl, 2-carboxyethyl, 1-carboxyethyl and 3-carboxypropyl; and |
| for (1-6C)alkoxycarbonyl-(1-6C)alkyl | methoxycarbonylmethyl, ethoxycarbonylmethyl, tert-butoxycarbonylmethyl and 2-methoxycarbonylethyl. |

As defined hereinbefore, in the group of the formula -OCH₂-Q², it is the oxygen atom, not the carbon atom, of the OC(H)₂ linking group which is attached to the phenyl ring in the Formula Ia and the carbon atom is attached to the Q² group

It is to be understood that certain compounds of the Formula Ia may exist in solvated as well as unsolvated forms such as, for example, hydrated forms. It is to be understood that the invention encompasses all such solvated forms which exhibit an inhibitory effect on an erbB receptor tyrosine kinase.

It is also to be understood that certain compounds of the Formula Ia may exhibit polymorphism, and that the invention encompasses all such forms which exhibit an inhibitory effect on an erbB receptor tyrosine kinase.

It is also to be understood that the invention relates to all tautomeric forms of the compounds of the Formula Ia forms which exhibit an inhibitory effect on an erbB receptor tyrosine kinase.

A suitable pharmaceutically-acceptable salt of a compound of the Formula Ia is, for example, an acid-addition salt of a compound of the Formula Ia, for example an acid-addition salt with an inorganic or organic acid such as hydrochloric, hydrobromic, sulfuric, trifluoroacetic, citric or maleic acid; or, for example, a salt of a compound of the Formula Ia which is sufficiently acidic, for example an alkali or alkaline earth metal salt such as a calcium or magnesium salt, or an ammonium salt, or a salt with an organic base such as methylamine, dimethylamine, trimethylamine, piperidine, morpholine or tris-(2-hydroxyethyl)amine.

Particular novel compounds of the invention include, for example, quinazoline derivatives of the Formula Ia, or pharmaceutically-acceptable salts thereof, wherein, unless otherwise stated, each of R², Q², X³, Y, and Z has any of the meanings defined hereinbefore or in paragraphs (a) to (nnn) hereinafter :-
(a) Y is selected from halogeno, (1-4C)alkyl, (1-4C)alkoxy, (2-4C)alkenyl and (2-4C)alkynyl;
(b) Y is selected from halogeno, (1-4C)alkyl, (1-4C)alkoxy and (2-4C)alkynyl;
(c) Y is selected from halogeno and (1-4C)alkyl;
(d) Y is halogeno;
(e) Y is (1-4C)alkyl (particularly (1-2C)alkyl);
(f) Y is selected from chloro and methyl;
(g) Y is chloro;
(h) Y is methyl;
(i) Q² is selected from phenyl, 2-pyridyl, 3-pyridyl, 2-pyrazinyl, 1,3-thiazol-2-yl, 1,3-thiazol-4-yl, 1,3-thiazol-5-yl, and isoxazol-3-yl,
   and wherein Q² optionally bears one or more substituents (for example 1, 2 or 3), which may be the same or different, selected from halogeno, hydroxy, cyano, carboxy, nitro, amino, (1-4C)alkyl, (1-4C)alkoxy, (2-4C)alkenyl, (2-4C)alkynyl, (1-4C)alkylthio, (1-4C)alkylsulfinyl, (1-4C)alkylsulfonyl, (2-4C)alkanoyl, N-(1-4C)alkylamino, N,N-di-[(1-4C)alkyl]amino, (1-4C)alkoxycarbonyl, carbamoyl, N-(1-4C)alkylcarbamoyl, N, N-di-[(1-4C)alkyl]carbamoyl, (2-4C)alkanoyloxy, (2-4C)alkanoylamino, N-(1-4C)alkyl-(2-4C)alkanoylamino, halogeno-(1-4C)alkyl, hydroxy-(1-4C)alkyl, (1-4C)alkoxy-(1-4C)alkyl, cyano-(1-4C)alkyl, carboxy-(1-4C)alkyl, amino-(1-4C)alkyl, N-(1-4C)alkylamino-(1-4C)alkyl and N, N-di-[(1-4C)alkyl]amino-(1-4C)alkyl;
(j) Q² is selected from phenyl, 2-pyridyl, 2-pyrazinyl, 1,3-thiazol-4-yl, 1,3-thiazol-5-yl, and isoxazol-3-yl,
   and wherein Q² optionally bears one or more substituents (for example 1, 2 or 3), which may be the same or different, as hereinbefore defined in (i);
(k) Q² is selected from phenyl, 2-pyridyl, 3-pyridyl and isoxazol-3-yl,
   and wherein Q² optionally bears one or more substituents (for example 1, 2 or 3), which may be the same or different, as hereinbefore defined in (i);
(l) Q² is selected from 2- or 3-pyridyl, 2-pyrazinyl, 1,3-thiazol-2-yl, 1,3-thiazol-4-yl and 3-isoxazolyl (particularly 3-pyridyl, 1,3-thiazol-4-yl and 3-isoxazolyl), and wherein Q² optionally bears one or more substituents (for example 1, 2 or 3), which may be the same or different, as hereinbefore defined in (i);
(m) Q² is selected from phenyl, 2-pyridyl, 3-pyridyl, 2-pyrazinyl, 1,3-thiazol-2-yl, 1,3-thiazol-4-yl, 1,3-thiazol-5-yl and isoxazol-3-yl, and wherein Q² optionally bears one or more substituents (for example 1, 2 or 3), which may be the same or different, selected from fluoro, chloro, bromo, hydroxy, carboxy, cyano, nitro, amino, methyl, ethyl, isopropyl, methoxy, ethoxy, vinyl, allyl, ethynyl, 2-propynyl, methylthio, methylsulfinyl, methylsulfonyl, acetyl, propionyl methylamino, ethylamino, N,N-dimethylamino, N,N-diethylamino, N-methyl-N-ethylamino methoxycarbonyl, ethoxycarbonyl, carbamoyl, N-methylcarbamoyl, N,N-dimethylcarbamoyl, acetoxy, acetamido, fluoromethyl, 2-fluoroethyl, chloromethyl, 2-chloroethyl, hydroxymethyl, 2-hydroxyethyl, methoxymethyl, 2-methoxyethyl, cyanomethyl, 2-cyanoethyl, carboxymethyl, 2-carboxymethyl, aminomethyl, methylaminomethyl, ethylaminomethyl, N,N-dimethylaminomethyl, N,N-diethylaminomethyl, N-methyl-N-ethylaminomethyl, 2-aminoethyl, 2-(methylamino)ethyl, 2-(ethylamino)ethyl, 2-(N,N-dimethylamino)ethyl, 2-(N,N-diethylamino)ethyl, 2-(N-methyl-N-ethylamino)ethyl, carbamoylmethyl, N-methylcarbamoylmethyl and N,N-dimethylcarbamoylmethyl;
(n) Q² is selected from phenyl, 2-pyridyl, 2-pyrazinyl, 1,3-thiazol-4-yl, 1,3-thiazol-5-yl, and isoxazol-3-yl, and wherein Q² optionally bears one or more substituents (for example 1, 2 or 3), which may be the same or different, as hereinbefore defined in (m);
(o) Q² is selected from phenyl, 2-pyridyl, 3-pyridyl and isoxazol-3-yl,
   and wherein Q² optionally bears one or more substituents (for example 1, 2 or 3), which may be the same or different, as hereinbefore defined in (m);
(p) Q² is selected from 2- or 3-pyridyl, 2-pyrazinyl, 1,3-thiazol-2-yl, 1,3-thiazol-4-yl and 3-isoxazolyl (particularly 3-pyridyl, 1,3-thiazol-4-yl and 3-isoxazolyl),
   and wherein Q² optionally bears one or more substituents (for example 1, 2 or 3), which may be the same or different, as hereinbefore defined in (m);
(q) Q² is selected from 2-pyridyl, 2-pyrazinyl, 1,3-thiazol-4-yl, 1,3-thiazol-5-yl and isoxazol-3-yl,
   and wherein Q² optionally bears 1, 2, or 3 substituents, which may be the same or different, as defined above in (m);
(r) Q² is selected from phenyl, 2-pyridyl, 3-pyridyl, 2-pyrazinyl, 1,3-thiazol-2-yl, 1,3-thiazol-4-yl, 1,3-thiazol-5-yl and isoxazol-3-yl,
   and wherein Q² optionally bears 1, 2, or 3 substituents, which may be the same or different, selected from fluoro, chloro, hydroxy, cyano, nitro, (1-4C)alkyl and (1-4C)alkoxy;
(s) Q² is selected from phenyl, 2-pyridyl, 2-pyrazinyl, 1,3-thiazol-4-yl, 1,3-thiazol-5-yl, and isoxazol-3-yl,
   and wherein Q² optionally bears one or more substituents (for example 1, 2 or 3), which may be the same or different, as hereinbefore defined in (r);
(t) Q² is selected from phenyl, 2-pyridyl, 3-pyridyl and isoxazol-3-yl,
   and wherein Q² optionally bears one or more substituents (for example 1, 2 or 3), which may be the same or different, as hereinbefore defined in (r);
(u) Q² is selected from 2- or 3-pyridyl, 2-pyrazinyl, 1,3-thiazol-2-yl, 1,3-thiazol-4-yl and 3-isoxazolyl (particularly 3-pyridyl, 1,3-thiazol-4-yl and 3-isoxazolyl),
   and wherein Q² optionally bears one or more substituents (for example 1, 2 or 3), which may be the same or different, as hereinbefore defined in (r);
(v) Q² is phenyl which optionally bears one or more substituents (for example 1, 2, or 3), which may be the same or different, selected from fluoro, chloro, bromo, cyano, methyl and methoxy;
(w) Q² is phenyl which bears 1 or 2 substituents, which may be the same or different, selected from halogeno (particularly fluoro and chloro, more particularly fluoro);
(x) Q² is selected from 2-fluorophenyl and 3-fluorophenyl;
(y) Q² is 3-fluorophenyl;
(z) Q² is 2-fluorophenyl;
(aa) Q² is selected from 2-pyridyl and 3-pyridyl,
   and wherein Q² optionally bears 1 or 2 substituents selected from fluoro, chloro, hydroxy, (1-4C)alkyl and (1-4C)allcoxy;
(bb) Q² is selected from 2-pyridyl and 3-pyridyl,
   and wherein Q² bears 1 or 2 substituents selected from hydroxy, (1-4C)alkyl and (1-4C)alkoxy;
(cc) Q² is 2-pyridyl which optionally bears 1 or 2 substituents selected from fluoro, chloro, hydroxy, (1-4C)alkyl and (1-4C)alkoxy (particularly (1-4C)alkyl);
(dd) Q² is 3-pyridyl which optionally bears 1 or 2 substituents selected from fluoro, chloro, hydroxy, (1-4C)alkyl and (1-4C)alkoxy (particularly (1-4C)alkyl);
(ee) Q² is selected from 2-pyridyl, 3-pyridyl, 6-methylpyrid-2-yl and 6-methylpyrid-3yl;
(ff) Q² is 2-pyridyl;
(gg) Q² is 3-pyridyl;
(hh) Q² is 6-methylpyrid-2-yl;
(ii) Q² is 6-methylpyrid-3yl;
(jj) Q² is 2-pyrazinyl which optionally bears 1 or 2 substituents, which may be the same or different, selected from fluoro, chloro, hydroxy, (1-4C)alkyl and (1-4C)alkoxy;
(kk) Q² is 2-pyrazinyl;
(ll) Q² is 3-isoxazolyl which optionally bears 1 or 2 substituents, which may be the same or different, selected from fluoro, chloro, hydroxy, (1-4C)alkyl and (1-4C)alkoxy;
(mm) Q² is 3-isoxazolyl which bears 1 or 2 substituents, which may be the same or different, selected from hydroxy, (1-4C)alkyl and (1-4C)alkoxy;
(nn) Q² is 3-isoxazolyl which optionally bears 1 or 2 substituents, which may be the same or different, selected from (1-4C)alkyl;
(oo) Q² is selected from 3-isoxazolyl and 5-methyl-3-isoxazolyl;
(pp) Q² is 3-isoxazolyl;
(qq) Q² is 5-methyl-3-isoxazolyl;
(rr) Q² is selected from 1,3-thiazol-2-yl, 1,3-thiazol-4-yl and 1,3-thiazol-5-yl,
   and wherein Q² optionally bears 1 or 2 substituents, which may be the same or different, selected from fluoro, chloro, hydroxy, (1-4C)alkyl and (1-4C)alkoxy;
(ss) Q² is selected from 1,3-thiazol-2-yl, 1,3-thiazol-4-yl and 1,3-thiazol-5-yl;
(tt) Q² is 1,3-thiazol-2-yl;
(uu) Q² is 1,3-thiazol-4-yl;
(vv) Q² is 1,3-thiazol-5-yl;
(ww) Q² is selected from 2-fluorophenyl, 3-fluorophenyl, 2-pyridyl, 3-pyridyl, 6-methylpyrid-2-yl, 6-methylpyrid-3-yl, 2-pyrazinyl, 1,3-thiazol-2-yl, 1,3-thiazol-4-yl, 1,3-thiazol-5-yl and 5-methyl-3-isoxazolyl;
(xx) Q² is selected from 2-pyridyl, 3-pyridyl, 6-methylpyrid-2-yl, 6-methylpyrid-3-yl, 2-pyrazinyl, 1,3-thiazol-2-yl, 1,3-thiazol-4-yl, 1,3-thiazol-5-yl and 5-methyl-3-isoxazolyl;
(yy) Q² is selected from 2-pyridyl, 6-methylpyrid-3-yl, 2-pyrazinyl, 1,3-thiazol-2-yl, 1,3-thiazol-4-yl and 5-methyl-3-isoxazolyl (particularly 6-methylpyrid-3-yl, 1,3-thiazol-4-yl and 5-methyl-3-isoxazolyl);
(zz) Q² is selected from 2-fluorophenyl, 3-fluorophenyl, 2-pyridyl, 6-methylpyrid-2-yl, 6-methylpyrid-3-yl, 2-pyrazinyl, 1,3-thiazol-4-yl and 5-methyl-3-isoxazolyl (particularly2-fluorophenyl, 3-fluorophenyl, 2-pyridyl, 6-methylpyrid-3-yl, 2-pyrazinyl and 1,3-thiazol-4-yl);
(aaa) Q² is selected from 2-pyridyl, 2-pyrazinyl, 1,3-thiazol-4-yl, 1,3-thiazol-5-yl and 5-methyl-3-isoxazolyl;
(bbb) Q² is selected from phenyl, 2-pyridyl, 2-pyrazinyl, 1,3-thiazol-2-yl, 1,3-thiazol-4-yl, 1,3-thiazol-5-yl and isoxazol-3-yl,
   and wherein Q² optionally bears 1, 2, or 3 substituents, which may be the same or different, selected from fluoro, chloro, hydroxy, cyano, nitro, amino, (1-4C)alkyl, (1-4C)alkoxy, N-(1-4C)alkylamino and N, N-di-[(1-4C)alkyl]amino;
(ccc) Q² is selected from phenyl, 2-pyridyl, 2-pyrazinyl, 1,3-thiazol-2-yl, 1,3-thiazol-4-yl, 1,3-thiazol-5-yl and isoxazol-3-yl,
   and wherein Q² optionally bears 1, 2, or 3 substituents, which may be the same or different, selected from fluoro, chloro, hydroxy, cyano, nitro, amino, (1-4C)alkyl, (1-4C)alkoxy, N-(1-4C)alkylamino and N, N-di-[(1-4C)alkyl]amino,
   and Y is selected from halogeno (particularly chloro) and (1-4C)alkyl (particularly methyl);
(ddd) Q² is selected from 2-pyridyl, 2-pyrazinyl, 1,3-thiazol-4-yl, 1,3-thiazol-5-yl and isoxazol-3-yl,
   and wherein Q² optionally bears 1, 2, or 3 substituents, which may be the same or different, selected from fluoro, chloro, hydroxy, cyano, nitro, amino, (1-4C)alkyl, (1-4C)alkoxy, N-(1-4C)alkylamino and N, N-di-[(1-4C)alkyl]amino;
(eee) Q² is selected from phenyl, 2-pyridyl, 2-pyrazinyl, 1,3-thiazol-2-yl, 1,3-thiazol-4-yl, 1,3-thiazol-5-yl and isoxazol-3-yl,
   and wherein Q² optionally bears 1, 2, or 3 substituents, which may be the same or different, selected from fluoro, chloro, hydroxy, (1-4C)alkyl and (1-4C)alkoxy;
(fff) Q² is selected from phenyl, 2-pyridyl, 2-pyrazinyl, 1,3-thiazol-2-yl, 1,3-thiazol-4-yl, 1,3-thiazol-5-yl and isoxazol-3-yl,
   and wherein Q² optionally bears 1, 2, or 3 substituents, which may be the same or different, selected from fluoro, chloro, hydroxy, (1-4C)alkyl and (1-4C)alkoxy,
   and Y is selected from chloro and methyl;
(ggg) Q² is selected from phenyl, 2-pyridyl, 2-pyrazinyl, 1,3-thiazol-2-yl, 1,3-thiazol-4-yl, 1,3-thiazol-5-yl and isoxazol-3-yl,
   and wherein Q² optionally bears 1, 2, or 3 substituents, which may be the same or different, selected from fluoro, chloro, hydroxy, cyano, nitro, amino, (1-4C)alkyl, (1-4C)alkoxy, N-(1-4C)alkylamino and N, N-di-[(1-4C)alkyl]amino,
   and R² is hydrogen;
(hhh) Q² is selected from 2-pyridyl, 2-pyrazinyl, 1,3-thiazol-4-yl, 1,3-thiazol-5-yl and isoxazol-3-yl,
   and wherein Q² optionally bears 1, 2, or 3 substituents, which may be the same or different, selected from fluoro, chloro, hydroxy, cyano, nitro, amino, (1-4C)alkyl, (1-4C)alkoxy, N-(1-4C)alkylamino and N, N-di-[(1-4C)alkyl]amino,
   and R² is hydrogen;
(iii) X³ is cyclopropylidene;
(jjj) X³ is selected from -CH₂-, -CH(CH₃)-, -C(CH₃)₂-_{,} -C(CH₃)₂CH₂-, -CH₂C(CH₃)₂-, CH(CH₃)CH₂-, -CH₂CH(CH₃)- and cyclopropylidene (particularly -CH₂-, -CH(CH₃)-, - C(CH₃)₂-, -CH₂C(CH₃)₂- and cyclopropylidene);
(kkk) Z is hydroxy;
(lll) Z is (1-6C)alkoxy (particularly methoxy or ethoxy);
(mmm)Z is dimethylamino;
(nnn) Z-X³ is hydroxymethyl;

In the quinazoline derivative of the Formula Ia: 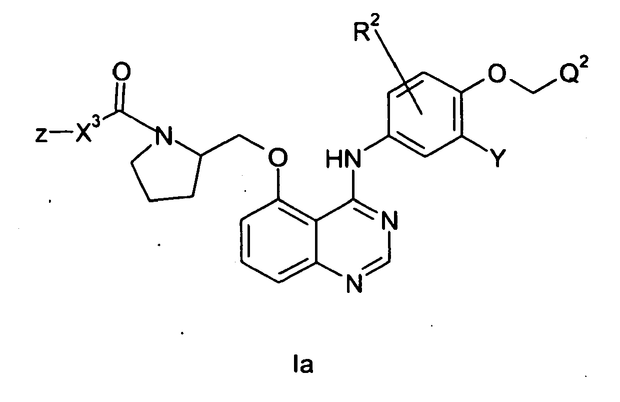 wherein:
**R²** is selected from hydrogen and halogeno;
**Y** is selected from halogeno, (1-4C)alkyl, (1-4C)alkoxy, (2-4C)alkenyl and (2-4C)alkynyl (particularly Y is halogeno or methyl, more particularly Y is chloro);
**Q²** is selected from phenyl, pyridyl, pyrazinyl, 1,3-thiazolyl and isoxazolyl (more particularly Q² is selected from phenyl, 2-pyridyl, 2-pyrazinyl, 1,3-thiazol-4-yl, 1,3-thiazol-5-yl and 3-isoxazolyl),
   and wherein Q² optionally bears one or more substituents (for example 1, 2 or 3), which may be the same or different, selected from halogeno, hydroxy, cyano, carboxy, nitro, amino, (1-4C)alkyl, (1-4C)alkoxy, (2-4C)alkenyl, (2-4C)alkynyl, (1-4C)alkylthio, (1-4C)alkylsulfinyl, (1-4C)alkylsulfonyl, (2-4C)alkanoyl, N-(1-4C)alkylamino, N, N-di-[(1-4C)alkyl]amino, (1-4C)alkoxycarbonyl, carbamoyl, N-(1-4C)alkylcarbamoyl, N, N-di-[(l-4C)alkyl]carbamoyl, (2-4C)alkanoyloxy, (2-4C)alkanoylamino, N-(1-4C)alkyl-(2-4C)alkanoylamino, halogeno-(1-4C)alkyl, hydroxy-(1-4C)alkyl, (1-4C)alkoxy-(1-4C)alkyl, cyano-(1-4C)alkyl, carboxy-(1-4C)alkyl, amino-(1-4C)alkyl, N-(1-4C)alkylamino-(1-4C)alkyl and N, N-di-[(1-4C)alkyl]amino-(1-4C)alkyl; and
X³ is selected from -CH₂-, -CH₂CH₂-, -(CR⁸R⁹)-, -(CR⁸R⁹CH₂)-, - (CH₂CR⁸R⁹)- or (3-6C)cycloalkylene (for example cyclopropylene such as cyclopropylidene),
wherein each of R⁸ and R⁹, which may be the same or different, is selected from hydrogen and methyl.

Z is selected from hydroxy, di-[(1-3C)alkyl]amino (for example dimethylamino) and (1-6C)alkoxy (for example methoxy or ethoxy), still more particularly Z is hydroxy;
or a pharmaceutically acceptable salt thereof.

Another embodiment is a quinazoline derivative of the Formula Ia wherein:
**R²** is selected from hydrogen and halogeno;
**Y** is selected from halogeno, (1-4C)alkyl, (1-4C)alkoxy, (2-4C)alkenyl and (2-4C)alkynyl (particularly Y is halogeno or methyl, more particularly Y is chloro);
**Q²** is selected from pyridyl, pyrazinyl, 1,3-thiazolyl and isoxazolyl (more particularly Q² is selected from 2-pyridyl, 2-pyrazinyl, 1,3-thiazol-4-yl, 1,3-thiazol-5-yl and 3-isoxazolyl),
   and wherein Q² optionally bears one or more substituents (for example 1, 2 or 3), which may be the same or different, selected from halogeno, hydroxy, cyano, carboxy, nitro, amino, (1-4C)alkyl, (1-4C)alkoxy, (2-4C)alkenyl, (2-4C)alkynyl, (1-4C)alkylthio, (1-4C)alkylsulfinyl, (1-4C)alkylsulfonyl, (2-4C)alkanoyl, N-(1-4C)alkylamino, N, N-di-[(1-4C)alkyl]amino, (1-4C)alkoxycarbonyl, carbamoyl, N-(1-4C)alkylcarbamoyl, N, N-di-[(1-4C)alkyl]carbamoyl, (2-4C)alkanoyloxy, (2-4C)alkanoylamino, N-(1-4C)alkyl-(2-4C)alkanoylamino, halogeno-(1-4C)alkyl, hydroxy-(1-4C)alkyl, (1-4C)alkoxy-(1-4C)alkyl, cyano-(1-4C)alkyl, carboxy-(1-4C)alkyl, amino-(1-4C)alkyl, N-(1-4C)alkylarnino-(1-4C)alkyl and N, N-di-[(1-4C)alkyl]amino-(1-4C)alkyl; and
X³ is selected from -CH₂-, -CH₂CH₂-, -(CR⁸R⁹)-, -(CR⁸R⁹CH₂)-, -(CH₂CR⁸R⁹)- or (3-6C)cycloalkylene (for example cyclopropylene such as cyclopropylidene),
   wherein each of R⁸ and R⁹, which may be the same or different, is selected from hydrogen and methyl;
Z is selected from hydroxy and di-[(1-3C)alkyl]amino (for example methylamino), still more particularly Z is hydroxy;
or a pharmaceutically acceptable salt thereof.

In this embodiment a further particular value for X³ is -CH₂- or -CH₂CH₂-, and Z is hydroxy or di-[(1-3C)alkyl]amino (particularly Z-X³ is hydroxymethyl).

Particular compounds of the invention are, for example, one or more quinazoline derivatives of the Formula Ia selected from:
2-((2*R*)-2-{[(4-{[3-chloro-4-(pyridin-2-ylmethoxy)phenyl]amino} quinazolin-5-yl)oxy]methyl}pyrrolidin-1-yl)-2-oxoethanol;
2-((2*S*)-2-{[(4-{[3-chloro-4-(pyridin-2-ylmethoxy)phenyl]amino} quinazolin-5-yl)oxy]methyl}pyrrolidin-1-yl)-2-oxoethanol;
*N*-[3-chloro-4-(pyridin-2-ylmethoxy)phenyl]-5-({(2*R*)-1-[(dimethylamino)acetyl] pyrrolidin-2-yl}methoxy)quinazolin-4-amine;
*N*-[3-chloro-4-(pyridin-2-ylmethoxy)phenyl]-5-({(2S)-1-[(dimethylamino)acetyl] pyrrolidin-2-yl}methoxy)quinazolin-4-amine;
1-((2*R*)-2-{[(4-{[3-chloro-4-(pyridin-2-ylmethoxy)phenyl]amino} quinazolin-5-yl)oxy]methyl}pyrrolidin-1-yl)-2-methyl-1-oxopropan-2-ol;
1-[((2*R*)-2-{[(4-{[3-chloro-4-(pyridin-2-ylmethoxy)phenyl]amino}quinazolin-5-yl)oxy]methyl}pyrrolidin-1-yl)carbonyl]cyclopropanol;
3-((2*R*)-2-{[(4-{[3-chloro-4-(pyridin-2-ylmethoxy)phenyl]amino}quinazolin-5-yl)oxy]methyl}pyrrolidin-1-yl)-2,2-dimethyl-3-oxopropan-1-ol;
(2*S*)-1-((2*R*)-2-{[(4-{[3-chloro-4-(pyridin-2-ylmethoxy)phenyl]amino}quinazolin-5-yl)oxy]methyl}pyrroolidin-1-yl)-1-oxopropan-2-ol;
*N*-[3-chloro-4-(pyridin-2-ylmethoxy)phenyl]-5-{[(*2R*)*-*1-(ethoxyacetyl)pyrrolidin-2-yl]methoxy}quinazolin-4-amine;
*N*-[3-chloro-4-(pyridin-2-ylmethoxy)phenyl]-5-{[(2*R*)-1-(methoxyacetyl)pyrrolidin-2-yl]methoxy} quinazolin-4-amine;
*N*-[3-chloro-4-(1,3-thiazol-4-ylmethoxy)phenyl]-5-({(2*R*)-1-[(dimethylamino)acetyl] pyrrolidin-2-yl}methoxy)quinazolin-4-amine;
2-((2*R*)-2-{[(4-{[3-chloro-4-(1,3-thiazol-4-ylmethoxy)phenyl]amino} quinazolin-5-yl)oxy]methyl}pyrrolidin-1-yl)-2-oxoethanol;
*N*-{3-chloro-4-[(5-methylisoxazol-3-yl)methoxy]phenyl}-5-({(2*R*)-1-[(dimethylamino)acetyl]pyrrolidin-2-yl}methoxy)quinazolin-4-amine;
2-[(2*R*)-2-({[4-({3-chloro-4-[(5-methylisoxazol-3-yl)methoxy]phenyl}amino) quinazolin-5-yl]oxy}methyl)pyrrolidin-1-yl]-2-oxoethanol;
*N*-[3-chloro-4-(1,3-thiazol-5-ylmethoxy)phenyl]-5-({(2*R*)-1-[(dimethylamino)acetyl] pyrrolidin-2-yl}methoxy)quinazolin-4-amine;
2-((2*R*)-2-{[(4-{[3-chloro-4-(1,3-thiazol-5-ylmethoxy)phenyl]amino}quinazolin-5-yl)oxy]methyl}pyrrolidin-1-yl)-2-oxoethanol;
*N*-[3-chloro-4-(pyrazin-2-ylmethoxy)phenyl]-5-({(2*R*)-1-[(dimethylamino)acetyl] pyrrolidin-2-yl}methoxy)quinazolin-4-amine;
2-((2*R*)-2-{[(4-([3-chloro-4-(pyrazin-2-ylmethoxy)phenyl]amino}quinazolin-5-yl)oxy]methyl}pyrrolidin-1-yl)-2-oxoethanol;
(2*R*)-1-((2*R*)-2-{[(4-{[3-chloro-4-(pyridin-2-ylmethoxy)phenyl]amino}quinazolin-5-yl)oxy]methyl}pyrrolidin-1-yl)-1-oxopropan-2-ol;
2-[(2*S*)-2-({[4-({3-chloro-4-[(6-methylpyridin-2-yl)methoxy]phenyl}amino) quinazolin-5-yl]oxy}methyl)pyrrolidin-1-yl]-2-oxoethanol;
2-[(2*S*)-2-({[4-({3-chloro-4-[(2-fluorobenzyl)oxy]phenyl}amino)quinazolin-5-yl]oxy}methyl)pyrrolidin-1-yl]-2-oxoethanol;
2-[(2*S*)-2-({[4-({3-chloro-4-[(3-fluorobenzyl)oxy]phenyl}amino)quinazolin-5-yl]oxy}methyl)pyrrolidin-1-yl]-2-oxoethanol;
2-((2*S*)-2-{[(4-{[3-chloro-4-(1,3-thiazol-4-ylmethoxy)phenyl]amino}quinazolin-5-yl)oxy]methyl}pyrrolidin-1-yl)-2-oxoethanol;
2-((2*S*)-2-{[(4-{[3-chloro-4-(pyrazin-2-ylmethoxy)phenyl]amino}quinazolin-5-yl)oxy]methyl}pyrrolidin-1-yl)-2-oxoethanol;
*N*-{3-chloro-4-[(6-methylpyridin-2-yl)methoxy]phenyl}-5-({(2*S*)-1-[(dimethylamino)acetyl]pyrrolidin-2-yl}methoxy)quinazolin-4-amine;
*N*-{3-chloro-4-[(2-fluorobenzyl)oxy]phenyl}-5-({(2*S*)-1-[(dimethylamino)acetyl] pyrrolidin-2-yl}methoxy)quinazolin-4-amine;
*N*-(3-chloro-4-[(3-fluorobenzyl)oxy]phenyl)-5-({(2*S*)-1-[(dimethylamino)acetyl] pyrrolidin-2-yl}methoxy)quinazolin-4-amine;
*N*-[3-chloro-4-(pyrazin-2-ylmethoxy)phenyl]-5-({(2*S*)-1-[(dimethylamino)acetyl] pyrrolidin-2-yl}methoxy)quinazolin-4-amine;
*N*-[3-chloro-4-(1,3-thiazol-4-ylmethoxy)phenyl]-5-({(2*S*)-1-[(dimethylamino)acetyl] pyrrolidin-2-yl}methoxy)quinazolin-4-amine;
2-((2*S*)-2-{[(4-{[3-methyl-4-(pyridin-2-ylmethoxy)phenyl]amino}quinazolin-5-yl)oxy]methyl}pyrrolidin-1-yl)-2-oxoethanol;
2-((2*S*)-2-{[(4-{[3-methyl-4-(pyrazin-2-ylmethoxy)phenyl]amino} quinazolin-5-yl)oxy]methyl}pyrrolidin-1-yl)-2-oxoethanol;
2-((2*R*)-2-{[(4-{[3-methyl-4-(pyridin-2-ylmethoxy)phenyl]amino} quinazolin-5-yl)oxy]methyl}pyrrolidin-1-yl)-2-oxoethanol;
2-((2*R*)-2-{[(4-{[3-methyl-4-(pyrazin-2-ylmethoxy)phenyl]amino}quinazolin-5-yl)oxy]methyl}pyrrolidin-1-yl)-2-oxoethanol;
2-((2*R*)-2-{[(4-{[3-methyl-4-(1,3-thiazol-4-ylmethoxy)phenyl]amino}quinazolin-5-yl)oxy]methyl}pyrrolidin-1-yl)-2-oxoethanol;
2-[(2*R*)-2-({[4-({3-methyl-4-[(5-methylisoxazol-3-yl)methoxy]phenyl} amino) quinazolin-5-yl]oxy} methyl)pyrrolidin-1-yl]-2-oxoethanol;
5-{[(2*R*)-1-(methoxyacetyl)pyrrolidin-2-yl]methoxy}-*N*-(3-methyl-4-[(5-methylisoxazol-3-yl)methoxy]phenyl)quinazolin-4-amine;
5-{[(2*R*)-1-(methoxyacetyl)pyrrolidin-2-yl]methoxy}-*N*-[3-methyl-4-(pyridin-2-ylmethoxy)phenyl]quinazolin-4-amine; and
5-{[(2*R*)-1-(methoxyacetyl)pyrrolidin-2-yl]methoxy}-*N*-[3-methyl-4-(1,3-thiazol-4-ylmethoxy)phenyl]quinazolin-4-amine;
or a pharmaceutically acceptable salt thereof.

Further particular compounds of the invention are, for example, one or more quinazoline derivatives of the Formula Ia selected from:
2-((2R)-2-{[(4-{[3-chloro-4-(pyridin-2-ylmethoxy)phenyl]amino}quinazolin-5-yl)oxy]methyl}pyrrolidin-1-yl)-2-oxoethanol;
2-((2*S*)-2-{[(4-{[3-chloro-4-(pyridin-2-ylmethoxy)phenyl]amino}quinazolin-5-yl)oxy]methyl}pyrrolidin-1-yl)-2-oxoethanol;
*N*-[3-chloro-4-(pyridin-2-ylmethoxy)phenyl]-5-({(2*R*)-1-[(dimethylamino)acetyl] pyrrolidin-2-yl}methoxy)quinazolin-4-amine;
*N*-[3-chloro-4-(pyridin-2-ylmethoxy)phenyl]-5-({(2*S*)-1-[(dimethylamino)acetyl] pyrrolidin-2-yl}methoxy)quinazolin-4-amine;
1-((2*R*)-2-{[(4-{[3-chloro-4-(pyridin-2-ylmethoxy)phenyl]amino}quinazolin-5-yl)oxy]methyl}pyrrolidin-1-yl)-2-methyl-1-oxopropan-2-ol;
1-[((2*R*)-2-{[(4-{[3-chloro-4-(pyridin-2-ylmethoxy)phenyl]amino}quinazolin-5-yl)oxy]methyl}pyrrolidin-1-yl)carbonyl]cyclopropanol;
(2*S*)-1-((2*R*)-2-{[(4-{[3-chloro-4-(pyridin-2-ylmethoxy)phenyl]amino}quinazolin-5-yl)oxy]methyl}pyrrolidin-1-yl)-1-oxopropan-2-ol;
*N*-[3-chloro-4-(1,3-thiazol-4-ylmethoxy)phenyl]-5-({(2*R*)-1-[(dimethylamino)acetyl] pyrrolidin-2-yl}methoxy)quinazolin-4-amine;
2-((2*R*)-2-{[(4-{[3-chloro-4-(1,3-thiazol-4-ylmethoxy)phenyl]amino}quinazolin-5-yl)oxy]methyl}pyrrolidin-1-yl)-2-oxoethanol;
*N*-{3-chloro-4-[(5-methylisoxazol-3-yl)methoxy]phenyl}-5-({(2*R*)-1-[(dimethylamino)acetyl]pyrrolidin-2-yl}methoxy)quinazolin-4-amine;
2-[(2*R*)-2-({[4-({3-chloro-4-[(5-methylisoxazol-3-yl)methoxy]phenyl}amino) quinazolin-5-yl]oxy}methyl)pyrrolidin-1-yl]-2-oxoethanol;
2-((2*R*)-2-{[(4-{[3-chloro-4-(1,3-thiazol-5-ylmethoxy)phenyl]amino}quinazolin-5-yl)oxy]methyl}pyrrolidin-1-yl)-2-oxoethanol; and
2-((2*R*)-2-{[(4-{[3-chloro-4-(pyrazin-2-ylmethoxy)phenyl]amino}quinazolin-5-yl)oxy]methyl}pyrrolidin-1-yl)-2-oxoethanol;
or a pharmaceutically acceptable salt thereof.

Further particular compounds of the invention are, for example, one or more quinazoline derivatives of the Formula Ia selected from:
2-((2R)-2-{[(4-{[3-chloro-4-(pyridin-2-ylmethoxy)phenyl]amino}quinazolin-5-yl)oxy]methyl}pyrrolidin-1-yl)-2-oxoethanol;
2-((2*S*)-2-{[(4-{[3-chloro-4-(pyridin-2-ylmethoxy)phenyl]amino}quinazolin-5-yl)oxy]methyl}pyrrolidin-1-yl)-2-oxoethanol;
1-((2*R*)-2-{[(4-{[3-chloro-4-(pyridin-2-ylmethoxy)phenyl]amino} quinazolin-5-yl)oxy]methyl}pyrrolidin-1-yl)-2-methyl-1-oxopropan-2-ol;
1-[((2*R*)-2-{[(4-{[3-chloro-4-(pyridin-2-ylmethoxy)phenyl]amino}quinazolin-5-yl)oxy]methyl}pyrrolidin-1-yl)carbonyl]cyclopropanol;
3-((2*R*)-2-{[(4-{[3-chloro-4-(pyridin-2-ylmethoxy)phenyl]amino}quinazolin-5-yl)oxy]methyl}pyrrolidin-1-yl)-2,2-dimethyl-3-oxopropan-1-ol;
(2*S*)-1-((2*R*)-2-{[(4-{[3-chloro-4-(pyridin-2-ylmethoxy)pheriyl]amino}quinazolin-5-yl)oxy]methyl}pyrrolidin-1-yl)-1-oxopropan-2-ol;
*N*-[3-Chloro-4-(pyridin-2-ylmethoxy)phenyl]-5-{[(2*R*)-1-(ethoxyacetyl)pyrrolidin-2-yl]methoxy}quinazolin-4-amine;
*N*-[3-Chloro-4-(pyridin-2-ylmethoxy)phenyl]-5-{[(2*R*)-1-(methoxyacetyl)pyrrolidin-2-yl]methoxy}quinazolin-4-amine;
2-((2*R*)-2-{[(4-{[3-chloro-4-(1,3-thiazol-4-ylmethoxy)phenyl]amino} quinazolin-5-yl)oxy]methyl}pyrrolidin-1-yl)-2-oxoethanol;
2-[(2*R*)-2-({[4-({3-chloro-4-[(5-methylisoxazol-3-yl)methoxy]phenyl}amino) quinazolin-5-yl]oxy}methyl)pyrrolidin-1-yl]-2-oxoethanol;
2-((2*R*)-2-{[(4-{[3-chloro-4-(1,3-thiazol-5-ylmethoxy)phenyl]amino}quinazolin-5-yl)oxy]methyl}pyrrolidin-1-yl)-2-oxoethanol;
2-((2*R*)-2-{[(4-{(3-chloro-4-(pyrazin-2-ylmethoxy)phenyl]amino}quinazolin-5-yl)oxy]methyl}pyrrolidin-1-yl)-2-oxoethanol;
(2*R*)-1-((2*R*)-2-{[(4-{[3-chloro-4-(pyridin-2-ylmethoxy)phenyl]amino}quinazolin-5-yl)oxy]methyl}pyrrolidin-1-yl)-1-oxopropan-2-ol;
2-[(2*S*)-2-({[4-({3-chloro-4-[(6-methylpyridin-2-yl)methoxy]phenyl}amino) quinazolin-5-yl]oxy}methyl)pyrrolidin-1-yl]-2-oxoethanol;
2-[(2*S*)-2-({[4-({3-chloro-4-[(2-fluorobenzyl)oxy]phenyl}amino)quinazolin-5-yl]oxy}methyl)pyrrolidin-1-yl]-2-oxoethanol;
2-[(2*S*)-2-({[4-({3-chloro-4-[(3-fluorobenzyl)oxy]phenyl}amino)quinazolin-5-yl]oxy}methyl)pyrrolidin-1-yl]-2-oxoethanol;
2-((2*S*)-2-{[(4-{[3-chloro-4-(1,3-thiazol-4-ylmethoxy)phenyl]amino}quinazolin-5-yl)oxy]methyl}pyrrolidin-1-yl)-2-oxoethanol;
2-((2*S*)-2-{[(4-{[3-chloro-4-(pyrazin-2-ylmethoxy)phenyl]amino}quinazolin-5-yl)oxy]methyl}pyrrolidin-1-yl)-2-oxoethanol;
2-((2*S*)-2-{[(4-{[3-methyl-4-(pyridin-2-ylmethoxy)phenyl]amino}quinazolin-5-yl)oxy]methyl}pyrrolidin-1-yl)-2-oxoethanol;
2-((2*S*)-2-{[(4-{[3-methyl-4-(pyrazin-2-ylmethoxy)phenyl]amino}quinazolin-5-yl)oxy]methyl}pyrrolidin-1-yl)-2-oxoethanol;
2-((2*R*)-2-{[(4-{[3-methyl-4-(pyridin-2-ylmethoxy)phenyl]amino}quinazolin-5-yl)oxy]methyl}pyrrolidin-1-yl)-2-oxoethanol;
2-((2*R*)-2-{[(4-{[3-methyl-4-(pyrazin-2-ylmethoxy)phenyl]amino}quinazolin-5-yl)oxy]methyl}pyrrolidin-1-yl)-2-oxoethanol;
2-((2*R*)-2-{[(4-{[3-methyl-4-(1,3-thiazol-4-ylmethoxy)phenyl]amino}quinazolin-5-yl)oxy]methyl}pyrrolidin-1-yl)-2-oxoethanol;
2-[(2*R*)-2-({[4-({3-methyl-4-[(5-methylisoxazol-3-yl)methoxy]phenyl}amino) quinazolin-5-yl]oxy}methyl)pyrrolidin-1-yl]-2-oxoethanol;
5-{[(2*R*)-1-(methoxyacetyl)pyrrolidin-2-yl]methoxy}-*N*-{3-methyl-4-[(5-methylisoxazol-3-yl)methoxy]phenyl}quinazolin-4-amine;
5-{[(2*R*)-1-(methoxyacetyl)pyrrolidin-2-yl]methoxy}-*N*-[3-methyl-4-(pyridin-2-ylmethoxy)phenyl]quinazolin-4-amine; and
5-{[(2*R*)-1-(methoxyacetyl)pyrrolidin-2-yl]methoxy}-*N*-[3-methyl-4-(1,3-thiazol-4-ylmethoxy)phenyl]quinazolin-4-amine;
or a pharmaceutically acceptable salt thereof.

Further particular compounds of the invention are, for example, one or more quinazoline derivatives of the Formula Ia selected from:
2-((2*R*)-2-{[(4-{[3-chloro-4-(pyridin-2-ylmethoxy)phenyl]amino}quinazolin-5-yl)oxy]methyl}pyrrolidin-1-yl)-2-oxoethanol;
1-((2*R*)-2-{[(4-{[3-chloro-4-(pyridin-2-ylmethoxy)phenyl]amino}quinazolin-5-yl)oxy]methyl}pyrrolidin-1-yl)-2-methyl-1-oxopropan-2-ol;
1-[((2*R*)-2-{[(4-{[3-chloro-4-(pyridin-2-ylmethoxy)phenyl]amino}quinazolin-5-yl)oxy]methyl}pyrrolidin-1-yl)carbonyl]cyclopropanol;
3-((2*R*)-2-{[(4-{[3-chloro-4-(pyridin-2-ylmethoxy)phenyl]amino}quinazolin-5-yl)oxy]methyl}pyrrolidin-1-yl)-2,2-dimethyl-3-oxopropan-1-ol;
2-((2*R*)-2-{[(4-{[3-chloro-4-(1,3-thiazol-4-ylmethoxy)phenyl]amino}quinazolin-5-yl)oxy]methyl}pyrrolidin-1-yl)-2-oxoethanol;
2-((2*R*)-2-{[(4-{[3-chloro-4-(pyrazin-2-ylmethoxy)phenyl]amino}quinazolin-5-yl)oxy]methyl}pyrrolidin-1-yl)-2-oxoethanol;
(2*R*)-1-((2*R*)-2-{[(4-{[3-chloro-4-(pyridin-2-ylmethoxy)phenyl]amino}quinazolin-5-yl)oxy]methyl}pyrrolidin-1-yl)-1-oxopropan-2-ol;
2-[(2*S*)-2-({[4-({3-chloro-4-[(2-fluorobenzyl)oxy]phenyl}amino)quinazolin-5-yl]oxy}methyl)pyrrolidin-1-yl]-2-oxoethanol;
2-[(2*S*)-2-({[4-({3-chloro-4-[(3-fluorobenzyl)oxy]phenyl}amino)quinazolin-5-yl]oxy}methyl)pyrrolidin-1-yl]-2-oxoethanol;
2-((2*S*)-2-{[(4-{[3-chloro-4-(1,3-thiazol-4-ylmethoxy)phenyl]amino} quinazolin-5-yl)oxy]methyl}pyrrolidin-1-yl)-2-oxoethanol;
*N*-[3-chloro-4-(pyrazin-2-ylmethoxy)phenyl]-5-({(2*S*)-1-[(dimethylamino)acetyl] pyrrolidin-2-yl}methoxy)quinazolin-4-amine;
2-((2*R*)-2-{[(4-{[3-methyl-4-(pyridin-2-ylmethoxy)phenyl]amino}quinazolin-5-yl)oxy]methyl}pyrrolidin-1-yl)-2-oxoethanol;
2-((2*R*)-2-{[(4-{[3-methyl-4-(1,3-thiazol-4-ylmethoxy)phenyl]amino}quinazolin-5-yl)oxy]methyl}pyrrolidin-1-yl)-2-oxoethanol; and
2-[(2*R*)-2-({[4-({3-methyl-4-[(5-methylisoxazol-3-yl)methoxy]phenyl}amino) quinazolin-5-yl]oxy}methyl)pyrrolidin-1-yl]-2-oxoethanol;
or a pharmaceutically active salt thereof.

Further particular compounds of the invention are, for example, one or more quinazoline derivatives of the Formula Ia selected from:
2-((2*R*)-2-{[(4-{[3-chloro-4-(pyridin-2-ylmethoxy)phenyl]amino}quinazolin-5-yl)oxy]methyl}pyrrolidin-1-yl)-2-oxoethanol;
3-((2*R*)-2-{[(4-{[3-chloro-4-(pyridin-2-ylmethoxy)phenyl]amino}quinazolin-5-yl)oxy]methyl}pyrrolidin-1-yl)-2,2-dimethyl-3-oxopropan-1-ol;
2-((2*R*)-2-{[(4-{[3-chloro-4-(pyrazin-2-ylmethoxy)phenyl]amino}quinazolin-5-yl)oxy]methyl}pyrrolidin-1-yl)-2-oxoethanol;
(2*R*)-1-((2*R*)-2-{[(4-{[3-chloro-4-(pyridin-2-ylmethoxy)phenyl]amino}quinazolin-5-yl)oxy]methyl}pyrrolidin-1-yl)-1-oxopropan-2-ol;
or a pharmaceutically active salt thereof.

Further particular compounds of the invention are, for example, one or more quinazoline derivatives of the Formula Ia selected from:
2-((2*S*)-2-{[(4-{[3-chloro-4-(pyrazin-2-ylmethoxy)phenyl]amino}quinazolin-5-yl)oxy]methyl}pynolidin-1-yl)-2-oxoethanol;
5-({(2*S*)-1-[(dimethylamino)acetyl]pyrrolidin-2-yl}methoxy)-*N*-{3-methyl-4-[(6-methylpyridin-3-yl)oxy]phenyl}quinazolin-4-amine;
2-[(2*R*)-2-({[4-({3-methyl-4-[(5-methylisoxazol-3-yl)methoxy]phenyl}amino) quinazolin-5-yl]oxy}methyl)pyrrolidin-1-yl]-2-oxoethanol;
5-{[(2*R*)-1-(methoxyacetyl)pyrrolidin-2-yl]methoxy}-*N*-{3-methyl-4-[(5-methylisoxazol-3-yl)methoxy]phenyl}quinazolin-4-amine;
or a pharmaceutically active salt thereof.

Further particular compounds of the invention are, for example, one or more quinazoline derivatives of the Formula Ia selected from:
2-[(2*R*)-2-(([4-((3-methyl-4-[(5-methylisoxazol-3-yl)methoxy]phenyl)amino) quinazolin-5-yl]oxy)methyl)pyrrolidin-1-yl]-2-oxoethanol;
or a pharmaceutically active salt thereof.

Particular compounds of the invention are, for example, one or more quinazoline derivatives of the Formula la selected from:
*N*-[3-chloro-4-(pyridin-2-ylmethoxy)phenyl]-5-({(2*R*)-1-[(dimethylamino)acetyl] pyrrolidin-2-yl}methoxy)quinazolin-4-amine;
*N*-[3-chloro-4-(pyridin-2-ylmethoxy)phenyl]-5-({(2*S*)-1-[(dimethylamino)acetyl] pyrrolidin-2-yl}methoxy)quinazolin-4-amine;
*N*-[3-chloro-4-(1,3-thiazol-4-ylmethoxy)phenyl]-5-({(2*R*)-1-[(dimethylamino)acetyl] pyrrolidin-2-yl}methoxy)quinazolin-4-amine;
*N*-(3-chloro-4-[(5-methylisoxazol-3-yl)methoxy]phenyl)-5-({(2*R*)-1-[(dimethylamino)acetyl]pyrrolidin-2-yl}methoxy)quinazolin-4-amine;
*N*-[3-chloro-4-(1,3-thiazol-5-ylmethoxy)phenyl]-5-({(2*R*)-1-[(dimethylarnino)acetyl] pyrrolidin-2-yl}methoxy)quinazolin-4-amine;
*N*-[3-chloro-4-(pyrazin-2-ylmethoxy)phenyl]-5-({(2*R*)-1-[(dimethylamino)acetyl] pyrrolidin-2-yl}methoxy)quinazolin-4-amine;
*N*-{3-chloro-4-[(6-methylpyridin-2-yl)methoxy]phenyl}-5-({(2*S*)-1-[(dimethylamino)acetyl]pyrrolidin-2-yl}methoxy)quinazolin-4-amine;
*N*-(3-chloro-4-[(2-fluorobenzyl)oxy]phenyl)-5-({(2*S*)-1-[(dimethylamino)acetyl] pyrrolidin-2-yl}methoxy)quinazolin-4-amine;
*N*-{3-chloro-4-[(3-fluorobenzyl)oxy]phenyl}-5-({(2*S*)-1-[(dimethylamino)acetyl] pyrrolidin-2-yl}methoxy)quinazolin-4-amine;
*N*-[3-chloro-4-(pyrazin-2-ylmethoxy)phenyl]-5-({(2*S*)-1-[(dimethylamino)acetyl] pyrrolidin-2-yl}methoxy)quinazolin-4-amine;
*N*-[3-chloro-4-(1,3-thiazol-4-ylmethoxy)phenyl]-5-({(2*S*)-1-[(dimethylamino)acetyl] pyrrolidin-2-yl}methoxy)quinazolin-4-amine;
or a pharmaceutically active salt thereof.

A quinazoline derivative of the Formula Ia, or a pharmaceutically-acceptable salt thereof, may be prepared by any process known to be applicable to the preparation of chemically-related compounds. Suitable processes include, for example, those illustrated in International Patent Applications WO 96/15118, WO01/94341, WO03/040108 and WO03/040109. Such processes, when used to prepare a quinazoline derivative of the Formula Ia are provided as a further feature of the invention and are illustrated by the following representative process variants in which, unless otherwise stated, R², X³, Y and Z have any of the meanings defined hereinbefore. Necessary starting materials may be obtained by standard procedures of organic chemistry. The preparation of such starting materials is described in conjunction with the following representative process variants and within the accompanying Examples. Alternatively necessary starting materials are obtainable by analogous procedures to those illustrated which are within the ordinary skill of an organic chemist.

Process (a) The coupling, conveniently in the presence of a suitable base, of a quinazoline of the formula **II:** 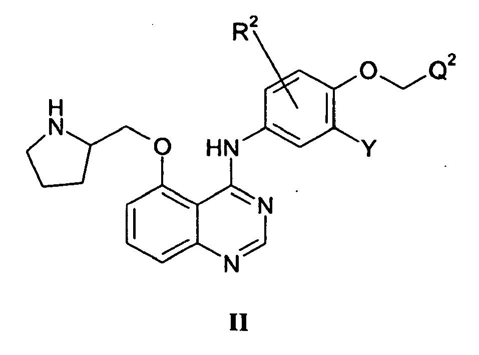 wherein R², Y, and Q² have any of the meanings defined hereinbefore except that any functional group is protected if necessary, with a carboxylic acid of the formula **III**, or a reactive derivative thereof:

Z-X³-COOH **III**

wherein Z and X³ have any of the meanings defined hereinbefore except that any functional group is protected if necessary;
or

Process (b) the reaction, conveniently in the presence of a suitable base, of a quinazoline of the formula **IV:** 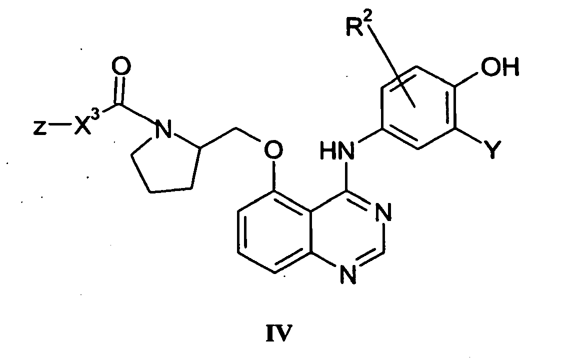 wherein R², X³, Z and Y have any of the meanings defined hereinbefore except that any functional group is protected if necessary, with a compound of the formula **V:**

Q²-C(H)₂-L¹ **V**

wherein L¹ is a suitable displaceable group and Q² has any of the meanings defined hereinbefore except that any functional group is protected if necessary; or

Process (c) The coupling of a quinazoline of the formula **VI:** 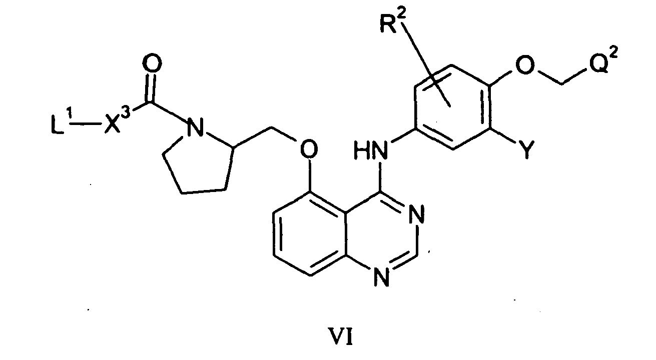 wherein L¹ is a suitable displaceable group and R², X³, Y and Q² have any of the meanings defined hereinbefore except that any functional group is protected if necessary, with a compound of the formula VII, or a reactive derivative thereof:

Z-H **VII**

wherein Z has any of the meanings defined hereinbefore except that any functional group is protected if necessary; or
and thereafter, if necessary:
(i) converting a quinazoline derivative of the formula Ia into another quinazoline derivative of the formula Ia;
(ii) removing any protecting group that is present by conventional means;
(iii) forming a pharmaceutically acceptable salt.

Specific conditions for the above reactions are as follows:

### Process (a)

The coupling reaction is conveniently carried out in the presence of a suitable coupling agent, such as a carbodiimide, or a suitable peptide coupling agent, for example O-(7-azabenzotriazol-1-yl)-N,N,N',N'-tetramethyluronium hexafluoro-phosphate (HATU) or a carbodiimide such as dicyclohexylcarbodiimide, optionally in the presence of a catalyst such as dimethylaminopyridine or 4-pyrrolidinopyridine .

The coupling reaction is conveniently carried out in the presence of a suitable base. A suitable base is, for example, an organic amine base such as, for example, pyridine, 2,6-lutidine, collidine, 4-dimethylaminopyridine, triethylamine, di-isopropylethylamine, N-methylmorpholine or diazabicyclo[5.4.0]undec-7-ene, or, for example, an alkali or alkaline earth metal carbonate, for example sodium carbonate, potassium carbonate, cesium carbonate, calcium carbonate.

The reaction is conveniently carried out in the presence of a suitable inert solvent or diluent, for example an ester such as ethyl acetate, a halogenated solvent such as methylene chloride, chloroform or carbon tetrachloride, an ether such as tetrahydrofuran or 1,4-dioxan, an aromatic solvent such as toluene, or a dipolar aprotic solvent such as N,N-dimethylformamide, N,N-dimethylacetamide, N-methylpyrrolidin-2-one or dimethylsulfoxide. The reaction is conveniently carried out at a temperature in the range, for example, from 0 to 120°C, conveniently at or near ambient temperature.

By the term "reactive derivative" of the carboxylic acid of the formula **III** is meant a carboxylic acid derivative that will react with the quinazoline of formula **II** to give the corresponding amide. A suitable reactive derivative of a carboxylic acid of the formula **III** is, for example, an acyl halide, for example an acyl chloride formed by the reaction of the acid and an inorganic acid chloride, for example thionyl chloride; a mixed anhydride, for example an anhydride formed by the reaction of the acid and a chloroformate such as isobutyl chloroformate; an active ester, for example an ester formed by the reaction of the acid and a phenol such as pentafluorophenol, an ester such as pentafluorophenyl trifluoroacetate or an alcohol such as methanol, ethanol, isopropanol, butanol or N-hydroxybenzotriazole; or an acyl azide, for example an azide formed by the reaction of the acid and azide such as diphenylphosphoryl azide; an acyl cyanide, for example a cyanide formed by the reaction of an acid and a cyanide such as diethylphosphoryl cyanide. The reaction of such reactive derivatives of carboxylic acid with amines (such as a compound of the formula **II**) is well known in the art, for example they may be reacted in the presence of a base, such as those described above, and in a suitable solvent, such as those described above. The reaction may conveniently be performed at a temperature as described above.

The quinazoline of the formula **II** may be obtained by conventional procedures. For example, the reaction, conveniently in the presence of a suitable base, of a quinazoline of the formula **IIa:** 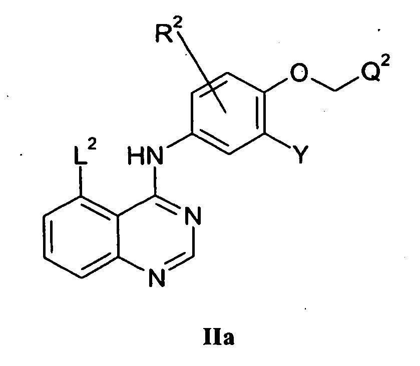 wherein R², Q² and Y have any of the meanings defined hereinbefore except that any functional group is protected if necessary, and L² is a suitable displaceable group, with an alcohol of the formula **IIb:** 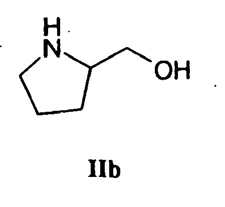 wherein any functional group is protected if necessary; and thereafter, if necessary removing any protecting group that is present by conventional means.

A suitable displaceable group L² in the quinazoline of formula **IIa** is for example halogeno or a sulfonyloxy group, for example fluoro, chloro, methylsulfonyloxy or toluene-4-sulfonyloxy group. A particular group L is fluoro or chloro, more particularly flouro.

A suitable base for the reaction a quinazoline of the formula **IIa** and the alcohol of the formula **IIb** includes, for example a strong non-nucleophilic base such as an alkali metal hydride, for example sodium hydride, or an alkali metal amide, for example lithium di-isopropylamide (LDA).

The reaction a quinazoline of the formula **IIa** and the alcohol of the formula **IIb** is conveniently carried out in the presence of a suitable inert solvent or diluent, for example a halogenated solvent such as methylene chloride, chloroform or carbon tetrachloride, an ether such as tetrahydrofuran or 1,4-dioxane, an aromatic solvent such as toluene, or a dipolar aprotic solvent such as N,N-dimethylformamide, N,N-dimethylacetamide, N-methylpyrrolidin-2-one or dimethylsulfoxide. The reaction is conveniently carried out at a temperature in the range of, for example, 10 to 250°C, preferably in the range 40 to 150°C. Conveniently, this reaction may also be performed by heating the reactants in a sealed vessel using a suitable heating apparatus such as a microwave heater.

Conveniently, the reaction a quinazoline of the formula **IIa** and the alcohol of the formula **IIb** is performed in the presence of a suitable catalyst, for example a crown ether such as 15-crown-5.

Alcohols of the formula **IIb** are commercially available compounds or they are known in the literature, or they can be can be prepared by standard processes known in the art. For example by the reduction of the corresponding acid or ester thereof as illustrated in *Reaction Scheme 1*:

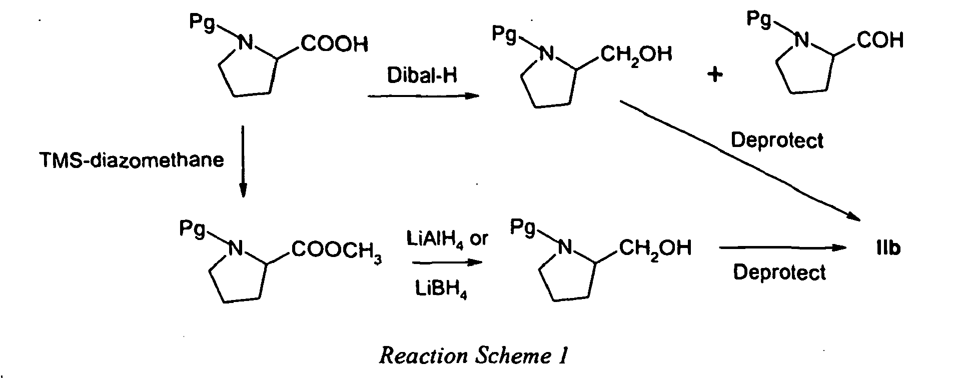

The quinazoline of the formula **IIa** may be obtained by conventional procedures. For example, a quinazoline of the formula **IIc:** 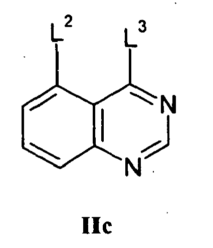 wherein L² and L³ are displaceable groups, and L³ is more labile than L², may be reacted with a compound of the formula **IId:** 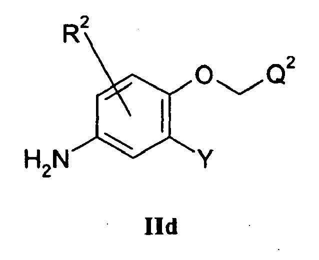 wherein Q², R² and Y have any of the meanings defined hereinbefore except that any functional group is protected if necessary, whereafter any protecting group that is present is removed by conventional means.

A suitable displaceable group L² is as hereinbefore defined, particularly fluoro. A suitable displaceable group L³ is, for example, a halogeno (particularly chloro), alkoxy, aryloxy, mercapto, alkylthio, arylthio, alkylsulfinyl, arylsulfinyl, alkylsulfonyl, arylsulfonyl, alkylsulfonyloxy or arylsulfonyloxy group, for example a chloro, bromo, methoxy, phenoxy, pentafluorophenoxy, methylthio, methanesulfonyl, methanesulfonyloxy or toluene-4-sulfonyloxy group.

The reaction is conveniently carried out in the presence of an acid. Suitable acids include, for example hydrogen chloride gas (conveniently dissolved in diethyl ether or dioxane) or hydrochloric acid.

Alternatively the quinazoline derivative of the formula **IIc,** wherein L³ is halogeno (for example chloro), may be reacted with the compound of the formula **IId** in the absence of an acid or a base. In this reaction displacement of the halogeno leaving group L³ results in the formation of the acid HL³ in-situ and the autocatalysis of the reaction.

Alternatively, the reaction of the quinazoline of formula **IIc** with the compound of formula **IId** may be carried out in the presence of a suitable base. A suitable base is, for example, an organic amine base such as, for example, pyridine, 2,6-lutidine, collidine, 4-dimethylaminopyridine, triethylamine, di-isopropylethylamine, N-methylmorpholine or diazabicyclo[5.4.0]undec-7-ene, or, for example, an alkali or alkaline earth metal carbonate, for example sodium carbonate, potassium carbonate, cesium carbonate, calcium carbonate, or, for example, an alkali metal hydride, for example sodium hydride.

The above reactions are conveniently carried out in the presence of a suitable inert solvent or diluent, for example an alcohol or ester such as methanol, ethanol, isopropanol or ethyl acetate, a halogenated solvent such as methylene chloride, chloroform or carbon tetrachloride, an ether such as tetrahydrofuran or 1,4-dioxan, an aromatic solvent such as toluene, or a dipolar aprotic solvent such as N,N-dimethylformamide, N,N-dimethylacetamide, N-methylpyrrolidin-2-one or dimethylsulfoxide. The above reactions are conveniently carried out at a temperature in the range, for example, 0 to 250°C, conveniently in the range 40 to 80°C or, preferably, at or near the reflux temperature of the solvent when used.

The quinazoline of formula **IIc** may be obtained using conventional methods, for example, when L² is fluoro and L³ is halogeno, 5-fluoro-3,4-dihydroquinazolin-4-one may be reacted with a suitable halogenating agent such as thionyl chloride, phosphoryl chloride or a mixture of carbon tetrachloride and triphenylphosphine. The 5-fluoro-3,4-dihydroquinazoline starting material is commercially available or can be prepared using conventional methods, for example as described in J. Org. Chem. 1952, 17, 164-176.

Compounds of the formula **IId** are commercially available compounds or they are known in the literature, or they can be can be prepared by standard processes known in the art. For example, the compound of the formula **IId** may be prepared in accordance with *Reaction Scheme 2*: 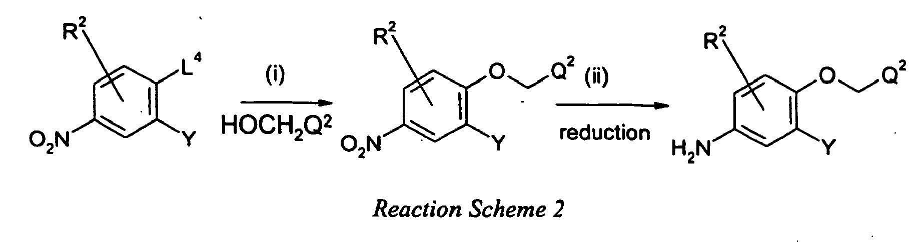 wherein L⁴ is a suitable displaceable group as hereinbefore defined (for example halogeno such as chloro) and Q², Y and R² are as hereinbefore defined, except any functional group is protected if necessary, and whereafter any protecting group that is present is removed by conventional means.
(i) The compounds of the formula HOCH₂Q² are commercially available, or they are known in the literature, or can be prepared using well known processes in the art. For example compounds of the formula Q²CH₂OH may be prepared using known methods, for example by reduction of the corresponding ester of the formula Q²COOR, wherein R is, for example (1-6C)alkyl, or benzyl, with a suitable reducing agent, for example sodium borohydride, followed by ester hydrolysis.
(ii) The reduction of the nitro group in step (ii) may be carried out under standard conditions, for example by catalytic hydrogenation over a platinum/carbon, palladium/carbon or nickel catalyst, treatment with a metal such as iron, titanium chloride, tin II chloride or indium, or treatment with another suitable reducing agent such as sodium dithionite:

Compounds of the formula **IId** may, for example, be prepared in accordance with *Reaction Scheme 3:* 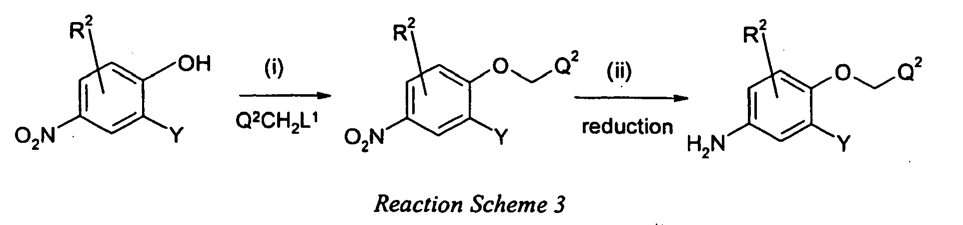 wherein L¹ is a suitable leaving group as defined hereinafter in relation to Process (b).

### Step (i): Analogous conditions to those used in Process (b)

### Step (ii) Analogous conditions to those used in Reaction Scheme 2.

Compounds of the formula **IId** may also be prepared by coupling the appropriate starting nitro phenol in *Reaction Scheme 3* with a compound of the formula Q²C(H)₂OH, conveniently in the presence of a suitable dehydrating agent. A suitable dehydrating agent is, for example, a carbodiimide reagent such as dicyclohexylcarbodiimide or 1-(3-dimethylaminopropyl)-3-ethylcarbodiimide or a mixture of an azo compound such as diethyl or di-tert-butyl azodicarboxylate and a phosphine such as triphenylphosphine. The reaction is conveniently carried out in the presence of a suitable inert solvent or diluent, for example a halogenated solvent such as methylene chloride, chloroform or carbon tetrachloride and at a temperature in the range, for example, 0 to 150°C, preferably at or near ambient temperature.

### Process (b)

A suitable displaceable group L¹ in the compound of the formula **V** is for example halogeno or a sulfonyloxy group, for example fluoro, chloro, methylsulfonyloxy or toluene-4-sulfonyloxy group. A particular group L is fluoro or chloro or methylsulfonyloxy.

The reaction of the quinazoline of formula **IV** with the compound of formula **V** is conveniently carried out in the presence of a suitable base such as, for example, a base as described in relation to Process (a) such as an alkali or alkaline earth metal carbonate, for example potassium carbonate.

The reaction a quinazoline of the formula **IV** and the compound of the formula **V** is conveniently carried out in the presence of a suitable inert solvent or diluent, for example a halogenated solvent such as methylene chloride, chloroform or carbon tetrachloride, an ether such as tetrahydrofuran or 1,4-dioxane, an aromatic solvent such as toluene, or a dipolar aprotic solvent such as N,N-dimethylformamide, N,N-dimethylacetamide, N-methylpyrrolidin-2-one or dimethylsulfoxide. The reaction is conveniently carried out at a temperature in the range of, for example, from 25 to 100°C, conveniently at or near ambient temperature.

The reaction a quinazoline of the formula **IV** and the compound of the formula **V** is conveniently carried out in the presence of a suitable catalyst, for example a crown ether such as 18-crown-6.

Compounds of the formula **V** are commercially available compounds or they are known in the literature, or they can be can be prepared by standard processes known in the art. The quinazoline of the formula **IV** may be prepared using conventional methods, for example, by reacting a compound of the formula **IVb:** 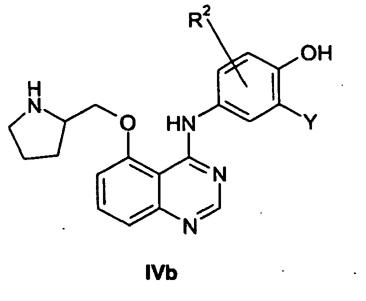 wherein R² and Y are as hereinbefore defined except that any functional group is protected if necessary, with a carboxylic acid of the formula **III**, or a reactive derivative thereof:

Z-X³- COOH **III**

wherein Z and X³ have any of the meanings defined hereinbefore except that any functional group is protected if necessary and whereafter any protecting group that is present is removed by conventional means.

The reaction of the quinazoline of the formula **IVb** and the compound of formula **III** is conveniently carried using analogous conditions to those described above for Process (a).

The quinazoline of the formula **IVb** may be prepared according to *Reaction Scheme 5.* 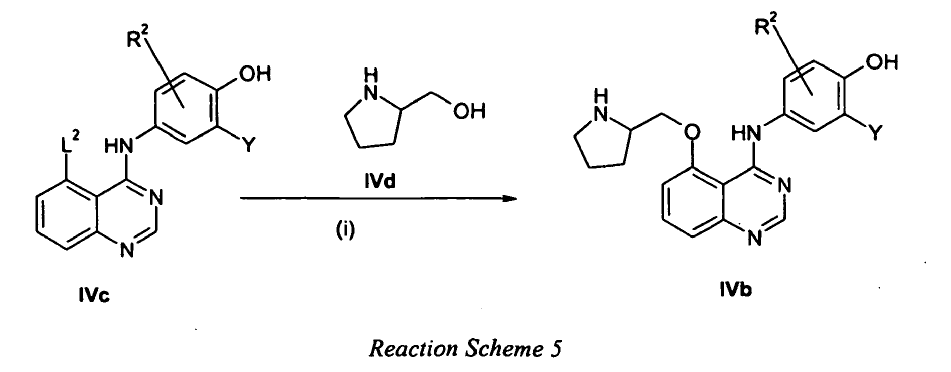 wherein R² and Y are as hereinbefore defined except that any functional group is protected if necessary, whereafter any protecting group that is present is removed by conventional means.

### Notes:

Step (i): Analogous conditions to those used in the reaction of the compound of formula **IIa** with the alcohol of the formula **IIb** described above in relation to process (a).

The compound of formula **IVc** may be prepared using *Reaction Scheme 4.*

The alcohol of the formula IVd are commercially available compounds or they are known in the literature, or they can be can be prepared by standard processes known in the art.

### Process (c)

A suitable displaceable group L¹ in the compound of the formula **VI** is for example halogeno or a sulfonyloxy group, for example fluoro, chloro, methylsulfonyloxy or toluene-4-sulfonyloxy group. A particular group L¹ is fluoro or chloro or methylsulfonyloxy.

The reaction of the quinazoline of formula **VI** with the compound of formula **VII** is conveniently carried out in the presence of a suitable catalyst such as, for example, tetra-n-butylammonium iodide or potassium iodide.

The reaction of a quinazoline of the formula **IV** and the compound of the formula **V** is conveniently carried out in the presence of a suitable inert solvent or diluent, for example an ether such as tetrahydrofuran or 1,4-dioxane, an aromatic solvent such as toluene, or a dipolar aprotic solvent such as N,N-dimethylformamide, N,N-dimethylacetamide, N-methylpyrrolidin-2-one or dimethylsulfoxide. The reaction is conveniently carried out at a temperature in the range of, for example, from 25 to 150°C, conveniently at about 100°C.

The quinazoline of the formula **VI** may be prepared using conventional methods, for example, as discussed above.

Compounds of the formula **VII** are commercially available compounds or they are known in the literature, or they can be can be prepared by standard processes known in the art.

The quinazoline derivative of the Formula Ia may be obtained from the above processes in the form of the free base or alternatively it may be obtained in the form of a salt, an acid addition salt. When it is desired to obtain the free base from a salt of the compound of Formula Ia, the salt may be treated with a suitable base, for example, an alkali or alkaline earth metal carbonate or hydroxide, for example sodium carbonate, potassium carbonate, calcium carbonate, sodium hydroxide or potassium hydroxide, or by treatment with ammonia for example using a methanolic ammonia solution such as 7N ammonia in methanol.

The protecting groups used in the processes above may in general be chosen from any of the groups described in the literature or known to the skilled chemist as appropriate for the protection of the group in question and may be introduced by conventional methods. Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Specific examples of protecting groups are given below for the sake of convenience, in which "lower", as in, for example, lower alkyl, signifies that the group to which it is applied preferably has 1-4 carbon atoms. It will be understood that these examples are not exhaustive. Where specific examples of methods for the removal of protecting groups are given below these are similarly not exhaustive. The use of protecting groups and methods of deprotection not specifically mentioned are, of course, within the scope of the invention.

A carboxy protecting group may be the residue of an ester-forming aliphatic or arylaliphatic alcohol or of an ester-forming silanol (the said alcohol or silanol preferably containing 1-20 carbon atoms). Examples of carboxy protecting groups include straight or branched chain (1-12C)alkyl groups (for example isopropyl, and tert-butyl); lower alkoxy- lower alkyl groups (for example methoxymethyl, ethoxymethyl and isobutoxymethyl); lower acyloxy-lower alkyl groups, (for example acetoxymethyl, propionyloxymethyl, butyryloxymethyl and pivaloyloxymethyl); lower alkoxycarbonyloxy-lower alkyl groups (for example 1-methoxycarbonyloxyethyl and 1-ethoxycarbonyloxyethyl); aryl-lower alkyl groups (for example benzyl, 4-methoxybenzyl, 2-nitrobenzyl, 4-nitrobenzyl, benzhydryl and phthalidyl); tri(lower alkyl)silyl groups (for example trimethylsilyl and tert-butyldimethylsilyl); tri(lower akyl)silyl-lower alkyl groups (for example trimethylsilylethyl); and (2-6C)alkenyl groups (for example allyl). Methods particularly appropriate for the removal of carboxyl protecting groups include for example acid-, base-, metal- or enzymically-catalysed cleavage.

Examples of hydroxy protecting groups include lower alkyl groups (for example tert-butyl), lower alkenyl groups (for example allyl); lower alkanoyl groups (for example acetyl); lower alkoxycarbonyl groups (for example tert-butoxycarbonyl); lower alkenyloxycarbonyl groups (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitrobenzyloxycarbonyl); tri(lower allcyl)silyl (for example trimethylsilyl and tert-butyldimethylsilyl) and aryl-lower alkyl (for example benzyl) groups.

Examples of amino protecting groups include formyl, aryl-lower alkyl groups (for example benzyl and substituted benzyl, 4-methoxybenzyl, 2-nitrobenzyl and 2,4-dimethoxybenzyl, and triphenylmethyl); di-4-anisylmethyl and furylmethyl groups; lower alkoxycarbonyl (for example tert-butoxycarbonyl); lower alkenyloxycarbonyl (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitrobenzyloxycarbonyl); lower alkanoyloxyalkyl groups (for example pivaloyloxymethyl); trialkylsilyl (for example trimethylsilyl and tert-butyldimethylsilyl); alkylidene (for example methylidene) and benzylidene and substituted benzylidene groups.

Methods appropriate for removal of hydroxy and amino protecting groups include, for example, acid-, base-, metal- or enzymically-catalysed hydrolysis for groups such as 2-nitrobenzyloxycarbonyl, hydrogenation for groups such as benzyl and photolytically for groups such as 2-nitrobenzyloxycarbonyl. For example a tert butoxycarbonyl protecting group may be removed from an amino group by an acid catalysed hydrolysis using trifluoroacetic acid.

The reader is referred to Advanced Organic Chemistry, 4th Edition, by J. March, published by John Wiley & Sons 1992, for general guidance on reaction conditions and reagents and to Protective Groups in Organic Synthesis, 2nd Edition, by T. Green et al., also published by John Wiley & Son, for general guidance on protecting groups.

It will be appreciated that certain of the various ring substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogeno group.

When a pharmaceutically-acceptable salt of a quinazoline derivative of the formula la is required, for example an acid-addition salt, it may be obtained by, for example, reaction of said quinazoline derivative with a suitable acid using a conventional procedure.

As mentioned hereinbefore some of the compounds according to the present invention may contain one or more chiral centers and may therefore exist as stereoisomers. Stereoisomers may be separated using conventional techniques, e.g. chromatography or fractional crystallisation. The enantiomers may be isolated by separation of a racemate for example by fractional crystallisation, resolution or HPLC. The diastereoisomers may be isolated by separation by virtue of the different physical properties of the diastereoisomers, for example, by fractional crystallisation, HPLC or flash chromatography. Alternatively particular stereoisomers may be made by chiral synthesis from chiral starting materials under conditions which will not cause racemisation or epimerisation, or by derivatisation, with a chiral reagent. When a specific stereoisomer is isolated it is suitably isolated substantially free for other stereoisomers, for example containing less than 20%, particularly less than 10% and more particularly less than 5% by weight of other stereoisomers.

In the section above relating to the preparation of the quinazoline derivative of Formula Ia, the expression "inert solvent" refers to a solvent which does not react with the starting materials, reagents, intermediates or products in a manner which adversely affects the yield of the desired product.

Persons skilled in the art will appreciate that, in order to obtain compounds of the invention in an alternative and in some occasions, more convenient manner, the individual process steps mentioned hereinbefore may be performed in different order, and/or the individual reactions may be performed at different stage in the overall route (i.e. chemical transformations may be performed upon different intermediates to those associated hereinbefore with a particular reaction).

Certain intermediates used in the processes described above are novel and form a further feature of the present invention. Accordingly there is provided a compound selected from a compound the formulae **II, IV, IVb** and **IVc** as hereinbefore defined, or a salt thereof. The intermediate may be in the form of a salt of the intermediate. Such salts need not be a pharmaceutically acceptable salt. For example it may be useful to prepare an intermediate in the form of a pharmaceutically non-acceptable salt if, for example, such salts are useful in the manufacture of a compound of Formula Ia.

### Biological Assays

The inhibitory activities of compounds were assessed in non-cell based protein tyrosine kinase assays as well as in cell based proliferation assays before their *in vivo* activity was assessed in Xenograft studies.

### a) Protein Tyrosine Kinase phosphorylation Assays

This test measures the ability of a test compound to inhibit the phosphorylation of a tyrosine containing polypeptide substrate by EGFR tyrosine kinase enzyme.

Recombinant intracellular fragments of EGFR, erbB2 and erbB4 (accession numbers X00588, X03363 and L07868 respectively) were cloned and expressed in the baculovirus/Sf21 system. Lysates were prepared from these cells by treatment with ice-cold lysis buffer (20mM N-2-hydroxyethylpiperizine-N'-2-ethanesulfonic acid (HEPES) pH7.5, 150mM NaCl, 10% glycerol, 1% Triton X-100, 1.5mM MgCl₂, 1mM ethylene glycol-bis(β-aminoethyl ether) N',N',N',N'-tetraacetic acid (EGTA), plus protease inhibitors and then cleared by centrifugation.

Constitutive kinase activity of these recombinant proteins was determined by their ability to phosphorylate a synthetic peptide (made up of a random co-polymer of Glutamic Acid, Alanine and Tyrosine in the ratio of 6:3:1). Specifically, Maxisorb^{™} 96-well immunoplates were coated with synthetic peptide (0.2µg of peptide in a 200µl phosphate buffered saline (PBS) solution and incubated at 4°C overnight). Plates were washed in 50mM HEPES pH 7.4 at room temperature to remove any excess unbound synthetic peptide. EGFR or erbB2 activities were assessed by incubation in peptide coated plates for 20 minutes at room temperature in 100mM HEPES pH 7.4 at room temperature, adenosine trisphosphate (ATP) at Km concentration for the respective enzyme, 10mM MnCl₂, 0.1mM Na₃VO₄, 0.2mM DL-dithiothreitol (DTT), 0.1% Triton X-100 with test compound in DMSO (final concentration of 2.5%). Reactions were terminated by the removal of the liquid components of the assay followed by washing of the plates with PBS-T (phosphate buffered saline with 0.5% Tween 20).

The immobilised phospho-peptide product of the reaction was detected by immunological methods. Firstly, plates were incubated for 90 minutes at room temperature with anti-phosphotyrosine primary antibodies that were raised in the mouse (4G10 from Upstate Biotechnology). Following extensive washing, plates were treated with Horseradish Peroxidase (HRP) conjugated sheep anti-mouse secondary antibody (NXA931 from Amersham) for 60 minutes at room temperature. After further washing, HRP activity in each well of the plate was measured colorimetrically using 22'-Azino-di-[3-ethylbenzthiazoline sulfonate (6)] diammonium salt crystals (ABTS™ from Roche) as a substrate. Quantification of colour development and thus enzyme activity was achieved by the measurement of absorbance at 405nm on a Molecular Devices ThermoMax microplate reader. Kinase inhibition for a given compound was expressed as an IC₅₀ value. This was determined by calculation of the concentration of compound that was required to give 50% inhibition of phosphorylation in this assay. The range of phosphorylation was calculated from the positive (vehicle plus ATP) and negative (vehicle minus ATP) control values.

### b) EGFR driven KB cell proliferation assay

This assay measures the ability of a test compound to inhibit the proliferation of KB cells (human naso-pharangeal carcinoma obtained from the American Type Culture Collection. (ATCC)).

KB cells were cultured in Dulbecco's modified Eagle's medium (DMEM) containing 10% foetal calf serum, 2 mM glutamine and non-essential amino acids at 37°C in a 7.5% CO₂ air incubator. Cells were harvested from the stock flasks usingTrypsin / ethylaminediaminetetraacetic acid (EDTA). Cell density was measured using a haemocytometer and viability was calculated using trypan blue solution before being seeded at a density of 1.25x10³ cells per well of a 96 well plate in DMEM containing 2.5% charcoal stripped serum, 1mM glutamine and non-essential amino acids at 37°C in 7.5% CO₂ and allowed to settle for 4 hours.

Following adhesion to the plate, the cells are treated with or without EGF (final concentration of 1ng/ml) and with or without compound at a range of concentrations in dimethylsulfoxide (DMSO) (0.1 % final) before incubation for 4 days. Following the incubation period, cell numbers were determined by addition of 50µl of 3-(4,5-Dimethylthiazol-2-yl)-2,5-diphenyltetrazolium bromide (MTT) (stock 5mg/ml) for 2 hours. MTT solution was then tipped off, the plate gently tapped dry and the cells dissolved upon the addition of 100µl of DMSO.

Absorbance of the solubilised cells was read at 540nm using a Molecular Devices ThernnoMax microplate reader. Inhibition of proliferation was expressed as an IC₅₀ value. This was determined by calculation of the concentration of compound that was required to give 50% inhibition of proliferation. The range of proliferation was calculated from the positive (vehicle plus EGF) and negative (vehicle minus EGF) control values.

### c) Clone 24 phospho-erbB2 cell assay

This immunofluorescence end point assay measures the ability of a test compound to inhibit the phosphorylation of erbB2 in a MCF7 (breast carcinoma) derived cell line which was generated by transfecting MCF7 cells with the full length erbB2 gene using standard methods to give a cell line that overexpresses full length wild type erbB2 protein (hereinafter 'Clone 24' cells).

Clone 24 cells were cultured in Growth Medium (phenol red free Dulbecco's modified Eagle's medium (DMEM) containing 10% foetal bovine serum, 2 mM glutamine and 1.2mg/ml G418) in a 7.5% CO₂ air incubator at 37°C. Cells were harvested from T75 stock flasks by washing once in PBS (phosphate buffered saline, pH7.4, Gibco No. 10010-015) and harvested using 2mls of Trypsin (1.25mg/ml) / ethylaminediaminetetraacetic acid (EDTA) (0.8mg/ml) solution. The cells were resuspended in Growth Medium. Cell density was measured using a haemocytometer and viability was calculated using Trypan Blue solution before being further diluted in Growth Medium and seeded at a density of 1x10⁴ cells per well (in 100ul) into clear bottomed 96 well plates (Packard, No. 6005182).

3 days later, Growth Medium was removed from the wells and replaced with 100ul, Assay Medium (phenol red free DMEM, 2mM glutamine, 1.2mg/ml G418) either with or without erbB inhibitor compound. Plates were returned to the incubator for 4hrs and then 20µl of 20% formaldehyde solution in PBS was added to each well and the plate was left at room temperature for 30 minutes. This fixative solution was removed with a multichannel pipette, 100µl of PBS was added to each well and then removed with a multichannel pipette and then 50µl PBS was added to each well. Plates were then sealed and stored for up to 2 weeks at 4°C.

Immunostaining was performed at room temperature. Wells were washed once with 200µl PBS / Tween 20 (made by adding 1 sachet of PBS / Tween dry powder (Sigma, No. P3563) to 1L of double distilled H₂O) using a plate washer then 200µl Blocking Solution (5% Marvel dried skimmed milk (Nestle) in PBS /Tween 20) was added and incubated for 10 minutes. Blocking Solution was removed using a plate washer and 200µl of 0.5% Triton X-100 / PBS was added to permeabalise the cells. After 10 minutes, the plate was washed with 200µl PBS / Tween 20 and then 200µl Blocking Solution was added once again and incubated for 15 minutes. Following removal of the Blocking Solution with a plate washer, 30µl of rabbit polyclonal anti-phospho ErbB2 IgG antibody (epitope phospho-Tyr 1248, SantaCruz, No. SC-12352-R), diluted 1:250 in Blocking Solution, was added to each well and incubated for 2 hours. Then this primary antibody solution was removed from the wells using a plate washer followed by two 200µl PBS / Tween 20 washes using a plate washer. Then 30µl of Alexa-Fluor 488 goat anti-rabbit IgG secondary antibody (Molecular Probes, No. A-11008), diluted 1:750 in Blocking Solution, was added to each well. From now onwards, wherever possible, plates were protected from light exposure, at this stage by sealing with black backing tape. The plates were incubated for 45 minutes and then the secondary antibody solution was removed from the wells followed by two 200ul PBS / Tween 20 washes using a plate washer. Then 100µl PBS was added to each plate, incubated for 10 minutes and then removed using a plate washer. Then a further 100µl PBS was added to each plate and then, without prolonged incubation, removed using a plate washer. Then 50µl of PBS was added to each well and plates were resealed with black backing tape and stored for up to 2 days at 4°C before analysis.

The Fluorescence signal is each well was measured using an Acumen Explorer Instrument (Acumen Bioscience Ltd.), a plate reader that can be used to rapidly quantitate features of images generated by laser-scanning. The instrument was set to measure the number of fluorescent objects above a pre-set threshold value and this provided a measure of the phosphorylation status of erbB2 protein. Fluorescence dose response data obtained with each compound was exported into a suitable software package (such as Origin) to perform curve fitting analysis. Inhibition of erbB2 phosphorylation was expressed as an IC₅₀ value. This was determined by calculation of the concentration of compound that was required to give 50% inhibition of erbB2 phosphorylation signal.

### d) In vivo BT-474 Xenograft assay

This assay measures the ability of a test compound to inhibit the growth of a BT-474 tumour cell xenograft (human mammary carcinoma obtained from Dr Baselga, Laboratorio Recerca Oncologica, Paseo Vall D'Hebron 119-129, Barcelona 08035, Spain) in Female Swiss athymic mice (Alderley Park, *nu*/*nu* genotype) (Baselga, J. et al. (1998) Cancer Research, 58, 2825-2831).

Female Swiss athymic (*nu*/*nu* genotype) mice were bred and maintained in Alderley Park in negative pressure Isolators (PFI Systems Ltd.). Mice were housed in a barrier facility with 12hr light/dark cycles and provided with sterilised food and water *ad libitum.* All procedures were performed on mice of at least 8 weeks of age. BT-474 tumour cell xenografts were established in the hind flank of donor mice by sub-cutaneous injections of 1x10⁷ freshly cultured cells in 100µl of serum free media with 50% Matrigel per animal. On day 14 post-implant, mice were randomised into groups of 10 prior to the treatment with compound or vehicle control that was administered once daily at 0.1ml/10g body weight. Tumour volume was assessed twice weekly by bilateral Vernier calliper measurement, using the formula (length x width) x √(length x width) x (π/6), where length was the longest diameter across the tumour, and width was the corresponding perpendicular. Growth inhibition from start of treatment was calculated by comparison of the mean changes in tumour volume for the control and treated groups, and statistical significance between the two groups was evaluated using a Students t test.

Although the pharmacological properties of the compounds of the Formula Ia vary with structural change as expected, in general activity possessed by compounds of the Formula Ia, may be demonstrated at the following concentrations or doses in one or more of the above tests (a), (b) and (c):-

| | |
|---|---|
| Test (a):- | IC₅₀ in the range, for example, 0.001 - 1 µM; |
| Test (b):- | IC₅₀ in the range, for example, 0.001 - 5 µM; |
| Test (c):- | IC₅₀ in the range, for example, 0.001 - 5 µM; |
| Test (d):- | activity in the range, for example, 1-200 mg/kg/day; |

No physiologically unacceptable toxicity was observed in Test (d) at the effective dose for compounds tested of the present invention. Accordingly no untoward toxicological effects are expected when a compound of Formula Ia, or a pharmaceutically-acceptable salt thereof, as defined hereinbefore is administered at the dosage ranges defined hereinafter.

By way of example, Table A illustrates the activity of representative compounds according to the invention. Column 2 of Table A shows IC₅₀ data from Test (a) for the inhibition of EGFR tyrosine kinase protein phosphorylation; column 3 shows IC₅₀ data from Test (a) for the inhibition of erbB2 tyrosine kinase protein phosphorylation; and column 4 shows IC₅₀ data for inhibition of phosphorylation of erbB2 in a MCF7 derived cell line in Test (c) described above:

**Table A**

| **Example Number** | **IC₅₀ (**µ**M) Test (a): Inhibition of EGFR tyrosine kinase protein phosphorylation** | **IC₅₀ (**µ**M) Test (a): Inhibition of erbB2 tyrosine kinase protein phosphorylation** | **IC₅₀ (**µ**M) Test (c): Inhibition of erbB2 tyrosine kinase protein phosphorylation** |
|---|---|---|---|
| 25 | 0.26 | 0.048 | 1.40 |
| 40 | 33.0 | 0.63 | 2.40 |
| 78 | 2.40 | 0.034 | 0.19 |

According to a further aspect of the invention there is provided a pharmaceutical composition which comprises a quinazoline derivative of the formula Ia, or a pharmaceutically-acceptable thereof, as defined hereinbefore in association with a pharmaceutically-acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a quinazoline derivative of the formula Ia will naturally vary according to the nature and severity of the conditions, the age and sex of the animal or patient and the route of administration, according to well known principles of medicine.

In using a quinazoline derivative of the formula Ia for therapeutic or prophylactic purposes it will generally be administered so that a daily dose in the range, for example, 0.1 mg/kg to 75 mg/kg body weight is received, given if required in divided doses. In general lower doses will be administered when a parenteral route is employed. Thus, for example, for intravenous administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration is however preferred, particularly in tablet form. Typically, unit dosage forms will contain about 0.5 mg to 0.5 g of a compound of this invention.

We have found that the compounds of the present invention possess anti-proliferative properties such as anti-cancer properties that are believed to arise from their erb-B, particularly EGFR and more particularly erbB2 receptor tyrosine kinase inhibitory activity. Furthermore, certain of the compounds according to the present invention possess substantially better potency against the erbB2 receptor tyrosine kinase, than against other tyrosine kinases enzymes, such as EGFR tyrosine kinase. Such compounds possess sufficient potency against the erbB2 receptor tyrosine kinase that they may be used in an amount sufficient to inhibit erbB2 receptor tyrosine kinase whilst demonstrating little, or significantly lower, activity against other tyrosine kinases such as EGFR. Such compounds are likely to be useful for the selective inhibition of erbB2 receptor tyrosine kinase and are likely to be useful for the effective treatment of, for example erbB2 driven tumours. Accordingly, the compounds of the present invention are expected to be useful in the treatment of diseases or medical conditions mediated alone or in part by and erb-B, particularly erbB2 receptor tyrosine kinases, i.e. the compounds may be used to produce a erb-B, particularly an erbB2, receptor tyrosine kinase inhibitory effect in a warm-blooded animal in need of such treatment. Thus the compounds of the present invention provide a method for the treatment of malignant cells characterised by inhibition of the erb-B, particularly erbB2, receptor tyrosine kinase. Particularly the compounds of the invention may be used to produce an anti-proliferative and/or pro-apoptotic and/or anti-invasive effect mediated alone or in part by the inhibition of erb-B, particularly erbB2, receptor tyrosine kinases. Particularly, the compounds of the present invention are expected to be useful in the prevention or treatment of those tumours that are sensitive to inhibition of an erb-B, particularly the erbB2, receptor tyrosine kinase that are involved in the signal transduction steps which drive proliferation and survival of these tumour cells. Accordingly the compounds of the present invention are expected to be useful in the treatment and/or prevention of a number of hyperproliferative disorders by providing an anti-proliferative effect. These disorders include, for example psoriasis, benign prostatic hyperplasia (BPH), atherosclerosis and restenosis and, in particular, erb-B, more particularly erb-B2, receptor tyrosine kinase driven tumours. Such benign or malignant tumours may affect any tissue and include non-solid tumours such as leukaemia, multiple myeloma or lymphoma, and also solid tumours, for example bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval tumours.

According to this aspect of the invention there is provided a quinazoline derivative of the formula Ia, or a pharmaceutically acceptable salt thereof, for use as a medicament.

Thus according to this aspect of the invention there is provided the use of a quinazoline derivative of the formula Ia, or a pharmaceutically-acceptable salt thereof, as defined hereinbefore in the manufacture of a medicament for use in the production of an anti-proliferative effect in a warm-blooded animal such as man.

A method for producing an anti-proliferative effect in a warm-blooded animal, such as man, in need of such treatment may comprise administering to said animal an effective amount of a quinazoline derivative of the formula Ia, or a pharmaceutically acceptable salt thereof, as hereinbefore defined.

According to a further aspect of the invention there is provided a quinazoline derivative of the formula Ia, or a pharmaceutically acceptable salt thereof, for use in the production of an anti-proliferative effect in a warm-blooded animal such as man.

According to a further aspect of the invention there is provided the use of a quinazoline derivative of the formula Ia, or a pharmaceutically-acceptable salt thereof, as defined hereinbefore in the manufacture of a medicament for use in the production of an anti-proliferative effect which effect is produced alone or in part by inhibiting erbB2 receptor tyrosine kinase in a warm-blooded animal such as man.

A method for producing an anti-proliferative effect which effect is produced alone or in part by inhibiting erbB2 receptor tyrosine kinase in a warm-blooded animal, such as man, in need of such treatment may comprise administering to said animal an effective amount of a quinazoline derivative of the formula Ia, or a pharmaceutically acceptable salt thereof, as hereinbefore defined.

According to a further aspect of the invention there is provided a quinazoline derivative of the formula Ia, or a pharmaceutically acceptable salt thereof, for use in the production of an anti-proliferative effect which effect is produced alone or in part by inhibiting erbB2 receptor tyrosine kinase in a warm-blooded animal such as man.

According to a further aspect of the present invention there is provided the use of a quinazoline derivative of the formula Ia, or a pharmaceutically-acceptable salt thereof, as defined hereinbefore in the manufacture of a medicament for use in the treatment of a disease or medical condition (for example a cancer as mentioned herein) mediated alone or in part by erb-B, particularly erbB2, receptor tyrosine kinase.

A method for treating a disease or medical condition (for example a cancer as mentioned herein) mediated alone or in part by erb-B, particularly erbB2, receptor tyrosine kinase in a warm-blooded animal, such as man, in need of such treatment, may comprise administering to said animal an effective amount of a quinazoline derivative of the formula Ia, or a pharmaceutically-acceptable salt thereof, as defined hereinbefore.

According to a further aspect of the invention there is provided a quinazoline derivative of the formula Ia, or a pharmaceutically acceptable salt thereof, for use in the treatment of a disease or medical condition (for example a cancer as mentioned herein) mediated alone or in part by erb-B, particularly erbB2, receptor tyrosine kinase.

According to a further aspect of the invention there is provided the use of a quinazoline derivative of the formula Ia, or a pharmaceutically-acceptable salt thereof, as defined hereinbefore in the manufacture of a medicament for use in the prevention or treatment of those tumours which are sensitive to inhibition of erbB2 receptor tyrosine kinase that is involved in the signal transduction steps which lead to the proliferation of tumour cells.

A method for the prevention or treatment of those tumours which are sensitive to inhibition of erbB2 receptor tyrosine kinase, that is involved in the signal transduction steps which lead to the proliferation and/or survival of tumour cells in a warm-blooded animal, such as man, in need of such treatment, may comprise administering to said animal an effective amount of a quinazoline derivative of the formula Ia, or a pharmaceutically-acceptable salt thereof, as defined hereinbefore.

According to a further aspect of the invention there is provided a quinazoline derivative of the formula Ia, or a pharmaceutically acceptable salt thereof, for use in the prevention or treatment of those tumours which are sensitive to inhibition of the erbB2 receptor tyrosine kinase, that is involved in the signal transduction steps which lead to the proliferation and/or survival of tumour cells. According to a further aspect of the invention there is provided the use of a quinazoline derivative of the formula I, or a pharmaceutically-acceptable salt thereof, as defined hereinbefore in the manufacture of a medicament for use in providing a erbB2 receptor tyrosine kinase inhibitory effect.

A method for providing an erbB2 receptor tyrosine kinase inhibitory effect in a warm-blooded animal, such as man, in need of such treatment, may comprise administering to said animal an effective amount of a quinazoline derivative of the formula Ia, or a pharmaceutically-acceptable salt thereof, as defined hereinbefore.

According to a further aspect of the invention there is provided a quinazoline derivative of the formula Ia, or a pharmaceutically acceptable salt thereof, for use in providing an erbB2 receptor tyrosine kinase inhibitory effect.

According to a further aspect of the invention there is provided the use of a quinazoline derivative of the formula Ia, or a pharmaceutically-acceptable salt thereof, as defined hereinbefore in the manufacture of a medicament for use in providing a selective erbB2 kinase inhibitory effect.

A method for providing a selective erbB2 kinase inhibitory effect in a warm-blooded animal, such as man, in need of such treatment, may comprise administering to said animal an effective amount of a quinazoline derivative of the formula la, or a pharmaceutically-acceptable salt thereof, as defined hereinbefore.

According to a further aspect of the invention there is provided a quinazoline derivative of the formula Ia, or a pharmaceutically acceptable salt thereof, for use in providing a selective erbB2 kinase inhibitory effect.

By "a selective erbB2 kinase inhibitory effect" is meant that the quinazoline derivative of Formula Ia is more potent against erbB2 receptor tyrosine kinase than it is against other kinases. In particular some of the compounds according to the invention are more potent against erbB2 receptor kinase than it is against other tyrosine kinases such as other erb-B receptor tyrosine kinases, particularly EGFR tyrosine kinase. For example a selective erb-B2 kinase inhibitor according to the invention is at least 5 times, preferably at least 10 times more potent against erbB2 receptor tyrosine kinase than it is against EGFR tyrosine kinase, as determined from the relative IC₅₀ values in suitable assays (for example the by comparing the IC₅₀ value from the Clone 24 phospho-erbB2 cell assay (a measure of the erb-B2 tyrosine kinase inhibitory activity in cells) with the IC₅₀ from the KB cell assay (a measure of the EGFR tyrosine kinase inhibitory activity in cells) for a given test compound as described above).

According to a further aspect of the present invention there is provided the use of a quinazoline derivative of the formula Ia, or a pharmaceutically-acceptable salt thereof, as defined hereinbefore in the manufacture of a medicament for use in the treatment of a cancer, for example a cancer selected from leukaemia, multiple myeloma, lymphoma, bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval cancer.

A method for treating a cancer, for example a cancer selected from selected from leukaemia, multiple myeloma, lymphoma, bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval cancer in a warm-blooded animal, such as man, in need of such treatment, may comprise administering to said animal an effective amount of a quinazoline derivative of the formula Ia, or a pharmaceutically-acceptable salt thereof, as defined hereinbefore.

According to a further aspect of the invention there is provided a quinazoline derivative of the formula Ia, or a pharmaceutically acceptable salt thereof, for use in the treatment of a cancer, for example a cancer selected from leukaemia, multiple myeloma, lymphoma, bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval cancer.

The anti-proliferative treatment defined hereinbefore may be applied as a sole therapy or may involve, in addition to the quinazoline derivative of the invention, conventional surgery or radiotherapy or chemotherapy. Such chemotherapy may include one or more of the following categories of anti-tumour agents:
As mentioned above the size of the dose required for the therapeutic or prophlyactic treatment of a particular disease will necessarily be varied depending upon, amongst other things, the host treated, the route of administration and the severity of the illness being treated.

The anti-proliferative treatment defined hereinbefore may be applied as a sole therapy or may involve, in addition to the quinazoline derivative of the invention, conventional surgery or radiotherapy or chemotherapy. Such chemotherapy may include one or more of the following categories of anti-tumour agents:-
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulfan and nitrosoureas); antimetabolites (for example antifolates such as fluoropyrimidines like 5-fluorouracil and tegafur, raltitrexed, methotrexate, cytosine arabinoside and hydroxyurea; antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) agents which inhibit cancer cell invasion (for example metalloproteinase inhibitors like marimastat and inhibitors of urokinase plasminogen activator receptor function);
(iv) inhibitors of growth factor function, for example such inhibitors include growth factor antibodies, growth factor receptor antibodies (for example the anti-erbB2 antibody trastuzumab [Herceptin™] and the anti-erbB1 antibody cetuximab [C225]), farnesyl transferase inhibitors, tyrosine kinase inhibitors and serine/threonine kinase inhibitors, for example other inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab [Avastin™], compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin);
(vi) vascular damaging agents such as Combretastatin A4 and compounds disclosed in International Patent Applications WO 99/02166, WO00/40529, WO 00/41669, WO01/92224, WO02/04434 and WO02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

According to this aspect of the invention there is provided a pharmaceutical product comprising a quinazoline derivative of the Formula Ia as defined hereinbefore and an additional anti-tumour agent as defined hereinbefore for the conjoint treatment of cancer.

Although the compounds of the Formula Ia are primarily of value as therapeutic agents for use in warm-blooded animals (including man), they are also useful whenever it is required to inhibit the effects of the erbB receptor tyrosine protein kinases. Thus, they are useful as pharmacological standards for use in the development of new biological tests and in the search for new pharmacological agents.

The invention will now be illustrated by the following non limiting examples in which, unless stated otherwise:
(i) temperatures are given in degrees Celsius (°C); operations were carried out at room or ambient temperature, that is, at a temperature in the range of 18-25°C;
(ii) organic solutions were dried over anhydrous magnesium sulfate; evaporation of solvent was carried out using a rotary evaporator under reduced pressure (600-4000 Pascals; 4.5-30mmHg) with a bath temperature of up to 60°C;
(iii) chromatography means flash chromatography on silica gel; thin layer chromatography (TLC) was carried out on silica gel plates;
(iv) in general, the course of reactions was followed by TLC and / or analytical LC-MS, and reaction times are given for illustration only;
(v) final products had satisfactory proton nuclear magnetic resonance (NMR) spectra and/or mass spectral data;
(vi) yields are given for illustration only and are not necessarily those which can be obtained by diligent process development; preparations were repeated if more material was required;
(vii) when given, NMR data is in the form of delta values for major diagnostic protons, given in parts per million (ppm) relative to tetramethylsilane (TMS) as an internal standard, determined at 300 MHz using perdeuterio dimethyl sulfoxide (DMSO-d₆) as solvent unless otherwise indicated; the following abbreviations have been used: s, singlet; d, doublet; t, triplet; q, quartet; m, multiplet; b, broad;
(viii) chemical symbols have their usual meanings; SI units and symbols are used;
(ix) solvent ratios are given in volume:volume (v/v) terms; and
(x) mass spectra were run with an electron energy of 70 electron volts in the chemical ionization (CI) mode using a direct exposure probe; where indicated ionization was effected by electron impact (EI), fast atom bombardment (FAB) or electrospray (ESP); values for m/z are given; generally, only ions which indicate the parent mass are reported; and unless otherwise stated, the mass ion quoted is (MH)⁺ which refers to the protonated mass ion; reference to M⁺ is to the mass ion generated by loss of an electron; and reference to M-H⁺ is to the mass ion generated by loss of a proton;
(xi) unless stated otherwise compounds containing an asymmetrically substituted carbon and/or sulfur atom have not been resolved;
(xii) where a synthesis is described as being analogous to that described in a previous example the amounts used are the millimolar ratio equivalents to those used in the previous example;
(xiii) all microwave reactions were carried out in a CEM Discover™ microwave synthesisor;
(xiv) preparative high performance liquid chromatography (HPLC) was performed on a Gilson instrument using the following conditions:

| | |
|---|---|
| Column: | 21 mm x 10 cm Hichrom RPB |
| Solvent A: | Water + 0.1 % trifluoroacetic acid, |
| Solvent B: | Acetonitrile + 0.1% trifluoroacetic acid |
| Flow rate: | 18 ml/min |
| Run time: | 15 minutes with a 10 minute gradient from 5-95% B |
| Wavelength: | 254 nm, bandwidth 10 nm |
| Injection volume | 2.0-4.0 ml; |

(xv) the following abbreviations have been used:

| | |
|---|---|
| HATU | O-(7-Azabenzotriazol-1-yl)-N,N,N',N'-Tetramethylurorunm Hexafluoro-Phosphate; and |
| THF | tetrahydrofuran; |
| DMF | *N*,*N*-dimethylformamide; |
| DMA | *N*,*N*-dimethylacetamide; |
| DCM | dichloromethane; |
| DMSO | dimethylsulfoxide; |
| IPA | Isopropyl alcohol; |
| ether | diethyl ether; |
| TFA | trifluoroacetic acid. |

Examples 5 to 10, 17 to 20, 29 to 32, 34 to 39, 50 to 63 and 73 to 101 are included for illustrative purposes only and do not form part of the present invention.

### Example 1

### 2-((2R)-2-{[(4-{[3-Chloro-4-(pyridin-2-ylmethoxy)phenyl]amino}quinazolin-5-yl)oxy]methyl}pyrrolidin-1-yl)-2-oxoethanol

A mixture of HATU (69 mg), diisopropylethylamine (58 µl), glycolic acid (13 mg) and *N*-[3-chloro-4-(pyridin-2-ylmethoxy)phenyl]-5-[(2*R*)-pyrrolidin-2-ylmethoxy]quinazolin-4-amine (70 mg) in DCM (5 ml) was stirred overnight. The solution was concentrated *in vacuo* and the residue purified by chromatography using DCM - 5% methanol as eluent to give the title compound as a white solid (48 mg, 61%); NMR spectrum (DMSO-d6) 1.85 - 2.10 (m, 4H), 3.42 (m, 2H), 4.04 (m, 2H), 4.20 (dd, 1H), 4.45 (dd, 1H), 4.52 (t, 1H), 4.60 (m, 1H), 5.30 (s, 2H), 7.25 (d, 2H), 7.38 (m, 2H), 7.58 (m, 2H), 7.73 (t, 1H), 7.89 (t, 1H), 8.05 (d, 1H), 8.48 (s, 1H), 8.60 (d, 1H), 9.98 (bs, 1H); Mass spectrum MH⁺ 520.

The *N*-[3-chloro-4-(pyridin-2-ylmethoxy)phenyl]-5-[(2*R*)-pyrrolidin-2-ylmethoxy]quinazolin-4-amine used as starting material was prepared as follows:
DMF (0.2 ml) was added to a suspension of 5-fluoro-3,4-dihydro-3*H*-quinazolin-4-one (1.64 g) in thionyl chloride (10 ml) and the mixture was stirred and heated at 80°C for 6 hours. Volatile material was removed by evaporation and the residue was azeotroped with toluene (20 ml). The resulting solid was added portionwise to a vigorously stirred mixture of saturated sodium bicarbonate (50 ml), crushed ice (50 g) and DCM (50 ml) such that the temperature was kept below 5°C. The organic phase was separated, dried and concentrated to give 4-chloro-5-fluoroquinazoline (1.82 g, 99%) as a solid which was used without purification; NMR spectrum (CDCl₃) 7.35 - 7.45 (m, 1H), 7.85 - 7.95 (m, 2H), 9.0 (s, 1H).

4-Chloro-5-fluoroquinazoline (6.75 g) was added to stirred solution of 3-chloro-4-(2-pyridylmethoxy)aniline (9.27 g, obtained as described in Example 13 of WO 96/15118) in IPA (200 ml), and the solution was stirred and heated at reflux for 8 hours. The solution was allowed to cool to ambient temperature overnight and the precipitated solid was filtered off, washed with acetone and dried. The solid was added to 50% aqueous methanol (400 ml) and the mixture was heated on a steam bath until all the solid had dissolved. The solution was basified by careful addition of aqueous ammonia (0.880), and the mixture was concentrated to remove methanol. Water (300 ml) was added and the mixture was extracted with DCM (600 ml). The extract was washed with water, and brine, and dried. The solvent was removed by evaporation to give a solid, which was re-precipitated from a mixture of ethyl acetate, tetrahydrofuran and isohexane to give *N*-[3-chloro-4-(pyridin-2-ylmethoxy)phenyl]-5-fluoroquinazolin-4-amine as a beige solid (6.75 g, 48%); NMR spectrum (DMSO-d6) 5.3 (s, 2H), 7.2 - 7.3 (d, 1H), 7.35 - 7.5 (m, 2H), 7.5 - 7.65 (m, 3H), 7.8 - 7.95 (m, 3H), 8.55 (s, 1H), 8.55 - 8.6 (d, 1H), 9.1 - 9.2 (bs, 1H); Mass spectrum MH⁺ 381.

Sodium hydride (60% dispersion in mineral oil, 0.16 g) was added to R-prolinol (0.39 ml) and *N*-[3-chloro-4-(pyridin-2-ylmethoxy)phenyl]-5-fluoroquinazolin-4-amine (0.5 g) in DMA (4 ml) and the reaction heated at 95°C for 4 hours. The reaction was cooled, quenched with water, and concentrated *in vacuo.* The residue was purified by chromatography using DCM - 5% methanol / 7N ammonia as eluent to give *N*-[3-chloro-4-(pyridin-2-ylmethoxy)phenyl]-5-[(2*R*)-pyrrolidin-2-ylmethoxy]quinazolin-4-amine as a beige solid (0.27 g, 45%); NMR spectrum (DMSO-d6) 1.47 (m, 1H), 1.69 (m, 2H), 1.86 (m, 1H), 2.85 (m, 2H), 3.53 (m, 1H), 4.05 (dt, 1H), 4.31 (dd, 1H), 5.27 (s, 2H), 7.12 (d, 1H), 7.23 (d, 1H), 7.31 (d, 1H), 7.35 (dd, 1H), 7.57 (d, 1H), 7.70 (t, 1H), 7.79 (dd, 1H), 7.86 (dt, 1H), 8.14 (d, 1H), 8.50 (s, 1H), 8.58 (d, 1H), 10.55 (bs, 1H); Mass spectrum M⁺ 462.

### Example 2

### 2-((2S)-2-{[(4-{[3-Chloro-4-(pyridin-2-ylmethoxy)phenyl]amino}quinazolin-5-yl)oxy]methyl}pyrrolidin-1-yl)-2-oxoethanol

The procedure described in example 1 was repeated using glycolic acid and *N*-[3-chloro-4-(pyridin-2-ylinethoxy)phenyl]-5-[(2*S*)-pyrrolidin-2-ylmethoxy]quinazolin-4-amine in 75% yield; NMR spectrum (DMSO-d6) 1.98 (m, 4H), 3.40 (m, 2H), 4.02 (m, 2H), 4.18 (dd, 1H), 4.43 (dd, 1H), 4.52 (m, 1H), 5.26 (s, 2H), 7.23 (d, 2H), 7.34 (m, 2H), 7.57 (d, 2H), 7.71 (t, 1H), 7.86 (d, 1H), 8.02 (d, 1H), 8.46 (s, 1H), 8.59 (d, 1H), 9.95 (bs, 1H); Mass spectrum MH⁺ 520.

The *N*-[3-chloro-4-(pyridin-2-ylmethoxy)phenyl]-5-[(2*S*)-pyrrolidin-2-ylmethoxy]quinazolin-4-amine used as starting material was prepared as described in example 1 (preparation of starting materials) using S-prolinol and *N*-[3-chloro-4-(pyridin-2-ylmethoxy)phenyl]-5-fluoroquinazolin-4-amine in 51% yield; NMR spectrum (DMSO-d6) 1.47 (m, 1H), 1.69 (m, 2H), 1.86 (m, 1H), 2.85 (m, 2H), 3.53 (m, 1H), 4.05 (dt, 1H), 4.31 (dd, 1H), 5.27 (s, 2H), 7.12 (d, 1H), 7.23 (d, 1H), 7.31 (d, 1H), 7.35 (dd, 1H), 7.57 (d, 1H), 7.70 (t, 1H), 7.79 (dd, 1H), 7.86 (dt, 1H), 8.14 (d, 1H), 8.50 (s, 1H), 8.58 (d, 1H), 10.55 (bs, 1H); Mass spectrum M⁺ 462.

### Example 3

### N-[3-Chloro-4-(pyridin-2-ylmethoxy)phenyl]-5-({(2R)-1-[(dimethylamino)acetyl]pyrrolidin-2-yl}methoxy)quinazolin-4-amine

The procedure described in example 1 was repeated using *N,N*-dimethylglycine and *N-*[3-chloro-4-(pyridin-2-ylmethoxy)phenyl]-5-[(2*R*)-pyrrolidin-2-ylmethoxy]quinazolin-4-amine (obtained as described in example 1, preparation of starting materials) in 84% yield; Mass spectrum M⁺ 547.

### Example 4

### N-[3-Chloro-4-(pyridin-2-ylmethoxy)phenyl]-5-({(2S)-1-[(dimethylamino)acetyl]pyrrolidin-2-yl}methoxy)quinazolin-4-amine

The procedure described in example 1 was repeated using *N,N-*dimethylglycine and *N-*[3-chloro-4-(pyridin-2-ylmethoxy)phenyl]-5-[(2*S*)-pyrrolidin-2-ylmethoxy]quinazolin-4-amine (obtained as described in example 2, preparation of starting materials) in 86% yield; Mass spectrum M⁺ 547.

### Example 5

### 2-((3S)-3-{[(4-{[3-Chloro-4-(pyridin-2-ylmethoxy)phenyl]amino}quinazolin-5-yl)oxy]methyl}morpholin-4-yl)-2-oxoethanol

The procedure described in example 1 was repeated using glycolic acid and *N*-[3-chloro-4-(pyridin-2-ylmethoxy)phenyl]-5-[(3*S*)-morpholin-3-ylmethoxy]quinazolin-4-amine in 40% yield; NMR spectrum (DMSO-d6) 3.5 (m, 2H), 3.6 (m, 1H), 3.8 (m, 1H), 3.9 - 4.2 (m, 4H), 4.5 (m, 2H), 4.6 (t, 1H), 4.8 (m, 1H), 5.3 (s, 2H), 7.2 (d, 2H), 7.4 (m, 2H), 7.5 (d, 1H), 7.6 (d, 1H), 7.7 (t, 1H), 7.9 (m, 2H), 8.4 (s, 1H), 8.6 (d, 1H), 9.7 (bs, 1H); Mass spectrum M⁺ 536.

The *N*-[3-chloro-4-(pyridin-2-ylmethoxy)phenyl]-5-[(3*S*)-morpholin-3-ylmethoxy]quinazolin-4-amine used as starting material was prepared as described in example 1 (preparation of starting materials) using (3*R*)-morpholin-3-ylmethanol (obtained as described in J. Chem. Soc. Perkin Trans. 1, 2577-2580 (1985)) and *N*-[3-chloro-4-(pyridin-2-ylmethoxy)phenyl]-5-fluoroquinazolin-4-amine in 53% yield; NMR spectrum (DMSO-d6) 2.90 (3H, m+bs), 3.23 (m, 1H), 3.34 (m, 2H), 3.68 (dd, 1H), 3.80 (dd, 1H), 4.22 (m, 2H), 5.28 (s, 2H), 7.08 (d, 1H), 7.28 (t, 1H), 7.34 (m, 2H), 7.57 (d, 1H), 7.71 (t, 1H), 7.83 (m, 2H), 8.08 (d, 1H), 8.50 (s, 1H), 8.58 (d, 1H), 10.53 (bs, 1H); Mass spectrum M⁺ 478.

### Example 6

### N-[3-Chloro-4-(pyridin-2-ylmethoxy)phenyl]-5-({(3S)-4-[(dimethylamino)acetyl]morpholin-3-yl}methoxy)quinazolin-4-amine

The procedure described in example 1 was repeated using *N,N*-dimethylglycine and *N-*[3-chloro-4-(pyridin-2-ylmethoxy)phenyl]-5-[(3*S*)-morpholin-3-ylmethoxy]quinazolin-4-amine (obtained as described in example 5, preparation of starting materials) in 77% yield; Mass spectrum M⁺ 563.

### Example 7

### 2-((2R)-2-{[(4-{[3-Chloro-4-(pyridin-2-ylmethoxy)phenyl]amino}quinazolin-5-yl)oxy]methyl}piperidin-1-yl)-2-oxoethanol

The procedure described in example 1 was repeated using glycolic acid and *N*-[3-chloro-4-(pyridin-2-ylmethoxy)phenyl]-5-[(2*R*)-piperidin-2-ylmethoxy]quinazolin-4-amine in 51% yield; NMR spectrum (DMSO-d6) 1.49 (m, 1H), 1.60 - 1.77 (m, 4H), 1.88 (m, 1H), 3.14 (t, 1H), 3.79 (m, 1H), 3.96 - 4.18 (m, 3H), 4.45 (dd, 1H), 4.65 (t, 1H), 4.93 (bs, 1H), 5.28 (s, 2H), 7.24 (m, 2H), 7.32 (dd, 1H), 7.38 (d, 1H), 7.55 (dd, 1H), 7.58 (d, 1H), 7.72 (t, 1H), 7.83 (t, 1H), 7.94 (d, 1H), 8.45 (s, 1H), 8.58 (d, 1H), 9.60 (s, 1H); Mass spectrum M⁺ 534.

The *N*-[3-chloro-4-(pyridin-2-ylmethoxy)phenyl]-5-[(2*R*)-piperidin-2-ylmethoxy]quinazolin-4-amine used as starting material was prepared as described in example 1 (preparation of starting materials) using *N*-[3-chloro-4-(pyridin-2-ylmethoxy)phenyl]-5-fluoroquinazolin-4-amine and (2*R*)-piperidin-2-ylmethanol in 33% yield; NMR spectrum (DMSO-d6) 1.30 - 1.39 (m, 2H), 1.56 (m, 1H), 1.57 (m, 1H), 1.69 (dd, 1H), 1.82 (m, 1H), 2.67 (m, 1H), 3.02 (m, 1H), 3.08 (m, 1H), 4.15 (dd, 1H), 4.27 (dd, 1H), 5.27 (s, 2H), 7.09 (d, 1H), 7.24 (d, 1H), 7.31 (m, 2H), 7.56 (d, 1H), 7.66 (t, 1H), 7.85 (m, 2H), 8.07 (d, 1H), 8.49 (s, 1H), 8.57 (d, 1H), 10.42 (bs, 1H); Mass spectrum M⁺ 477.

The (2*R*)-piperidin-2-ylmethanol used as starting material was prepared as follows: Trifluoroacetic acid (3 ml) was carefully added to a stirring solution of *tert*-butyl (2R)-2-(hydroxymethyl)piperidine-1-carboxylate (1.15 g, obtained as described in Tetrahedron, 58 (2002), 1343 - 1354) in DCM (3 ml) and stirred at room temperature for 1 hour. Volatiles were removed *in vacuo* and the oil thus obtained dissolved in methanol (60 ml), and neutralized by addition of MP-Carbonate resin (polymer supported carbonate reagent ex. Argonaut Technologies Inc.) (approximately 1g) whilst stirring at room temperature for 2 hours. The resin was filtered, washed with methanol (3 x 30 ml) and the filtrate concentrated. The resulting oil was dissolved in DCM (30 ml) and dried (MgSO₄) before filtration and solvent removal to afford a grey oil (615 mg, 100%); NMR spectrum (DMSO-d6) 1.44 - 1.51 (m, 2H), 1.61 (m, 1H), 1.70 - 1.78 (m, 3H), 2.84 (m, 1H), 3.03 (m, 1H), 3.21 (d, 1H), 3.49 (m, 1H), 3.57 (dd, 1H), 5.01 (bs, 1H), 7.65 (bs, 1H); Mass spectrum M⁺ 116.

### Example 8

### 2-((2S)-2-{[(4-{[3-Chloro-4-(pyridin-2-ylmethoxy)phenyl]amino}quinazolin-5-yl)oxy]methyl}piperidin-1-yl)-2-oxoethanol

The procedure described in example 1 was repeated using glycolic acid and N-[3-chloro-4-(pyridin-2-ylmethoxy)phenyl]-5-[(2*S*)-piperidin-2-ylmethoxy]quinazolin-4-amine in 50% yield; NMR spectrum (DMSO-d6) 1.48 (m, 1H), 1.62 - 1.75 (m, 4H), 1.90 (m, 1H), 3.15 (t, 1H), 3.78 (m, 1H), 3.98 - 4.05 (m, 3H), 4.42 (dd, 1H), 4.65 (t, 1H), 4.95 (bs, 1H), 5.29 (s, 2H), 7.24 (t, 2H), 7.34 (dd, 1H), 7.35 (t, 1H), 7.55 (dd, 1H), 7.58 (d, 1H), 7.71 (t, 1H), 7.84 (m, 1H), 7.94 (d, 1H), 8.45 (s, 1H), 8.57 (d, 1H), 9.62 (s, 1H); Mass spectrum M⁺ 535.

The *N*-[3-chloro-4-(pyridin-2-ylmethoxy)phenyl]-5-[(2*S*)-piperidin-2-ylmethoxy]quinazolin-4-amine used as starting material was prepared as described in example 1 (preparation of starting materials) using *N*-[3-chloro-4-(pyridin-2-ylmethoxy)phenyl]-5-fluoroquinazolin-4-amine and (2*S*)-piperidin-2-ylmethanol in 24% yield; NMR spectrum (DMSO-d6) 1.29 - 1.48 (m, 3H), 1.55 (d, 1H), 1.62 (d, 1H), 1.83 (d, 1H), 2.67 (t, 1H), 2.99 (m, 1H), 3.09 (d, 1H), 4.14 (dd, 1H), 4.27 (dd, 1H), 5.29 (s, 2H), 7.09 (d, 1H), 7.27 (d, 1H), 7.35 (d, 1H), 7.38 (dd, 1H), 7.58 (d, 1H), 7.72 (t, 1H), 7.86 (t, H), 7.95 (dd, 1H), 8.09 (d, 1H), 8.52 (s, 1H), 8.59 (d, 1H), 10.61 (s, 1H); Mass spectrum M⁺ 476.

The (2*S*)-piperidin-2-ylmethanol used as starting material was obtained as described in example 7 (preparation of starting materials) using *tert*-butyl (2*S*)-2-(hydroxymethyl)piperidine-1-carboxylate (obtained as described in Tetrahedron, 58 (2002), 1343 -1354) in 100% yield; NMR spectrum (DMSO-d6) 1.44 - 1.51 (m, 2H), 1.61 (m, 1H), 1.70 - 1.79 (m, 3H), 2.86 (m, 1H), 3.06 (m, 1H), 3.23 (d, 1H), 3.48 (m, 1H), 3.58 (dd, 1H), 5.01 (bs, 1H), 7.80 (bs, 1H); Mass spectrum M⁺ 116.

### Example 9

### N-[3-Chloro-4-(pyridin-2-ylmethoxy)phenyl]-5-({(2R)-1-[(dimethylamino)acetyl]piperidin-2-yl}methoxy)quinazolin-4-amine

The procedure described in example 1 was repeated using *N,N*-dimethylglycine and *N-*[3-chloro-4-(pyridin-2-ylmethoxy)phenyl]-5-[(2*R*)-piperidin-2-ylmethoxy]quinazolin-4-amine (obtained as described in example 7, preparation of starting materials) in 39% yield; NMR spectrum (DMSO-d6) 1.45 (m, 1H), 1.61 - 1.75 (m, 4H), 1.89 (m, 1H), 2.10 (s, 6H), 2.98 (s, 2H), 3.14 (t, 1H), 4.11 (d, 1H), 4.44 (t, 1H), 4.68 (t, 1H), 5.05 (bs, 1H), 5.27 (s, 2H), 7.22 (t, 2H), 7.28 (d, 1H), 7.30 (d, 1H), 7.56 (dd, 2H), 7.72 (t, 1H), 7.85 (t, 1H), 7.94 (d, 1H), 8.43 (s, 1H), 8.59 (d, 1H), 9.66 (s, 1H); Mass spectrum M⁺ 562.

### Example 10

### N-[3-Chloro-4-(pyridin-2-ylmethoxy)phenyl]-5-({(2S)-1-[(dimethylamino)acetyl]piperidin-2-yl}methoxy)quinazolin-4-amine

The procedure described in example 1 was repeated using *N,N*-dimethylglycine and *N-*[3-chloro-4-(pyridin-2-ylmethoxy)phenyl]-5-[(2*S*)-piperidin-2-ylmethoxy]quinazolin-4-amine (obtained as described in example 8, preparation of starting materials) in 64% yield; NMR spectrum (DMSO-d6) 1.48 (m, 1H), 1.61 - 1.73 (m, 4H), 1.87 (m, 1H), 2.12 (s, 6H), 3.08 - 3.18 (m, 3H), 4.03 (m, 1H), 4.45 (m, 1H), 4.67 (m, 1H), 5.05 (bs, 1H), 5.27 (s, 2H), 7.25 (t, 2H), 7.33 (d, 1H), 7.35 (t, 1H), 7.58 (dd, 2H), 7.72 (t, 1H), 7.84 (t, 1H), 7.96 (d, 1H), 8.45 (s, 1H), 8.5 (d, 1H), 9.65 (s, 1H); Mass spectrum M⁺ 562.

### Example 11

### 1-((2R)-2-{[(4-{[3-Chloro-4-(pyridin-2-ylmethoxy)phenyl]amino}quinazolin-5-yl)oxy]methyl}pyrrolidin-1-yl)-2-methyl-1-oxopropan-2-ol

The procedure described in example 1 was repeated using *N*-[3-chloro-4-(pyridin-2-ylmethoxy)phenyl]-5-[(2*R*)-pyrrolidin-2-ylmethoxy]quinazolin-4-amine and 2-methyllactic acid in 40% yield; NMR spectrum (DMSO-d6) 1.29 (s, 6H), 1.88 (m, 2H), 1.99 (m, 2H), 3.67 (m, 1H), 4.02 (m, 1H), 4.20 (m, 1H), 4.42 (m, 1H), 4.64 (bs, 1H), 5.19 (s, 1H), 5.30 (s, 2H), 7.28 (d, 2H), 7.33 (d, 1H), 7.38 (dd, 1H), 7.58 (m, 2H), 7.73 (t, 1H), 7.89 (t, 1H), 8.0 (s, 1H), 8.47 (s, 1H), 8.59 (d, 1H), 9.98 (s, 1H); Mass spectrum M⁺ 549.

### Example 12

### 1-[((2R)-2-{[(4-{[3-Chloro-4-(pyridin-2-ylmethoxy)phenyl]amino}quinazolin-5-yl)oxy]methyl}pyrrolidin-1-yl)carbonyl]cyclopropanol

The procedure described in example 1 was repeated using 1-hydroxy-1-cyclopropanecarboxylic acid and *N*-[3-chloro-4-(pyridin-2-ylmethoxy)phenyl]-5-[(2*R*)-pyrrolidin-2-ylmethoxy]quinazolin-4-amine in 41% yield; NMR spectrum (DMSO-d6) 0.70 (m, 1H), 0.82 (m, 2H), 1.06 (m, 1H), 1.88 (m, 2H), 2.05 (m, 2H), 3.76 (m, 1H), 3.91 (m, 1H), 4.22 (m, 1H), 4.40 (m, 1H) 4.61 (m, 1H), 5.32 (s, 2H), 6.14 (s, 1H), 7.22 (t, 2H), 7.34 (m, 2H), 7.53 (dd, 1H), 7.56 (d, 1H), 7.73 (t, 1H), 7.88 (t, 1H), 7.97 (s, 1H), 8.45 (s, 1H), 8.59 (d, 1H), 9.97 (s, 1H); Mass spectrum M⁺ 547.

### Example 13

### 3-((2R)-2-{[(4-{[3-Chloro-4-(pyridin-2-ylmethoxy)phenyl]amino}quinazolin-5-yl)oxy]methyl}pyrrolidin-1-yl)-2,2-dimethyl-3-oxopropan-1-ol

The procedure described in example 1 was repeated using 2,2-dimethyl-3-hydroxypropionic acid and *N*-[3-chloro-4-(pyridin-2-ylmethoxy)phenyl]-5-[(2*R*)-pyrrolidin-2-ylmethoxy]quinazolin-4-amine in 56% yield; NMR spectrum (DMSO-d6) 1.11 (s, 6H), 1.89 (m, 2H), 2.02 (m, 2H), 3.42 (m, 2H), 3.58 (m, 1H), 3.75 (m, 1H), 4.18 (m, 1H), 4.45 (dd, 1H), 4.58 (t, 1H), 4.64 (m, 1H), 5.31 (s, 2H), 7.26 (dd, 2H), 7.32 (dd, 1H), 7.38 (m, 1H), 7.58 (m, 2H), 7.72 (t, 1H), 7.89 (m, 1H), 8.06 (d, 1H), 8.48 (s, 1H), 8.61 (d, 1H), 9.96 (s, 1H); Mass spectrum M⁺ 562.

### Example 14

### (2S)-1-((2R)-2-{[(4-{[3-Chloro-4-(pyridin-2-ylmethoxy)phenyl]amino}quinazolin-5-yl)oxy]methyl}pyrrolidin-1-yl)-1-oxopropan-2-ol

The procedure described in example 1 was repeated using *L*-lactic acid and *N*-[3-chloro-4-(pyridin-2-ylmethoxy)phenyl]-5-[(2R)-pyrrolidin-2-ylmethoxy]quinazolin-4-amine in 60% yield; NMR spectrum (DMSO-d6) 1.13 (d, 3H), 1.91 (m, 2H), 2.03 (m, 2H), 3.58 (m, 2H), 3.66 (m, 1H), 4.28 (m, 2H), 4.45 (m, 1H), 4.59 (m, 1H), 5.33 (s, 2H), 7.33 (t, 1H), 7.38 (s, 1H), 7.39 (dd, 1H), 7.51-7.62 (m, 3H), 7.86 (d, 1H), 7.91 (m, 1H), 8.0 (t, 1H), 8.61 (d, 1H), 8.79 (s, 1H), 11.12 (s, 1H); Mass spectrum M⁺ 534.

### Example 15

### N-[3-Chloro-4-(pyridin-2-ylmethoxy)phenyl]-5-{[(2R)-1-(ethoxyacetyl)pyrrolidin-2-yl]methoxy}quinazolin-4-amine

The procedure described in example 1 was repeated using ethoxyacetic acid and *N*-[3-chloro-4-(pyridin-2-ylmethoxy)phenyl]-5-[(2*R*)-pyrrolidin-2-ylmethoxy]quinazolin-4-amine in 49% yield; NMR spectrum (DMSO-d6) 1.05 (t, 3H), 1.92 (m, 2H), 2.05 (m, 2H), 3.44 (q, 2H), 3.73 (m, 2H), 4.01 (s, 2H), 4.32 (dd, 1H), 4.53 (dd, 1H), 4.64 (m, 1H), 5.31 (s, 2H), 7.31 (d, 1H), 7.38 (dd, 1H), 7.45 (d, 1H), 7.51 (d, 1H), 7.57 (dd, 1H), 7.62 (d, 1H), 7.83 (s, 1H), 7.90 (m, 1H), 7.95 (t, 1H), 8.60 (d, 1H), 8.69 (d, 1H), 10.92 (bs, 1H); Mass spectrum M⁺ 549.

### Example 16

### N-[3-Chloro-4-(pyridin-2-ylmethoxy)phenyl]-5-{[(2R)-1-(methoxyacetyl)pyrrolidin-2-yl]methoxy}quinazolin-4-amine

The procedure described in example 1 was repeated using methoxyacetic acid and *N-*[3-chloro-4-(pyridin-2-ylmethoxy)phenyl]-5-[(2*R*)-pyrrolidin-2-ylmethoxy]quinazolin-4-amine in 36% yield; NMR spectrum (DMSO-d6) 1.92 (m, 2H), 2.05 (m, 2H), 3.25 (s, 3H), 4.0 (d, 2H), 4.35 (dd, 1H), 4.49 (dd, 1H), 4.63 (m, 1H), 5.36 (s, 2H), 7.32 (d, 1H), 7.38 (dd, 1), 7.43 (d, 1H), 7.47 (d, 1H), 7.57 (m, 2H), 7.85 (s, 1H), 7.88 (t, 1H), 7.96 (t, 1H), 8.59 (d, 1H), 8.73 (s, 1H), 10.94 (bs, 1H); Mass spectrum M⁺ 535.

### Example 17

### 2-{(3S)-3-[(4-{[3-Chloro-4-(pyridin-2-ylmethoxy)phenyl]amino}quinazolin-5-yl)oxy]pyrrolidin-1-yl}-2-oxoethanol

The procedure described in example 1 was repeated using glycolic acid and *N*-[3-chloro-4-(pyridin-2-ylmethoxy)phenyl]-5-[(3*S*)-pyrrolidin-3-yloxy]quinazolin-4-amine in 45% yield; NMR spectrum (DMSO-d6) 2.35 - 2.45 (bs, 2H), 3.57 - 3.67 (m, 1H), 3.7 - 3.8 (m, 1H), 3.8 - 4.0 (m, 2H), 4.0 - 4.1 (m, 2H), 4.2 - 4.3 (bs, 1H), 5.3 (s, 1H), 5.4 - 5.5 (bs, 1H), 7.2 - 7.3 (dd, 2H), 7.3 - 7.4 (m, 1H), 7.4 - 7.5 (m, 2H), 7.6 - 7.65 (d, 1H), 7.7 - 7.8 (t, 1H), 7.8 - 7.9 (m, 1H), 8.1 (s, 1H), 8.5 (s, 1H), 8.6 (d, 1H), 9.7 (bs, 1H); Mass spectrum MH⁺ 507.

The *N*-[3-chloro-4-(pyridine-2-ylmethoxy)-phenyl]-5-[(3*S*)-pyrrolidin-3-yloxy] quinazolin-4-amine used as starting material was prepared as follows:

Sodium hydride (60% dispersion in oil, 220 mg) was added to a solution of *N*-*tert-*butoxycarbonyl-(3*S*)-hydroxypyrrolidine (1.02 g), 15-crown-5 (10 mg) and *N*-[3-chloro-4-pyridin-2-ylmethoxy)phenyl]-5-fluoroquinazolin-4-amine (0.95 g, obtained as described in example 1, preparation of starting materials) in 1,4-dioxane (40 ml) and heated at 150°C for 20 minutes in a sealed vessel using a microwave synthesisor. The solution was cooled and p*H* adjusted to 7 by addition of glacial acetic acid and then concentrated *in vacuo.* The residue was purified by chromatography on silica using ethyl acetate - 5% methanol as eluent to give *tent*-butyl (3*S*)-3-[(4-{[3-chloro-4-(pyridin-2-ylmethoxy)phenyl]amino}quinazolin-5-yl)oxy]pyrrolidine-1-carboxylate (1.6 g) as an oil; Mass spectrum MH⁺ 548.

*tert*-Butyl (3*S*)-3-[(4-{[3-chloro-4-(pyridin-2-ylmethoxy)phenyl]amino}quinazolin-5-yl)oxy]pyrrolidine-1-carboxylate (1.6 g) was dissolved in trifluoroacetic acid (50 ml) and stood at ambient temperature for 17 hours. The solution was concentrated *in vacuo* and the residue dissolved in water (100 ml) and pH adjusted to 8 by addition of 880 ammonia solution. The precipitate was extracted into hot ethyl acetate and washed with water, dried (MgSO₄) and evaporated to give *N*-[3-chloro-4-(pyridine-2-ylmethoxy)-phenyl]-5-[(3*S*)-pyrrolidin-3-yloxy]-quinazolin-4-amine (0.52 g, 46%); NMR spectrum (DMSO-d6) 1.9 - 2.1 (bs, 1H), 2.15 - 2.25 (m, 1H), 2.9 - 3.3 (m, 3H), 5.25 (s, 2H), 7.1 (d, 1H), 7.2 (d, 1H), 7.3 - 7.4 (m, 2H), 7.5 - 7.65 (m, 2H), 7.8 - 7.85 (t, 1H), 8.1 - 8.2 (bs, 1H), 8.5 (s, 1H), 8.55 (d, 1H), 10.0 - 10.1 (bs, 1H); Mass spectrum MH⁺ 448.

### Example 18

### N-[3-Chloro-4-(pyridin-2-ylmethoxy)phenyl]-5-({(3S)-1-[(dimethylamino)acetyl]pyrrolidin-3-yl}oxy)quinazolin-4-amine

The procedure described in example 1 was repeated using *N,N*-dimethylglycine and *N-*[3-chloro-4-(pyridine-2-ylmethoxy)-phenyl]-5-[(3*S*)-pyrrolidin-3-yloxy]quinazolin-4-amine (obtained as described in example 17, preparation of starting materials) in 29% yield; NMR spectrum (DMSO-d6) 2.2 (s, 6H), 2.3 - 2.5 (bs, 2H), 3.0 (s, 2H), 3.4 - 3.9 (bs, 2H), 3.9 - 4.2 (bs, 2H), 5.3 (s, 1H), 5.4 - 5.5 (bs, 1H), 7.2 - 7.3 (t, 2H), 7.3 - 7.4 (m, 1H), 7.4 - 7.45 (d, 1H), 7.45 - 7.5 (dd, 1H), 7.6 - 7.65 (d, 1H), 7.7 - 7.8 (t, 1H), 7. 8 - 7.9 (t, 1H), 8.0 - 8.1 (bs, 1H), 8.5 (s, 1H), 8.6 (s, 1H), 9.7 - 9.8 (bs, 1H); Mass spectrum MH⁺ 534.

### Example 19

### 2-{(3R)-3-[(4-{[3-Chloro-4-(pyridin-2-ylmethoxy)phenyl]amino}quinazolin-5-yl)oxy]pyrrolidin-1-yl}-2-oxoethanol

The procedure described in example 1 was repeated using glycolic acid and *N*-[3-chloro-4-(pyridine-2-ylmethoxy)-phenyl]-5-[(3*R*)-pyrrolidin-3-yloxy] quinazolin-4-amine in 25% yield; NMR spectrum (DMSO-d6) 2.35 - 2.45 (bs, 2H), 3.57 - 3.67 (m, 1H), 3.7 - 3.8 (m, 1H), 3.8 - 4.0 (m, 2H), 4.0 - 4.1 (m, 2H), 4.2 - 4.3 (bs, 1H), 5.3 (s, 1H), 5.4 - 5.5 (bs, 1H), 7.2 - 7.3 (dd, 2H), 7.3 - 7.4 (m, 1H), 7.4 - 7.5 (m, 2H), 7.6 - 7.65 (d, 1H), 7.7 - 7.8 (t, 1H), 7.8 - 7.9 (m, 1H), 8.1 (s, 1H), 8.5 (s, 1H), 8.6 (d, 1H), 9.7 (bs, 1H); Mass spectrum MH⁺ 507.

The *N*-[3-chloro-4-(pyridine-2-ylmethoxy)-phenyl]-5-[(3*R*)-pyrrolidin-3-yloxy] quinazolin-4-amine used as starting material was prepared as described in example 17 (preparation of starting materials) using *N*-*tert*-butoxycarbonyl-(3*R*)-hydroxypyrrolidine and *N*-[3-chloro-4-pyridin-2-ylmethoxy)phenyl]-5-fluoroquinazolin-4-amine in 98% yield; NMR spectrum (CDCl₃) 1.6 - 1.9 (bs, 2H), 2.1 2.3 (m, 1H), 2.3 - 2.5 (m, 1H), 3.2 - 3.6 (m, 2H), 5.2 - 5.3 (bs, 1H), 5.4 (s, 1H), 6.9 - 7.0 (d, 1H), 7.05 - 7.15 (d, 1H), 7.15 - 7.2 (m, 1H), 7.5 - 7.6 (d, 1 H), 7.6 - 7.7 (dd, 1H), 7.7 - 7.8 (m, 2H), 7.8 - 7.9 (dt, 1H), 8.15 (d, 1H), 8.7 - 8.75 (d, 1H), 8.75 (s, 1H), 10.1 (bs, 1H); Mass spectrum MH⁺ 448.

### Example 20

### N-[3-Chloro-4-(pyridin-2-ylmethoxy)phenyl]-5-{[(3R)-1-(methoxyacetyl)pyrrolidin-3-yl]oxy}quinazolin-4-amine

The procedure described in example 1 was repeated using methoxyacetic acid and *N-*[3-chloro-4-(pyridine-2-ylmethoxy)-phenyl]-5-[(3*R*)-pyrrolidin-3-yloxy] quinazolin-4-amine (obtained as described in example 19, preparation of starting materials) to give the title compound in 33% yield; NMR spectrum (DMSO-d6) 2.3 - 2.5 (bs, 2H), 3.2 (s, 3H), 3.5 - 3.7 (m, 2H), 3.7 - 4.1 (m, 4H), 5.25 (s, 2H), 5.4 - 5.5 (bs, 1H), 7.2 - 7.25 (d, 2H), 7.3 - 7.35 (m, 1H), 7.35 - 7.4 (d, 1H), 7.4 - 7.5 (dd, 1H), 7.55 - 7.6 (d, 1H), 7.7 - 7.8 (t, 1H), 7. 8 - 7.9 (t, 1H), 8.0 (s, 1H), 8.55 (s, 1H), 8.55 - 8.65 (d, 1H), 9.6 - 9.8 (bs, 1H); Mass spectrum MH⁺ 521.

### Example 21

### N-[3-Chloro-4-(1,3-thiazol-4-ylmethoxy)phenyl]-5-({(2R)-1-[(dimethylamino)acetyl]pyrrolidin-2-yl}methoxy)quinazolin-4-amine

A mixture of 2-chloro-4-[(5-{[(2*R*)-1-(*N,N*-dimethylglycyl)pyrrolidin-2-yl]methoxy}quinazolin-4-yl)amino]phenol (70 mg), potassium carbonate (138 mg) and 18-crown-6 (10 mg) in DMA (5 ml) was stirred and sonicated for 5 minutes. 4-(chloromethyl)thiazole hydrochloride (29 mg) was added, and the mixture was stirred at room temperature for 16 hours. The mixture was concentrated *in vacuo*, and the residue partitioned between DCM (15 ml) and water (15 ml). The DCM fraction was purified by chromatography using 2 - 5 % of 10: 1 methanol / aqueous ammonia (0.880) in DCM as eluent. The appropriate fractions were evaporated, and the residue was triturated with diethyl ether to give the title product as a white solid (24 mg, 28%); NMR spectrum (DMSO-d6) 1.85 - 2.10 (m, 4H), 2.19 (s, 6H), 2.99 (d, 1H), 3.11 (d, 1H), 3.56 (m, 2H), 4.21 (dd, 1H), 4.41 (dd, 1H), 4.60 (m, 1H), 5.35 (s, 2H), 7.22 (d, 1H), 7.32 (d, 1H), 7.33 (d, 1H), 7.60 (dd, 1H), 7.72 (dd, 1H), 7.81 (s, 1H), 8.00 (d, 1H), 8.47 (s, 1H), 9.15 (s, 1H), 9.98 (s, 1H); Mass spectrum MH⁺ 554.

The 2-chloro-4-[(5-{[(2*R*)-1-(*N,N*-dimethylglycyl)pyrrolidin-2-yl]methoxy}quinazolin-4-yl)amino]phenol used as starting material was prepared as follows:

4-Amino-2-chlorophenol (8.65 g) was dissolved in iso-propanol (200 ml) and 4-chloro-5-fluoroquinazoline (prepared as described in example 1, preparation of starting materials, 10.00g) was added. The mixture was heated under reflux for 2 hours, causing a yellow solid to precipitate. The mixture was cooled to ambient temperature; the solid was collected by filtration and washed with cold isopropanol (100 ml). The solid was dissolved in a boiling mixture of methanol (550 ml) and water (100 ml). With vigorous stirring, the solution was basified with aqueous ammonia (0.880, 20 ml), causing a pale pink solid to precipitate. The mixture was concentrated *in vacuo* to such a volume that all of the methanol had been removed, leaving the product as a suspension in aqueous solution. The suspension was cooled; the solid was collected by filtration, triturated with ethyl acetate and dried to give 2-chloro-4-[(5-fluoroquinazolin-4-yl)amino]phenol as a pale pink solid (13.5 g, 85%); NMR spectrum (DMSO-d6) 6.97 (d, 1H), 7.38 (dd, 1H), 7.42 (dd, 1H), 7.59 (d, 1H), 7.73 (d, 1H), 7.81 (ddd, 1H), 8.51 (s, 1H), 9.03 (d, 1H), 10.07 (bs, 1H); Mass spectrum MH⁺ 290.

Sodium hydride (60% dispersion in mineral oil, 1.50 g) was suspended in DMA (100 ml), and D-prolinol (3.70 ml) was added dropwise under a nitrogen atmosphere. The mixture was stirred for 30 minutes at ambient temperature, and the 2-chloro-4-[(5-fluoroquinazolin-4-yl)amino]phenol (4.34 g) was added. The mixture was heated to 110°C for 2 hours under a nitrogen atmosphere, and then cooled to ambient temperature. Saturated ammonium chloride solution (15 ml) was added, and the mixture concentrated *in vacuo.* The residue was treated with saturated sodium hydrogen carbonate solution (150 ml), and the mixture stirred and sonicated to give a granular precipitate. The solid was collected by filtration, triturated with ethyl acetate, and dried to give 2-chloro-4-({5-[(2*R*)-pyrrolidin-2-ylmethoxy]quinazolin-4-yl}amino)phenol as a cream coloured solid (4.72 g, 85%); NMR spectrum (DMSO-d6) 1.50 (m, 1H), 1.60 - 1.77 (m, 2H), 1.88 (m, 1H), 2.80 - 2.95 (m, 2H), 3.57 (m, 1H), 4.07 (dd, 1H), 4.32 (dd, 1H), 6.99 (d, 1H), 7.12 (d, 1H), 7.31 (d, 1H), 7.62 (dd, 1H), 7.70 (dd, 1H), 8.00 (d, 1H), 8.49 (s, 1H), 10.50 (bs, 1H); Mass spectrum MH⁺ 371.

HATU (1.14 g) was added to a mixture of the 2-chloro-4-({5-[(2*R*)-pyrrolidin-2-ylmethoxy]quinazolin-4-yl}amino)phenol (1.11 g) and *N,N*-dimethylglycine (340 mg) in DMF (70 ml). The mixture was stirred at ambient temperature for 16 hours, and then concentrated *in vacuo.* The residue was treated with water to give a pale yellow solid that was collected by filtration, and washed with water. The solid was treated with a mixture of DCM (10 ml), methanol (10 ml) and aqueous ammonia (0.880, 1 ml), and the mixture stirred and sonicated for 10 minutes. The resulting white solid was collected by filtration to give 2-chloro-4-[(5-{[(2*R*)-1-(*N,N*-dimethylglycyl)pyrrolidin-2-yl]methoxy}quinazolin-4-yl)amino]phenol as a white solid (600 mg, 44%); NMR spectrum (DMSO-d6) 1.90 - 2.15 (m, 4H), 3.47 (m, 2H), 3.88 (s, 2H), 4.25 (dd, 1H), 4.47 (dd, 1H), 4.60 (m, 1H), 7.00 (d, 1H), 7.24 (d, 1H), 7.34 (d, 1H), 7.44 (dd, 1H), 7.72 (dd, 1H), 7.90 (d, 1H), 8.47 (s, 1H), 9.88 (s, 1H), 10.04 (s, 1H); Mass spectrum MH⁺ 457.

### Example 22

### 2-((2R)-2-{[(4-{[3-Chloro-4-(1,3-thiazol-4-ylmethoxy)phenyl]amino}quinazolin-5-yl)oxy]methyl}pyrrolidin-1-yl)-2-oxoethanol

The procedure described in example 21 was repeated using 2-chloro-4-[(5-{[(2*R*)-1-glycoloylpyrrolidin-2-yl]methoxy}quinazolin-4-yl)amino]phenol and 4-(chloromethyl)thiazole hydrochloride in 50% yield; NMR spectrum (DMSO-d6) 1.90 - 2.10 (m, 4H), 3.40 (m, 2H), 4.05 (m, 2H), 4.21 (dd, 1H), 4.47 (dd, 1H), 4.53 (t, 1H), 4.60 (m, 1H), 5.35 (s, 2H), 7.27 (d, 1H), 7.34 (d, 1H), 7.35 (d, 1H), 7.60 (dd, 1H), 7.72 (dd, 1H), 7.81 (s, 1H), 8.02 (d, 1H), 8.49 (s, 1H), 9.16 (s, 1H), 9.98 (s, 1H); Mass spectrum MH⁺ 527.

The 2-chloro-4-[(5-{[(2*R*)-1-glycoloylpyrrolidin-2-yl]methoxy}quinazolin-4-yl)amino]phenol used as starting material was prepared as described in example 21 (preparation of starting materials) using glycolic acid and 2-chloro-4-({5-[(2*R*)-pyrrolidin-2-ylmethoxy]quinazolin-4-yl}amino)phenol in 62% yield; NMR spectrum (DMSO-d6) 1.85 - 2.10 (m, 4H), 3.10 - 3.30 (m, 2H), 4.05 (q, 2H), 4.30 (dd, 1H), 4.48 (dd, 1H), 4.62 (m, 1H), 7.05 (d, 1H), 7.3 8 (d, 1H), 7.40 (dd, 1H), 7.52 (d, 1H), 7.71 (d, 1H), 7.99 (dd, 1H), 8.79 (s, 1H), 10.40 (s, 1H), 11.03 (s, 1H); Mass spectrum MH⁺ 429.

### Example 23

### N-{3-Chloro-4-[(5-methylisoxazol-3-yl)methoxy]phenyl}-5-({(2R)-1-[(dimethylamino)acetyl]pyrrolidin-2-yl}methoxy)quinazolin-4-amine

The procedure described in example 21 was repeated using 2-chloro-4-[(5-{[(2*R*)-1-(*N,N*-dimethylglycyl)pyrrolidin-2-yl]methoxy}quinazolin-4-yl)amino]phenol and 3-(chloromethyl)-5-methylisoxazole in 38% yield; NMR spectrum (DMSO-d6) 1.85 - 2.10 (m, 4H), 2.20 (s, 6H), 2.41 (s, 3H), 3.00 (d, 1H), 3.11 (d, 1H), 3.58 (m, 2H), 4.16 (dd, 1H), 4.46 (dd, 1H), 4.60 (m, 1H), 5.23 (s, 2H), 6.30 (s, 1H), 7.20 (d, 1H), 7.27 (d, 1H), 7.34 (d, 1H), 7.60 (dd, 1H), 7.70 (dd, 1H), 8.00 (d, 1H), 8.47 (s, 1H), 9.90 (s, 1H); Mass spectrum MH⁺ 552.

### Example 24

### 2-[(2R)-2-({[4-({3-Chloro-4-[(5-methylisoxazol-3-yl)methoxy]phenyl}amino)quinazolin-5-yl]oxy}methyl)pyrrolidin-1-yl]-2-oxoethanol

The procedure described in example 21 was repeated using 2-chloro-4-[(5-{[(2*R*)-1-glycoloylpyrrolidin-2-yl]methoxy}quinazolin-4-yl)amino]phenol (obtained as described in example 22, preparation of starting materials) and 3-(chloromethyl)-5-methylisoxazole in 28% yield; NMR spectrum (DMSO-d6) 1.90 - 2.10 (m, 4H), 2.45 (s, 3H), 3.41 (m, 2H), 4.04 (m, 2H), 4.21 (dd, 1H), 4.47 (dd, 1H), 4.53 (t, 1H), 4.60 (m, 1H), 5.28 (s, 2H), 6.37 (s, 1H), 7.26 (d, 1H), 7.30 (d, 1H), 7.35 (d, 1H), 7.60 (dd, 1H), 7.72 (dd, 1H), 8.48 (s, 1H), 10.00 (s, 1H); Mass spectrum MH⁺ 525.

### Example 25

### N-[3-Chloro-4-(1,3-thiazol-5-ylmethoxy)phenyl]-5-({(2R)-1-[(dimethylamino)acetyl]pyrrolidin-2-yl}methoxy)quinazolin-4-amine

Methanesulfonyl chloride (20 µl) was added to a solution of 1,3-thiazol-5-ylmethanol (26 mg) and *N,N*-diisopropylethylamine (44 µl) in DCM (5 ml) at 0°C. The mixture was heated to 40°C for 6 hours, and then concentrated *in vacuo.* The residue was dissolved in DMA (5 ml), and added to a suspension of 2-chloro-4-[(5-{[(2*R*)-1-(*N,N-*dimethylglycyl)pyrrolidin-2-yl]methoxy}quinazolin-4-yl)amino]phenol (prepared as described in example 21, preparation of starting materials, 68 mg), potassium carbonate (104 mg), and 18-crown-6 (10 mg) in DMA (5 ml). The mixture was stirred at room temperature for 16 hours, then concentrated *in vacuo.* The residue was partitioned between DCM (15 ml) and water (15 ml). The DCM fraction was loaded onto a silica column, which was purified by chromatography using 2 - 5% 10:1 methanol / aqueous ammonia (0.880) in DCM as eluent. The appropriate fractions were concentrated and the residue triturated with diethyl ether to give the title product as a pale yellow solid (23 mg, 28%); NMR spectrum (DMSO-d6) 1.90 - 2.10 (m, 4H), 2.22 (s, 6H), 3.02 (d, 1H), 3.18 (d, 1H), 3.61 (m, 2H), 4.26 (dd, 1H), 4.47 (dd, 1H), 4.63 (m, 1H), 5.58 (s, 2H), 7.28 (d, 1H), 7.39 (d, 1H), 7.40 (d, 1H), 7.68 (dd, 1H), 7.78 (dd, 1H), 8.06 (d, 1H), 8.09 (s, 1H), 8.52 (s, 1H), 9.19 (s, 1H), 10.04 (s, 1H); Mass spectrum MH⁺ 554.

### Example 26

### 2-((2R)-2-{[(4-{[3-Chloro-4-(1,3-thiazol-5-ylmethoxy)phenyl]amino}quinazolin-5-yl)oxy]methyl}pyrrolidin-1-yl)-2-oxoethanol

The procedure described in example 25 was repeated using 2-chloro-4-[(5-{[(2*R*)-1-glycoloylpyrrolidin-2-yl]methoxy}quinazolin-4-yl)amino]phenol (prepared as described in example 22, preparation of starting materials) and 1,3-thiazol-5-ylmethanol in 36% yield; NMR spectrum (DMSO-d6) 1.90 - 2.10 (m, 4H), 3.40 (m, 2H), 4.05 (m, 2H), 4.21 (dd, 1H), 4.47 (dd, 1H), 4.53 (t, 1H), 4.60 (m, 1H), 5.51 (s, 2H), 7.26 (d, 1H), 7.33 (d, 1H), 7.34 (d, 1H), 7.61 (dd, 1H), 7.73 (dd, 1H), 8.02 (d, 1H), 8.03 (s, 1H), 8.49 (s, 1H), 9.14 (s, 1H), 10.00 (s, 1H); Mass spectrum MH⁺ 526.

### Example 27

### N-[3-Chloro-4-(pyrazin-2-ylmethoxy)phenyl]-5-({(2R)-1-[(dimethylamino)acetyl]pyrrolidin-2-yl}methoxy)quinazolin-4-amine

The procedure described in example 25 was repeated using pyrazin-2-ylmethanol and 2-chloro-4-[(5-{[(2*R*)-1-(*N,N*-dimethylglycyl)pyrrolidin-2-yl]methoxy}quinazolin-4-yl)amino]phenol (prepared as described in example 21, preparation of starting materials) in 44% yield; NMR spectrum (DMSO-d6) 1.85 - 2.10 (m, 4H), 2.19 (s, 6H), 3.00 (d, 1H), 3.12 (d, 1H), 3.56 (m, 2H), 4.20 (dd, 1H), 4.41 (dd, 1H), 4.59 (m, 1H), 5.40 (s, 2H), 7.23 (d, 1H), 7.31 (d, 1H), 7.34 (d, 1H), 7.62 (dd, 1H), 7.72 (dd, 1H), 8.03 (d, 1H), 8.48 (s, 1H), 8.67 (d, 1H), 8.70 (d, 1H), 8.87 (s, 1H), 10.00 (s, 1H); Mass spectrum MH⁺ 549.

### Example 28

### 2-((2R)-2-{[(4-{[3-Chloro-4-(pyrazin-2-ylmethoxy)phenyl]amino}quinazolin-5-yl)oxy]methyl}pyrrolidin-1-yl)-2-oxoethanol

The procedure described in example 25 was repeated using pyrazin-2-ylmethanol and 2-chloro-4-[(5-{[(2*R*)-1-glycoloylpyrrolidin-2-yl]methoxy}quinazolin-4-yl)amino]phenol (prepared as described in example 22, preparation of starting materials) in 36% yield; NMR spectrum (DMSO-d6) 1.85 - 2.10 (m, 4H), 3.41 (m, 2H), 4.05 (m, 2H), 4.21 (dd, 1H), 4.46 (dd, 1H), 4.53 (t, 1H), 4.61 (m, 1H), 5.40 (s, 2H), 7.27 (d, 1H), 7.32 (d, 1H), 7.36 (d, 1H), 7.62 (dd, 1H), 7.73 (dd, 1H), 8.07 (d, 1H), 8.49 (s, 1H), 8.67 (d, 1H), 8.70 (d, 1H), 8.88 (s, 1H), 10.00 (s, 1H); Mass spectrum MH⁺ 522.

### Example 29

### 2-{(3S)-3-[3-Chloro-4-(pyridin-2-ylmethoxy)phenyl]amino}quinazolin-5-yl)oxy]piperidin-1-yl}-2-oxoethanol

The procedure described in example 1 was repeated using glycolic acid and *N*-[3-chloro-4-(pyridin-2-ylmethoxy)phenyl]-5-[(3*S*)-piperidin-3-yloxy]quinazolin-4-amine in 36% yield; NMR spectrum (CDCl₃) 1.76 - 1.97 (m, 2H), 2.04 - 2.23 (m, 4H), 3.19 - 3.30 (m, 1H), 3.42 - 3.67 (m, 1H), 3.71 - 3.88 (m, 1H), 3.90 - 4.14 (m, 2H), 4.68 (s, 1H), 5.21 (s, 2H), 6.79 - 7.00 (m, 2H), 7.13 - 7.21 (m, 2H), 7.40 (d, 1H), 7.52 - 7.62 (m, 2H), 7.64 - 7.73 (m, 1H), 7.75 (s, 1H),, 8.46 - 8.55 (m, 2H), 9.50 (s, 1H); Mass spectrum MH⁺ 520.

The *N*-[3-chloro-4-(pyridin-2-ylmethoxy)phenyl]-5-[(3*S*)-piperidin-3-yloxy]quinazolin-4-amine used as starting material was prepared as follows:
(*S*)-3-Hydroxypiperidine hydrochloride (15 g), diisopropylethylamine (20.9 ml) and 4-dimethylaminopyridine (1.33 g) were stirred in DCM (150 ml) and cooled to 0°C. A solution of di-*tert*-butyldicarbonate (26.2 g) in DCM (50 ml) was added slowly and the reaction mixture was stirred at 0°C for 2 hours. The reaction mixture was allowed to warm to room temperature and stirred for 64 hours, then was washed with 1N citric acid (2 x 200 ml) and water (2 x 200 ml). The organic layer was dried (MgSO₄) and concentrated to give *tert*-butyl-(3*S*)-3-hydroxypiperidine-1-carboxylate as an oil (21.48 g, 98%); NMR spectrum (DMSO-d6 at 373K) 1.20 - 1.33 (m, 2H), 1.38 (s, 9H), 1.54 - 1.67 (m, 1H), 1.72 - 1.85 (m, 1H), 2.53 - 2.68 (m, 1H), 2.69 - 2.83 (m, 1H), 3.30 - 3.42 (m, 1H), 3.53 - 3.64 (m, 1H), 3.68 - 3.79 (m, 1H), 4.79 (d, 1H).

Sodium hydride (60% dispersion in mineral oil, 623 mg) was added portionwise to a stirred solution of the *tert*-butyl-(3*S*)-3-hydroxypiperidine-1-carboxylate (3.06 g) in DMA (50 ml). Upon complete addition the reaction mixture was stirred for ½ hour. 15-Crown-5 (50 mg) was added followed by 2-chloro-4-[(5-fluoroquinazolin-4-yl)amino]phenol (obtained as described in example 21, preparation of starting materials) and the mixture heated to 95°C for 2 hours. Saturated ammonium chloride solution (10 ml) was added and the DMA was removed *in vacuo.* Water (150 ml) was added to the residue and stirred vigorously. The resulting solid was filtered and dried. This was then stirred in hot diethyl ether (100 ml) for 10 minutes and the cooled mixture filtered to give *tert*-butyl (3*S*)-3-({4-[(3-chloro-4-hydroxyphenyl)amino]quinazolin-5-yl}oxy)piperidine-1-carboxylate as a yellow solid (2.4 g, 82%); NMR spectrum (DMSO-d6) 1.12 (s, 9H), 1.57 - 1.84 (m, 2H), 2.00 - 2.18 (m, 2H), 3.08 - 3.24 (m, 1H), 3.43 - 3.57 (m, 1H), 3.64 - 3.76 (m, 1H), 3.86 - 4.03 (m, 1H), 4.87 - 5.00 (m, 1H), 6.95 - 7.04 (m, 1H), 7.18 - 7.41 (m, 3H), 7.68 - 7.85 (m, 2H), 8.49 (s, 1H), 9.80 (s, 2H); Mass spectrum MH⁺ 471.

*Tert*-butyl (3*S*)-3-({4-[(3-chloro-4-hydroxyphenyl)amino]quinazolin-5-yl}oxy)piperidine-1-carboxylate (1 g), picolyl chloride hydrochloride (384 mg) and potassium carbonate (737 mg) were stirred in DMF to which was added 18-crown-6 (0.1 g). The reaction was stirred at room temperature for 2 days. The DMF was removed *in-vacuo* and the residue was partitioned between water and ethyl acetate. The ethyl acetate was washed with water (3 x 100 ml) and brine (3 x 50 ml). This was dried (MgSO₄) and concentrated to give *tert*-butyl (3*S*)-3-[(4-{[3-chloro-4-(pyridin-2-ylmethoxy)phenyl]amino}quinazolin-5-yl)oxy]piperidine-1-carboxylate as a foam (1.1 g, 92%); NMR spectrum (CDCl₃) 1.21 (s, 9H), 1.54 - 1.71 (m, 1H), 1.75 - 1.89 (m, 1H), 1.91 - 2.05 (m, 1H), 2.07 - 2.21 (m, 1H), 3.17 - 3.93 (m, 4H), 4.56 - 4.69 (m, 1H), 5.23 (s, 2H), 6.83 - 6.99 (m, 2H), 7.11 - 7.20 (m, 1H), 7.29 - 7.43 (m, 2H), 7.52 - 7.63 (m, 2H), 7.64 - 7.72 (m, 1H), 7.83 - 7.90 (m, 1H), 8.47 - 8.57 (m, 2H), 9.71 (s, 1H); Mass spectrum MH⁺ 562.

*tert*-butyl (3*S*)-3-[(4-{[3-chloro-4-(pyridin-2-ylmethoxy)phenyl]amino}quinazolin-5-yl)oxy]piperidine-1-carboxylate (1.1 g) was stirred in TFA (15 ml) for 3 hours. The reaction mixture was concentrated and saturated potassium carbonate solution was added until the mixture was basic. This was diluted with water (20 ml) and stirred overnight. The precipitate was filtered and washed with water (100 ml). This was dried to give *N*-[3-chloro-4-(pyridin-2-ylmethoxy)phenyl]-5-[(3*S*)-piperidin-3-yloxy]quinazolin-4-amine as a solid (0.89 g, 99%); NMR spectrum (DMSO-d6) 1.37 - 1.51 (m, 1H), 1.52 - 1.81 (m, 2H), 1.90 - 2.07 (m, 1H), 2.53 - 2.76 (m, 2H), 2.83 - 3.01 (m, 2H), 3.07 - 3.18 (m, 1H), 4.88 - 4.98 (m, 1H), 5.27 (s, 2H), 7.14 - 7.25 (m, 2H), 7.26 - 7.40 (m, 3H), 7.53 - 7.62 (m, 1H), 7.65 - 7.74 (m, 1H), 7.75 - 7.82 (m, 1H), 7.83 - 7.93 (m, 1H), 8.50 (s, 1H), 8.58 (d, 1H), 10.74 (s, 1H); Mass spectrum MH⁺ 462.

### Example 30

### 2-{(3R)-3-[3-Chloro-4-(pyridin-2-ylmethoxy)phenyl]amino}quinazolin-5-yl)oxy]piperidin-1-yl}-2-oxoethanol

The procedure described in example 1 was repeated using glycolic acid and *N*-[3-chloro-4-(pyridin-2-ylmethoxy)phenyl]-5-[(3*R*)-piperidin-3-yloxy]quinazolin-4-amine (32 mg, 29%); NMR spectrum (CDCl₃) 1.74 - 1.93 (m, 2H), 2.01 - 2.17 (m, 4H), 3.19 - 3.30 (m, 1H), 3.47 - 3.67 (m, 1H), 3.70 - 3.85 (m, 1H), 3.90 - 4.10 (m, 2H), 4.60 - 4.70 (m, 1H), 5.22 (s, 2H), 6.85 - 6.97 (m, 2H), 7.11 - 7.18 (m, 1H), 7.22 - 7.35 (m, 1H), 7.38 - 7.47 (m, 1H), 7.52 - 7.62 (m, 2H), 7.63 - 7.72 (m, 1H), 7.76 (s, 1H), 8.48 - 8.58 (m, 2H), 9.41 - 9.52 (m, 1H); Mass spectrum MH⁺ 520.

The *N*-[3-chloro-4-(pyridin-2-ylmethoxy)phenyl]-5-[(3*R*)-piperidin-3-yloxy]quinazolin-4-amine used as starting material was prepared as described in example 29 (preparation of starting materials) using *tert*-butyl (3R)-3-[(4-{[3-chloro-4-(pyridin-2-ylmethoxy)phenyl]amino}quinazolin-5-yl)oxy]piperidine-1-carboxylate in 100% yield; NMR spectrum (DMSO-d6) 1.37 - 1.51 (m, 1H), 1.52 - 1.81 (m, 2H), 1.90 - 2.07 (m, 1H), 2.53 - 2.76 (m, 2H), 2.83 - 3.01 (m, 2H), 3.07 - 3.18 (m, 1H), 4.88 - 4.98 (m, 1H), 5.27 (s, 2H), 7.14 - 7.25 (m, 2H), 7.26 - 7.40 (m, 3H), 7.53 - 7.62 (m, 1H), 7.65 - 7.74 (m, 1H), 7.75 - 7.82 (m, 1H), 7.83 - 7.93 (m, 1H), 8.50 (s, 1H), 8.58 (d, 1H), 10.74 (s, 1H); Mass spectrum MH⁺ 462.

The *tert*-butyl (3R)-3-[(4-{[3-chloro-4-(pyridin-2-ylmethoxy)phenyl]amino}quinazolin-5-yl)oxy]piperidine-1-carboxylate used as starting material was prepared as described in example 29 (preparation of starting materials) using *tert*-butyl (3*R*)-3-({4-[(3-chloro-4-hydroxyphenyl)amino]quinazolin-5-yl}oxy)piperidine-1-carboxylate in 92% yield; NMR spectrum (CDCl₃) 1.21 (s, 9H), 1.54 - 1.71 (m, 1H), 1.75 - 1.89 (m, 1H), 1.91 - 2.05 (m, 1H), 2.07 - 2.21 (m, 1H), 3.17 - 3.93 (m, 4H), 4.56 - 4.69 (m, 1H), 5.23 (s, 2H), 6.83 - 6.99 (m, 2H), 7.11 - 7.20 (m, 1H), 7.29 - 7.43 (m, 2H), 7.52 - 7.63 (m, 2H), 7.64 - 7.72 (m, 1H), 7.83 - 7.90 (m, 1H), 8.47 - 8.57 (m, 2H), 9.71 (s, 1H); Mass spectrum MH⁺ 562.

The *tert*-butyl (3*R*)-3-({4-[(3-chloro-4-hydroxyphenyl)amino]quinazolin-5-yl}oxy)piperidine-1-carboxylate used as starting material was prepared as described in example 29 (preparation of starting materials) using *tert*-butyl-(3*R*)-3-hydroxypiperidine-1-carboxylate and 2-chloro-4-[(5-fluoroquinazolin-4-yl)amino]phenol (obtained as described in example 21, preparation of starting materials) in 72% yield; NMR spectrum (DMSO-d6 at 373K) 1.12 (s, 9H), 1.57 - 1.84 (m, 2H), 2.00 - 2.18 (m, 2H), 3.08 - 3.24 (m, 1H), 3.43 - 3.57 (m, 1H), 3.64 - 3.76 (m, 1H), 3.86 - 4.03 (m, 1H), 4.87 - 5.00 (m, 1H), 6.95 - 7.04 (m, 1H), 7.18 - 7.41 (m, 3H), 7.68 - 7.85 (m, 2H), 8.49 (s, 1H), 9.80 (s, 2H); Mass spectrum MH⁺ 471.

The *tert*-butyl-(3*R*)-3-hydroxypiperidine-1-carboxylate used as starting material was prepared as described in example 29 (preparation of starting materials) using (R)-3-hydoxypiperidine hydrochloride; NMR spectrum (DMSO-d6) 1.20 - 1.33 (m, 2H), 1.38 (s, 9H), 1.54 - 1.67 (m, 1H), 1.72 - 1.85 (m, 1H), 2.53 - 2.68 (m, 1H), 2.69 - 2.83 (m, 1H), 3.30 - 3.42 (m, 1H), 3.53 - 3.64 (m, 1H), 3.68 - 3.79 (m, 1H), 4.79 (d, 1H).

### Example 31

### N-13-Chloro-4-(pyridin-2-ylmethoxy)phenyl]-5-({(3S)-1-[dimethylamino)acetyl]piperidin-3-yl}oxy)quinazolin4-amine

The procedure described in example 1 was repeated using *N,N-*dimethylglycine and *N-*[3-chloro-4-(pyridin-2-ylmethoxy)phenyl]-5-[(3*S*)-piperidin-3-yloxy]quinazolin-4-amine (prepared as described in example 29, preparation of starting materials) in 17% yield; NMR spectrum (CDCl₃) 1.82 - 2.00 (m, 2H), 2.17 (s, 6H), 2.22 - 2.45 (m, 1H), 2.72 - 2.85 (m, 1H), 2.94 - 3.14 (m, 2H), 3.26 - 3.47 (m, 2H), 3.69 - 4.20 (m, 1H), 4.28 - 4.47 (m, 1H), 4.49 - 4.65 (m, 1H), 5.22 (s, 2H), 6.94 (d, 1H), 7.13 - 7.26 (m, 3H), 7.34 - 7.44 (m, 2H), 7.52 - 7.63 (m, 2H), 7.64 - 7.74 (m, 1H), 7.79 - 7.91 (m, 1H), 8.48 - 8.60 (m, 2H); Mass spectrum MH⁺ 547.

### Example 32

### N-[3-Chloro-4-(pyridin-2-ylmethoxy)phenyl]-5-(1(3R)-1-[dimethylamino)acetyl]piperidin-3-yl}oxy)quinazolin-4-amine

The procedure described in example 1 was repeated using *N*,*N*-dimethylglycine and *N-*[3-chloro-4-(pyridin-2-ylmethoxy)phenyl]-5-[(3*R*)-piperidin-3-yloxy]quinazolin-4-amine (prepared as described in example 30, preparation of starting materials) in 36% yield; NMR spectrum (CDCl₃) 1.81 - 2.00 (m, 2H), 2.15 (s, 6H), 2.21 - 2.42 (m, 1H), 2.70 - 2.84 (m, 1H), 2.94 - 3.25 (m, 2H), 3.28 - 3.41 (m, 2H), 3.68 - 4.18 (m, 1H), 4.28 - 4.46 (m, 1H), 4.48 - 4.64 (m, 1H), 5.22 (s, 2H), 6.93 (d, 1H), 7.10 - 7.25 (m, 2H), 7.30 - 7.41 (m, 2H), 7.52 - 7.63 (m, 2H), 7.64 - 7.72 (m, 1H), 7.79 - 7.90 (m, 1H), 8.48 - 8.57 (m, 2H), 9.58 - 9.77 (m, 1H); Mass spectrum MH⁺ 547.

### Example 33

### (2R)-1-((2R)-2-{[(4-{[3-chloro-4-(pyridin-2-ylmethoxy)phenyl]amino}quinazolin-5-yl)oxy]methyl}pyrrolidin-1-yl)-1-oxopropan-2-ol

The procedure described in example 1 was repeated using D-lactic acid and *N*-[3-chloro-4-(pyridin-2-ylmethoxy)phenyl]-5-[(2*R*)-pyrrolidin-2-ylinethoxy]quinazolin-4-amine (obtained as described example 1, preparation of starting materials) to give the title compound in 50% yield; NMR spectrum (DMSO-d6) 1.14 (d, 3H), 1.98 (m, 3H), 3.44 (m, 1H), 3.70 (m, 1H), 4.26 (m, 2H), 4.42 (m, 1H), 4.60 (bs, 1H), 4.78 (d, 1H), 5.30 (s, 2H), 7.24 (d, 1H), 7.27 (d, 1H), 7.35 (m, 2H), 7.58 (m, 2H), 7.72 (t, 1H), 7.88 (dt, 1H), 8.01 (d, 1H), 8.47 (s, 1H), 8.59 (d, 1H), 9.94 (bs, 1H); Mass spectrum MH⁺ 534.5.

### Example 34

### 2-((3R)-3-{[(4-{[3-chloro-4-(pyridin-2-ylmethoxy)phenyl]amino}quinazolin-5-yl)oxy]methyl}4-methylpiperazin-1-yl)-2-oxoethanol

The procedure described in example 1 was repeated using glycolic acid and *N*-[3-chloro-4-(pyridin-2-ylmethoxy)phenyl]-5-{[(2*R*)-1-methylpiperazin-2-yl]methoxy}quinazolin-4-amine hydrochloride to give the title compound in 28% yield; Mass spectrum MH⁺ 549.2.

The *N*-[3-chloro-4-(pyridin-2-ylmethoxy)phenyl]-5-{[(2*R*)-1-methylpiperazin-2-yl]methoxy}quinazolin-4-amine hydrochloride used as starting material was prepared as follows:
Trimethylsilyl diazomethane (2M in hexane, 14 ml) was added dropwise to a solution of (2*R*)-1-(*tert*-butoxycarbonyl)piperazine-2-carboxylic acid (5 g) in methanol (100 ml) and DCM (115 ml), and the solution stirred at room temperature for 16 hours. The solvent was concentrated *in vacuo* and the residue purified by chromatography, eluting with ethyl acetate then 5% methanol / 7N ammonia in ethyl acetate, to give 1-*tert*-butyl 2-methyl (2*R*)-piperazine-1,2-dicarboxylate as an oil (2.55 g, 48%); NMR spectrum (DMSO-d6) 1.40 (s, 9H), 2.10 (bs, 1H), 2.52 (m, 1H), 2.72 (dd, 1H), 2.82 (d, 1H), 2.97 (m, 1H), 3.29 (d, 1H), 3.61 (d, 1H), 3.67 (s, 3H), 4.43 (m, 1H); Mass spectrum MH⁺ 245.
Lithium aluminium hydride (1M in THF, 26 ml) was added to a solution of 1*-tert-*butyl 2-methyl (2*R*)-piperazine-1,2-dicarboxylate (2.55 g) in THF (70 ml) at -40°C, then the reaction was warmed to room temperature. The solution was stirred for 1 hour, then cooled to 0°C and quenched by sequential addition of water (1 ml), sodium hydroxide (2N, 1 ml) and then water (2 ml). The resulting slurry was filtered and concentrated *in vacuo* to give *tert-*butyl (2*R*)-2-(hydroxymethyl)piperazine-1-carboxylate (2.37 g, > 100%); NMR spectrum (DMSO-d6, 373K) 1.40 (s, 9H), 2.58 (m, 1H), 2.82 (m, 3H), 2.92 (bs, 1H), 2.98 (d, 1H), 3.43 (m, 1H), 3.65 (m, 2H), 3.80 (m, 1H); Mass spectrum MH⁺ 217.
Lithium aluminium hydride (1M in THF, 17.6 ml) was added to a solution of *tert-*butyl (2*R*)-2-(hydroxymethyl)piperazine-1-carboxylate (1.27 g) in THF (40 ml) at 0°C, then the reaction was warmed to room temperature. The solution was stirred for 3 hours, then heated at reflux for 1 hour, cooled to 0°C and quenched by sequential addition of water (0.2 ml), sodium hydroxide (2N, 0.2 ml) and then water (0.4 ml). The resulting slurry was filtered and concentrated *in vacuo* to give [(2*R*)-1-methylpiperazin-2-yl]methanol (0.44 g); Mass spectrum MH⁺ 131.
Sodium hydride (60% dispersion in mineral oil, 0.27 g) was added to [(2*R*)-1-methylpiperazin-2-yl]methanol (0.44 g) and *N*-[3-chloro-4-(pyridin-2-ylmthoxy)phenyl]-5-fluoroquinazolin-4-amine (0.43 g, obtained as described in example 1, preparation of starting materials) in DMA (20 ml) and the reaction heated at 90°C for 16 hours. The reaction was cooled, quenched with water, and concentrated *in vacuo.* The residue was purified by reverse phase chromatography, using 10 - 40% acetonitrile in water with 0.2% TFA modifier as eluent, to give after acidification with HCl in ether, *N*-[3-chloro-4-(pyridin-2-ylmethoxy)phenyl]-5-{[(2*R*)-1-methylpiperazin-2-yl]methoxy}quinazolin-4-amine hydrochloride as a white solid (82 mg, 15%); NMR spectrum (DMSO-d6) 2.40 (s, 3H), 2.69 (dt, 1H), 2.82 (dt, 1H), 2.83 (m, 1H), 2.98 (m, 1H), 3.15 (t, 1H), 3.27 (m, 1H), 3.37 (m, 1H), 4.52 (m, 2H), 5.31 (s, 2H), 7.32 (m, 3H), 7.47 (d, 1H), 7.58 (d, 1H), 7.67 (dd, 1H), 7.85 (dt, 1H), 7.87 (d, 1H), 7.92 (t, 1H), 8.57 (d, 1H), 8.72 (s, 1H); Mass spectrum MH⁺ 491.4.

### Example 35

### 2-((3S)-3-{[(4-{[3-chloro-4-(pyridin-2-ylmethoxy)phenyl]amino}quinazolin-5-yl)oxy]methyl}4-methylpiperazin-1-yl)-2-oxoethanol

The procedure described in example 1 was repeated using glycolic acid and *N*-[3-chloro-4-(pyridin-2-ylmethoxy)phenyl]-5-{[(2*S*)-1-methylpiperazin-2-yl]methoxy}quinazolin-4-amine to give the title compound in 34% yield; Mass spectrum MH⁺ 549.3.

The *N*-[3-chloro-4-(pyridin-2-ylmethoxy)phenyl]-5-{[(2S*R*)-1-methylpiperazin-2-yl]methoxy}quinazolin-4-amine used as starting material was prepared as follows:
Lithium aluminium hydride (1M in THF, 19.5 ml) was added to a solution of (2*S*)-1-(*tert*-butoxycarbonyl)piperazine-2-carboxylic acid (1.5 g) in THF (20 ml) at 0°C, then the reaction was warmed to room temperature and heated at 60°C for 16 hours. The reaction was cooled to 0°C and quenched by sequential addition of water (0.75 ml), sodium hydroxide (2N, 0.75 ml) and then water (1.5 ml). The resulting slurry was filtered and concentrated *in vacuo* and the residue purified by chromatography using DCM to 20% 7N ammonia in methanol in DCM to [(2*S*)-1-methylpiperazin-2-yl]methanol as a white solid (454 mg, 53%); NMR spectrum (DMSO-d6) 1.83 (m, 1H), 1.98 (dt, 1H), 2.14 (s, 3H), 2.31 (dd, 1H), 2.56 (m, 2H), 2.68 (m, 1H), 2.84 (dd, 1H), 3.23 (dd, 1H), 3.52 (dd, 1H).

*N*-[3-chloro-4-(pyridin-2-ylmethoxy)phenyl]-5-{[(2S*R*)-1-methylpiperazin-2-yl]methoxy}quinazolin-4-amine was prepared as described in example 34 (preparation of starting materials) using [(2*S*)-1-methylpiperazin-2-yl]methanol and *N*-[3-chloro-4-(pyridin-2-ylmethoxy)phenyl]-5-fluoroquinazolin-4-amine (obtained as described in example 1, preparation of starting materials) in 17% yield; NMR spectrum (DMSO-d6) 2.23 (dt, 1H), 2.30 (s, 3H), 2.32 (m, 1H), 2.60 (dt, 1H), 2.76 (m, 3H), 2.87 (dd, 1H), 4.24 (d, 1H), 4.44 (dd, 1H), 5.25 (s, 2H), 7.09 (dd, 1H), 7.26 (d, 1H), 7.34 (m, 2H), 7.57 (d, 1H), 7.71 (t, 1H), 7.88 (m, 2H), 7.98 (d, 1H), 8.51 (s, 1H), 8.58 (m, 1H), 10.42 (bs, 1H); Mass spectrum MH⁺ 491.

### Example 36

### 2-((2R)-2-{[(4-{[3-chloro-4-(pyridin-2-ylmethoxy)phenyl]amino}quinazolin-5-yl)oxy]methyl}4-methylpiperazin-1-yl)-2-oxoethanol

The procedure described in example 1 was repeated using glycolic acid and *N*-[3-chloro-4-(pyridin-2-ylmethoxy)phenyl]-5-{[(2*R*)-4-methylpiperazin-2-yl]methoxy}quinazolin-4-amine in 31% yield; Mass spectrum M⁺ 548.9.

The *N*-[3-chloro-4-(pyridin-2-ylmethoxy)phenyl]-5-{[(2*R*)-4-methylpiperazin-2-yl]methoxy} quinazolin-4-amine used as starting material was prepared as follows:
[(2R)-4-methylpiperazin-2-yl]methanol was prepared as described in example 35 (preparation of starting materials) using (2*R*)-4-(*tert*-butoxycarbonyl)piperazine-2-carboxylic acid as starting material in 61% yield; NMR spectrum (DMSO-d6, 373K) 1.63 (t, 1H), 1.88 (dt, 1H), 2.13 (s, 3H), 2.52 (m, 1H), 2.60 (m, 1H), 2.67 (m, 2H), 2.81 (dt, 1H), 3.30 (d, 2H).

*N*-[3-chloro-4-(pyridin-2-ylmethoxy)phenyl]-5-{[(2*R*)-4-methylpiperazin-2-yl]methoxy}quinazolin-4-amine was prepared as described in example 34 (preparation of starting materials) using [(2*R*)-4-methylpiperazin-2-yl]methanol and *N*-[3-chloro-4-(pyridin-2-ylmethoxy)phenyl]-5-fluoroquinazolin-4-amine (obtained as described in example 1, preparation of starting materials) in 51% yield; NMR spectrum (DMSO-d6) 1.88 (m, 2H), 2.14 (s, 3H), 2.62 (d, 1H), 2.73 (d, 1H), 2.81 (m, 1H), 2.98 (d, 1H), 3.20 (m, 1H), 4.21 (m, 1H), 4.31 (m, 1H), 5.29 (s, 2H), 7.10 (d, 1H), 7.27 (d, 1H), 7.35 (d, 1H), 7.37 (m, 1H), 7.58 (d, 1H), 7.72 (t, 1H), 7.90 (dt, 1H), 7.92 (dd, 1H), 8.09 (d, 1H), 8.52 (s, 1H), 8.60 (d, 1H), 10.58 (s, 1H); Mass spectrum MH⁺ 491.

### Example 37

### 2-((2S)-2-{[(4-{[3-chloro-4-(pyridin-2-ylmethoxy)phenyl]amino}quinazolin-5-yl)oxy]methyl}4-methylpiperazin-1-yl)-2-oxoethanol

The procedure described in example 1 was repeated using glycolic acid and *N*-[3-chloro-4-(pyridin-2-ylmethoxy)phenyl]-5- {[(2*S*)-4-methylpiperazin-2-yl]methoxy}quinazolin-4-amine in 44% yield; Mass spectrum M⁺ 548.9.

The *N*-[3-chloro-4-(pyridin-2-ylmethoxy)phenyl]-5-{[(2*S*)-4-methylpiperazin-2-yl]methoxy}quinazolin-4-amine used as starting material was prepared as follows:
[(2*S*)-4-methylpiperazin-2-yl]methanol was prepared as described in example 36, preparation of starting materials, for the *R*-antipode using (*2S*)*-*4*-*(*tert-*butoxycarbonyl)piperazine-2-carboxylic acid as starting material in 52% yield.

*N*-[3-chloro-4-(pyridin-2-ylmethoxy)phenyl]-5-{[(2*S*)-4-methylpiperazin-2-yl]methoxy}quinazolin-4-amine was prepared as described in example 36, preparation of starting materials, for the *R*-antipode using [(2*S*)-4-methylpiperazin-2-yl]methanol as starting material in 43% yield.

### Example 38

### 2-((2R)-2-{(1S)-1-[(4-{[3-Chloro-4-(pyridin-2-ylmethoxy)phenyl]amino}quinazolin-5-yl)oxy]ethyl}pyrrolidin-1-yl)-2-oxoethanol

The procedure described in example 21 was repeated using 2-(chloromethyl)pyridine and 2-chloro-4-[(5-{(1*S*)-1-[(2*R*)-1-glycoloylpyrrolidin-2-yl]ethoxy}quinazolin-4-yl)amino]phenol to give the title compound in 17% yield; NMR spectrum (DMSO-d6) 1.35 (d, 3H), 1.95 (m, 4H), 3.44 (t, 2H), 4.01 (dq, 2H), 4.55 (m, 2H), 5.15 (m, 1H), 5.30 (s, 2H), 7.24 (d, 1H), 7.32 (d, 1H), 7.35 (m, 1H), 7.43 (d, 1H), 7.57 (d, 1H), 7.62 (dd, 1H), 7.73 (dd, 1H), 7.87 (dt, 1H), 8.17 (d, 1H), 8.48 (s, 1H), 8.58 (m, 1H), 10.10 (s, 1H); Mass spectrum MH⁺ 534.

The 2-chloro-4-[(5-{(1*S*)-1-[(2*R*)-1-glycoloylpyrrolidin-2-yl]ethoxy}quinazolin-4-yl)amino]phenol used as starting material was prepared as follows:

1-(*tert*-butoxycarbonyl)-D-proline (10.24 g) in DCM (240 ml) and triethylamine (6.94 ml) were vigorously stirred at -5°C. Isobutylchloroformate (6.6 g) was added dropwise maintaining the temperature below-7°C. The solution was stirred for 30 minutes. *N*,*O-*Dimethylhydroxylamine hydrochloride (4.8 g) was added, then triethylamine (6.9 ml) dropwise. The solution was stirred for 1 hour and allowed to warm to 0°C. The solution was stirred for a further 3 hours whereupon the temperature had reached ambient. The solution was washed with saturated sodium hydrogen carbonate solution, water, brine and was dried (MgSO₄) and evaporated to givel-(*tert*-butoxycarbonyl)-*N*-methoxy-*N*-methyl-D-prolinamide (9.4 g, 76%); NMR spectrum (CDCl₃) 1.40 and 1.43 (each s, together 9H), 2.00 (m, 4H), 3.20 (s, 3H), 3.50 (m, 2H), 3.70 and 3.79 (each s, together 3H), 4.64 (ddd, 1H).

1-(*tert*-Butoxycarbonyl)-*N*-methoxy-*N*-methyl-D-prolinamide (6.67 g) in THF (70 ml) at -9 °C under nitrogen was treated with methylmagnesium bromide (3.0 M in diethyl ether) (13 ml) dropwise keeping the temperature below -8°C. The ice bath was left in place and stirring continued overnight. Ethyl acetate (60 ml) was added followed by 2N HCl and the solution extracted with ethyl acetate (2x). The combined organic extracts were washed with saturated sodium hydrogen carbonate solution, water, brine and dried (MgSO₄) and evaporated to give *tert*-butyl (2*R*)-2-acetylpyrrolidine-1-carboxylate (4.82 g, 87%); NMR spectrum (CDCl₃) 1.42 (s, 9H), 1.85 (m, 4H), 2.12 (s, 3H), 3.51 (m, 2H), 4.26 (m, 1H).

*tert*-Butyl (2*R*)-2-acetylpyrrolidine-1-carboxylate (4.14 g) in THF (90 ml) at 0°C was treated with lithium aluminium hydride (1.0 M in THF) (5.79 ml) and stirred for 50 minutes. Water (0.22 ml) was added followed by 15% NaOH (0.22 ml) then water (0.66 ml) and the mixture filtered and evaporated to give *tert*-butyl (2*R*)-2-(1-hydroxyethyl)pyrrolidine-1-carboxylate (3.72 g, 89%); NMR spectrum (DMSO-d6) 0.95 and 1.01 (each d, together 6H), 1.42 (s, 18H), 1.80 (m, 8H), 3.19 (m, 2H), 3.39 (m, 2H), 3.58 (m, 2H), 3.75 (m, 2H), 3.88 (m, 2H), 4.11 (d, 1H), 4.20 (d, 1H).

*tert*-Butyl (2*R*)-2-(1-hydroxyethyl)pyrrolidine-1-carboxylate (2.05 g) in diethyl ether (15 ml) was treated with HCl (1.0 M in diethyl ether) (35 ml) and stirred overnight. The solution was then taken to pH10 with ammonium hydroxide, extracted with DCM (8x) and the extracts purified by chromatography using DCM - 10% methanol/NH₄OH as eluant to give 1-[(2*R*)-pyrrolidin-2-yl]ethanol as a 1:1 mixture of diastereoisomers (0.86 g, 78%); NMR spectrum (DMSO-d6) 1.07 (d, *J* = 2.0 Hz, 3H), 1.09 (d, *J* = 2.3 Hz, 3H), 1.36 (m, 1H), 1.69 (m, 7H), 2.76 (m, 1H), 2.88 (m, 8H), 3.06 (m, 1H), 3.29 (m, 1H), 3.75 (m, 1H).

2-Chloro-4-[(5-fluoroquinazolin-4-yl)amino]phenol (obtained as described in example 21, preparation of starting materials, 2.0 g) in DMA (30 g) was treated with 1-[(2R)-pyrrolidin-2-yl]ethanol (1.98 g) and sodium hydride (60% in oil) (0.69 g) and heated in a microwave at 140°C for 15 minutes over several batches. The mixture was evaporated and purified by chromatography using DCM - 10% methanol/NH₄OH as eluant to give 2-chloro-4-[(5-{(1*S*)-1-[(2*R*)-pyrrolidin-2-yl]ethoxy}quinazolin-4-yl)amino]phenol (0.171 g, 6%); NMR spectrum (DMSO-d6) 1.38 (d, 3H), 1.50 (m, 1H), 1.60 (m, 2H), 1.82 (m, 1H), 2.78 (m, 2H), 3.28 (m, 2H), 4.70 (m, 1H), 6.95 (d, 1H), 7.13 (d, 1H), 7.29 (m, 2H), 7.60 (dd, 1H), 7.67 (t, 1H), 7.95 (d, 1H), 8.44 (s, 1H), 10.66 (s, 1H); and 2-chloro-4-[(5-{(1*R*)-1-[(2*R*)-pyrrolidin-2-yl]ethoxy}quinazolin-4-yl)amino]phenol (0.094 g, 4%); NMR spectrum (DMSO-d6) 1.35 (d, 3H), 1.53 (m, 1H), 1.68 (m, 2H), 1.90 (m, 1H), 2.80 (m, 1H), 2.85 (m, 1H), 3.30 (m, 2H), 3.50 (m, 1H), 4.75 (m, 1H), 7.00 (d, 1H), 7.20 (d, 1H), 7.28 (d, 1H), 7.50 (d, 1H), 7.69 (t, 1H), 7.98 (s, 1H), 8.47 (s, 1H), 10.35 (s, 1H).

2-chloro-4-[(5-{(1*S*)-1-[(2*R*)-1-glycoloylpyrrolidin-2-yl]ethoxy}quinazolin-4-yl)amino]phenol was prepared as described in example 21 (preparation of starting materials) using 2-chloro-4-[(5-{(1*S*)-1-[(2*R*)-pyrrolidin-2-yl]ethoxy}quinazolin-4-yl)amino]phenol and glycolic acid in 63% yield.

### Example 39

### 2-((2R)-2-{(1R)-1-[(4-{[3-Chloro-4-(pyridin-2-ylmethoxy)phenyl]amino}quinazolin-5-yl)oxy]ethyl}pyrrolidin-1-yl)-2-oxoethanol

The procedure described in example 21 was repeated using 2-(chloromethyl)pyridine and 2-chloro-4-[(5-{(1*R*)-1-[(2*R*)-1-glycoloylpyrrolidin-2-yl]ethoxy}quinazolin-4-y1)amino]phenol to give the title compound in 31% yield; NMR spectrum (DMSO-d6) 1.40 (d, 3H), 1.80 (m, 2H), 2.00 (m, 1H), 2.10 (m, 1H), 3.12 (m, 1H), 3.42 (m, 1H), 3.85 (dd, 1H), 4.03 (dd, 1H), 4.33 (m, 1H), 4.51 (m, 1H), 5.25 (dd, 1H), 5.30 (s, 2H), 7.08 (d, 1H), 7.26 (d, 1H), 7.32 (d, 1H), 7.35 (m, 1H), 7.51 (dd, 1H), 7.58 (d, 1H), 7.69 (t, 1H), 7.87 (dt, 1H), 8.07 (dd, 1H), 8.48 (s, 1H), 8.58 (d, 1H), 10.01 (s, 1H); Mass spectrum MH⁺ 534.

The 2-chloro-4-[(5-{(1*R*)-1-[(2*R*)-1-glycoloylpyrrolidin-2-yl]ethoxy}quinazolin-4-yl)amino]phenol used as starting material was prepared as described in example 38 (preparation of starting materials) using 2-chloro-4-[(5-{(1*R*)-1-[(2*R*)-pyrrolidin-2-yl]ethoxy}quinazolin-4-yl)amino]phenol (obtained as described in example 38, preparation of starting materials) and glycolic acid in 61% yield.

### Example 40

### 2-[(2S)-2-({[4-({3-Chloro4-[(6-methylpyridin-2-yl)methoxy]phenyl}amino)quinazolin-5-yl]oxy}methyl)pyrrolidin-1-yl]-2-oxoethanol

The procedure described in example 25 was repeated using (6-methylpyridin-2-yl)methanol and 2-chloro-4-[(5-{[(2*S*)-1-glycoloylpyrrolidin-2-yl]methoxy}quinazolin-4-yl)amino]phenol to give the title compound in 65% yield; NMR spectrum (DMSO-d6) 1.94 - 2.12 (m, 4H), 3.43 - 3.50 (m, 2H), 3.61 (s, 1H), 4.03 - 4.15 (m, 1H), 4.23 - 4.29 (m, 1H), 4.48 - 4.54 (m, 1H), 4.56 - 4.62 (m, 1H), 4.63 - 4.69 (m, 1H), 5.31 (s, 2H), 7.26 - 7.34 (m, 3H), 7.38 - 7.46 (m, 2H), 7.62 - 7.67 (m, 1H), 7.76 - 7.85 (m, 2H), 8.09 (d, 1H), 8.54 (s, 1H), 10.45 (s, 1H); Mass spectrum MH⁺ 534.

The 2-chloro-4-[(5-{[(2*S*)-1-glycoloylpyrrolidin-2-yl]methoxy}quinazolin-4-yl)amino]phenol used as starting material was prepared as follows:

2-Chloro-4-({5-[(2*S*)-pyrrolidin-2-ylmethoxy]quinazolin-4-yl}amino)phenol was prepared as described in example 21 (preparation of starting materials) using 2-chloro-4-[(5-fluoroquinazolin-4-yl)amino]phenol and (2*S*)-pyrrolidin-2-ylmethanol in 88% yield; NMR spectrum (DMSO-d6) 1.53 - 1.68 (m, 1H),1.71 - 1.89 (m, 2H),1.93 - 2.09 (m, 1H),3.02 (t, 2H),3.18 - 3,42 (m, 2H),3.7.4 - 3.88 (m, 1H),4.29 (t, 1H),4.36 - 4.45 (m, 1H),6.98 (d, 1H),7.15 (d, 1H),7.33 (d, 1H),7.50 - 7.58 (m, 1H),7.66 - 7.75 (m, 1H),7.93 - 7.97 (m, 1H),8.48 (s, 1H),10.05 (s, 1H); Mass spectrum MH⁺ 371.

2-Chloro-4-[(5-{[(2*S*)-1-glycoloylpyrrolidin-2-yl]methoxy}quinazolin-4-yl)amino]phenol was prepared as described in example 21 (preparation of starting materials) using 2-chloro-4-({5-[(2*S*)-pyrrolidin-2-ylmethoxy]quinazolin-4-yl}amino)phenol and glycolic acid in 98% yield; NMR spectrum (DMSO-d6) 1.84 - 2.09 (m, 5H), 3.94 - 4.10 (m, 2H), 4.22 - 4.31 (m, 1H), 4.46 (t, 1H), 4.56 - 4.66 (m, 1H), 7.03 - 7.10 (m, 2H), 7.34 - 7.51 (m, 4H), 7.70 (s, 1H), 7.90 - 7.99 (m, 1H), 8.73 (s, 1H), 10.45 (s, 1H), 10.92 (s, 1H); Mass spectrum MH⁺ 429.

### Example 41

### 2-[(2S)-2-({[4-({3-Chloro-4-[(2-fluorobenzyl)oxy]phenyl}amino)quinazolin-5-yl]oxy}methyl)pyrrolidin-1-yl]-2-oxoethanol

The procedure described in example 21 was repeated using 1-(chloromethyl)-2-fluorobenzene and 2-chloro-4-[(5-{[(2*S*)-1-glycoloylpyrrolidin-2-yl]methoxy}quinazolin-4-yl)amino]phenol (obtained as described in example 40, preparation of starting materials) to give the title compound in 61% yield; NMR spectrum (DMSO-d6) 1.88 - 2.07 (m, 4H), 3.38 - 3.45 (m, 2H), 3.55 (s, 1H), 3.98 - 4.11 (m, 1H), 4.18 - 4.25 (m, 1H), 4.43 - 4.49 (m, 1H), 4.51 - 4.56 (m, 1H), 4.57 - 4.64 (m, 1H), 5.27 (s, 2H), 7.24 - 7.38 (m, 5H), 7.42 - 7.48 (m, 1H), 7.59 - 7.65 (m, 2H), 7.70 - 7.76 (m, 1H), 8.02 (d, 1H), 8.48 (s, 1H), 9.99 (s, 1H); Mass spectrum MH⁺ 537.

### Example 42

### 2-[(2S)-2-({[4-({3-Chloro-4-[(3-fluorobenzyl)oxy]phenyl}amino)quinazolin-5-yl]oxy}methyl)pyrrolidin-1-yl]-2-oxoethanol

The procedure described in example 21 was repeated using 1-(chloromethyl)-3-fluorobenzene and 2-chloro-4-[(5-{[(2*S*)-1-glycoloylpyrrolidin-2-yl]methoxy}quinazolin-4-yl)amino]phenol (obtained as described in example 40, preparation of starting materials) to give the title compound in 61% yield; NMR spectrum (DMSO-d6) 1.94 - 2.12 (m, 4H), 3.42 - 3.50 (m, 2H), 3.61 (s, 1H), 4.03 - 4.15 (m, 1H), 4.23 - 4.29 (m, 1H), 4.48 - 4.54 (m, 1H), 4.56 - 4.61 (m, 1H), 4.63 - 4.69 (m, 1H), 5.32 (s, 2H), 7.21 - 7.27 (m, 1H), 7.28 - 7.34 (m, 2H), 7.35 - 7.44 (m, 3H), 7.50 - 7.56 (m, 1H), 7.63 - 7.68 (m, 1H), 7.76 - 7.82 (m, 1H), 8.08 (d, 1H), 8.53 (s, 1H), 10.04 (s, 1H); Mass spectrum MH⁺ 537.

### Example 43

### 2-((2S)-2-{[(4-{[3-Chloro4-(1,3-thiazol4-ylmethoxy)phenyl]amino}quinazolin-5-yl)oxy]methyl}pyrrolidin-1-yl)-2-oxoethanol

The procedure described in example 21 was repeated using 4-(chloromethyl)-1,3-thiazole and 2-chloro-4-[(5-{[(2*S*)-1-g1ycoloylpyrrolidin-2-yl]methoxy}quinazolin-4-yl)amino]phenol (obtained as described in example 40, preparation of starting materials) to give the title compound in 49% yield; NMR spectrum (DMSO-d6) 1.89 - 2.07 (m, 4H), 3.38 - 3.44 (m, 2H), 3.55 (s, 1H), 4.04 (t, 1H), 4.18 - 4.24 (m, 1H), 4.43 - 4.49 (m, 1H), 4.53 (t, 1H), 4.57 - 4.63 (m, 1H), 5.35 (s, 2H), 7.26 (d, 1H), 7.35 (d, 2H), 7.58 - 7.63 (m, 1H), 7.70 - 7.76 (m, 1H), 7.82 (d, 1H), 8.02 (d, 1H), 8.48 (s, 1H), 9.16 (d, 1H), 9.98 (s, 1H); Mass spectrum MH⁺ 526.

### Example 44

### 2-((2S)-2-{[(4-{[3-Chloro-4-(pyrazin-2-ylmethoxy)phenyl]amino}quinazolin-5-yl)oxy]methyl}pyrrolidin-1-yl)-2-oxoethanol

The procedure described in example 25 was repeated using pyrazin-2-ylmethanol and 2-chloro-4-[(5-{[(2*S*)-1-glycoloylpyrrolidin-2-yl]methoxy}quinazolin-4-yl)amino]phenol (obtained as described in example 40, preparation of starting materials) to give the title compound in 57% yield; NMR spectrum (DMSO-d6) 1.89 - 2.07 (m, 4H), 3.38 - 3.44 (m, 2H), 3.55 (s, 0H), 4.04 (t, 1H), 4.19 - 4.24 (m, 1H), 4.43 - 4.49 (m, 1H), 4.51 - 4.55 (m, 1H), 4.57 - 4.65 (m, 1H), 5.39 (s, 2H), 7.26 (d, 1H), 7.31 - 7.37 (m, 2H), 7.60 - 7.64 (m, 1H), 7.70 - 7.76 (m, 1H), 8.05 (d, 1H), 8.48 (s, 1H), 8.65 - 8.71 (m, 2H), 8.86 - 8.89 (m, 1H), 10.00 (s, 1H); Mass spectrum MH⁺ 521.

### Example 45

### N-{3-Chloro-4-[(6-methylpyridin-2-yl)methoxy]phenyl}-5-({(2S)-1-[(dimethylamino)acetyl]pyrrolidin-2-yl}methoxy)quinazolin-4-amine

The procedure described in example 25 was repeated using (6-methylpyridin-2-yl)methanol and 2-chloro-4-[(5-{[(2*S*)-1-(*N*,*N*-dimethylglycyl)pyrrolidin-2-yl]methoxy}quinazolin-4-yl)amino]phenol to give the title compound in 24% yield; NMR spectrum (DMSO-d6) 1.90 - 2.11 (m, 4H), 2.20 - 2.26 (m, 6H), 3.04 (d, 1H), 3.17 (d, 1H), 3.56 - 3.65 (m, 2H), 4.23 - 4.30 (m, 1H), 4.44 - 4.49 (m, 1H), 4.60 - 4.67 (m, 1H), 5.30 (s, 2H), 7.28 (d, 3H), 7.37 - 7.44 (m, 2H), 7.63 - 7.67 (m, 1H), 7.75 - 7.84 (m, 2H), 8.08 (d, 1H), 8.51 (s, 1H), 10.03 (s, 1H), 2.56 (s, 3H); Mass spectrum MH⁺ 561.

The 2-chloro-4-[(5-{[(2*S*)-1-(*N*,*N*-dimethylglycyl)pyrrolidin-2-yl]methoxy}quinazolin-4-yl)amino]phenol used as starting material was prepared as described in example 21 (preparation of starting materials) using *N*,*N*-dimethylglycine and 2-chloro-4-({5-[(2*S*)-pyrrolidin-2-ylmethoxy]quinazolin-4-yl}amino)phenol (obtained as described in example 40, preparation of starting materials) in 93% yield; NMR spectrum (DMSO-d6) 1.85 - 2.08 (m, 4H), 2.33 (s, 6H), 3.23 - 3.40 (m, 2H), 3.46 - 3.54 (m, 2H), 4.15 - 4.24 (m, 1H), 4.36 - 4.45 (m, 1H), 4.51 - 4.61 (m, 1H), 6.98 (d, 1H), 7.20 (d, 1H), 7.27 - 7.33 (m, 1H), 7.37 - 7.43 (m, 1H), 7.65 - 7.73 (m, 1H), 7.86 (d, 1H), 8.42 (s, 1H), 9.85 (s, 1H), 10.06 (s, 1H); Mass spectrum MH⁺ 456.

### Example 46

### N-{3-Chloro-4-[(2-fluorobenzyl)oxy]phenyl}-5-({(2S)-1-[(dimethylamino)acetyl]pyrrolidin-2-yl}methoxy)quinazolin4-amine

The procedure described in example 21 was repeated using 1-(chloromethyl)-2-fluorobenzene and 2-chloro-4-[(5-{[(2*S*)-1-(*N*,*N*-dimethylglycyl)pyrrolidin-2-yl]methoxy}quinazolin-4-yl)amino]phenol (obtained as described in example 45, preparation of starting materials) to give the title compound in 26% yield; NMR spectrum (DMSO-d6) 1.85 - 2.07 (m, 4H), 2.17 - 2.22 (m, 6H), 3.01 (d, 1H), 3.14 (d, 1H), 3.52 - 3.59 (m, 2H), 4.19 - 4.24 (m, 1H), 4.39 - 4.45 (m, 1H), 4.56 - 4.62 (m, 1H), 5.27 (s, 2H), 7.21 - 7.37 (m, 5H), 7.42 - 7.48 (m, 1H), 7.58 - 7.65 (m, 2H), 7.70 - 7.75 (m, 1H), 8.00 (d, 1H), 8.47 (s, 1H), 9.98 (s, 1H); Mass spectrum MH⁺ 564.

### Example 47

### N-{3-Chloro4-[(3-fluorobenzyl)oxy]phenyl}-5-({(2S)-1-[(dimethylamino)acetyl]pyrrolidin-2-yl}methoxy)quinazolin-4-amine

The procedure described in example 21 was repeated using 1-(chloromethyl)-3-fluorobenzene and 2-chloro-4-[(5-{[(2*S*)-1-(*N*,*N*-dimethylglycyl)pyrrolidin-2-yl]methoxy}quinazolin-4-yl)amino]phenol (obtained as described in example 45, preparation of starting materials) to give the title compound in14 % yield; NMR spectrum (DMSO-d6) 1.87 - 2.08 (m, 4H), 2.25 - 2.33 (m, 6H), 3.27 (s, 2H), 3.50 - 3.56 (m, 2H), 4.19 - 4.25 (m, 1H), 4.40 - 4.46 (m, 1H), 4.56 - 4.64 (m, 1H), 5.27 (s, 2H), 7.15 - 7.27 (m, 3H), 7.29 - 7.36 (m, 3H), 7.44 - 7.51 (m, 1H), 7.59 - 7.63 (m, 1H), 7.70 - 7.75 (m, 1H), 8.01 (d, 1H), 8.47 (s, 1H), 9.98 (s, 1H); Mass spectrum MH⁺ 564.

### Example 48

### N-[3-Chloro-4-(pyrazin-2-ylmethoxy)phenyl]-5-({(2S)-1-[(dimethylamino)acetyl]pyrrolidin-2-yl}methoxy)quinazolin-4-amine

The procedure described in example 25 was repeated using pyrazin-2-ylmethanol and 2-chloro-4-[(5-{[(2*S*)-1-(*N*,*N*-dimethylglycyl)pyrrolidin-2-yl]methoxy}quinazolin-4-yl)amino]phenol (obtained as described in example 45, preparation of starting materials) to give the title compound in 19% yield; NMR spectrum (DMSO-d6) 1.86 - 2.07 (m, 4H), 2.20 (s, 6H), 3.02 (d, 1H), 3.15 (d, 1H), 3.52 - 3.59 (m, 2H), 4.18 - 4.25 (m, 1H), 4.39 - 4.45 (m, 1H), 4.56 - 4.63 (m, 1H), 5.40 (s, 2H), 7.23 (d, 1H), 7.30 - 7.36 (m, 2H), 7.60 - 7.65 (m, 1H), 7.70 - 7.75 (m, 1H), 8.03 (d, 1H), 8.47 (s, 1H), 8.65 - 8.72 (m, 2H), 8.87 (s, 1H), 10.00 (s, 1H); Mass·spectrum MH⁺ 548.

### Example 49

### N-[3-Chloro-4-(1,3-thiazol-4-ylmethoxy)phenyl]-5-({(2S)-1-[(dimethylamino)acetyl]pyrrolidin-2-yl}methoxy)quinazolin-4-amine

The procedure described in example 21 was repeated using 4-(chloromethyl)-1,3-thiazole and 2-chloro-4-[(5-{[(2*S*)-1-(*N*,*N*-dimethylglycyl)pyrrolidin-2-yl]methoxy}quinazolin-4-yl)amino]phenol (obtained as described in example 45, preparation of starting materials) in to give the title compound 1% yield; Mass spectrum MH⁺ 553.

### Example 50

### 2-[(2R)-2-({[4-({3-Chloro-4-[(6-methylpyridin-2-yl)methoxy]phenyl}amino)quinazolin-5-yl]oxy}methyl)piperidin-1-yl]-2-oxoethanol

The procedure described in example 25 was repeated using (6-methylpyridin-2-yl)methanol and 2-chloro-4-[(5-{[(2*R*)-1-glycoloylpiperidin-2-yl]methoxy}quinazolin-4-yl)amino]phenol to give the title compound in 35% yield; NMR spectrum (DMSO-d6) 1.34 - 1.52 (m, 1H), 1.57 - 1.76 (m, 4H), 1.82 - 1.91 (m, 1H), 3.27 - 3.32 (m, 4H), 3.50 - 3.65 (m, 1H), 3.92 - 4.14 (m, 2H), 4.18 - 4.45 (m, 2H), 4.71 (t, 1H), 5.11 - 5.22 (m, 1H), 5.25 (s, 2H), 7.22 (d, 1H), 7.24 (d, 1H), 7.29 (d, 1H), 7.33 - 7.40 (m, 2H), 7.54 (d, 1H), 7.71 - 7.79 (m, 2H), 7.95 (s, 1H), 8.45 (s, 1H), 9.66 (s, 1H); Mass spectrum MH⁺ 548.

The 2-chloro-4-[(5-{[(2R)-1-glycoloylpiperidin-2-yl]methoxy}quinazolin-4-yl)amino]phenol used as starting material was prepared as follows:

2-Chloro-4-({5-[(2*R*)-piperidin-2-ylmethoxy]quinazolin-4-yl} amino)phenol was prepared as described in example 21 (preparation of starting materials) using (2*R*)-piperidin-2-ylmethanol and 2-chloro-4-[(5-fluoroquinazolin-4-yl)amino]phenol in 71 % yield; Mass spectrum MH⁺ 384.

2-Chloro-4-[(5-{[(2*R*)-1-glycoloylpiperidin-2-yl]methoxy} quinazolin-4-yl)amino]phenol.was prepared as described in example 21 (preparation of starting materials) using glycolic acid and 2-chloro-4-({5-[(2*R*)-piperidin-2-ylmethoxy]quinazolin-4-yl}amino)phenol in 73% yield; Mass spectrum MH⁺ 443.

### Example 51

### 2-[(2R)-2-({[4-({3-Chloro4-[(2-fluorobenzyl)oxy]phenyl}amino)quinazolin-5-yl]oxy}methyl)piperidin-1-yl]-2-oxoethanol

The procedure described in example 21 was repeated using 1-(chloromethyl)-2-fluorobenzene and 2-chloro-4-[(5-{[(2*R*)-1-glycoloylpiperidin-2-yl]methoxy} quinazolin-4-yl)amino]phenol (obtained as described in example 50, preparation of starting materials) to give the title compound in 40% yield; NMR spectrum (DMSO-d6) 1.34 - 1.51 (m, 1H), 1.57 - 1.78 (m, 4H), 1.82 - 1.93 (m, 1H), 3.49 - 3.65 (m, 3H), 3.91 - 4.16 (m, 2H), 4.19 - 4.45 (m, 2H), 4.72 (t, 1H), 5.27 (s, 2H), 7.23 - 7.39 (m, 5H), 7.41 - 7.50 (m, 1H), 7.52 - 7.66 (m, 2H), 7.69 - 7.79 (m, 1H), 7.93 (s, 1H), 8.41 - 8.50 (m, 1H), 9.68 (s, 1H); Mass spectrum MH⁺ 551.

### Example 52

### 2-[(2R)-2-({[4-({3-Chloro-4-[(3-fluorobenzyl)oxy]phenyl}amino)quinazolin-5-yl]oxy}methyl)piperidin-1-yl]-2-oxoethanol

The procedure described in example 21 was repeated using 1-(chloromethyl)-3-fluorobenzene and 2-chloro-4-[(5-{[(2*R*)-1-glycoloylpiperidin-2-yl]methoxy}quinazolin-4-yl)amino]phenol (obtained as described in example 50, preparation of starting materials) in to give the title compound 44% yield; NMR spectrum (DMSO-d6) 1.35 - 1.51 (m, 1H), 1.57 - 1.77 (m, 4H), 1.82 - 1.91 (m, 1H), 3.51 - 3.59 (m, 3H), 3.91 - 4.15 (m, 2H), 4.17 - 4.42 (m, 2H), 4.72 (t, 1H), 5.27 (s, 2H), 7.14 - 7.40 (m, 6H), 7.44 - 7.59 (m, 2H), 7.70 - 7.78 (m, 1H), 7.90 - 7.97 (m, 1H), 8.45 (s, 1H), 9.66 (s, 1H); Mass spectrum MH⁺ 551.

### Example 53

### 2-((2R)-2-{[(4-{[3-Chloro-4-(1,3-thiazol-4-ylmethoxy)phenyl]amino}quinazolin-5-yl)oxy]methyl}piperidin-1-yl)-2-oxoethanol

The procedure described in example 21 was repeated using 4-(chloromethyl)-1,3-thiazole and 2-chloro-4-[(5-{[(2*R*)-1-glycoloylpiperidin-2-yl]methoxy}quinazolin-4-yl)amino]phenol (obtained as described in example 50, preparation of starting materials) to give the title compound in 34% yield; NMR spectrum (DMSO-d6) 1.33 - 1.52 (m, 1H), 1.56 - 1.78 (m, 4H), 1.81 - 1.93 (m, 1H), 3.55 (s, 3H), 3.90 - 4.16 (m, 2H), 4.17 - 4.45 (m, 2H), 4.71 (t, 1H), 5.34 (s, 2H), 7.23 - 7.42 (m, 3H), 7.55 (d, 1H), 7.69 - 7.78 (m, 1H), 7.82 (s, 1H), 7.93 (s, 1H), 8.45 (s, 1H), 9.16 (s, 1H), 9.66 (s, 1H); Mass spectrum M-H⁺ 539.

### Example 54

### 2-((2R)-2-{[(4-{[3-Chloro4-(pyrazin-2-ylmethoxy)phenyl]amino}quinazolin-5-yl)oxy]methyl}piperidin-1-yl)-2-oxoethanol

The procedure described in example 25 was repeated using pyrazin-2-ylmethanol and 2-chloro-4-[(5-{[(2*R*)-1-glycoloylpiperidin-2-yl]methoxy}quinazolin-4-yl)amino]phenol (obtained as described in example 50, preparation of starting materials) to give the title compound in 43% yield; NMR spectrum (DMSO-d6) 1.35 - 1.51 (m, 1H), 1.58 - 1.76 (m, 4H), 1.82 - 1.91 (m, 1H), 3.28 (s, 2H), 3.55 (s, 1H), 3.90 - 4.15 (m, 2H), 4.19 - 4.45 (m, 2H), 4.72 (t, 1H), 5.39 (s, 2H), 7.25 - 7.38 (m, 3H), 7.54 - 7.60 (m, 1H), 7.71 - 7.78 (m, 1H), 7.96 (s, 1H), 8.46 (s, 1H), 8.65 - 8.72 (m, 1H), 8.88 (s, 1H), 9.67 (s, 1H); Mass spectrum MH⁺ 535.

### Example 55

### 2-[(2R)-2-({[4-({3-Methyl4-[(6-methylpyridin-3-yl)oxy]phenyl}amino)quinazolin-5-yl]oxy}methyl)pyrrolidin-1-yl]-2-oxoethanol

Acetoxyacetyl chloride (50 µl, 0.5 mmol) was added dropwise to an ice-cooled solution of *N*-{3-methyl-4-[(6-methylpyridin-3-yl)oxy]phenyl}-5-[(2*R*)-pyrrolidin-2-ylmethoxy]quinazolin-4-amine (200 mg, 0.45 mmol) and triethylamine (80 µl, 0.54 mmol) in DCM (5 ml). The mixture was stirred at room temperature for 2 hours. After evaporation of the mixture to dryness, pyrrolidine (0.19 ml, 2.26 mmol) was added and the mixture was stirred at 80°C for 1 hour. After evaporation of the solvents, the residue was purified on an HPLC column (C18, 5 microns, 19 mm diameter, 100 mm length) of a preparative HPLC-MS system eluting with a mixture of water and acetonitrile containing 2g/l of ammonium formate (gradient), then triturated in ether to give the title compound (55 mg, 24%) as a pale solid; NMR spectrum (CDCl₃) 2.2-2.1 (m, 4H), 2.29 (s, 3H), 2.53 (s, 3H), 3.30 (m, 1H), 3.43-3.35 (m, 2H), 4.12 (m, 3H), 4.55 (m, 1H), 4.76 (m, 1H), 6.91 (d, 1H), 6.99 (d, 1H), 7.09 (d, 1H), 7.14 (d, 1H), 7.44 (d, 1H), 7.50 (d, 1H), 7.63 (m, 2H), 8.28 (s, 1H), 8.62 (s, 1H), 9.78 (s br, 1H); Mass spectrum: MH⁺ 500.

The *N*-{3-methyl-4-[(6-methylpyridin-3-yl)oxy]phenyl}-5-[(2*R*)-pyrrolidin-2-ylmethoxy]quinazolin-4-amine used as starting material was made as follows:
Sodium hydride (25.6 g, 60% dispersion in oil, 0.64 mol) was added portion wise to a solution of 5-hydroxy-2-methylpyridine (70 g, 0.64 mol) in DMA (700 ml) while keeping the temperature below 40°C. At the end of the addition, the mixture was stirred at room temperature for 1 hour and 2-fluoro-5-nitrotoluene (91.3 g, 0.59 mol) in DMA (100 ml) was added slowly. The mixture was stirred at 80°C for 3 hours and then cooled. The solvents were evaporated under vacuum and the residue was partitioned between ethyl acetate and water. The organic layer was washed with water and brine, dried over MgSO₄. After evaporation of the solvents, the residue was purified by chromatography on silica gel (eluant: 30% ethyl acetate in petroleum ether) to give 2-methyl-5-(2-methyl-4-nitrophenoxy)pyridine (141 g, 98%) as an oil; NMR spectrum (CDCl₃); 2.43 (s, 3H), 2.59 (s, 3H), 6.74 (d, 1H), 7.21 (d, 1H), 7.27 (d, 1H), 8.00 (d, 1H), 8.17 (s, 1H), 8.32 (s, 1H).
A mixture of 2-methyl-5-(2-methyl-4-nitrophenoxy)pyridine (141 g, 0.58 mol) and 10% palladium on charcoal (13 g) in ethyl acetate (200 ml) and ethanol (700 ml) was stirred under an atmosphere of hydrogen (1.2 bar) for 5 hours. After reaction completion, the mixture was purged with nitrogen and the catalyst was filtered off. The filtrate was evaporated to dryness to give 3-methyl-4-[(6-methylpyridin-3-yl)oxy]aniline (120.6 g, 98%) as a white solid; Mass spectrum MH⁺ 215.

3-Methyl-4-[(6-methylpyridin-3-yl)oxy]aniline (6.42 g, 30 mmol) and 4N hydrogen chloride in dioxane (7.55 ml, 30 mmol) were added to a suspension of 4-chloro-5-fluoroquinazoline (5 g, 27.5 mmol; PCT Int. Appl. WO2001094341, AstraZeneca) in acetonitrile (100 ml). The mixture was stirred at 80°C for 2 hours. After cooling, the precipitate was washed with acetonitrile. This precipitate was partitioned between DCM and 5% aqueous sodium bicarbonate and the pH was adjusted to 8. The organic layer was washed with brine and dried over MgSO₄. Evaporation of the solvents gave 5-fluoro-*N*-{3-methyl-4-[(6-methylpyridin-3-yl)oxy]phenyl}quinazolin-4-amine (9.3 g, 94%) as a dark gum which crystallised on standing; NMR spectrum (CDCl₃); 2.30 (s, 3H), 2.54 (s, 3H), 6.93 (d, 1H), 7.15-7.08 (m, 2H), 7.22 (m, 1H), 7.56 (d, 1H), 7.63 (s, 1H), 7.71 (m, 2H), 8.27 (s, 1H), 8.37 (d, 1H), 8.71 (s, 1H).

Sodium hydride (228 mg, 60% dispersion in oil, 5.7 mmol) was added portion wise to a solution of (R)-2-pyrrolidinemethanol (562 mg, 5.56 mmol) in THF (20 ml). 15-Crown-5 (120 mg, 0.56 mmol) was added and the mixture was stirred at room temperature for 15 minutes. 5-Fluoro-*N*-{3-methyl-4-[(6-methylpyridin-3-yl)oxy]phenyl}quinazolin-4-amine (1 g, 2.78 mmol) was added. The mixture was heated at reflux for 15 hours. After cooling, the solvents were evaporated under vacuum and brine was added. The mixture was extracted with DCM. The organic layer was dried over MgSO₄. After evaporation of the solvents, the residue was purified by chromatography on silica gel (eluant: 7 to 10% methanol in DCM), then triturated in ether to give *N*-{3-methyl-4-[(6-methylpyridin-3-yl)oxy]phenyl}-5-[(2*R*)-pyrrolidin-2-ylmethoxy]quinazolin-4-amine (930 mg, 76%) as a beige solid; NMR spectrum (CDCl₃) 1.66 (m, 1H), 2.0-1.8 (m, 2H), 2.03 (m, 1H), 2.28 (s, 3H), 2.53 (s, 3H), 3.02 (m, 2H), 3.74 (m, 1H), 4.08 (m, 1H), 4.20 (m, 1H), 6.90 (m, 2H), 7.08 (d, 1H), 7.13 (d, 1H), 7.44 (d, 1H), 7.62 (m, 2H), 7.75 (s, 1H), 8.27 (s, 1H), 8.63 (s, 1H); Mass spectrum: MH⁺ 442.

### Example 56

### 5-({(2R)-1-[(Dimethylamino)acetyl]pyrrolidin-2-yl}methoxy)-N-{3-methyl4-[(6-methylpyridin-3-yl)oxy]phenyl}quinazolin-4-amine

Dimethylaminoacetyl chloride (126 mg, 0.8 mmol) was added dropwise to an ice-cooled solution of *N*-{3-methyl-4-[(6-methylpyridin-3-yl)oxy]phenyl}-5-[(2*R*)-pyrrolidin-2-ylmethoxy]quinazolin-4-amine (200 mg, 0.45 mmol) and triethylamine (260 µl, 1.8 mmol) in DCM (5 ml). The mixture was stirred at room temperature for 2 hours. After evaporation of the mixture to dryness, the residue was purified on an HPLC column (C18, 5 microns, 19 mm diameter, 100 mm length) of a preparative HPLC-MS system eluting with a mixture of water and acetonitrile containing 2g/l of ammonium formate (gradient), then triturated in ether to give the title compound (170 mg, 71%) as a pale solid; NMR spectrum (CDCl₃) 2.16-2.03 (m, 4H), 2.29 (s, 3H), 2.33 (s, 6H), 2.53 (s, 3H), 3.06 (d, 1H), 3.14 (d, 1H), 3.60 (m, 2H), 4.06 (t, 1H), 4.62 (m, 1H), 4.68 (m, 1H), 6.91 (d, 1H), 7.14-7.07 (m, 3H), 7.47 (m, 2H), 7.64 (m, 2H), 8.27 (s, 1H), 8.62 (s, 1H), 9.80 (s br, 1H); Mass spectrum: MH⁺ 527.

### Example 57

### 2-[(2S)-2-({[4-({3-Methyl-4-[(6-methylpyridin-3-yl)oxy]phenyl}amino)quinazolin-5-yl]oxy}methyl)pyrrolidin-1-yl]-2-oxoethanol

The procedure described in example 55 was repeated with *N*-{3-methyl-4-[(6-methylpyridin-3-yl)oxy]phenyl}-5-[(2*S*)-pyrrolidin-2-ylmethoxy]quinazolin-4-amine (200 mg, 0.45 mmol) to give the title compound (85 mg, 35%) as a pale solid; NMR spectrum (CDCl₃) 2.2-2.1 (m, 4H), 2.30 (s, 3H), 2.54 (s, 3H), 3.28 (m, 1H), 3.43-3.35 (m, 2H), 4.12 (m, 3H), 4.55 (m, 1H), 4.76 (m, 1H), 6.91 (d, 1H), 6.99 (d, 1H), 7.09 (d, 1H), 7.14 (d, 1H), 7.44 (d, 1H), 7.50 (d, 1H), 7.63 (m, 2H), 8.28 (s, 1H), 8.62 (s, 1H), 9.81 (s br, 1H); Mass spectrum: MH⁺ 500.

The *N*-{3-methyl-4-[(6-methylpyridin-3-yl)oxy]phenyl}-5-[(2*S*)-pyrrolidin-2-ylmethoxy]quinazolin-4-amine used as starting material was made from (*S*)-2-pyrrolidinemethanol (562 mg, 5.56 mmol) and 5-fluoro-*N*-{3-methyl-4-[(6-methylpyridin-3-yl)oxy]phenyl}quinazolin-4-amine (1 g, 2.78 mmol) according to the procedure described in Example 55 starting material (889 mg, 72%); Mass spectrum: MH⁺ 442.

### Example 58

### 5-({(2S)-1-[(Dimethylamino)acetyl]pyrrolidin-2-yl}methoxy)-N-{3-methyl-4-[(6-methylpyridin-3-yl)oxy]phenyl}quinazolin4-amine

The procedure described in example 56 was repeated with *N*-{3-methyl-4-[(6-methylpyridin-3-yl)oxy]phenyl}-5-[(2*S*)-pyrrolidin-2-ylmethoxy]quinazolin-4-amine (200 mg, 0.45 mmol) to give the title compound (140 mg, 58%) as a pale solid; NMR spectrum (CDCl₃) 2.16-2.03 (m, 4H), 2.29 (s, 3H), 2.34 (s, 6H), 2.53 (s, 3H), 3.07 (d, 1H), 3.14 (d, 1H), 3.60 (m, 2H), 4.06 (t, 1H), 4.62 (m, 1H), 4.68 (m, 1H), 6.91 (d, 1H), 7.14-7.07 (m, 3H), 7.47 (m, 2H), 7.64 (m, 2H), 8.27 (s, 1H), 8.62 (s, 1H), 9.80 (s br, 1H); Mass spectrum: MH⁺ 527.

### Example 59

### 2-(4-{[(4-{[3-chloro-4-(pyridin-2-ylmethoxy)phenyl]amino}quinazolin-5-yl)oxy]methyl}piperidin-1-yl)-2-oxoethanol

The procedure described in example 21 was repeated using 2-chloro-4-({5-[(1-glycoloylpiperidin-4-yl)methoxy]quinazolin-4-yl}amino)phenol and 2-picolyl chloride hydrochloride to give the title compound in 32% yield; NMR spectrum (DMSO-d6) 1.25-1.40 (m, 2H), 1.87 (m, 2H), 2.35 (m, 1H), 2.73 (m, 1H), 3.05 (m, 1H), 3.75 (m, 1H), 4.09 (dd, 2H), 4.24 (d, 2H), 4.44 (m, 1H), 4.47 (t, 1H), 5.30 (s, 2H), 7.17 (d, 1H), 7.28 (d, 1H), 7.36 (d, 1H), 7.37 (dd, 1H), 7.47 (dd, 1H), 7.59 (d, 1H), 7.74 (dd, 1H), 7.89 (ddd, 1H), 8.19 (d, 1H), 8.55 (s, 1H), 8.60 (d, 1H), 9.94 (s, 1H); Mass spectrum MH⁺ 534.4, 536.4.

The 2-chloro-4-({5-[(1-glycoloylpiperidin-4-yl)methoxy]quinazolin-4-yl}amino)phenol used as starting material was obtained as follows:
Sodium hydride (60% dispersion in mineral oil, 864 mg, 21.6 mmol) was suspended in DMA (40 ml), and 4-(hydroxymethyl)piperidine (2.48 g, 21.6 mmol) was added dropwise as a solution in DMA (20 ml) under a nitrogen atmosphere. The mixture was stirred for 20 minutes at ambient temperature, and 2-chloro-4-[(5-fluoroquinazolin-4-yl)amino]phenol (obtained as described in example 21, preparation of starting materials, 2.50 g, 8.65 mmol) was added. The mixture was heated to 110°C for 5 hours under a nitrogen atmosphere, and then cooled to ambient temperature. Saturated ammonium chloride solution (10 ml) was added, and the mixture concentrated *in vacuo.* The residue was treated with saturated sodium hydrogen carbonate solution (75 ml), and the mixture stirred and sonicated to give a granular precipitate. The solid was collected by filtration, washed with water, and dried to give 2-chloro-4-{[5-(piperidin-4-ylmethoxy)quinazolin-4-yl]amino}phenol as a cream coloured solid (3.35 g, quantitative); NMR spectrum (DMSO-d6) 1.32 (m, 2H), 1.78 (m, 2H), 2.15 (m, 1H), 2.60 (m, 2H), 3.04 (m, 2H), 4.17 (d, 2H), 7.00 (d, 1H), 7.14 (d, 1H), 7.34 (d, 1H), 7.48 (dd, 1H), 7.72 (dd, 1H), 7.96 (d, 1H), 8.51 (s, 1H), 9.94 (s, 1H); Mass spectrum MH⁺ 385.0, 387.0.

HATU (1.09 g, 2.86 mmol) was added to a mixture of 2-chloro-4-{[5-(piperidin-4-ylmethoxy)quinazolin-4-yl]amino}phenol (1.00 g, 2.60 mmol) and glycolic acid (217 mg, 2.86 mmol) in DMA (30 ml). The mixture was stirred at ambient temperature for 16 hours, and then concentrated *in vacuo.* The residue was purified by chromatography, eluting with 4 to 6% (10:1 MeOH / conc. NH_{3(aq)}) in DCM to give 2-chloro-4-({5-[(1-glycoloylpiperidin-4-yl)methoxy]quinazolin-4-yl}amino)phenol as a yellow solid (810 mg, 70%); NMR spectrum (DMSO-d6) 1.25-1.40 (m, 2H), 1.86 (m, 2H), 2.34 (m, 1H), 2.74 (m, 1H), 3.05 (m, 1H), 3.75 (m, 1H), 4.08 (s, 2H), 4.23 (d, 2H), 4.42 (t, 1H), 4.42 (m, 1H), 7.01 (d, 1H), 7.16 (d, 1H), 7.33 (dd, 1H), 7.34 (d, 1H), 7.73 (dd, 1H), 8.04 (d, 1H), 8.51 (s, 1H), 9.87 (s, 1H), 10.05 (br.s, 1H); Mass spectrum MH⁺ 443.0, 445.0.

### Example 60

### 2-(4-{[(4-{[3-chloro-4-(1,3-thiazol-4-ylmethoxy)phenyl]amino}quinazolin-5-yl)oxy]methyl}piperidin-1-yl)-2-oxoethanol

The procedure described in example 21 was repeated using 2-chloro-4-({5-[(1-glycoloylpiperidin-4-yl)methoxy]quinazolin-4-yl}amino)phenol (obtained as described in example 59, preparation of starting materials) and 4-(chloromethyl)-1,3-thiazole hydrochloride to give the title compound in 26% yield; NMR spectrum (DMSO-d6); 1.25-1.45 (m, 2H), 1.87 (m, 2H), 2.36 (m, 1H), 2.73 (m, 1H), 3.05 (m, 1H), 3.76 (m, 1H), 4.10 (dd, 2H), 4.24 (d, 2H), 4.43 (m, 1H), 4.49 (t, 1H), 5.34 (s, 2H), 7.17 (d, 1H), 7.3 6 (d, 1H), 7.36 (d, 1H), 7.48 (dd, 1H), 7.75 (dd, 1H), 7.83 (d, 1H), 8.17 (d, 1H), 8.55 (s, 1H), 9.15 (d, 1H), 9.95 (s, 1H); Mass spectrum MH⁺ 540.4, 542.4.

### Example 61

### 2-(4-{[(4-{[3-chloro-4-(1,3-thiazol-2-ylmethoxy)phenyl]amino}quinazolin-5-yl)oxy]methyl}piperidin-1-yl)-2-oxoethanol

Methanesulfonyl chloride (19 µl, 0.25 mmol) was added to a solution of 1,3-thiazol-2-ylmethanol (29 mg, 0.25 mmol) and *N,N*-diisopropylethylamine (44 µl, 0.25 mmol) in DCM (5 ml) at 0°C. The mixture was allowed to warm to ambient temperature, stirred for 2 hours and concentrated *in vacuo.* The residue was dissolved in DMA (5 ml), and added to a suspension of 2-chloro-4-({5-[(1-glycoloylpiperidin-4-yl)methoxy]quinazolin-4-yl}amino)phenol (obtained as described in example 59, preparation of starting materials, 89 mg, 0.20 mmol) and potassium carbonate (138 mg, 1.00 mmol) in DMA (5 ml). The mixture was stirred at room temperature for 16 hours, then concentrated *in vacuo.* The residue was partitioned between DCM (15 ml) and water (15 ml). The DCM fraction was loaded onto a silica column, which was eluted with 2 to 4% (10:1 MeOH / conc_{.} NH_{3(aq)}) in DCM. The appropriate fractions were concentrated and the residue crystallised twice from ethyl acetate to give the title product as a white solid (30 mg, 28%); NMR spectrum (DMSO-d6) 1.25-1.45 (m, 2H), 1.87 (m, 2H), 2.36 (m, 1H), 2.73 (m, 1H), 3.05 (m, 1H), 3.75 (m, 1H), 4.09 (dd, 2H), 4.24 (d, 2H), 4.43 (m, 1H), 4.48 (t, 1H), 5.56 (s, 2H), 7.17 (d, 1H), 7.35 (d, 1H), 7.37 (d, 1H), 7.50 (dd, 1H), 7.75 (dd, 1H), 7.81 (d, 1H), 7.87 (d, 1H), 8.20 (d, 1H), 8.56 (s, 1H), 9.96 (s, 1H); Mass spectrum MH⁺ 540.2, 542.2.

### Example 62

### 2-[4-({[4-({3-chloro-4-[(3-fluorobenzyl)oxy]phenyl}amino)quinazolin-5-yl]oxy}methyl)piperidin-1-yl]-2-oxoethanol

The procedure described in example 21 was repeated using 2-chloro-4-({5-[(1-glycoloylpiperidin-4-yl)methoxy]quinazolin-4-yl}amino)phenol (obtained as described in example 59, preparation of starting materials) and 3-fluorobenzyl chloride to give the title compound in 46% yield; NMR spectrum (DMSO-d6) 1.25-1.45 (m, 2H), 1.87 (m, 2H), 2.35 (m, 1H), 2.73 (m, 1H), 3.05 (m, 1H), 3.76 (m, 1H), 4.09 (dd, 2H), 4.24 (d, 2H), 4.43 (m, 1H), 4.48 (t, 1H), 5.26 (s, 2H), 7.17 (d, 1H), 7.18 (ddd, 1H), 7.27 (d, 1H), 7.30-7.35 (m, 2H), 7.36 (d, 1H), 7.47 (dd, 1H), 7.48 (ddd, 1H), 7.75 (dd, 1H), 8.18 (d, 1H), 8.55 (s, 1H), 9.94 (s, 1H); Mass spectrum MH⁺ 551.2, 553.2

### Example 63

### 2-[4-({[4-({3-chloro-4-[(6-methylpyridin-2-yl)methoxy]phenyl}amino)quinazolin-5-yl]oxy}methyl)piperidin-1-yl]-2-oxoethanol

Methanesulfonyl chloride (19 µl, 0.25 mmol) was added to a solution of 6-methylpyridin-2-ylmethanol (31 mg, 0.25 mmol) aud *N,N*-diisopropylethylamine (44 µl, 0.25 mmol) in DCM (5 ml) at 0°C. The mixture was allowed to warm to ambient temperature, was stirred for 2 hours and concentrated *in vacuo.* The residue was dissolved in DMA (5 ml), and added to a suspension of 2-chloro-4-({5-[(1-glycoloylpiperidin-4-yl)methoxy]quinazolin-4-yl}amino)phenol (obtained as described in example 59, preparation of starting materials, 89 mg, 0.20 mmol) and potassium carbonate (138 mg,1.00 mmol) in DMA (5 ml). The mixture was stirred at room temperature for 16 hours, then concentrated *in vacuo.* The residue was partitioned between DCM (15 ml) and water (15 ml). The DCM fraction was loaded onto a silica column, which was purified by chromatography, eluting with 2 to 4.5% (10:1 MeOH / conc. NH_{3(aq)}) in DCM. The appropriate fractions were concentrated *in vacuo* and the residue purified by reverse phase HPLC, eluting with 5 to 50% MeCN in water containing 0.2% TFA. The appropriate fractions were basified with 2N sodium hydroxide solution (1 ml), and extracted with DCM (2 x 15 ml). The combined extractions were filtered through a silicone- treated filter paper and concentrated *in vacuo.* The residue was crystallised from ethyl acetate / *iso*-hexane to give the title compound as a white solid (20 mg, 18%); NMR spectrum (DMSO-d6) 1.25-1.40 (m, 2H), 1.87 (m, 2H), 2.36 (m, 1H), 2.51 (s, 3H), 2.73 (m, 1H), 3.05 (m, 1H), 3.76 (m, 1 H), 4.09 (dd, 2H), 4.24 (d, 2H), 4.43 (m, 1H), 4.47 (t, 1H), 5.25 (s, 2H), 7.17 (d, 1H), 7.22 (d, 1H), 7.27 (d, 1H), 7.36 (d, 1H), 7.37 (d, 1H), 7.47 (dd, 1H), 7.75 (dd, 1H), 7.77 (dd, 1H), 8.19 (d, 1H), 8.55 (s, 1H), 9.95 (s, 1H); Mass spectrum MH⁺ 548.4, 550.4.

### Example 64

### 2-((2S)-2-{[(4-{[3-methyl-4-(pyridin-2-ylmethoxy)phenyl]amino}quinazolin-5-yl)oxy]methyl}pyrrolidin-1-yl)-2-oxoethanol

The procedure described in example 21 was repeated using 4-[(5-{[(2*S*)-1-glycoloylpyrrolidin-2-yl]methoxy} quinazolin-4-yl)amino]-2-methylphenol and 2-picolyl chloride hydrochloride to give the title compound as a white solid in 15% yield; NMR spectrum (DMSO) 1.98 (m, 4H), 2.27 (s, 3H), 3.38 (m, 2H), 4.02 (m, 2H), 4.21 (dd, 1H), 4.43 (dd, 1H), 4.55 (m, 2H), 5.20 (s, 2H), 7.01 (d, 1H), 7.22 (d, 1H), 7.29 (d, 1H), 7.33 (dd, 1H), 7.50 (m, 3H), 7.68 (t, 1H), 7.85 (td, 1H), 8.41 (s, 1H), 8.57 (d, 1H), 9.84 (s, 1H); Mass spectrum MH⁺ 500.

The 4-[(5-{[(2*S*)-1-glycoloylpyrrolidin-2-yl]methoxy}quinazolin-4-yl)amino]-2-methylphenol used as starting material was prepared as described in example 21 (preparation of starting materials) using 2-methyl-4-({5-[(2*S*)-pyrrolidin-2-ylmethoxy]quinazolin-4-yl}amino)phenol and glycolic acid in 88% yield; NMR spectrum (DMSO) 1.96 (m, 4H), 2.16 (s, 3H), 3.33 (m, 2H), 4.01 (d, 2H), 4.29 (m, 1H), 4.46 (dd, 1H), 4.57 (m, 1H), 6.83 (d, 1H), 7.23 (d, 1H), 7.31 (m, 2H), 7.50 (d, 1H), 7.94 (t, 1H), 8.72 (s, 1H), 9.53 (s, 1H), 10.91 (s, 1H); Mass spectrum MH⁺ 409.

The 2-methyl-4-({5-[(2*S*)-pyrrolidin-2-ylmethoxy]quinazolin-4-yl}amino)phenol used as starting material was prepared as described in example 21 (preparation of starting materials) using 2-methyl-4-[(5-fluoroquinazolin-4-yl)amino]phenol and *D*-prolinol in 92% yield; NMR spectrum (DMSO) 1.49 (m, 1H), 1.66 (m, 2H), 1.85 (m, 1H), 2.16 (s, 3H), 2.83 (t, 2H), 3.57 (m, 1H), 4.05 (dd, 1H), 4.26 (dd, 1H), 6.77 (d, 1H), 7.09 (d, 1H), 7.27 (d, 1H), 7.49 (m, 2H), 7.66 (t, 1H), 8.39 (s, 1H), 10.31 (s, 1H); Mass spectrum MH⁺ 351.

The 2-methyl-4-[(5-fluoroquinazolin-4-yl)amino]phenol used as starting material was prepared as described in example 21 (preparation of starting materials) using 4-chloro-5-fluoroquinazoline (prepared as described in example 1, preparation of starting materials) and 4-amino-2-methyl-phenol in 82% yield; NMR spectrum (DMSO-d6) 3.30 (s, 3H), 6.78 (d, 1H), 7.28 (m, 2H), 7:38 (dd, 1H), 7.57 (d, 1H), 7.78 (m, 1H), 8.43 (s, 1H), 8.88 (d, 1H), 9.22 (s, 1H); Mass spectrum MH⁺ 270.

### Example 65

### 2-((2S)-2-{[(4-{[3-methyl-4-(pyrazin-2-ylmethoxy)phenyl]amino}quinazolin-5-yl)oxy]methyl}pyrrolidin-1-yl)-2-oxoethanol

The procedure described in example 25 was repeated using 4-[(5-{[(2*S*)-1-glycoloylpyrrolidin-2-yl]methoxy}quinazolin-4-yl)amino]-2-methylphenol (obtained as described in example 64, preparation of starting materials) and pyrazin-2-ylmethanol to give the title compound as a pale yellow solid in 25% yield; NMR spectrum (DMSO) 1.96 (m, 4H), 2.24 (s, 3H), 3.37 (m, 2H), 3.98 (m, 2H), 4.18 (dd, 1H), 4.42 (dd, 1H), 4.49 (m, 2H), 5.25 (s, 2H), 7.03 (d, 1H), 7.20 (d, 1H), 7.27 (d, 1H), 7.46 (m, 2H), 7.67 (t, 1H), 8.39 (s, 1H), 8.61 (d, 1H), 8.65 (d, 1H), 8.82 (s, 1H), 9.83 (s, 1H): Mass spectrum MH⁺ 501.

### Example 66

### 2-((2R)-2-{[(4-{[3-methyl-4-(pyridin-2-ylmethoxy)phenyl]amino}quinazolin-5-yl)oxy]methyl}pyrrolidin-1-yl)-2-oxoethanol

The procedure described in example 21 was repeated using 4-[(5-{[(2*R*)-1-glycoloylpyrrolidin-2-yl]methoxy} quinazolin-4-yl)amino]-2-methylphenol and 2-picolyl chloride hydrochloride to give the title compound as a yellow solid in 35% yield; NMR spectrum (DMSO) 1.98 (m, 4H), 2.27 (s, 3H), 3.38 (m, 2H), 4.02 (m, 2H), 4.21 (dd, 1H), 4.43 (dd, 1H), 4.55 (m, 2H), 5.20 (s, 2H), 7.01 (d, 1H), 7.22 (d, 1H), 7.29 (d, 1H), 7.33 (dd, 1H), 7.50 (m, 3H), 7.68 (t, 1H), 7.85 (td, 1H), 8.41 (s, 1H), 8.57 (d, 1H), 9.84 (s, 1H); Mass spectrum MH⁺ 500.

The 4-[(5-{[(2*R*)-1-glycoloylpyrrolidin-2-yl]methoxy}quinazolin-4-yl)amino]-2-methylphenol used as starting material was prepared as described in example 21 (preparation of starting materials) using 2-methyl-4-({5-[(2*R*)-pyrrolidin-2-ylmethoxy]quinazolin-4-yl}amino)phenol and glycolic acid in 95% yield; NMR spectrum (DMSO) 1.96 (m, 4H), 2.16 (s, 3H), 3.33 (m, 2H), 4.01 (d, 2H), 4.29 (m, 1H), 4.46 (dd, 1H), 4.57 (m, 1H), 6.83 (d, 1H), 7.23 (d, 1H), 7.31 (m, 2H), 7.50 (d, 1H), 7.94 (t, 1H), 8.72 (s, 1H), 9.53 (s, 1H), 10.91 (s, 1H); Mass spectrum MH⁺ 409.

The 2-methyl-4-({5-[(2*R*)-pyrrolidin-2-ylmethoxy]quinazolin-4-yl}amino)phenol used as starting material was prepared as described in example 21 (preparation of starting materials) using 2-methyl-4-[(5-fluoroquinazolin-4-yl)amino]phenol (prepared as described in example 64, preparation of starting materials) and *L*-prolinol in 20% yield; NMR spectrum (DMSO) 1.49 (m, 1H), 1.66 (m, 2H), 1.85 (m, 1H), 2.16 (s, 3H), 2.83 (t, 2H), 3.57 (m, 1H), 4.05 (dd, 1H), 4.26 (dd, 1H), 6.77 (d, 1H), 7.09 (d, 1H), 7.27 (d, 1H), 7.49 (m, 2H), 7.66 (t, 1H), 8.39 (s, 1H), 10.31 (s, 1H); Mass spectrum MH⁺ 351.

### Example 67

### 2-((2R)-2-{[(4-{[3-methyl-4-(pyrazin-2-ylmethoxy)phenyl]amino}quinazolin-5-yl)oxy]methyl}pyrrolidin-1-yl)-2-oxoethanol

The procedure described in example 25 was repeated using 4-[(5-{[(2*R*)-1-glycoloylpyrrolidin-2-yl]methoxy} quinazolin-4-yl)amino]-2-methylphenol (obtained as described in example 66, preparation of starting materials) and pyrazin-2-ylmethanol to give the title compound as a white solid in 38% yield; NMR spectrum (DMSO) 1.96 (m, 4H), 2.24 (s, 3H), 3.37 (m, 2H), 3.98 (m, 2H), 4.18 (dd, 1H), 4.42 (dd, 1H), 4.49 (m, 2H), 5.25 (s, 2H), 7.03 (d, 1H), 7.20 (d, 1H), 7.27 (d, 1H), 7.46 (m, 2H), 7.67 (t, 1H), 8.39 (s, 1H), 8.61 (d, 1H), 8.65 (d, 1H), 8.82 (s, 1H), 9.83 (s, 1H); Mass spectrum MH⁺ 501.

### Example 68

### 2-((2R)-2-{[(4-{[3-methyl-4-(1,3-thiazol-4-ylmethozy)phenyl]amino}quinaxolin-5-yl)oxy]methyl}pyrrolidin-1-yl)-2-oxoethanol

The procedure described in example 21 was repeated using 4-[(5-{[(2*R*)-1-glycoloylpyrrolidin-2-yl]methoxy}quinazolin-4-yl)amino]-2-methylphenol (obtained as described in example 66, preparation of starting materials) and 4-(chloromethyl)thiazole hydrochloride to give the title compound as a yellow solid in 29% yield; NMR spectrum (DMSO) 1.99 (m, 4H), 2.21 (s, 3H), 3.37 (m, 2H), 4.02 (m, 2H), 4.21 (dd, 1H), 4.43 (dd, 1H), 4.55 (m, 2H), 5.24 (s, 2H), 7.09 (d, 1H), 7.24 (d, 1H), 7.32 (d, 1H), 7.47 (m, 2H), 7.67 (t, 1H), 7.77 (d, 1H), 8.41 (s, 1H), 9.13 (d, 1H), 9.84 (s, 1H); Mass spectrum MH⁺ 506.

### Example 69

### 2-[(2R)-2-({[4-({3-methyl-4-[(5-methylisoxazol-3-yl)methoxy]phenyl}amino)quinazolin-5-yl]oxy}methyl)pyrrolidin-1-yl]-2-oxoethanol

The procedure described in example 21 was repeated using 4-[(5-{[(2*R*)-1-glycoloylpyrrolidin-2-yl]methoxy}quinazolin4-yl)amino]-2-methylphenol (obtained as described in example 66, preparation of starting materials) and 3-(chloromethyl)-5-methylisoxazole to give the title compound as a pale yellow solid in 45% yield; NMR spectrum (DMSO) 1.98 (m, 4H), 2.19 (s, 3H), 2.41 (s, 3H), 3.37 (m, 2H), 4.02 (m, 2H), 4.19 (dd, 1H), 4.45 (dd, 1H), 4.56 (m, 2H), 5.16 (s, 2H), 6.35 (s, 1H), 7.06 (d, 1H), 7.24 (d, 1H), 7.31 (d, 1H), 7.47 (m, 2H), 7.68 (t, 1H), 8.42 (s, 1H), 9.84 (s, 1H); Mass spectrum MH⁺ 504.

### Example 70

### 5-{[(2R)-1-(methoxyacetyl)pyrrolidin-2-yl]methoxy}-N-{3-methyl-4-[(5-methylisoxazol-3-yl)methoxy]phenyl}quinazolin-4-amine

The procedure described in example 21 was repeated using 4-[(5-{[(2*R*)-1-(methoxyacetyl)pyrrolidin-2-yl]methoxy}quinazolin-4-yl)amino]-2-methylphenol and 3-(chloromethyl)-5-methylisoxazole to give the title compound as a white solid in 30% yield; NMR spectrum (DMSO) 1.98 (m, 4H), 2.18 (s, 3H), 2.42 (s, 3H), 3.26 (s, 3H), 3.41 (m, 2H), 4.01 (s, 2H), 4.18 (dd, 1H), 4.44 (dd, 1H), 4.55 (m, 1H), 5.16 (s, 2H), 6.35 (s, 1H), 7.05 (d, 1H), 7.22 (d, 1H), 7.32 (d, 1H), 7.49 (m, 2H), 7.67 (t, 1H), 8.41 (s, 1H), 9.83 (s, 1H); Mass spectrum MH⁺ 518.

The 4-[(5-{[(2*R*)-1-(methoxyacetyl)pyrrolidin-2-yl]methoxy}quinazolin-4-yl)amino]-2-methylphenol used as starting material was prepared as described in example 21 (preparation of starting materials) using 2-methyl-4-({5-[(2*R*)-pyrrolidin-2-ylinethoxy]quinazolin-4-yl}amino)phenol (obtained as described in example 66, preparation of starting materials) and methoxyacetic acid in 100% yield; NMR spectrum (DMSO) 1.93 (m, 4H), 2.17 (s, 3H), 3.24 (s, 3H), 3.41 (m, 2H), 3.98 (d, 1H), 4.02 (d, 1H), 4.27 (dd, 1H), 4.45 (dd, 1H), 4.57 (m, 1H), 6.82 (d, 1H), 7.24 (dd, 1H), 7.33 (m, 2H), 7.48 (d, 1H), 7.96 (t, 1H), 8.73 (s, 1H), 9.53 (s, 1H), 10.99 (s, 1H); Mass spectrum MH⁺ 422.

### Example 71

### 5-{[(2R)-1-(methoxyacetyl)pyrrolidin-2-yl]methoxy}-N-[3-methyl-4-(pyridin-2-ylmethoxy)phenyl]quinazolin-4-amine

The procedure described in example 21 was repeated using 4-[(5-{[(2*R*)-1-(methoxyacetyl)pyrrolidin-2-yl]methoxy}quinazolin-4-yl)amino]-2-methylphenol (obtained as described in example 70, preparation of starting materials) and 2-picolyl chloride hydrochloride to give the title compound as a yellow solid in 31% yield; NMR spectrum (DMSO) 1.98 (m, 4H), 2.26 (s, 3H), 3.25 (s, 3H), 3.40 (m, 2H), 4.02 (s, 2H), 4.18 (dd, 1H), 4.43 (dd, 1H), 4.56 (m, 1H), 5.20 (s, 2H), 7.00 (d, 1H), 7.21 (d, 1H), 7.30 (d, 1H), 7.34 (dd, 1H), 7.53 (m, 3H), 7.67 (t, 1H), 7.86 (td, 1H), 8.40 (s, 1H), 8.58 (d, 1H), 9.85 (s, 1H); Mass spectrum MH⁺ 514.

### Example 72

### 5-{[(2R)-1-(methoxyacetyl)pyrrolidin-2-yl]methoxy}-N-[3-methyl-4-(1,3-thiazol-4-ylmethoxy)phenyl]quinazolin-4-amine

The procedure described in example 21 was repeated using 4-[(5-{[(2*R*)-1-(methoxyacetyl)pyrrolidin-2-yl]methoxy}quinazolin-4-yl)amino]-2-methylphenol (obtained as described in example 70, preparation of starting materials) and 4-(chloromethyl)thiazole hydrochloride to give the title compound as a white solid in 27% yield; NMR spectrum (DMSO) 1.99 (m, 4H), 2.22 (s, 3H), 3.24 (s, 3H), 3.42 (m, 2H), 4.01 (s, 2H), 4.20 (dd, 1H), 4.42 (dd, 1H), 4.55 (m, 1H), 5.25 (s, 2H), 7.10 (d, 1H), 7.22 (d, 1H), 7.32 (d, 1H), 7.49 (m, 2H), 7.70 (t, 1H), 7.78 (d, 1H), 8.42 (s, 1H), 9.14 (d, 1H), 9.87 (s, 1H); Mass spectrum MH⁺ 520.

### Example 73

### 2-(2R)-2-{[(4-{[3-methyl-4-(pyridin-2-ylmethoxy)phenyl]amino}quinazolin-5-yl)oxy]methyl}piperidin-1-yl)-2-oxoethanol

The procedure described in example 21 was repeated using 4-[(5-{[(2*R*)-1-glycoloylpiperidin-2-yl]methoxy}quinazolin-4-yl)amino]-2-methylphenol and 2-picolyl chloride hydrochloride to give the title compound as a white solid in 44% yield; NMR spectrum (DMSO-d6, 100°C) 1.45 (m, 1H), 1.67 (m, 4H), 1.87 (m, 1H), 2.26 (s, 3H), 3.17 (m, 1H), 3.82 (m, 1H), 4.05 (m, 2H), 4.47 (dd, 1H), 4.63 (dd, 1H), 4.95 (m, 1H), 5.20 (s, 2H), 7.01 (d, 1H), 7.23 (d, 1H), 7.34 (m, 2H), 7.47 (m, 2H), 7.55 (d, 1H), 7.67 (t, 1H), 7.83 (td, 1H), 8.40 (s, 1H), 8.57 (d, 1H), 9.57 (s, 1H); Mass spectrum MH⁺ 514.

The 4-[(5-{[(2*R*)-1-glycoloylpiperidin-2-yl]methoxy}quinazolin-4-yl)amino]-2-methylphenol used as starting material was prepared as described in example 21 (preparation of starting materials) using 2-methyl-4-({5-[(2*R*)-piperidin-2-ylmethoxy]quinazolin-4-yl}amino)phenol and glycolic acid in 40% yield; NMR spectrum (DMSO-d6, 100°C); 1.45 (m, 1H), 1.66 (m, 4H), 1.86 (m, 1H), 2.17 (s, 3H), 3.10 (m, 1H), 3.80 (m, 1H), 4.04 (m, 2H), 4.46 (dd, 1H), 4.60 (dd, 1H), 4.94 (m, 1H), 6.77 (d, 1H), 7.21 (d, 1H), 7.31 (m, 3H), 7.66 (t, 1H), 8.37 (s, 1H), 8.77 (s, 1H), 9.47 (s, 1H); Mass spectrum MH⁺ 423.

The 2-methyl-4-({5-[(2*R*)-piperidin-2-ylmethoxy]quinazolin-4-yl}amino)phenol used as starting material was prepared as described in example 21 (preparation of starting materials) using 2-methyl-4-[(5-fluoroquinazolin-4-yl)amino]phenol (prepared as described in example 64, preparation of starting materials) and (2*R*)-piperidin-2-ylmethanol (prepared as described in example 7, preparation of starting materials) in 99% yield; NMR spectrum (DMSO) 1.30 (m, 3H), 1.48 (br d, 1H), 1.62 (br d, 1H), 1,77 (br d, 1H), 2.16 (s, 3H), 3.10 (m, 1H), 2.61 (br t, 1H), 3.00 (m, 2H), 4.11 (dd, 1H), 4.23 (dd, 1H), 6.75 (d, 1H), 7.04 (d, 1H), 7.26 (d; 1H), 7.48 (d, 1H), 7.62 (dd, 1H), 7.66 (t, 1H), 8.41 (s, 1H), 9.16 (s, 1H), 10.40 (s, 1H); Mass spectrum MH⁺ 365.

### Example 74

### 2-((3R)-3-{[(4-{[3-chloro-4-(pyridin-2-ylmethoxy)phenyl]amino}quinazolin-5-yl)oxy]methyl}piperidin-1-yl)-2-oxoethanol

HATU (121 mg, 0.32 mmol) was added to a solution of *N-*[3-chloro-4-(pyridin-2-ylmethoxy)phenyl]-5-[(3*R*)-piperidin-3-ylmethoxy]quinazolin-4-amine (150 mg, 0.316 mmol) and glycolic acid (26 mg, 0.32 mmol) in dry DMF (5 ml). The reaction was stirred at ambient temperature for 2 days. The reaction mixture was concentrated and the residue was stirred in water. The resultant precipitate was filtered and purified by chromatography eluting with 3 - 10% (10:1 MeOH / conc. NH_{3 (aq)}) in DCM to give a gum. This was triturated with ether to give the title compound as a solid (95 mg, 57%); NMR spectrum (DMSO-d6, 373K) 1.45 - 1.58 (m, 2H), 1.68 - 1.79 (m, 1H), 1.93 - 2.03 (m, 1H), 2.20 - 2.34 (m, 1H), 3.00 - 3.16 (m, 2H), 3.67 - 3.90 (m, 1H), 4.00 - 4.20 (m, 3H), 4.23 - 4.39 (m, 2H), 5.28 (s, 2H), 7.16 (d, 1H), 7.25 (d, 1H), 7.29 - 7.41 (m, 2H), 7.54 - 7.64 (m, 2H), 7.71 (t, 1H), 7.85 (t, 1H), 8.04 (s, 1H), 8.52 (s, 1H), 8.58 (d, 1H), 9.79 (s, 1H); Mass spectrum MH⁺ 534.

The starting *N*-[3-chloro-4-(pyridin-2-ylinethoxy)phenyl]-5-[(3*R*)-piperidin-3-ylmethoxy]quinazolin-4-amine was prepared as follows:
*tert*-Butyl (3*R*)-3-[({4-[(3-chloro-4-hydroxyphenyl)amino]quinazolin-5-yl}oxy)methyl]piperidine-1-carboxylate was prepared as described in example 21 (preparation of starting materials) using *tert*-butyl (3*R*)-3-(hydroxymethyl)piperidine-1-carboxylate and 2-chloro-4-[(5-fluoroquinazolin-4-yl)amino]phenol in quantitative yield; NMR spectrum (CDCl₃) 0.74 - 0.85 (m, 1H), 1.13 -1.60 (m, 11H), 1.62 -1.76 (m, 1H), 1.88 - 2.01 (m, 1H), 2.10 - 2.26 (m, 1H), 2.86 - 3.10 (m, 2H), 3.67 - 3.80 (m, 1H), 3.91- 4.19 (m, 3H), 6.84 (d, 1H), 6.97 (d, 1H), 7.25 - 7.37 (m, 1H), 7.51 (d, 1H), 7.59 (t, 1H), 7.88 (d, 1H), 8.55 (s, 1H), 9.83 (s, 1H); Mass spectrum MH⁺ 485.
*tert*-Butyl (3*R*)-3-{[(4-{[3-chloro-4-(pyridin-2-ylmethoxy)phenyl]amino}quinazolin-5-yl)oxy]methyl}piperidine-1-carboxylate was prepared as described in example 21 (preparation of starting materials) using *tert*-butyl (3*R*)-3-[({4-[(3-chloro-4-hydroxyphenyl)amino]quinazolin-5-yl}oxy)methyl]piperidine-1-carboxylate and 2-picolyl chloride hydrochloride in 76% yield; NMR spectrum (CDCl₃ 300MHz) 1.14 -1.59 (m, 10H), 1.60 -1.82 (m, 2H), 1.90 - 2.02 (m, 1H), 2.11 2.26 (m, 1H), 2.86 - 3.08 (m, 2H), 3.68 - 3.82 (m, 1H), 3.95 - 4.17 (m, 3H), 5.22 (s, 2H), 6.81 (d, 1H), 6.94 (d, 1H), 7.13 - 7.18 (m, 1H), 7.39 - 7.50 (m, 2H), 7.52 - 7.62 (m, 2H), 7.64 - 7.73 (m, 1H), 7.90 (d, 1H), 8.52 (d, 1H), 8.56 (s, 1H), 9.74 (s, 1H); Mass spectrum MH⁺ 576.

*N*-[3-chloro-4-(pyridin-2-ylmethoxy)phenyl]-5-[(3*R*)-piperidin-3-ylmethoxy]quinazolin-4-amine was prepared as described in example 29 (preparation of starting materials) using *tert*-butyl (3*R*)-3-{[(4-{[3-chloro-4-(pyridin-2-ylmethoxy)phenyl]amino}quinazolin-5-yl)oxy]methyl}piperidine-1-carboxylate in 82% yield); NMR spectrum (DMSO-d6) 1.30-1.42 (m, 1H), 1.47 -1.76 (m, 1H), 1.68 - 1.78 (m, 1H), 1.88 -1.97 (m, 1H), 2.24 - 2.36 (m, 1H), 2.58 - 2.70 (m, 2H), 3.04 (d, 2H, partially obscured by DMSO), 4.21- 4.31 (m, 2H), 5.31 (s, 2H), 7.18 (d, 1H), 7.28 (d, 1H), 7.34 - 7.40 (m, 2H), 7.54 - 7.61 (m, 2H), 7.75 (t, 1H) 7.86 - 7.93 (m, 1H), 8.10 - 8.14 (m, 1H), 8.54 (s, 1H), 8.61 (d, 1H), 9.92 (s, 1H); Mass spectrum MH⁺ 476.

### Example 75

### 2-((3R)-3-{[(4-{[3-chloro-4-(1,3-thiazol-4-ylmethoxy)phenyl]amino}quinazolin-5-yl)oxy]methyl}piperidin-1-yl)-2-oxoethanol

The procedure described in example 74 was repeated using *N*-[3-chloro-4-(1,3-thiazol-4-ylmethoxy)phenyl]-5-[(3*R*)-piperidin-3-ylmethoxy]quinazolin-4-amine and glycolic acid to give the title compound in 35% yield; NMR spectrum (DMSO-d6 373K) 1.46 -1.62 (m, 2H), 1.71 - 1.83 (m, 1H), 1.96 - 2.03 (m, 1H), 2.22 - 2.34 (m, 1H), 3.00 - 3.14 (m, 2H), 3.68 - 3.91 (m, 1H), 4.00 - 4.19 (m, 3H), 4.25 - 4.40 (m, 2H), 5.32 (s, 2H), 7.19 (d, 1H), 7.32 (d, 1H), 7.39 (d, 1H), 7.63 (d, 1H), 7.69 - 7.80 (m, 2H), 8.03 (s, 1H), 8.37 (s, 1H), 9.08 (s, 1H), 9.80 (s, 1H); Mass spectrum MH⁺ 540.

The starting *N*-[3-chloro-4-(1,3-thiazol-4-ylmethoxy)phenyl]-5-[(3*R*)-piperidin-3-ylmethoxy]quinazolin-4-amine was prepared as follows:

*tert*-Butyl (3*R*)-3-{[(4-{[3-chloro-4-(1,3-thiazol-4-ylmethoxy)phenyl]amino}quinazolin-5-yl)oxy]methyl}piperidine-1-carboxylate was prepared as described in example 21 (preparation of starting materials) using *tert*-butyl (3*R*)-3-[({4-[(3-chloro-4-hydroxyphenyl)amino]quinazolin-5-yl}oxy)methyl]piperidine-1-carboxylate (obtained as described in example 74, preparation of starting materials) and 4-(chloromethyl)-1,3-thiazole. hydrochloride in 87% yield; NMR spectrum (CDCl₃) 1.14 - 1.60 (m, 10H), 1.61 -1.81 (m, 2H), 1.91- 2.04 (m, 1H), 2.11 2.27 (m, 1H), 2.87 - 3.12 (m, 2H), 3.68 - 3.83 (m, 1H), 3.92 - 4.19 (m, 3H), 5.29 (s, 2H), 6.82 (d, 1H), 6.99 (d, 1H), 7.3 8- 7.53 (m, 3H), 7.57 (t, 1H), 7.89 (d, 1H), 8.57 (d, 1H), 8.77 (d, 1H), 9.76 (s, 1H); Mass spectrum MH⁺ 582.

*N*-[3-chloro-4-(1,3-thiazol-4-ylmethoxy)phenyl]-5-[(3*R*)-piperidin-3-ylmethoxy]quinazolin-4-amine was prepared as described in example 29 (preparation of starting materials) using *tert*-butyl (3*R*)-3-{[(4-{[3-chloro-4-(pyridin-2-ylmethoxy)phenyl]ammo}quinazolin-5-yl)oxy]methyl}piperidine-1-carboxylate in 9 1 % yield; NMR spectrum (DMSO-d6) 1.32 -1.47 (m, 1H), 1.53 - 1.69 (m, 1H), 1.73 -1.84 (m, 1H), 1.89 - 2.01 (m, 1H), 2.31 - 2.44 (m, 1H), 2.72 (q, 2H), 3.13 (d, 1H), 3.30 (d, 1H), 4.22 - 4.36 (m, 2H), 5.35 (s, 2H), 7.18 (d, 1H), 7.32 - 7.44 (m, 1H), 7.59 (dd, 1H), 7.75 (t, 1H), 7.82(s, 1H), 8.07 (dd, 1H), 8.54 (s, 1H), 9.16 (dd, 1H), 9.87 (s, 1H); Mass spectrum MH⁺ 482.

### Example 76

### 2-((3R)-3-({[4-({3-chloro-4-[(5-methylisozazol-3-yl)methozy]phenyl}amino)quinazolin-5-yl]oxy}methyl)piperidin-1-yl]-2-oxoethanol

The procedure described in example 74 was repeated using *N*-{3-chloro-4-[(5-methylisoxazol-3-yl)methoxy]phenyl}-5-[(3*R*)-piperidin-3-ylmethoxy]quinazolin-4-amine and glycolic acid to give the title compound in 44% yield; NMR spectrum (DMSO-d6, 373K) 1.45 -1.60 (m, 2H), 1.71 - 1.81 (m, 1H), 1.94 - 2.04 (m, 1H), 2.21- 2.33 (m, 1H), 2.43 (s, 3H), 3.00 - 3.14 (m, 2H), 3.71- 3.88 (m, 1H), 4.00 - 4.18 (m, 3H), 4.24 - 4.37 (m, 2H), 5.21 (s, 2H), 6.31 (s, 1H), 7.16 (d, 1H), 7.27 (d, 1H), 7.37 (d, 1H), 7.61 (d, 1H), 7.71 (t, 1H), 8.04 (s 1H), 8.51 (s, 1H), 8.81 (s, 1H); Mass spectrum MH⁺ 538.

The starting *N*-{3-chloro-4-[(5-methylisoxazol-3-yl)methoxy]phenyl}-5-[(3*R*)-piperidin-3-ylmethoxy]quinazolin-4-amine was prepared as follows:

*tert*-Butyl (3*R*)-3-({[4-({3-chloro-4-[(5-methylisoxazol-3-yl)methoxy]phenyl}amino)quinazolin-5-yl]oxy}methyl)piperidine-1-carboxylate was prepared as described in example 21 (preparation of starting materials) using *tert*-butyl (3*R*)-3-[({4-[(3-chloro-4-hydroxyphenyl)amino]quinazolin-5-yl}oxy)methyl]piperidine-1-carboxylate (obtained as described in example 74, preparation of starting materials) and 3-(chloromethyl)-5-methylisoxazole in 90% yield; NMR spectrum (CDCl₃ 300MHz) 1.14 -1.59 (m, 10H), 1.60 -1.81 (m, 2H), 1.90 - 2.03 (m, 1H), 2.11- 2.24 (m, 1H), 2.37 (s, 3H), 2.86 - 3.11 (m, 2H), 3.68 - 3.82 (m, 1H), 3.94 - 4.17 (m, 3H), 5.12 (s, 2H), 6.12 (s, 1H), 6.81 (d, 1H), 6.98 (d, 1H), 7.37 - 7.50 (m, 2H), 7.57 (t, 1H), 7.91 (d, 1H), 8.56 (s, 1H), 9.75 (s, 1H); ,Mass spectrum MH⁺ 580.

*N*-{3-chloro-4-[(5-methylisoxazol-3-yl)methoxy]phenyl}-5-[(3*R*)-piperidin-3-ylmethoxy]quinazolin-4-amine was prepared as described in example 29 (preparation of starting materials) using *tert*-butyl (3*R*)-3-({[4-({3-chloro-4-[(5-methylisoxazol-3-yl)methoxy]phenyl}amino)quinazolin-5-yl]oxy}methyl)piperidine-1-carboxylate in 91% yield; NMR spectrum (DMSO-d6) 1.32 -1.47 (m, 1H), 1.56 -1.72 (m, 1H), 1.76 - 1.87 (m, 1H), 1.90 - 2.02 (m, 1H), 2.44 (s, 3H), 2.67 - 2.85 (m, 2H), 3.18 (d, 1H), 3.33 (d, 1H), 4.23 - 4.37 (m, 2H), 5.28 (s, 2H), 6.36 (s, 1H), 7.19 (d, 1H), 7.76 (t, 1H), 8.09 (d, 1H), 8.54 (s, 1H), 9.85 (s, 1H); Mass spectrum MH⁺ 480.

### Example 77

### 2-((3S)-3-{[(4-{[3-chloro-4-(pyridin-2-ylmethoxy)phenyl]amino}quinazolin-5-yl)oxy]methyl}piperidin-1-yl)-2-oxoethanol

The procedure described in example 74 was repeated using of *N*-[3-chloro-4-(pyridin-2-ylmethoxy)phenyl]-5-[(3*S*)-piperidin-3-ylmethoxy]quinazolin-4-amine and glycolic acid to give the title compound in 60% yield; NMR spectrum (DMSO-d6, 373K) 1.45 - 1.58 (m, 2H), 1.68 -1.79 (m, 1H), 1.93 - 2.03 (m, 1H), 2.20 - 2.34 (m, 1H), 3.00 - 3.16 (m, 2H), 3.67 - 3.90 (m, 1H), 4.00 - 4.20 (m, 3H), 4.23 - 4.39 (m, 2H), 5.28 (s, 2H), 7.16 (d, 1H), 7.25 (d, 1H), 7.29 - 7.41 (m, 2H), 7.54 - 7.64 (m, 2H), 7.71 (t, 1H), 7.85 (t, 1H), 8.04 (s, 1H), 8.52 (s, 1H), 8.58 (d, 1H), 9.79 (s, 1H): Mass spectrum MH⁺ 534.

The starting *N*-[3-chloro-4-(pyridin-2-ylmethoxy)phenyl]-5-[(3*S*)-piperidin-3-ylmethoxy]quinazolin-4-amine was prepared as follows:

*tert*-Butyl (3*S*)-3-[({4-[(3-chloro-4-hydroxyphenyl)amino]quinazolin-5-yl}oxy)methyl]piperidine-1-carboxylate used was prepared as described in example 21 (preparation of starting materials) using *tert*-butyl (3*S*)-3-(hydroxymethyl)piperidine-1-carboxylate and 2-chloro-4-[(5-fluoroquinazolin-4-yl)amino]phenol in quantitative yield; NMR spectrum (CDCl₃) 0.74 - 0.85 (m, 1H), 1.13 - 1.60 (m, 11 H), 1.62 - 1.76 (m, 1H), 1.88 - 2.01 (m, 1H), 2.10 - 2.26 (m, 1H), 2.86 - 3.10 (m, 2H), 3.67 - 3.80 (m, 1H), 3.91- 4.19 (m, 3H), 6.84 (d, 1H), 6.97 (d, 1H), 7.25 - 7.37 (m, 1H), 7.51 (d, 1H), 7.59 (t, 1H), 7.88 (d, 1H), 8.55 (s, 1H), 9.83 (s, 1H); Mass spectrum MH⁺ 485.

*tert*-Butyl (3*S*)-3-{[(4-{[3-chloro-4-(pyridin-2-ylmethoxy)phenyl]amino}quinazolin-5-yl)oxy]methyl}piperidine-1-carboxylate was prepared as described in example 21 (preparation of starting materials) using *tert*-butyl (3*S*)-3-[({4-[(3-chloro-4-hydroxyphenyl)amino]quinazolin-5-yl}oxy)methyl]piperidine-1-carboxylate and 2-picolyl chloride hydrochloride in 76% yield; NMR spectrum (CDCl₃) 1.14 - 1.59 (m, 10H), 1.60 - 1.82 (m, 2H), 1.90 - 2.02 (m, 1H), 2.11 2.26 (m, 1H), 2.86 - 3.08 (m, 2H), 3.68 - 3.82 (m, 1H), 3.95 - 4.17 (m, 3H), 5.22 (s, 2H), 6.81 (d, 1 H), 6.94 (d, 1H), 7.13 - 7.18 (m, 1H), 7.39 - 7.50 (m, 2H), 7.52 - 7.62 (m, 2H), 7.64 - 7.73 (m, 1H), 7.90 (d, 1H), 8.52 (d, 1H), 8.56 (s, 1H), 9.74 (s, 1H); Mass spectrum MH⁺ 576.

*N*-[3-chloro-4-(pyridin-2-ylmethoxy)phenyl]-5-[(3*S*)-piperidin-3-ylmethoxy]quinazolin-4-amine was prepared as described in example 29 (preparation of starting materials) using *tert*-butyl (3*S*)-3-{[(4-{[3-chloro-4-(pyridin-2-ylmethoxy)phenyl]amino}quinazolin-5-yl)oxy]methyl}piperidine-1-carboxylate in 82% yield; NMR spectrum (DMSO-d6) 1.30 - 1.42 (m, 1H), 1.47 -1.76 (m, 1H), 1.68 -1.78 (m, 1H), 1.88 -1.97 (m, 1H), 2.24 - 2.36 (m, 1H), 2.58 - 2.70 (m, 2H), 3.04 (d, 2H, partially obscured by DMSO), 4.21- 4.31 (m, 2H), 5.31 (s, 2H), 7.18 (d, 1 H), 7.28 (d, 1H), 7.34 - 7.40 (m, 2H), 7.54 - 7.61 (m, 2H), 7.75 (t, 1H) 7.86 - 7.93 (m, 1H), 8.10 - 8.14 (m, 1H), 8.54 (s, 1H), 8.61 (d, 1H), 9.92 (s, 1H); Mass spectrum MH⁺ 476.

### Example 78

### 2-((3S)-3-{[(4-{[3-chloro-4-(1,3-thiazol-4-ylmethoxy)phenyl]amino}quinazolin-5-yl)oxy]methyl}piperidin-1-yl)-2-oxoethanol

The procedure described in example 74 was repeated using *N*-[3-chloro-4-(1,3-thiazol-4-ylmethoxy)phenyl]-5-[(3*S*)-piperidin-3-ylmethoxy]quinazolin-4-amine and glycolic acid to give the title compound in 35% yield; NMR spectrum (DMSO-d6 373K) 1.46 -1.62 (m, 2H), 1.71 - 1.83 (m, 1H), 1.96 - 2.03 (m, 1H), 2.22 - 2.34 (m, 1H), 3.00 - 3.14 (m, 2H), 3.68 - 3.91 (m, 1H), 4.00 - 4.19 (m, 3H), 4.25 - 4.40 (m, 2H), 5.32 (s, 2H), 7.19 (d, 1H), 7.32 (d, 1H), 7.39 (d, 1H), 7.63 (d, 1H), 7.69 - 7.80 (m, 2H), 8.03 (s, 1H), 8.37 (s, 1H), 9.08 (s, 1H), 9.80 (s, 1H); Mass spectrum MH⁺ 540.

The starting *N*-[3-chloro-4-(1,3-thiazol-4-ylmethoxy)phenyl]-5-[(3*R*)-piperidin-3-ylmethoxy]quinazolin-4-amine was prepared as follows:

*tert*-Butyl (3*S*)-3-{[(4-{[3-chloro-4-(1,3-thiazol-4-ylmethoxy)phenyl]amino}quinazolin-5-yl)oxy]methyl}piperidine-1-carboxylate was prepared as described in example 21 (preparation of starting materials) using *tert*-butyl (3*S*)-3-[({4-[(3-chloro-4-hydroxyphenyl)amino]quinazolin-5-yl}oxy)methyl]piperidine-1-carboxylate (obtained as described in example 77, preparation of starting materials) and 4-(chloromethyl)-1,3-thiazale hydrochloride in 87% yield; NMR spectrum (CDCl₃) 1.14 - 1.60 (m, 10H), 1.61 -1.81 (m, 2H), 1.91 - 2.04 (m, 1H), 2.11 2.27 (m, 1H), 2.87 - 3.12 (m, 2H), 3.68 - 3.83 (m, 1H), 3.92 - 4.19 (m, 3H), 5.29 (s, 2H), 6.82 (d, 1H), 6.99 (d, 1H), 7.3 8- 7.53 (m, 3H), 7.57 (t, 1H), 7.89 (d, 1H), 8.57 (d, 1H), 8.77 (d, 1H), 9.76 (s, 1H); Mass spectrum MH⁺ 582.

*N*-[3-chloro-4-(1,3-thiazol-4-ylmethoxy)phenyl]-5-[(3*S*)-piperidin-3-ylmethoxy]quinazolin-4-amine was prepared as described in example 29 (preparation of starting materials) using *tert*-butyl (3*S*)-3-{[(4-{[3-chloro-4-(pyridin-2-ylmethoxy)phenyl]amino}quinazolin-5-yl)oxy]methyl}piperidine-1-carboxylate in 91% yield; NMR spectrum (DMSO-d6) 1.32 -1.47 (m, 1H), 1.53 -1.69 (m, 1H), 1.73 -1.84 (m, 1H), 1.89 - 2.01 (m, 1H), 2.31- 2.44 (m, 1H), 2.72 (q, 2H), 3.13 (d, 1H), 3.30 (d, 1H), 4.22 - 4.36 (m, 2H), 5.35 (s, 2H), 7.18 (d, 1H), 7.32-7.44 (m, 1H), 7.59 (dd, 1H), 7.75 (t, 1H), 7.82(s, 1H), 8.07 (dd, 1H), 8.54 (s, 1H), 9.16 (dd, 1H), 9.87 (s, 1H); Mass spectrum MH⁺ 482.

### Example 79

### 2-(3R)-3-{[(4-{[3-chloro-4-(pyridin-2-ylmethoxy)phenyl]amino}quinazolin-5-yl)oxy]methyl}pyrrolidin-1-yl)-2-oxoethanol

*N*-[3-chloro-4-(pyridin-2-ylmethoxy)phenyl]-5-[(3*R*)-pyrrolidin-3-ylmethoxy]quinazolin-4-amine (150 mg, 0.33 mmol) and glycolic acid (31mg, 0.41 mmol) were dissolved in DMF (5 ml). HATU (149 mg, 0.42 mmol) was added and the resulting yellow solution stirred at 25°C for 18 hours. The solvent was removed *in vacuo,* water (20 ml) and DCM (20 ml) were added. The suspension was sonicated before filtering the solid. This was washed well with water and dried under vacuum to give the title compound as a yellow solid (160 mg, 95% yield). NMR spectrum (DMSO-d6) 1.70-1.90 (m, 1H), 2.00-2.20 (m, 1H), 2.90-3.00 (m, 1H), 3.30-3.60 (m, 4H), 4.00 (d, 2H), 4.40 (m, 2H), 5.40 (s, 2H), 7.30-7.50 (m, 5H), 7.60 (d, 1H), 7.80-8.00 (m, 3H), 8.60 (d, 1H), 8.80 (s, 1H), 10.66 (br s, 1H); Mass spectrum MH⁺ 520.

The *N*-[3-chloro-4-(pyridin-2-ylmethoxy)phenyl]-5-[(3*R*)-pyrrolidin-3-ylmethoxy]quinazolin-4-amine used as starting material was prepared as follows:

Trifluoroacetic acid (10 ml) was added to *tert*-butyl (3*R*)-3-(hydroxymethyl)pyrrolidine-1-carboxylate (1.00 g) dissolved in DCM (10 ml). The resulting solution was stirred for 1 hour and the solvent removed *in vacuo* to give a pale brown oil. This was dissolved in MeOH and MP-Carbonate resin (5.00 g) was added to give a basic methanolic solution. The mixture was stirred for 1 hour, filtered, the resin washed and the filtrate evaporated to give (3*R*)-pyrrolidin-3-ylmethanol as an orange oil (400 mg, 80%). NMR spectrum (DMSO-d6) 1.60 (m, 1H), 1.90 (m, 1H), 2.30 (m, 1H), 2.90 (m, 1H), 3.00-3.40 (m, 5H), 4.80 (m, 1H), 8.80 (br s, 1H).

Sodium hydride (60% dispersion in mineral oil, 0.32 g) was added to (3*R*)-pyrrolidin-3-ylmethanol (0.33 g) and *N*-[3-chloro-4-(pyridin-2-ylmethoxy)phenyl]-5-fluoroquinazolin-4-amine (obtained as described in example 1, preparation of starting materials, 0.50 g) in DMA (4 ml) and the reaction heated at 120°C for 4 hours. The reaction was cooled, quenched with water, and concentrated *in vacuo.* The residue was purified by chromatography using DCM - 5% methanol / 7N ammonia as eluent to give *N*-[3-chloro-4-(pyridin-2-ylmethoxy)phenyl]-5-[(3*R*)-pyrrolidin-3-ylmethoxy]quinazolin-4-amine as a white solid (330 mg, 54%). NMR spectrum (DMSO-d6) 1.60 (m, 1H), 1.90 (m, 1H), 2.70-3.00 (m, 5H), 4.20 (m, 1H), 5.30 (s, 2H), 7.10 (d, 1H), 7.25 (d, 1H), 7.40 (m, 2H), 7.50 (d, 1H), 7.60 (d, 1H), 7.70 (t, 1H), 7.90 (t, 1H), 8.10 (m, 1H), 8.50 (s, 1H), 8.60 (d, 1H), 10.00 (br s, 1H); Mass spectrum M⁺ 462.

### Example 80

### N-[3-chloro4-(pyridin-2-ylmethoxy)phenyl]-5-({(3R)-1-[(dimethylamino)acetyl]pyrrolidin-3-yl}methoxy)quinazolin-4-amine

The procedure described in example 79 was repeated using *N*-[3-chloro-4-(pyridin-2-ylmethoxy)phenyl]-5-[(3*R*)-pyrrolidin-3-ylmethoxy]quinazolin-4-amine (60 mg, 0.13 mmol), HATU (64 mg, 0.17 mmol), and *N*,*N*-dimethylglycine (17.4 mg, 0.17 mmol) to give the title compound as a solid (51 mg, 79 %). NMR spectrum (DMSO-d6) 1.80-2.00 (m, 1H). 2.00-2.25 (m, 1H), 2.75 (s, 3H), 2.85 (s, 3H), 3.20-3.50 (m, 5H), 4.10 (d, 2H); 4.40 (d, 2H), 5.30 (s, 2H), 7.30 (m, 2H), 7.35 (m, 2H), 7.50 (m, 2H), 7.70 (t, 2H), 8.00 (m, 1H), 8.60 (d, 1H), 8.70 (d, 1H), 10.25 (br s, 1H); Mass spectrum MH⁺ 547.

### Example 81

### N-[3-chloro-4-(pyridin-2-ylmethoay)phenyl]-5-{[(3R)-1-(methozyacetyl)pyrrolidin-3-yl]methoxy}quinazolin4-amine

The procedure described in example 79 was repeated using *N*-[3-chloro-4-(pyridin-2-ylmethoxy)phenyl]-5-[(3*R*)-pymolidin-3-ylmethoxy]quinazolin-4-amine (60 mg, 0.13 mmol), HATU (64 mg, 0.17 mmol), and methoxyacetic acid (13 ul, 0.17 mmol) to give the title compound as a solid (37 mg, 63 %). NMR spectrum (DMSO-d6) 1.80-2.00 (m, 1H). 2.00-2.25 (m, 1H), 3.00 (m, 1H), 3.30 (s, 3H), 3.30-3.60 (m, 4H), 3.80 (d, 2H), 4.40 (m, 2H), 5.40 (s, 2H), 7.30-7.50 (m, 5H), 7.60 (d, 1H), 7.90 (m, 2H), 8.00 (t, 1H), 8.60 (d, 1H), 8.90 (s, 1H), 10.70 (br s, 1H); Mass spectrum MH⁺ 534.

### Example 82

### 2-((3S)-3-{[(4-{[3-chloro-4-(pyridin-2-ylmethoxy)phenyl]amino}quinazolin-5-yl)oxy]methyl}pyrrolidin-1-yl)-2-ozoethanol

The procedure described in example 79 was repeated using *N*-[3-chloro-4-(pyridin-2-ylmethoxy)phenyl]-5-[(3*S*)-pyrrolidin-3-ylmethoxy]quinazolin-4-amine (107 mg, 0.23 mmol), HATU (109 mg, 0.29 mmol), and glycolic acid (22 mg, 0.29 mmol) to give the title compound as a solid (58 mg, 48%). NMR spectrum (DMSO-d6) 1.75-2.00 (m, 1H). 2.00-2.20 (m, 1H), 3.00 (m, 1H), 3.30-3.70 (m, 4H), 4.00 (d, 2H), 4.40 (m, 2H), 5.40 (s, 2H), 7.30 7.50 (m, 5H), 7.60 (d, 1H), 7.90 (m, 2H), 8.00 (t, 1H), 8.60 (d, 1H), 8.90 (s, 1H), 10.70 (br s, 1H); Mass spectrum MH⁺ 520.

The *N*-[3-chloro-4-(pyridin-2-ylmethoxy)phenyl]-5-[(3*S*)-pyrrolidin-3-ylmethoxy]quinazolin-4-amine used as starting material was prepared as follows:

Sodium hydride (60% dispersion in mineral oil, 0.48 g) was added to (3*S*)-pyrrolidin-3-ylmethanol (obtained as described for the R-antipode in example 79, preparation of starting materials, 0.50 g) and *N*-[3-chloro-4-(pyridin-2-ylmethoxy)phenyl]-5-fluoroquinazolin-4-amine (0.75 g) in DMA (10 ml) and the reaction heated at 90°C for 2 hours. The reaction was cooled, quenched with water, filtered and dried under vacuum to afford *N*-[3-chloro-4-(pyridin-2-ylmethoxy)phenyl]-5-[(3*S*)-pyrrolidin-3-ylmethoxy]quinazolin-4-amine as a white solid (910 mg, 100%). NMR spectrum (DMSO-d6) 1.60 (m, 1H), 1.90 (m, 1H), 2.70-3.00 (m, 5H), 4.20 (d, 1H), 5.30 (s, 2H), 7.10 (d, 1H), 7.25 (d, 1H), 7.40 (m, 2H), 7.50 (dd, 1H), 7.60 (d, 1H), 7.70 (t, 1H), 7.90 (t, 1H), 8.10 (m, 1H), 8.50 (s, 1H), 8.60 (d, 1H), 10.00 (br s, 1H); Mass spectrum M⁺ 462.

### Example 83

### N-[3-chloro-4-(pyridin-2-ylmethoxy)phenyl]-5-({(3S)-1-[(dimethylamino)acetyl]pyrrolidin-3-yl}methoxy)quinazolin-4-amine

The procedure described in example 79 was repeated using *N*-[3-chloro-4-(pyridin-2-ylmethoxy)phenyl]-5-[(3*S*)-pyrrolidin-3-ylmethoxy]quinazolin4-amine (obtained as described in example 82, preparation of starting materials, 107 mg, 0.23 mmol), HATU (109 mg, 0.29 mmol), and *N,N*-dimetliylglycine (30 mg, 0.29 mmol) to give the title compound as a solid (64 mg, 50 %). NMR spectrum (DMSO-d6) 1.75-2.00 (m, 1H), 2.00-2.20 (m, 1H), 2.80 (d, 6H), 3.10 (m, 1H), 3.20-3.70 (m, 4H), 4.10 (d, 2H), 4.40 (m, 2H), 5.30 (s, 2H), 7.30-7.40 (m, 4H), 7.50-7.60 (m, 2H), 7.80-8.00 (m, 3H), 8.60 (d, 1H), 8.80 (s, 1H), 10.70 (br s, 1H); Mass spectrum MH⁺ 547.

### Example 84

### (N-[3-chloro-4-(pyridin-2-ylmethoxy)phenyl]-5-{[(3S)-1-(methoxyacetyl)pyrrolidin-3-yl]methoxy}quinazolin-4-amine

The procedure described in Example 79 was repeated using *N*-[3-chloro-4-(pyridin-2-ylmethoxy)phenyl]-5-[(3*S*)-pyrrolidin-3-ylmethoxy]quinazolin-4-amine (obtained as described in example 82, preparation of starting materials, 107 mg, 0.23 mmol), HATU (109 mg, 0.29 mmol), and 2-methoxyacetic acid (22 ul, 0.29 mmol) to give the title compound as a solid (85 mg, 69 %). NMR spectrum (DMSO-d6) 1.70-2.00 (m, 1H), 2.00-2.20 (m, 1H), 2.90 (m, 1H), 3.30 (s, 3H), 3.35-3.60 (m, 4H), 3.95 (d, 2H), 4.40 (m, 2H), 5.40 (s, 2H), 7.30 -7.50 (m, 5H), 7.60 (d, 1H), 7.90 (m, 2H), 8.00 (t, 1H), 8.60 (d, 1H), 8.85 (s, 1H), 10.72 (br s, 1H); Mass spectrum MH⁺ 534.

### Example 85

### N-[3-chloro-4-(pyridin-2-ylmethoxy)phenyl]-5-({(2R)4-[(dimethylamino)acetyl]morpholin-2-yl}methoxy)quinazolin-4-amine

The procedure described in example 79 was repeated using *N*-[3-chloro-4-(pyridin-2-ylinethoxy)phenyl]-5-[(2R)-morpholin-2-ylmethoxy]quinazolin-4-amine (100 mg, 0.21 mmol), HATU (104 mg, 0.28 mmol), and *N,N*-dimethylglycine (28 mg, 0.28 mmol) to give the title compound as a solid (48 mg, 41 %); NMR spectrum (DMSO-d6) 2.80 (s, 6H), 3.20-3.80 (m, 4H), 3.90 - 4.20 (m, 4H), 4.40 (m, 2H), 4.60 (m, 1H), 5.30 (s, 2H), 7.20 - 7.40 (m, 4H), 7.60 (d, 1H), 7.70 (t, 1H), 7.80 - 8.00 (m, 3H), 8.60 (d, 1H), 8.80 (s, 1H), 10.50-10.60 (br s, 1H); Mass spectrum MH⁺ 563

The *N*-[3-chloro-4-(pyridin-2-ylmethoxy)phenyl]-5-[(2R)-morpholin-2-ylmethoxy]quinazolin-4-amine used as starting material was prepared as follows:
Sodium hydride (60% dispersion in mineral oil, 1.01 g) was added to (2*R*)-morpholin-2-ylmethanol ((obtained as described in Journal of Medicinal Chemistry 1998, 41(11), 1934-1942, 0.50 g) and *N*-[3-chloro-4-(pyridin-2-ylmethoxy)phenyl]-5-fluoroquinazolin-4-amine (1.00 g) in DMA (20 ml) and the reaction heated at 110°C for 4 hours. The reaction was cooled, quenched with saturated NH₄Cl and filtered to give a solid. This was slurried in MeCN and stirred for 10 min. before filtration to give *N*-[3-chloro-4-(pyridin-2-ylmethoxy)phenyl]-5-[(2*R*)-morpholin-2-ylmethoxy]quinazolin-4-amine as a yellow solid (585 mg, 46%). NMR spectrum (DMSO-d6) 2.50-2.80 (m, 3H), 2.90 (d, 1H), 3.60 (dt, 1H), 3.80 (d, 1H), 4.00 (m, 1H), 4.20 (t, 1H), 4.40 (dd, 1H), 5.30 (s, 2H), 7.10 (d, 1H), 7.25-7.40 (m, 3H), 7.60 (d, 2H), 7.70 (m, 2H), 7.90 (t, 1H), 8.00 (d, 1H), 8.50 (s, 1H), 8.60 (d, 1H), 10.25 (br s, 1H); Mass spectrum MH⁺ 478.

### Example 86

### 2-((2R)-2-{[(4-{[3-chloro-4-(pyridin-2-ylmethoxy)phenyl]amino}quinazolin-5-yl)oxy]methyl}morpholin-4-yl)-2-oxoethanol

The procedure described in example 79 was repeated using *N*-[3-chloro-4-(pyridin-2-ylmethoxy)phenyl]-5-[(2*R*)-morpholin-2-ylmethoxy]quinazolin-4-amine (obtained as described in example 85, preparation of starting materials, 100 mg, 0.21 mmol), HATU (104 mg, 0.28 mmol), and glycolic acid (21 mg, 0.28 mmol) to give the title compound as a solid (32 mg, 29 %); NMR spectrum (DMSO-d6) 2.80 (m, 1H), 3.10 (m, 1H), 3.40 (m, 1H), 3.90-4.40 (m, 6H), 4.50 (m, 1H), 4.60 (m, 1H), 5.30 (s, 2H), 7.10 (d, 1H), 7.30 (d, 1H), 7.40 (d, 1H), 7.60 (d, 1H), 7.65 - 7.80 (m, 2H), 7.85 (t, 1H), 8.00 (d, 1H), 8.60 (m, 2H), 10.18 (br s, 1H); Mass spectrum MH⁺ 536.

### Example 87

### 2-((2S)-2-{[(4-{[3-Chloro-4-(pyridin-2-ylmethoxy)phenyl]amino}quinazolin-5-yl)oxy]methyl}morpholin-4-yl)-2-oxoethanol trifluoroacetate

The procedure described in example 1 was repeated using glycolic acid and *N*-[3-chloro-4-(pyridin-2-ylinethoxy)phenyl]-5-[(2*S*)-morpholin-2-ylmethoxy]quinazolin-4-amine, but purifying the product by preparative HPLC, to give the title compound as a yellow solid in 48% yield; NMR spectrum (DMSO-d6) 2.75 - 2.85 (m, 1H), 3.05 - 3.2 (m, 1H), 3.5 - 3.65 (m, 1H), 3.75 - 3.9 (m, 2H), 4.0 - 4.2 (m, 3H), 4.25 - 4.4 (m, 2H), 4.55 - 4.7 (m, 1H), 5.3 (s, 2H), 7.35 - 7.5 (m, 4H), 7.55 - 7.7 (m, 2H), 7.85 - 8.05 (m, 3H), 8.6 (d, 1H), 8.9 (s, 1H); 11.8 (br s, 1H); Mass spectrum MH⁺ 536.5.

The *N*-[3-chloro-4-(pyridin-2-ylmethoxy)phenyl]-5-[(2*S*)-morpholin-2-ylmethoxy]quinazolin-4-amine used as starting material was prepared as described in example 1 (preparation of starting materials) using (2*S*)-morpholin-2-ylmethanol (obtained as described in Journal of Medicinal Chemistry 1998, 41(11), 1934-1942) and *N*-[3-chloro-4-(pyridin-2-ylinethoxy)phenyl]-5-fluoroquinazolin-4-amine as a solid in 46% yield; NMR spectrum (DMSO-d6) 1.47 (m, 1H), 1.69 (m, 2H), 1.86 (m, 1H), 2.85 (m, 2H), 3.53 (m, 1H), 4.05 (dt, 1H), 4.31 (dd, 1H), 5.27 (s, 2H), 7.12 (d, 1H), 7.23 (d, 1H), 7.31 (d, 1H), 7.35 (dd, 1H), 7.57 (d, 1H), 7.70 (t, 1H), 7.79 (dd, 1H), 7.86 (dt, 1H), 8.14 (d, 1H), 8.50 (s, 1H), 8.58 (d, 1H), 10.55 (br s, 1H); Mass spectrum MH⁺ 478.

### Example 88

### N-[3-Chloro-4-(pyridin-2-ylmethoxy)phenyl]-5-({(2S)-4-[(dimethylamino)acetyl]morpholin-2-yl}methozy)quinazolin-4-amine trifluoroacetate

The procedure described in example 1 was repeated using *N, N*-dimethylglycine and *N*-[3-chloro-4-(pyridin-2-ylmethoxy)phenyl]-5-[(2*S*)-morpholin-2-ylmethoxy]quinazolin-4-amine (obtained as described in example 87, preparation of starting materials), but purifying the product by preparative HPLC, to give the title compound as a yellow solid in 38% yield; NMR spectrum (DMSO-d6) 2.8 (s, 6H), 3.15 - 3.3 (m, 1H), 3.45 - 3.75 (m, 2H), 3.9 - 4.0 (m, 1H), 4.05 - 4.2 (m, 3H), 4.3 - 4.45 (m, 3H), 4.6 (dd, 1H), 5.3 (s, 2H), 7.3 - 7.45 (m, 4H), 7.55 - 7.7 (m, 2H), 7.85 - 8.05 (m, 3H), 8.6 (d, 1H), 8.85 (s, 1H); 11.8 (br s, 1H); Mass spectrum MH⁺ 563.

### Example 89

### 2-((2S)-2-{[(4-{[3-Chloro4-(1,3-thiazol4-ylmethoxy)phenyl]amino}quinazolin-5-yl)oxy]methyl}piperidin-1-yl)-2-oxoethanol hydrochloride

The procedure described in example 21 was repeated using 2-chloro-4-[(5-{[(2*S*)-1-glycoloylpiperidin-2-yl]methoxy}quinazolin-4-yl)amino]phenol. The product obtained after chromatography was treated with 1 equivalent of 1M hydrogen chloride in ether to give the title compound as a solid in 35% yield; NMR spectrum (DMSO-d6) 1.4-1.55 (m, 1H), 1.6 - 1.8 (m, 4H), 1.8 - 1.95 (m, 1H), 3.15 - 3.3 (m, 2H), 3.95 (d, 1H), 4.05 - 4.15 (m, 1H), 4.3 - 4.4 (m, 1H), 4.9 (d, 1H), 5.15 - 5.25 (m, 1H), 5.4 (s, 2H), 7.4 - 7.6 (m, 4H), 7.75 (s, 1H), 7.85 (s, 1H), 8.0 (t, 1H), 8.8 (s, 1H); 9.2 (s, 1H), 10.7 (s, 1H); Mass spectrum MH⁺ 540.

The 2-chloro-4-[(5-{[(2*S*)-1-glycoloylpiperidin-2-yl]methoxy}quinazolin-4-yl)amino]phenol used as starting material was prepared as follows:
The procedure described in example 21 (preparation of starting materials) was repeated using (2*S*)-piperidin-2-ylmethanol (obtained as described for the R-antipode in example 7, preparation of starting materials) and 2-chloro-4-[(5-fluoroquinazolin-4-yl)amino]phenol to give 2-chloro-4-({5-[(2*S*)-piperidin-2-ylmethoxy]quinazolin-4-yl}amino)phenol as a light brown solid in 55% yield; NMR spectrum (DMSO-d6) 1.45 - 1.85 (m, 5H), 1.95 - 2.05 (m, 1H), 2.85 - 3.0 (m, 1H), 3.35 (d, 1H), 3.7 - 3.8 (m, 1H), 4.45 - 4.55 (m, 1H), 4.6 - 4.7 (m, 1H), 7.0 (d, 1H), 7.2 (d, 1H), 7.4 (d, 1H), 7.55 (dd, 1H), 7.75 (t, 1H), 8.0 (d, 1H), 8.5 (s, 1H), 9.6 (br s, 2H), 10.1 (br s 1H).
The procedure described in example 21 (preparation of starting materials) was repeated using glycolic acid and 2-chloro-4-({5-[(2*S*)-piperidin-2-ylmethoxy]quinazolin-4-yl}amino)phenol, but purifying the product by column chromatography, eluting with 0-10% methanol in DCM, to give 2-chloro-4-[(5-{[(2*S*)-1-glycoloylpiperidin-2-yl]methoxy}quinazolin-4-yl)amino]phenol as a yellow foam in 70% yield; NMR spectrum (DMSO-d6) 1.35-1.5 (m, 1H), 1.6 - 1.75 (m, 4H), 1.8 - 1.95 (m, 1H), 3.1 - 3.2 (m, 2H), 3.9 - 4.15 (m, 2H), 4.6 - 4.7 (m, 2H), 5.1 - 5.2 (m, 1H), 6.95(d, 1H), 7.2 - 7.4 (m, 3H), 7.65 - 7.8 (m, 2H), 8.4 (s, 1H), 9.6 (s, 1H); 10.0 (s, 1H).

### Example 90

### N-[3-Chloro-4-(1,3-thiazol-4-ylmethozy)phenyl]-5-({(2S)-1-[(dimethylamino)acetyl]piperidin-2-yl}methozy)quinazolin-4-amine hydrochloride

The procedure described in example 21 was repeated using 2-chloro-4-[(5-{[(2*S*)-1-(*N,N*-dimethylglycyl)piperidin-2-yl]methoxy}quinazolin-4-yl)amino]phenol. The product obtained after chromatography was treated with 1 equivalent of 1M hydrogen chloride in ether to give the title compound as a solid in 25% yield; NMR spectrum (DMSO-d6) 1.4-1.55 (m, 1H), 1.6 - 1.8 (m, 4H), 1.9 - 2.0 (m, 1H), 2.6 (s, 3H), 2.75 (s, 3H), 3.15 - 3.3 (m, 2H), 3.95 (d, 1H), 4.05 - 4.15 (m, 1H), 4.3 - 4.4 (m, 1H), 4.9 (d, 1 H), 5.15 - 5.25 (m, 1H), 5.4 (s, 2H), 7.4 - 7.6 (m, 4H), 7.75 (s, 1H), 7.85 (s, 1H), 8.0 (t, 1H), 8.8 (s, 1H); 9.2 (s, 1 H), 10.7 (s, 1H); Mass spectrum MH⁺ 567.

The 2-chloro-4-[(5-{[(2*S*)-1-(*N,N*-dimethylglycyl)piperidin-2-yl]methoxy}quinazolin-4-yl)amino]phenol used as starting material was prepared as a solid in 25% yield using the procedure described in example 21 (preparation of starting materials), but starting from chloro-4-({5-[(2*S*)-piperidin-2-ylmethoxy]quinazolin-4-yl}amino)phenol (obtained as described in example 89, preparation of starting materials) and purifying the product by column chromatography, eluting with 0-10% methanol in DCM; NMR spectrum (DMSO-d6) 1.4-1.55 (m, 1H), 1.6 -1.8 (m, 4H), 1.85 - 1.95 (m, 1H), 3.5 - 3.6 (m, 1H), 3.8 - 3.9 (m, 1H), 4.0 - 4.15 (m, 1H), 4.35 - 4.45 (m, 1H), 4.6 - 4.8 (m, 2H), 5.15 - 5.25 (m, 1H), 6.95(d, 1H), 7.25 - 7.45 (m, 3H), 7.65 - 7.8 (m, 1H), 7.85 (s, 1H), 8.4 (s, 1H), 9.6 (s, 1H); 10.0 (s, 1H).

### Example 91

### N-[3-Chloro-4-(pyridin-2-ylmethoxy)phenyl]-5-{[(2R)-1-(pyrrolidin-1-ylacetyl)pyrrolidin-2-yl]methoxy}quinazolin-4-amine hydrochloride

A solution of 5-{[(2*R*)-1-(chloxoacetyl)pyrrolidin-2-yl]methoxy}-*N*-[3-chloro-4-(pyridin-2-ylmethoxy)phenyl]quinazolin-4-amine (200 mg), pyrrolidine (0.31 ml) and tetra-n-butylammonium iodide (70 mg) in DMA (15 ml) was heated at 100°C for 2 hours. Volatile material was removed by evaporation and the residue was partitioned between DCM (10 mL) and water (5 mL). The organic phase was separated, washed with water (5 ml) and saturated sodium chloride solution (5 ml), and purified by chromatography, eluting with 0 - 5 % of 10:1 methanol / aqueous ammonia (0.880) in DCM. The appropriate fractions were combined and concentrated, and the residue was treated with 1 equivalent of 1M hydrogen chloride in ether to give the title compound as a solid (73 mg); NMR spectrum (DMSO-d6) 1.2 - 1.3 (m, 1H), 1.45 - 1.55 (m, 1H), 1.7 - 2.05 (m, 6H), 2.9 - 3.0 (m, 2H), 3.1 - 3.2 (m, 2H), 3.25 - 3.45 (m, 3H), 4.2 (s, 2H), 4.45 (t, 1H), 4.55 - 4.65 (m, 1H), 5.3 (s, 2H), 7.25 - 7.4 (m, 4H), 7.55 (d, 2H), 7.8 - 7.95 (m, 3H), 8.55 (d, 1H), 8.7 (s, 1H); 10.1 (br s, 1H), 10.8 (br s, 1H); Mass spectrum MH⁺ 573.

The 5-{[(2*R*)-1-(chloroacetyl)pyrrolidin-2-yl]methoxy}-*N*-[3-chloro-4-(pyridin-2-ylmethoxy)phenyl]quinazolin-4-amine used as starting material was obtained as follows:
Chloroacetyl chloride (0.54 ml) was added to a solution of *N*-[3-chloro-4-(pyridin-2-ylmethoxy)phenyl]-5-[(2*R*)-pyrrolidin-2-ylmethoxy]quinazolin-4-amine (3.0 g, obtained as described example 1, preparation of starting materials), and *N,N*-diisopropylethylamine (1.1 ml) in DMF (60 ml) and the mixture was stirred for 2 hours. Volatile material was removed by evaporation and the residue was triturated with water to give 5-{[(2*R*)-1-(chloroacetyl)pyrrolidin-2-yl]methoxy}-*N*-[3-chloro-4-(pyridin-2-ylmethoxy)phenyl]quinazolin-4-amine (2.95 g); NMR spectrum (DMSO-d6) 1.9 - 2.1 (m, 4H), 3.5 - 3.6 (m, 2H), 4.2(dd, 1H), 4.35 (s, 2H), 4.4 - 4.5 (m, 1H), 4.55 - 4.65 (m, I H), 5.3 (s, 2H), 7.25 - 7.4 (m, 4H), 7.5 - 7.6 (m, 2H), 7.8 (t, 1H), 7.9 (t, 1H), 8.0 (d, 1H); 8.55 (s, 1H), 8.6 (d, 1H), 10.25 (br s, 1H); Mass spectrum MH⁺ 538.5.

### Example 92

### N-{3-methyl-4-[(6-methylpyridin-3-yl)oxy]phenyl}-5-{[(2R)-1-(pyrrolidin-1-ylacetyl)pyrrolidin-2-yl]methoxy}quinazolin-4-amine

Chloroacetyl chloride (46 µl, 0.62 mmol) was added dropwise to a ice-cooled solution of *N*-{3-methyl-4-[(6-methylpyridin-3-yl)oxy]phenyl}-5-[(2*R*)-pyrrolidin-2-ylmethoxy]quinazolin-4-amine (240 mg, 0.54 mmol, Example 57 starting material) and diisopropylethylamine (108 µl, 0.62 mmol) in DCM (2 ml). The mixture was stirred at room temperature for 1 hour. Pyrrolidine (0.18 ml, 2.2 mmol) was added and the mixture was stirred at room temperature for 2 hours. After evaporation of the solvents to dryness, the residue was dissolved in DMSO, filtered and purified on an HPLC column (C18, 5 microns, 19 mm diameter, 100 mm length) of a preparative HPLC-MS system eluting with a mixture of water and acetonitrile containing 2g/l of ammonium formate (gradient). Additional purification by chromatography on silica gel (eluant: 8% 7N ammonia-methanol in DCM) afforded the title compound as a white solid (112 mg, 37%); NMR spectrum (CDCl₃) 1.81 (m, 4H), 2.20-2.00 (m, 4H), 2.29 (s, 3H), 2.53 (s, 3H), 2.64 (m, 2H), 2.69 (m, 2H), 3.26 (d, 1H), 3.39 (d, 1H), 3.56 (m, 2H), 4.06 (t, 1H), 4.62 (m, 1H), 4.69 (m, 1H), 6.91 (d, 1H), 7.14-7.07 (m, 3H), 7.47 (m, 2H), 7.64 (m, 2H), 8.27 (s, 1H), 8.62 (s, 1H), 9.81 (s br, 1H); Mass spectrum: MH⁺ 553.

### Example 93

### 2-[(2R)-2-({[4-({3-chloro-4-[(6-methylpyridin-3-yl)oxy]phenyl}amino)quinazolin-5-yl]oxy}methyl)pyrrolidin-1-yl]-2-oxoethanol

The procedure from example 57 was repeated using *N*-{3-chloro-4-[(6-methylpyridin-3-yl)oxy]phenyl}-5-[(2*R*)-pyrrolidin-2-ylmethoxy]quinazolin-4-amine (200 mg, 0.45 mmol) and acetoxyacetyl chloride to give the title compound (102 mg, 43%); NMR spectrum (CDCl₃) 2.2-2.1 (m, 4H), 2.55 (s, 3H), 3.27 (m, 1H), 3.46-3.37 (m, 2H), 4.17-4.07 (m, 3H), 4.54 (m, 1H), 4.78 (m, 1H), 7.00 (m, 2H), 7.12 (d, 1H), 7.20 (d, 1H), 7.54 (m, 2H), 7.66 (m, 1H), 7.97 (s, 1H), 8.31 (s, 1H), 8.64 (s, 1H), 9.93 (s br, 1H); Mass spectrum: MH⁺ 520.

The *N*-{3-chloro-4-[(6-methylpyridin-3-yl)oxy]phenyl}-5-[(2*R*)-pyrrolidin-2-ylinethoxy]quinazolin-4-amine was made from 5-hydroxy-2-methylpyridine, 3-chloro-4-fluoro-1-nitrobenzene, 4-chloro-5-fluoroquinazoline and (*R*)-2-pyrrolidinemethanol using a similar procedure as the one described in example 57 starting material:
5-(2-chloro-4-nitrophenoxy)-2-methylpyridine (4.5 g, 93%); Mass spectrum: MH⁺ 265.
3-chloro-4-[(6-methylpyridin-3-yl)oxy]aniline (1.33 g, 100%); Mass spectrum: MH⁺ 235. The reaction was carried out in ethanol in the presence of platinum oxide instead of palladium on charcoal.
*N*-{3-chloro-4-[(6-methylpyridin-3-yl)oxy]phenyl}-5-fluoroquinazolin-4-amine (1.83 g, 94%); Mass spectrum: MH⁺ 381.
*N*-{3-chloro-4-[(6-methylpyridin-3-yl)oxy]phenyl}-5-[(2*R*)-pyrrolidin-2-ylmethoxy]quinazolin-4-amine (720 mg, 64%); Mass spectrum: MH⁺ 462.

### Example 94

### 2-[(2S)-2-({[4-({3-chloro-4-[(6-methylpyridin-3-yl)oxy]phenyl}amino)quinazolin-5-yl]oxy}methyl)pyrrolidin-1-yl]-2-oxoethanol

The procedure from example 57 was repeated using *N*-{3-chloro-4-[(6-methylpyridin-3-yl)oxy]phenyl}-5-[(2*S*)-pyrrolidin-2-ylmethoxy]quinazolin-4-amine (200 mg, 0.45 mmol) and acetoxyacetyl chloride to give the title compound (112 mg, 47%); NMR spectrum (CDCl₃) 2.2-2.1 (m, 4H), 2.55 (s, 3H), 3.27 (m, 1H), 3.46-3.37 (m, 2H), 4.17-4.07 (m, 3H), 4.54 (m, 1H), 4.78 (m, 1H), 7.00 (m, 2H), 7.12 (d, 1H), 7.20 (d, 1H), 7.54 (m, 2H), 7.66 (m, 1H), 7.97 (s, 1H), 8.31 (s, 1H), 8.64 (s, 1H), 9.93 (s br, 1H); Mass spectrum: MH⁺ 520.

The *N*-{3-chloro-4-[(6-methylpyridin-3-yl)oxy]phenyl}-5-[(2*S*)-pyrrolidin-2-ylmethoxy]quinazolin-4-amine was made from *N*-{3-chloro-4-[(6-methylpyridin-3-yl)oxy]phenyl}-5-fluoroquinazolin-4-amine and (*S*)-2-pyrrolidinemethanol following procedure of Example 57 starting material:

*N*-{3-chloro-4-[(6-methylpyridin-3-yl)oxy]phenyl}-5-[(2*S*)-pyrrolidin-2-ylmethoxy]quinazolin-4-amine (690 mg, 65%); Mass spectrum: MH⁺ 462.

### Example 95

### 2-((2R)-2-{[(4-{[3-chloro-4-(pyridin-3-yloxy)phenyl]amino}quinazolin-5-yl)oxy]methyl}pyrrolidin-1-yl)-2-oxoethanol

The procedure from example 57 was repeated using *N*-[3-chloro-4-(pyridin-3-yloxy)phenyl]-5-[(2*R*)-pyrrolidin-2-ylmethoxy]quinazolin-4-amine (200 mg, 0.45 mmol) and acetoxyacetyl chloride to give the title compound (113 mg, 49%); NMR spectrum (CDCl₃) 2.3-2.1 (m, 4H), 3.46-3.37 (m, 2H), 4.15-4.05 (m, 3H), 4.53 (m, 1H), 4.79 (m, 1H), 6.99 (m, 1H), 7.09 (m, 1H), 7.29 (s, 2H); 7.50 (d, 1H), 7.67-7.58 (m, 2H), 8.03 (s, 1H), 8.36 (s, 1H), 8.41 (s, 1H), 8.65 (s, 1H), 10.0 (s br, 1H); Mass spectrum: MH⁺ 506.

The *N*-[3-chloro-4-(pyridin-3-yloxy)phenyl]-5-[(2*R*)-pyrrolidin-2-ylmethoxy]quinazolin-4-amine was made from 3-hydroxypyridine, 3-chloro-4-fluoro-1-nitrobenzene, 4-chloro-5-fluoroquinazoline and (*R*)-2-pyrrolidinemethanol using a similar procedure as the one described in example 57 starting material:

3-(2-chloro-4-nitrophenoxy)pyridine (4.8 g, 85%); Mass spectrum: MH⁺ 251.

3-chloro-4-(pyridin-3-yloxy)aniline (2.28 g, 58%); Mass spectrum: MH⁺ 235. The reaction was carried out in ethanol in the presence of platinum oxide instead of palladium on charcoal and after work-up, the residue was purified by chromatography on silica gel (eluant: 5% methanol in DCM).

*N*-[3-chloro-4-(pyridin-3-yloxy)phenyl]-5-fluoroquinazolin-4-amine (1.7 g, 81%); Mass spectrum: MH⁺ 3.67.

*N*-[3-chloro-4-(pyridin-3-yloxy)phenyl]-5-[(2*R*)-pyrrolidin-2-ylmethoxy]quinazolin-4-amine; Mass spectrum: MH⁺ 448.

### Example 96

### 2-((2S)-2-{[(4-{[3-chloro-4-(pyridin-3-yloxy)phenyl]amino}quinazolin-5-yl)oxy]methyl}pyrrolidin-1-yl)-2-oxoethanol

The procedure from example 57 was repeated using *N*-[3-chloro-4-(pyridin-3-yloxy)phenyl]-5-[(2*S*)-pyrrolidin-2-ylmethoxy]quinazolin-4-amine (200 mg, 0.45 mmol) and acetoxyacetyl chloride to give the title compound (113 mg, 49%); NMR spectrum (CDCl₃) 2.3-2.1 (m, 4H), 3.46-3.37 (m, 2H), 4.15-4.05 (m, 3H), 4.53 (m, 1H), 4.79 (m, 1H), 6.99 (m, 1H), 7.09 (m, 1H), 7.29 (s, 2H); 7.50 (d, 1H), 7.67-7.58 (m, 2H), 8.03 (s, 1H), 8.36 (s, 1H), 8.41 (s, 1H), 8.65 (s, 1H), 10.0 (s br, 1H); Mass spectrum: MH⁺ 506.

The *N*-[3-chloro-4-(pyridin-3-yloxy)phenyl]-5-[(2*S*)-pyrrolidin-2-ylmethoxy]quinazolin-4-amine was made from *N*-[3-chloro-4-(pyridin-3-yloxy)phenyl]-5-fluoroquinazolin-4-amine and (*S*)-2-pyrrolidinemethanol following procedure of Example 57 starting material:

*N*-{3-chloro-4-[(6-methylpyridin-3-yl)oxy]phenyl}-5-[(2*S*)-pyrrolidin-2-ylmethoxy]quinazolin-4-amine (448 mg, 61%); Mass spectrum: MH⁺ 462.

### Example 97

### 2-[(3R)-3-({[4-({3-methyl-4-[(6-methylpyridin-3-yl)oxy]phenyl}amino)quinazolin-5-yl]oxy}methyl)morpholin-4-yl]-2-oxoethanol

The procedure from example 57 was repeated using *N*-{3-methyl-4-[(6-methylpyridin-3-yl)oxy]phenyl}-5-[(3*R*)-morpholin-3-ylmethoxy]quinazolin-4-amine (250 mg, 0.57 mmol) and acetoxyacetyl chloride to give the title compound (70 mg, 24%); NMR spectrum (CDCl₃) 2.29 (s, 3H), 2.53 (s, 3H), 3.14 (s br, 1H), 3.25 (d, 1H), 3.57-3.49 (m, 2H), 3.85-3.78 (m, 2H), 4.06 (dd, 1H), 4.20 (m, 2H), 4.43 (m, 1H), 4.59 (m, 1H), 5.03 (m, 1H), 6.91 (d, 1H), 6.96 (d, 1H), 7.09 (d, 1H), 7.17 (m, 1H), 7.34 (m, 1H), 7.49 (m, 2H), 7.65 (t, 1H), 8.28 (s, 1H), 8.57 (s, 1H), 9.43 (s br, 1H); Mass spectrum: MH⁺ 516.

The *N*-{3-methyl-4-[(6-methylpyridin-3-yl)oxy]phenyl}-5-[(3*R*)-morpholin-3-ylmethoxy]quinazolin-4-amine used as starting material was made from 5-fluoro-*N*-{3-methyl-4-[(6-methylpyridin-3-yl)oxy]phenyl}quinazolin-4-amine and (3*S*)-morpholin-3-ylmethanol (J. Chem. Soc. Perkin Trans. 1, 2577-2580 (1985)) according to procedure from example 57 starting material:

*N*-{3-methyl-4-[(6-methylpyridin-3-yl)oxy]phenyl}-5-[(3*R*)-morpholin-3-ylmethoxy]quinazolin-4-amine (320 mg, 50%); Mass spectrum: MH⁺ 548.

### Example 98

### 2-((3R)-3-{[(4-{[3-Chloro-4-(pyridin-2-ylmethoxy)phenyl]amino}quinazolin-5-yl)oxy]methyl}morpholin-4-yl)-2-oxoethanol

The procedure from example 57 was repeated using *N*-[3-chloro-4-(pyridin-2-ylmethoxy)phenyl]-5-[(3*R*)-morpholin-3-ylmethoxy]quinazolin-4-amine (250 mg, 0.52 mmol) and acetoxyacetyl chloride to give the title compound (55 mg, 20%); NMR spectrum (CDCl₃) 3.17 (m, 1H), 3.25 (d, 1H), 3.55 (m, 2H), 3.80 (m, 2H), 4.08 (dd, 1H), 4.20 (m, 2H), 4.41 (m, 1H), 4.59 (t, 1H), 5.01 (m, 1H), 5.30 (s, 2H), 6.95 (d, 1H), 7.03 (d, 1H), 7.25 (m, 1H), 7.39 (dd, 1H), 7.51 (d, 1H), 7.70-7.63 (m, 3H), 7.75 (m, 1H), 8.56 (s, 1H), 8.59 (d, 1H), 9.43 (s br, 1H); Mass spectrum: MH⁺ 536.

The *N*-[3-chloro-4-(pyridin-2-ylmethoxy)phenyl]-5-[(3*R*)-morpholin-3-ylmethoxy]quinazolin-4-amine used as starting material was made from *N*-[3-chloro-4-(pyridin-2-ylmethoxy)phenyl]-5-fluoroquinazolin-4-amine and (3*S*)-morpholin-3-ylmethanol (J. Chem. Soc. Perkin Trans. 1, 2577-2580 (1985)) according to procedure from example 57 starting material:

*N*-[3-chloro-4-(pyridin-2-ylmethoxy)phenyl]-5-[(3*R*)-morpholin-3-ylmethoxy]quinazolin-4-amine (396 mg, 63%); Mass spectrum: MH⁺ 478.

### Example 99

### 2-[(2R)-2-({[4-({3-methyl-4-[(6-methylpyridin-3-yl)oxy]phenyl}amino)quinazolin-5-yl]oxy}methyl)piperidin-1-yl]-2-oxoethanol

The procedure from Example 1 was repeated using *N*-{3-methyl-4-[(6-methylpyridin-3-yl)oxy]phenyl}-5-[(2*R*)-piperidin-2-ylmethoxy]quin:azolin-4-amine (200 mg, 0.45 mmol) except that after evaporation of the solvents, the residue was injected on an HPLC column (C18, 5 microns, 19 mm diameter, 100 mm length) of a preparative HPLC-MS system eluting with a mixture of water and acetonitrile containing 2g/l of ammonium formate (gradient) to give the title compound (115 mg, 51%); NMR Spectrum: (DMSOd₆ and CF₃CO₂D) 1.45 (m, 1H), 1.68 (m, 4H), 1.87 (m, 1H), 2.28 (s, 3H), 2.71 (s, 3H), 3.24 (m, 1H), 3.56 (m, 1H), 3.94 (m, 1H), 4.11 (m, 1H), 4.92 (t, 1H), 5.28 (m, 1H), 7.21 (d, 1H), 7.44 (d, 1H), 7.7-7.5 (m, 3H), 7.94 (d, 1H), 8.11-8.04 (m, 2H), 8.72 (s, 1H), 8.85 (s, 1H); Mass spectrum: MH⁺ 514.

The *N*-{3-methyl-4-[(6-methylpyridin-3-yl)oxy]phenyl}-5-[(2*R*)-piperidin-2-ylmethoxy]quinazolin-4-amine used as starting material was made from 5-fluoro-*N*-{3-methyl-4-[(6-methylpyridin-3-yl)oxy]phenyl}quinazolin-4-amine and (2*R*)-piperidin-2-ylmethanol according to procedure from example 57 starting material: *N*-{3-methyl-4-[(6-methylpyridin-3-yl)oxy]phenyl}-5-[(2*R*)-piperidin-2-ylmethoxy]quinazolin-4-amine (415 mg, 73%); Mass spectrum: MH⁺ 456.

### Example 100

### 2-[(2S)-2-({[4-({3-methyl-4-[(6-methylpyridin-3-yl)oxy]phenyl}amino)quinazolin-5-yl]oxy}methyl)piperidin-1-yl]-2-oxoethanol

The procedure from Example 1 was repeated using *N*-{3-methyl-4-[(6-methylpyridin-3-yl)oxy]phenyl}-5-[(2*S*)-piperidin-2-ylmethoxy]quinazolin-4-amine (200 mg, 0.45 mmol) except that after evaporation of the solvents, the residue was injected on an HPLC column (C18, 5 microns, 19 mm diameter, 100 mm length) of a preparative HPLC-MS system eluting with a mixture of water and acetonitrile containing 2g/l of ammonium formate (gradient) to give the title compound (100 mg, 45%); NMR Spectrum: (DMSOd₆ and CF₃CO₂D) 1.45 (m, 1H), 1.68 (m, 4H), 1.87 (m, 1H), 2.28 (s, 3H), 2.71 (s, 3H), 3.24 (m, 1H), 3.56 (m, 1H), 3.94 (m, 1H), 4.11 (m, 1H), 4.92 (t, 1H), 5.28 (m, 1H), 7.21 (d, 1H), 7.44 (d, 1H), 7.7-7.5 (m, 3H), 7.94 (d, 1H), 8.11-8.04 (m, 2H), 8.72 (s, 1H), 8.85 (s, 1H); Mass spectrum: MH⁺ 514.

The *N*-{3-methyl-4-[(6-methylpyridin-3-yl)oxy]phenyl}-5-[(2*S*)-piperidin-2-ylmethoxy]quinazolin-4-amine used as starting material was made from 5-fluoro-*N*-{3-methyl-4-[(6-methylpyridin-3-yl)oxy]phenyl}quinazolin-4-amine and (2*S*)-piperidin-2-ylmethanol according to procedure from example 57 starting material: *N*-{3-methyl-4-[(6-methylpyridin-3-yl)oxy]phenyl}-5-[(2*S*)-piperidin-2-ylmethoxy]quinazolin-4-amine (391 mg, 52%); Mass spectrum: MH⁺ 456.

### Example 101

### 2-[(2R)-4-methyl-2-({[4-({3-methyl-4-[(6-methylpyridin-3-yl)oxy]phenyl}amino)quinazolin-5-yl]oxy}methyl)piperazin-1-yl]-2-oxoethanol

A mixture of *N*-{3-methyl-4-[(6-methylpyridin-3-yl)oxy]phenyl}-5-{[(2*R*)-4-methylpiperazin-2-yl]methoxy}quinazolin-4-amine (200 mg, 0.43 mmol), glycolic acid (36 mg, 0.47 mmol), diisopropylethylamine (0.22 ml, 1.28 mmol) and HATU (179 mg, 0.47 mmol) in DMF (2 ml) was stirred at room temperature for 16 hours. More HATU (36 mg) and glycolic acid (8 mg) were added. The mixture was stirred for 16 hours more. After evaporation of the solvents under high vacuum, the residue was taken in dichloromethane. The resulting solution was washed with saturated aqueous ammonium chloride, saturated aqueous sodium bicarbonate, dried over magnesium sulfate. After evaporation of the solvents, the residue was purified by chromatography on silica gel (eluant: 4% to 7% 7N ammonia-methanol in dichloromethane) to give the title compound (132 mg, 60%) as a beige solid; NMR spectrum (CDCl₃) 2.03 (m, 1H), 2.23 (s, 3H), 2.28 (m, 1H), 2.30 (s, 3H), 2.54 (s, 3H), 2.77 (m, 1H), 3.01 (m, 1H), 3.15 (m, 1H), 3.34 (m, 1H), 3.47 (m, 1H), 4.07 (dd, 1H), 4.20 (dd, 1H), 4.37 (dd, 1H), 4.62 (m, 1H), 5.20 (m, 1H), 6.92 (d, 1H), 6.97 (d, 1H), 7.10 (d, 1H), 7.18 (m, 1H), 7.32 (m, 1H), 7.45 (s, 1H), 7.52 (m, 1H), 7.65 (m, 1H), 8.29 (s, 1H), 8.56 (s, 1H), 9.62 (s br, 1H); Mass spectrum: MH⁺ 529.

The *N*-{3-methyl-4-[(6-methylpyridin-3-yl)oxy]phenyl}-5-{[(2*R*)-4-methylpiperazin-2-yl]methoxy}quinazolin-4-amine used as starting material was made from 5-fluoro-*N*-{3-methyl-4-[(6-methylpyridin-3-yl)oxy]phenyl}quinazolin-4-amine and [(2*R*)-4-methylpiperazin-2-yl]methanol (Example 36 starting material) according to procedure from Example 57 starting material:
*N*-{(3-methyl-4-[(6-methylpyridin-3-yl)oxy]phenyl)-5-{[(2*R*)-4-methylpiperazin-2-yl]methoxy}quinazolin-4-amine (536 mg, 33%); Mass spectrum: MH⁺ 471.

## Claims

1. A quinazoline derivative of the Formula Ia: 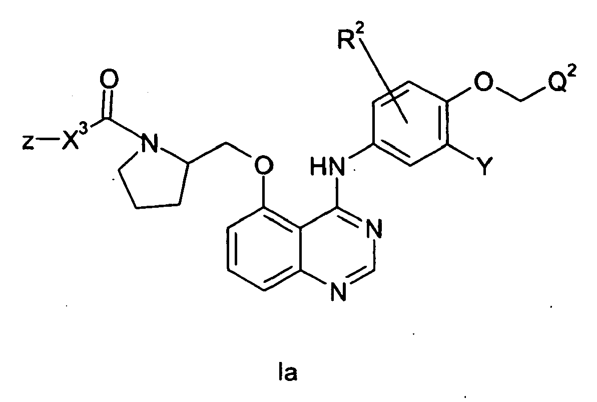 wherein:
**R²** is selected from hydrogen and halogeno;
**Y** is selected from halogeno, (1-4C)alkyl, (1-4C)alkoxy, (2-4C)alkenyl and (2-4C)alkynyl;
**Q²** is selected from phenyl, pyridyl, pyrazinyl, 1,3-thiazolyl and isoxazolyl
and wherein Q² optionally bears one or more substituents, which may be the same or different, selected from halogeno, hydroxy, cyano, carboxy, nitro, amino, (1-4C)alkyl, (1-4C)alkoxy, (2-4C)alkenyl, (2-4C)alkynyl, (1-4C)alkylthio, (1-4C)alkylsulfinyl, (1-4C)alkylsulfonyl, (2-4C)alkanoyl, N-(1-4C)alkylamino, N, N-di-[(1-4C)alkyl]amino, (1-4C)alkoxycarbonyl, carbamoyl, N-(1-4C)alkylcarbamoyl, N, N-di-[(1-4C)alkyl]carbamoyl, (2-4C)alkanoyloxy, (2-4C)alkanoylamino, N-(1-4C)alkyl-(2-4C)alkanoylamino, halogeno-(1-4C)alkyl, hydroxy-(1-4C)alkyl, (1-4C)alkoxy-(1-4C)alkyl, cyano-(1-4C)alkyl, carboxy-(1-4C)alkyl, amino-(1-4C)alkyl, N-(1-4C)alkylamino-(1-4C)alkyl and N, N-di-[(1-4C)alkyl]amino-(1-4C)alkyl;
**Z** is selected from hydroxy, di-[(1-3C)alkyl]amino and (1-6C)alkoxy;
**X³** is selected from -CH₂-, -CH₂CH₂-, -(CR⁸R⁹)-, -(CR⁸R⁹CH₂)-, -(CH₂CR⁸R⁹)- and (3-6C)cycloalkylene, wherein each of R⁸ and R⁹, which may be the same or different, is selected from hydrogen and methyl;
or a pharmaceutically acceptable salt thereof.

2. A quinazoline derivative of the Formula Ia as defined in claim 1, wherein Y is halogeno or methyl; or a pharmaceutically acceptable salt thereof.

3. A quinazoline derivative of the Formula Ia as defined in claim 2, wherein Y is chloro; or a pharmaceutically acceptable salt thereof.

4. A quinazoline derivative of the Formula Ia as defined in any one or more of claims 1 to 3, wherein Q² is selected from phenyl, 2-pyridyl, 2-pyrazinyl, 1,3-thiazol-4-yl, 1,3-thiazol-5-yl and 3-isoxazolyl; or a pharmaceutically acceptable salt thereof.

5. A quinazoline derivative of the Formula Ia as defined in any one or more of claims 1 to 4, wherein X³ is cyclopropylidene; or a pharmaceutically acceptable salt thereof.

6. A quinazoline derivative of the Formula Ia as defined in any one or more of claims 1 to 5, wherein Z is selected from hydroxy, dimethylamino, methoxy and ethoxy; or a pharmaceutically acceptable salt thereof.

7. A quinazoline derivative of the Formula Ia as defined in claim 7, wherein Z is hydroxy; or a pharmaceutically acceptable salt thereof.

8. A quinazoline derivative of the Formula Ia as defined in claim 1, wherein Q² is selected from pyridyl, pyrazinyl, 1,3-thiazolyl and isoxazolyl
and wherein Q² optionally bears one or more substituents, which may be the same or different, selected from halogeno, hydroxy, cyano, carboxy, nitro, amino, (1-4C)alkyl, (1-4C)alkoxy, (2-4C)alkenyl, (2-4C)alkynyl, (1-4C)alkylthio, (1-4C)alkylsulfinyl, (1-4C)alkylsulfonyl, (2-4C)alkanoyl, N-(1-4C)alkylamino, N, N-di-[(1-4C)alkyl]amino, (1-4C)alkoxycarbonyl, carbamoyl, N-(1-4C)alkylcarbamoyl, N, N-di-[(1-4C)alkyl]carbamoyl, (2-4C)alkanoyloxy, (2-4C)alkanoylamino, N-(1-4C)alkyl-(2-4C)alkanoylamino, halogeno-(1-4C)alkyl, hydroxy-(1-4C)alkyl, (1-4C)alkoxy-(1-4C)alkyl, cyano-(1-4C)alkyl, carboxy-(1-4C)alkyl, amino-(1-4C)alkyl, N-(1-4C)alkylamino-(1-4C)alkyl and N, N-di-[(1-4C)alkyl]amino-(1-4C)alkyl; and X³ is selected from -CH₂- and -CH₂CH₂-;
Z is selected from hydroxy and di-[(1-3C)alkyl]amino; and
R² and Y are as defined in claim 1;
or a pharmaceutically acceptable salt thereof.

9. A quinazoline derivative of the Formula Ia as defined in claim 8, wherein Y is selected from halogeno and methyl; or a pharmaceutically acceptable salt thereof.

10. A quinazoline derivative of the Formula Ia as defined in claim 9, wherein Y is chloro; or a pharmaceutically acceptable salt thereof.

11. A quinazoline derivative of the Formula Ia as defined in any one or more of claims 8 to 10, wherein Q² is selected from 2-pyridyl, 2-pyrazinyl, 1,3-thiazol-4-yl, 1,3-thiazol-5-yl and 3-isoxazolyl; or a pharmaceutically acceptable salt thereof.

12. A quinazoline derivative of the Formula Ia as defined in any one or more of claims 8 to 11, wherein Z-X³ is hydroxymethyl; or a pharmaceutically acceptable salt thereof.

13. A quinazoline derivative as defined in claim 1 selected from one or more of the following:
2-((2R)-2-{[(4-{[3-chloro-4-(pyridin-2-ylmethoxy)phenyl]amino} quinazolin-5-yl)oxy]methyl}pyrrolidin-1-yl)-2-oxoethanol;
2-((2*S*)-2-{[(4-{[3-chloro-4-(pyridin-2-ylmethoxy)phenyl]amino}quinazolin-5-yl)oxy]methyl}pyrrolidin-1-yl)-2-oxoethanol;
*N*-[3-chloro-4-(pyridin-2-ylmethoxy)phenyl]-5-({(2*R*)-1-[(dimethylamino)acetyl] pyrrolidin-2-yl}methoxy)quinazolin-4-amine;
*N*-[3-chloro-4-(pyridin-2-ylmethoxy)phenyl]-5-({(2*S*)-1-[(dimethylamino)acetyl] pyrrolidin-2-yl}methoxy)quinazolin-4-amine;
1-((2*R*)-2-{[(4-{[3-chloro-4-(pyridin-2-ylmethoxy)phenyl]amino}quinazolin-5-yl)oxy]methyl}pyrrolidin-1-yl)-2-methyl-1-oxopropan-2-ol;
1-[((2*R*)-2-{[(4-{[3-chloro-4-(pyridin-2-ylmethoxy)phenyl]amino}quinazolin-5-yl)oxy]methyl}pyrrolidin-1-yl)carbonyl]cyclopropanol;
3-((2*R*)-2-{[(4-{[3-chloro-4-(pyridin-2-ylmethoxy)phenyl]amino}quinazolin-5-yl)oxy]methyl}pyrrolidin-1-yl)-2,2-dimethyl-3-oxopropan-1-ol;
(2*S*)-1-((2*R*)-2-{[(4-{[3-chloro-4-(pyridin-2-ylmethoxy)phenyl]amino} quinazolin-5-yl)oxy]methyl}pyrrolidin-1-yl)-1-oxopropan-2-ol;
*N*-[3-chloro-4-(pyridin-2-ylmethoxy)phenyl]-5-{[(2*R*)-1-(ethoxyacetyl) pyrrolidin-2-yl]methoxy}quinazolin-4-amine;
*N*-[3-chloro-4-(pyridin-2-ylmethoxy)phenyl]-5-{[(2*R*)-1-(methoxyacetyl) pyrrolidin-2-yl]methoxy}quinazolin-4-amine;
*N*-[3-chloro-4-(1,3-thiazol-4-ylmethoxy)phenyl]-5-({(2*R*)-1-[(dimethylamino) acetyl]pyrrolidin-2-yl}methoxy)quinazolin-4-amine;
2-((2*R*)-2-{[(4-{[3-chloro-4-(1,3-thiazol-4-ylmethoxy)phenyl]amino} quinazolin-5-yl)oxy]methyl}pyrrolidin-1-yl)-2-oxoethanol;
*N*-{3-chloro-4-[(5-methylisoxazol-3-yl)methoxy]phenyl}-5-({(2*R*)-1-[(dimethylamino)acetyl]pyrrolidin-2-yl}methoxy)quinazolin-4-amine;
2-[(2*R*)-2-({[4-({3-chloro-4-[(5-methylisoxazol-3-yl)methoxy]phenyl} amino)quinazolin-5-yl]oxy}methyl)pyrrolidin-1-yl]-2-oxoethanol;
*N*-[3-chloro-4-(1,3-thiazol-5-ylmethoxy)phenyl]-5-({(2*R*)-1-[(dimethylamino)acetyl]pyrrolidin-2-yl}methoxy)quinazolin-4-amine;
2-((2*R*)-2-{[(4-{[3-chloro-4-(1,3-thiazol-5-ylmethoxy)phenyl]amino} quinazolin-5-yl)oxy]methyl}pyrrolidin-1-yl)-2-oxoethanol;
*N*-[3-chloro-4-(pyrazin-2-ylmethoxy)phenyl]-5-({(2*R*)-1-[(dimethylamino)acetyl]pyrrolidin-2-yl}methoxy)quinazolin-4-amine;
2-((2*R*)-2-{[(4-{[3-chloro-4-(pyrazin-2-ylmethoxy)phenyl]amino}quinazolin-5-yl)oxy]methyl}pyrrolidin-1-yl)-2-oxoethanol;
(2*R*)-1-((2*R*)-2-{[(4-{[3-chloro-4-(pyridin-2-ylmethoxy)phenyl]amino} quinazolin-5-yl)oxy]methyl}pyrrolidin-1-yl)-1-oxopropan-2-ol;
2-[(2*S*)-2-({[4-({3-chloro-4-[(6-methylpyridin-2-yl)methoxy]phenyl}amino) quinazolin-5-yl]oxy}methyl)pyrrolidin-1-yl]-2-oxoethanol;
2-[(2*S*)-2-({[4-({3-chloro-4-[(2-fluorobenzyl)oxy]phenyl}amino)quinazolin-5-yl]oxy}methyl)pyrrolidin-1-yl]-2-oxoethanol;
2-[(2*S*)-2-({[4-({3-chloro-4-[(3-fluorobenzyl)oxy]phenyl}amino)quinazolin-5-yl]oxy}methyl)pyrrolidin-1-yl]-2-oxoethanol;
2-((2*S*)-2-{[(4-{[3-chloro-4-(1,3-thiazol-4-ylmethoxy)phenyl]amino} quinazolin-5-yl)oxy]methyl}pyrrolidin-1-yl)-2-oxoethanol;
2-((2*S*)-2-{[(4-{[3-chloro-4-(pyrazin-2-ylmethoxy)phenyl]amino}quinazolin-5-yl)oxy]methyl}pyrrolidin-1-yl)-2-oxoethanol;
*N*-{3-chloro-4-[(6-methylpyridin-2-yl)methoxy]phenyl}-5-({(2*S*)-1-[(dimethylamino)acetyl]pyrrolidin-2-yl}methoxy)quinazolin-4-amine;
*N*-{3-chloro-4-[(2-fluorobenzyl)oxy]phenyl}-5-({(2*S*)-1-[(dimethylamino) acetyl]pyrrolidin-2-yl}methoxy)quinazolin-4-amine;
*N*-{3-chloro-4-[(3-fluorobenzyl)oxy]phenyl}-5-({(2*S*)-1-[(dimethylamino) acetyl]pyrrolidin-2-yl}methoxy)quinazolin-4-amine;
*N*-[3-chloro-4-(pyrazin-2-ylmethoxy)phenyl]-5-({(2*S*)-1-[(dimethylamino) acetyl]pyrrolidin-2-yl}methoxy)quinazolin-4-amine;
*N*-[3-chloro-4-(1,3-thiazol-4-ylmethoxy)phenyl]-5-({(2*S*)-1-[(dimethylamino) acetyl]pyrrolidin-2-yl}methoxy)quinazolin-4-amine;
2-((2*S*)-2-{[(4-{[3-methyl-4-(pyridin-2-ylmethoxy)phenyl]amino}quinazolin-5-yl)oxy]methyl}pyrrolidin-1-yl)-2-oxoethanol;
2-((2*S*)-2-{[(4-{[3-methyl-4-(pyrazin-2-ylmethoxy)phenyl]amino}quinazolin-5-yl)oxy]methyl}pyrrolidin-1-yl)-2-oxoethanol;
2-((2*R*)-2-{[(4-{[3-methyl-4-(pyridin-2-ylmethoxy)phenyl]amino}quinazolin-5-yl)oxy]methyl}pyrrolidin-1-yl)-2-oxoethanol;
2-((2*R*)-2-{[(4-{[3-methyl-4-(pyrazin-2-ylmethoxy)phenyl]amino}quinazolin-5-yl)oxy]methyl}pyrrolidin-1-yl)-2-oxoethanol;
2-((2*R*)-2-{[(4-{[3-methyl-4-(1,3-thiazol-4-ylmethoxy)phenyl]amino} quinazolin-5-yl)oxy]methyl}pyrrolidin-1-yl)-2-oxoethanol;
2-[(2*R*)-2-({[4-({3-methyl-4-[(5-methylisoxazol-3-yl)methoxy]phenyl}amino) quinazolin-5-yl]oxy}methyl)pyrrolidin-1-yl]-2-oxoethanol;
5-{[(2*R*)-1-(methoxyacetyl)pyrrolidin-2-yl]methoxy}-*N*-{3-methyl-4-[(5-methylisoxazol-3-yl)methoxy]phenyl}quinazolin-4-amine;
5-{[(2*R*)-1-(methoxyacetyl)pyrrolidin-2-yl]methoxy}-*N*-[3-methyl-4-(pyridin-2-ylmethoxy)phenyl]quinazolin-4-amine;
5-{[(2*R*)-1-(methoxyacetyl)pyrrolidin-2-yl]methoxy}-*N*-[3-methyl-4-(1,3-thiazol-4-ylmethoxy)phenyl]quinazolin-4-amine;
or a pharmaceutically acceptable salt thereof.

14. A pharmaceutical composition which comprises a quinazoline derivative of the Formula Ia, or a pharmaceutically acceptable salt thereof, as defined in any one of claims 1 to 13 in association with a pharmaceutically-acceptable diluent or carrier.

15. A quinazoline derivative of the Formula la, or a pharmaceutically acceptable salt thereof, as defined in any one of claims 1 to 13 for use as a medicament.

16. A quinazoline derivative of the Formula Ia, or a pharmaceutically acceptable salt thereof, as defined in any one of claims 1 to 13 for use in the production of an anti-proliferative effect which effect is produced alone or in part by inhibiting erbB2 receptor tyrosine kinase in a warm-blooded animal such as man.

17. A quinazoline derivative of the Formula Ia, or a pharmaceutically acceptable salt thereof, as defined in any one of claims 1 to 13 for use in the production of an erbB2 receptor tyrosine kinase inhibitory effect in a warm-blooded animal such as man.

18. A quinazoline derivative of the Formula Ia, or a pharmaceutically acceptable salt thereof, as defined in any one of claims 1 to 13 for use in the production of a selective erbB2 receptor tyrosine kinase inhibitory effect in a warm-blooded animal such as man.

19. A process for the preparation of a quinazoline derivative of the Formula Ia, or a pharmaceutically acceptable salt thereof, as defined in claim 1 which comprises:
(a) the coupling, conveniently in the presence of a suitable base, of a quinazoline of the formula **II**: 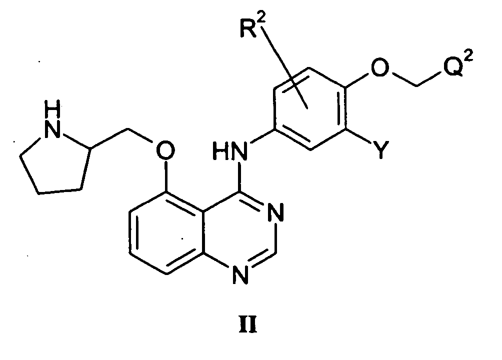 wherein R², Y and Q² have any of the meanings defined in any one of claim 1 to 12 except that any functional group is protected if necessary, with a carboxylic acid of the formula **III,** or a reactive derivative thereof:
Z-X³-COOH **III**
wherein Z and X³ have any of the meanings defined in any one of claims 1 to 12 except that any functional group is protected if necessary; or
(b) the reaction, conveniently in the presence of a suitable base, of a quinazoline of the formula **IV:** 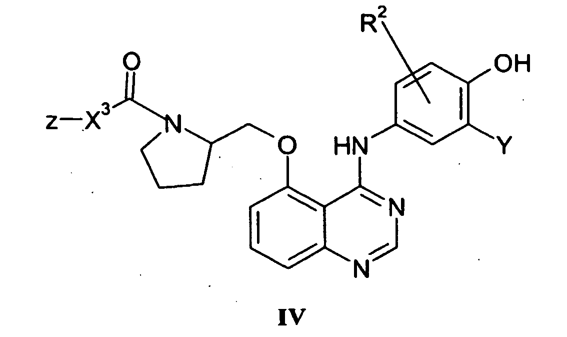 wherein R², X³, Z and Y have any of the meanings defined in any one of claims 1 to 12 except that any functional group is protected if necessary, with a compound of the formula **V**:
Q²-CH₂-L¹ **V**
wherein L¹ is a suitable displaceable group and Q² has any of the meanings defined in any one of claims 1 to 12 except that any functional group is protected if necessary;
(c) the coupling of a quinazoline of the formula **VI:** 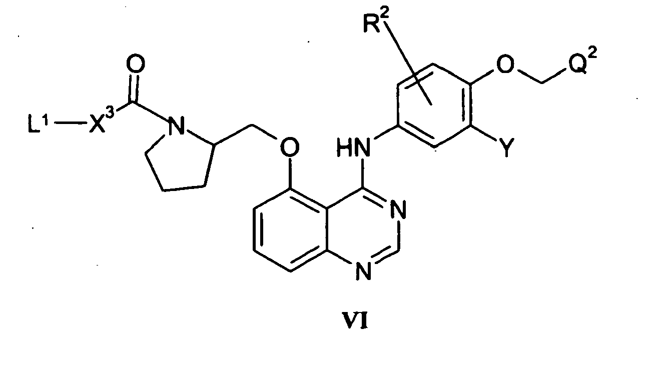 wherein L¹ is a suitable displaceable group and R², X³, Y and Q² have any of the meanings defined in any one of claims 1 to 12 except that any functional group is protected if necessary, with a compound of the formula **VII,** or a reactive derivative thereof:
Z-H **VII**
wherein Z has any of the meanings defined in any one of claims 1 to 12 except that any functional group is protected if necessary; and thereafter, if necessary:
(i) converting a quinazoline derivative of the formula I into another quinazoline derivative of the formula I;
(ii) removing any protecting group that is present by conventional means;
(iii) forming a pharmaceutically acceptable salt.

## Patentansprüche

1. Chinazolinderivat der Formel Ia: 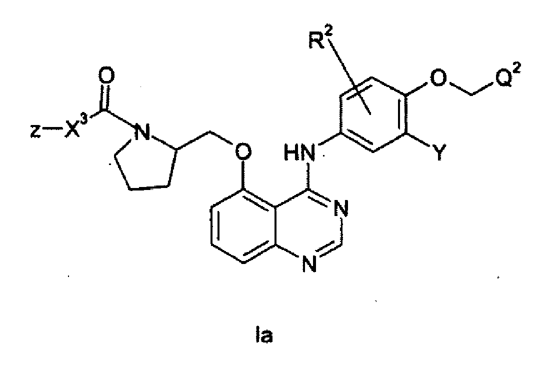 worin:
**R²** unter Wasserstoff und Halogen ausgewählt ist;
**Y** unter Halogen, C₁₋₄-Alkyl, C₁₋₄-Alkoxy, C₂₋₄-Alkenyl und C₂₋₄-Alkinyl ausgewählt ist;
**Q²** unter Phenyl, Pyridyl, Pyrazinyl, 1,3-Thiazolyl und Isoxazolyl ausgewählt ist
und worin Q² gegebenenfalls einen oder zwei Substituenten trägt, die gleich oder verschieden sein können und unter Halogen, Hydroxy, Cyano, Carboxy, Nitro, Amino, C₁₋₄-Alkyl, C₁₋₄-Alkoxy, C₂₋₄-Alkenyl, C₂₋₄-Alkinyl, C₁₋₄-Alkylthio, C₁₋₄-Alkylsulfinyl, C₁₋₄-Alkylsulfonyl, C₂₋₄-Alkanoyl, N-C₁₋₄-Alkylamino, N,N-Di(C₁₋₄-alkyl)amino, C₁₋₄-Alkoxycarbonyl, Carbamoyl, N-C₁₋₄-Alkylcarbamoyl, N,N-Di(C₁₋₄-alkyl)carbamoyl, C₂₋₄-Alkanoyloxy, C₂₋₄-Alkanoylamino, N-C₁₋₄-Alkyl-C₂₋₄-alkanoylamino, Halogen-C₁₋₄-alkyl, Hydroxy-C₁₋₄-alkyl, C₁₋₄-Alkoxy-C₁₋₄-alkyl, Cyano-C₁₋₄-alkyl, Carboxy-C₁₋₄-alkyl, Amino-C₁₋₄-alkyl, N-C₁₋₄-Alkylamino-C₁₋₄-alkyl und N,N-Di(C₁₋₄-alkyl)amino-C₁₋₄-alkyl ausgewählt sind;
**Z** unter Hydroxy, Di(C₁₋₃-alkyl)amino und C₁₋₆-Alkoxy ausgewählt ist;
**X³** unter -CH₂-, -CH₂CH₂-, -(CR⁸R⁹)-, -(CR⁸R⁹CH₂)-, -(CH₂CR⁸R⁹)- und C₃₋₆-Cycloalkylen, worin R⁸ und R⁹ jeweils gleich oder verschieden sein können und unter Wasserstoff und Methyl ausgewählt sind, ausgewählt ist;
oder ein pharmazeutisch annehmbares Salz davon.

2. Chinazolinderivat der Formel Ia nach Anspruch 1, worin Y für Halogen oder Methyl steht; oder ein pharmazeutisch annehmbares Salz davon.

3. Chinazolinderivat der Formel Ia nach Anspruch 2, worin Y für Chlor steht; oder ein pharmazeutisch annehmbares Salz davon.

4. Chinazolinderivat der Formel Ia nach einem oder mehreren der Ansprüche 1 bis 3, worin Q² unter Phenyl, 2-Pyridyl, 2-Pyrazinyl, 1,3-Thiazol-4-yl, 1,3-Thiazol-5-yl und 3-Isoxazolyl ausgewählt ist; oder ein pharmazeutisch annehmbares Salz davon.

5. Chinazolinderivat der Formel Ia nach einem oder mehreren der Ansprüche 1 bis 4, worin X³ für Cyclopropyliden steht; oder ein pharmazeutisch annehmbares Salz davon.

6. Chinazolinderivat der Formel Ia nach einem oder mehreren der Ansprüche 1 bis 5, worin Z unter Hydroxy, Dimethylamino, Methoxy und Ethoxy ausgewählt ist; oder ein pharmazeutisch annehmbares Salz davon.

7. Chinazolinderivat der Formel Ia nach Anspruch 7, worin Z für Hydroxy steht; oder ein pharmazeutisch annehmbares Salz davon.

8. Chinazolinderivat der Formel Ia nach Anspruch 1, worin
Q² unter Pyridyl, Pyrazinyl, 1,3-Thiazolyl und Isoxazolyl ausgewählt ist
und worin Q² gegebenenfalls einen oder zwei Substituenten trägt, die gleich oder verschieden sein können und unter Halogen, Hydroxy, Cyano, Carboxy, Nitro, Amino, C₁₋₄-Alkyl, C₁₋₄-Alkoxy, C₂₋₄-Alkenyl, C₂₋₄-Alkinyl, C₁₋₄-Alkylthio, C₁₋₄-Alkylsulfinyl, C₁₋₄-Alkylsulfonyl, C₂₋₄-Alkanoyl, N-C₁₋₄-Alkylamino, N,N-Di(C₁₋₄-alkyl)amino, C₁₋₄-Alkoxycarbonyl, Carbamoyl, N-C₁₋₄-Alkylcarbamoyl, N,N-Di(C₁₋₄-alkyl)carbamoyl, C₂₋₄-Alkanoyloxy, C₂₋₄-Alkanoylamino, N-C₁₋₄-Alkyl-C₂₋₄-alkanoylamino, Halogen-C₁₋₄-alkyl, Hydroxy-C₁₋₄-alkyl, C₁₋₄-Alkoxy-C₁₋₄-alkyl, Cyano-C₁₋₄-alkyl, Carboxy-C₁₋₄-alkyl, Amino-C₁₋₄-alkyl, N-C₁₋₄-Alkylamino-C₁₋₄-alkyl und N,N-Di(C₁₋₄-alkyl)amino-C₁₋₄-alkyl ausgewählt sind; **X³** unter -CH₂- und -CH₂CH₂- ausgewählt ist;
**Z** unter Hydroxy und Di(C₁₋₃-alkyl)amino ausgewählt ist und
R² und Y die in Anspruch 1 angegebene Bedeutung besitzen;
oder ein pharmazeutisch annehmbares Salz davon.

9. Chinazolinderivat der Formel Ia nach Anspruch 8, worin Y unter Halogen und Methyl ausgewählt ist; oder ein pharmazeutisch annehmbares Salz davon.

10. Chinazolinderivat der Formel Ia nach Anspruch 9, worin Y für Chlor steht; oder ein pharmazeutisch annehmbares Salz davon.

11. Chinazolinderivat der Formel Ia nach einem oder mehreren der Ansprüche 8 bis 10, worin Q² unter 2-Pyridyl, 2-Pyrazinyl, 1,3-Thiazol-4-yl, 1,3-Thiazol-5-yl und 3-Isoxazolyl ausgewählt ist; oder ein pharmazeutisch annehmbares Salz davon.

12. Chinazolinderivat der Formel Ia nach einem oder mehreren der Ansprüche 8 bis 11, worin Z-X³ für Hydroxymethyl steht; oder ein pharmazeutisch annehmbares Salz davon.

13. Chinazolinderivat nach Anspruch 1, ausgewählt unter einer oder mehreren der folgenden Verbindungen:
2-((2R)-2-{[(4-{[3-Chlor-4-(pyridin-2-ylmethoxy)-phenyl]amino}chinazolin-5-yl)oxy]methyl}-pyrrolidin-1-yl)-2-oxoethanol;
2-((2S)-2-{[(4-{[3-Chlor-4-(pyridin-2-ylmethoxy)-phenyl]amino}chinazolin-5-yl)oxy]methyl}-pyrrolidin-1-yl)-2-oxoethanol;
N-[3-Chlor-4-(pyridin-2-ylmethoxy)phenyl]-5-({(2R)-1-[(dimethylamino)acetyl]pyrrolidin-2-yl}-methoxy)chinazolin-4-amin;
N-[3-Chlor-4-(pyridin-2-ylmethoxy)phenyl]-5-({(2S)-1-[(dimethylamino)acetyl]pyrrolidin-2-yl}-methoxy)chinazolin-4-amin;
1-((2R)-2-{[(4-{[3-Chlor-4-(pyridin-2-ylmethoxy)-phenyl]amino}chinazolin-5-yl)oxy]methyl}-pyrrolidin-1-yl)-2-methyl-1-oxopropan-2-ol;
1-[((2R)-2-{[(4-{[3-Chlor-4-(pyridin-2-ylmethoxy)-phenyl]amino}chinazolin-5-yl)oxy]methyl}-pyrrolidin-1-yl)carbonyl]cyclopropanol;
3-((2R)-2-{[(4-{[3-Chlor-4-(pyridin-2-ylmethoxy)-phenyl]amino}chinazolin-5-yl)oxy]methyl}-pyrrolidin-1-yl)-2,2-dimethyl-3-oxopropan-1-ol;
(2S)-1-((2R)-2-{[(4-{[3-Chlor-4-(pyridin-2-ylmethoxy)phenyl]amino}chinazolin-5-yl)oxy]-methyl}pyrrolidin-1-yl)-1-oxopropan-2-ol;
N-[3-Chlor-4-(pyridin-2-ylmethoxy)phenyl]-5-({(2R)-1-[(ethoxyacetyl)pyrrolidin-2-yl}-methoxy)chinazolin-4-amin;
N-[3-Chlor-4-(pyridin-2-ylmethoxy)phenyl]-5-({(2R)-1-[(methoxyacetyl)pyrrolidin-2-yl}-methoxy)chinazolin-4-amin;
N-[3-Chlor-4-(1,3-thiazol-4-ylmethoxy)phenyl]-5-({(2R)-1-[(dimethylamino)acetyl]pyrrolidin-2-yl}-methoxy)chinazolin-4-amin;
2-((2R)-2-{[(4-{[3-Chlor-4-(1,3-thiazol-4-yl-methoxy)phenyl]amino}chinazolin-5-yl)oxy]methyl}-pyrrolidin-1-yl)-2-oxoethanol;
N-{3-Chlor-4-[(5-methylisoxazol-3-yl)methoxy]-phenyl}-5-({(2R)-1-[(dimethylamino)acetyl]-pyrrolidin-2-yl}methoxy)chinazolin-4-amin;
2-[(2R)-2-({[4-({3-Chlor-4-[(5-methylisoxazol-3-yl)methoxy]phenyl}amino)chinazolin-5-yl]oxy}-methyl)pyrrolidin-1-yl]-2-oxoethanol;
N-[3-Chlor-4-(1,3-thiazol-5-ylmethoxy)phenyl]-5-({(2R)-1-[(dimethylamino)acetyl]pyrrolidin-2-yl}-methoxy)chinazolin-4-amin;
2-((2R)-2-{[(4-{[3-Chlor-4-(1,3-thiazol-5-yl-methoxy)phenyl]amino}chinazolin-5-yl)oxy]methyl}-pyrrolidin-1-yl)-2-oxoethanol;
*N*-[3-Chlor-4-(pyrazin-2-ylmethoxy)phenyl]-5-({(2R)-1-[(dimethylamino)acetyl]pyrrolidin-2-yl}-methoxy)chinazolin-4-amin;
2-((2R)-2-{[(4-{[3-Chlor-4-(pyrazin-2-ylmethoxy)-phenyl]amino}chinazolin-5-yl)oxy]methyl}-pyrrolidin-1-yl)-2-oxoethanol;
(2R)-1-((2R)-2-{[(4-{[3-Chlor-4-(pyridin-2-ylmethoxy)phenyl]amino}chinazolin-5-yl)oxy]-methyl}pyrrolidin-1-yl)-1-oxopropan-2-ol;
2-[(2S)-2-({[4-({3-Chlor-4-[(6-methylpyridin-2-yl)methoxy]phenyl}amino)chinazolin-5-yl]oxy}-methyl)pyrrolidin-1-yl]-2-oxoethanol;
2-[(2S)-2-({[4-({3-Chlor-4-[(2-fluorbenzyl)oxy]-phenyl}amino)chinazolin-5-yl]oxy}methyl)-pyrrolidin-1-yl]-2-oxoethanol;
2-[(2S)-2-({[4-({3-Chlor-4-[(3-fluorbenzyl)oxy]-phenyl}amino)chinazolin-5-yl]oxy}methyl)-pyrrolidin-1-yl]-2-oxoethanol;
2-((2S)-2-{[(4-{[3-Chlor-4-(1,3-thiazol-4-ylmethoxy)phenyl]amino}chinazolin-5-yl)oxy]-methyl}pyrrolidin-1-yl)-2-oxoethanol;
2-((2S)-2-{[(4-{[3-Chlor-4-(pyrazin-2-ylmethoxy)-phenyl]amino}chinazolin-5-yl)oxy]methyl}-pyrrolidin-1-yl)-2-oxoethanol;
*N*-{3-Chlor-4-[(6-methylpiperidin-2-yl)methoxy]-phenyl}-5-({(2S)-1-[(dimethylamino)acetyl]-pyrrolidin-2-yl}methoxy)chinazolin-4-amin;
*N*-{3-Chlor-4-[(2-fluorbenzyl)oxy]phenyl}-5-({(2S)-1-[(dimethylamino)acetyl]pyrrolidin-2-yl}-methoxy)chinazolin-4-amin;
*N*-{3-Chlor-4-[(3-fluorbenzyl)oxy]phenyl}-5-({(2S)-1-[(dimethylamino)acetyl]pyrrolidin-2-yl}-methoxy)chinazolin-4-amin;
*N*-[3-Chlor-4-(pyrazin-2-ylmethoxy)phenyl]-5-({(2S)-1-[(dimethylamino)acetyl]pyrrolidin-2-yl}-methoxy)chinazolin-4-amin;
*N*-[3-Chlor-4-(1,3-thiazol-4-ylmethoxy)phenyl]-5-({(2S)-1-[(dimethylamino)acetyl]pyrrolidin-2-yl}-methoxy)chinazolin-4-amin;
2-((2S)-2-{[(4-{[3-Methyl-4-(pyridin-2-ylmethoxy)-phenyl]amino}chinazolin-5-yl)oxy]methyl}-pyrrolidin-1-yl)-2-oxoethanol;
2-((2S)-2-{[(4-{[3-Methyl-4-(pyrazin-2-ylmethoxy)-phenyl]amino}chinazolin-5-yl)oxy]methyl}-pyrrolidin-1-yl)-2-oxoethanol;
2-((2R)-2-{[(4-{[3-Methyl-4-(pyridin-2-ylmethoxy)-phenyl]amino}chinazolin-5-yl)oxy]methyl}-pyrrolidin-1-yl)-2-oxoethanol;
2-((2R)-2-{[(4-{[3-Methyl-4-(pyrazin-2-ylmethoxy)-phenyl]amino}chinazolin-5-yl)oxy]methyl}-pyrrolidin-1-yl)-2-oxoethanol;
2-((2R)-2-{[(4-{[3-Methyl-4-(1,3-thiazol-4-ylmethoxy)phenyl]amino}chinazolin-5-yl)oxy]-methyl}pyrrolidin-1-yl)-2-oxoethanol;
2-[(2R)-2-({[4-({3-Methyl-4-[(5-methylisoxazol-3-yl)methoxy]phenyl}amino)chinazolin-5-yl]oxy}-methyl)pyrrolidin-1-yl]-2-oxoethanol;
5-{[(2R)-1-(Methoxyacetyl)pyrrolidin-2-yl]-methoxy}-*N*-{3-methyl-4-[(5-methylisoxazol-3-yl)-methoxy]phenyl}chinazolin-4-amin;
5-{[(2R)-1-(Methoxyacetyl)pyrrolidin-2-yl]-methoxy}-*N*-[3-methyl-4-(pyridin-2-ylmethoxy)-phenyl]chinazolin-4-amin;
5-{[(2R)-1-(Methoxyacetyl)pyrrolidin-2-yl]-methoxy}-*N*-[3-methyl-4-(1,3-thiazol-4-ylmethoxy)-phenyl]chinazolin-4-amin;
oder ein pharmazeutisch annehmbares Salz davon.

14. Pharmazeutische Zusammensetzung, die ein Chinazolinderivat der Formel Ia oder ein pharmazeutisch annehmbares Salz davon nach einem der Ansprüche 1 bis 13 zusammen mit einem pharmazeutisch annehmbaren Verdünnungsmittel oder Träger enthält.

15. Chinazolinderivat der Formel Ia oder ein pharmazeutisch annehmbares Salz davon nach einem der Ansprüche 1 bis 13 zur Verwendung als Arzneimittel.

16. Chinazolinderivat der Formel Ia oder ein pharmazeutisch annehmbares Salz davon nach einem der Ansprüche 1 bis 13 zur Verwendung bei der Hervorrufung einer antiproliferativen Wirkung, wobei die Wirkung alleine oder teilweise durch Inhibierung von erbB2-Rezeptor-Tyrosinkinase hervorgerufen wird, bei einem Warmblüter wie dem Menschen.

17. Chinazolinderivat der Formel Ia oder ein pharmazeutisch annehmbares Salz davon nach einem der Ansprüche 1 bis 13 zur Verwendung bei der Hervorrufung einer Hemmwirkung gegenüber erbB2-Rezeptor-Tyrosinkinase bei einem Warmblüter wie dem Menschen.

18. Chinazolinderivat der Formel Ia oder ein pharmazeutisch annehmbares Salz davon nach einem der Ansprüche 1 bis 13 zur Verwendung bei der Hervorrufung einer selektiven Hemmwirkung gegenüber erbB2-Rezeptor-Tyrosinkinase bei einem Warmblüter wie dem Menschen.

19. Verfahren zur Herstellung eines Chinazolinderivats der Formel Ia oder eines pharmazeutisch annehmbaren Salzes davon nach Anspruch 1, bei dem man:
(a) ein Chinazolin der Formel **II:** 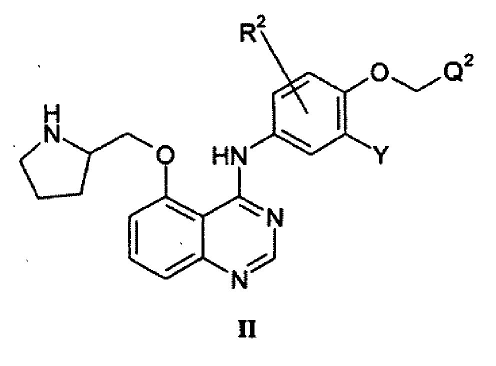 worin R², Y und Q² eine der in einem der Ansprüche 1 bis 12 angegebenen Bedeutungen besitzen, wobei jedoch jede funktionelle Gruppe gegebenenfalls geschützt ist, mit einer Carbonsäure der Formel **III** oder einem reaktiven Derivat davon:
2-X³-COOH **III**
worin Z und X³ eine der in einem der Ansprüche 1 bis 12 angegebenen Bedeutungen besitzen, wobei jedoch jede funktionelle Gruppe gegebenenfalls geschützt ist, kuppelt, zweckmäßigerweise in Gegenwart einer geeigneten Base; oder
(b) ein Chinazolin der Formel **IV:** 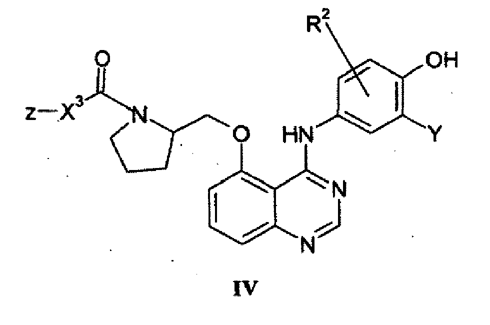 worin R², X³, Z und Y eine der in einem der Ansprüche 1 bis 12 angegebenen Bedeutungen besitzen, wobei jedoch jede funktionelle Gruppe gegebenenfalls geschützt ist, mit einer Verbindung der Formel **V:**
Q²-CH₂-L¹ **V**
worin L¹ für eine geeignete verdrängbare Gruppe steht und Q² eine der in einem der Ansprüche 1 bis 12 angegebenen Bedeutungen besitzt, wobei jedoch jede funktionelle Gruppe gegebenenfalls geschützt ist, umsetzt, zweckmäßigerweise in Gegenwart einer geeigneten Base;
(c) ein Chinazolin der Formel **VI:** 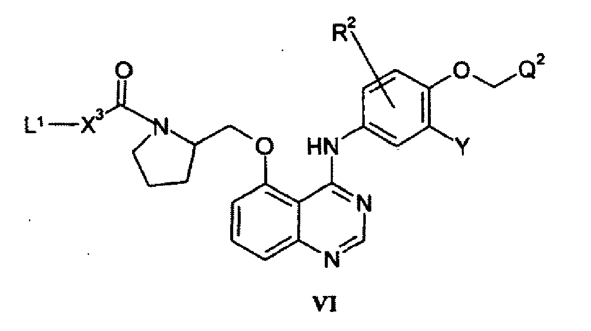
worin L¹ für eine geeignete verdrängbare Gruppe steht und R², X³, Y und Q² eine der in einem der Ansprüche 1 bis 12 angegebenen Bedeutungen besitzen, wobei jedoch jede funktionelle Gruppe gegebenenfalls geschützt ist, mit einer Verbindung der Formel **VII** oder einem reaktiven Derivat davon:
Z-H **VII**
worin Z eine der in einem der Ansprüche 1 bis 12 angegebenen Bedeutungen besitzt, wobei jedoch jede funktionelle Gruppe gegebenenfalls geschützt ist, kuppelt; und danach gegebenenfalls:
(i) ein Chinazolinderivat der Formel I in ein anderes Chinazolinderivat der Formel I umwandelt;
(ii) jede vorhandene Schutzgruppe mit herkömmlichen Mitteln abspaltet;
(iii) ein pharmazeutisch annehmbares Salz bildet.

## Revendications

1. Dérivé de la quinazoline de formule Ia: 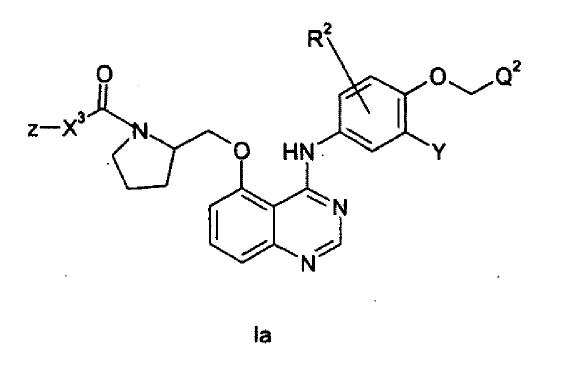 dans laquelle:
**R²** est choisi parmi hydrogène et halogéno;
**Y** est choisi parmi halogéno, C₁₋₄-alkyle, C₁₋₄-alcoxy, C₂₋₄-alcényle et C₂₋₄-alcynyle;
**Q²** est choisi parmi phényle, pyridyle, pyrazinyle, 1,3-thiazolyle et isoxazolyle
et où Q² porte éventuellement un ou plusieurs substituants, qui peuvent être identiques ou différents, choisis parmi halogéno, hydroxy, cyano, carboxy, nitro, amino, C₁₋₄-alkyle, C₁₋₄-alcoxy, C₂₋₄-alcényle, C₂₋₄-alcynyle, C₁₋₄-alkylthio, C₁₋₄-alkylsulfinyle, C₁₋₄-alkylsulfonyle, C₂₋₄-alcanoyle, N-C₁₋₄-alkylamino, N,N-di(C₁₋₄-alkyl)amino, C₁₋₄-alcoxycarbonyle, carbamoyle, N-C₁-₄-alkylcarbamoyle, N,N-di(C₁₋₄-alkyl)carbamoyle, C₂₋₄-alcanoyloxy, C₂₋₄-alcanoylamino, N-C₁₋₄-alkyl-C₂₋₄-alcanoylamino, halogéno-C₁₋₄-alkyle, hydroxy-C₁₋₄-alkyle, C₁₋₄-alcoxy-C₁₋₄-alkyle, cyano-C₁₋₄-alkyle, carboxy-C₁₋₄-alkyle, amino-C₁₋₄-alkyle, N-C₁₋₄-alkylamino-C₁₋₄-alkyle et N,N-di(C₁₋₄-alkyl)amino-C₁₋₄-alkyle;
**Z** est choisi parmi hydroxy, di(C₁₋₃-alkyl)amino et C₁₋₆-alcoxy;
**X³** est choisi parmi -CH₂-, -CH₂CH₂-, -(CR⁸R⁹)-, -(CR⁸R⁹CH₂)-, -(CH₂CR⁸R⁹)- et C₃₋₆-cycloalkylène, où chacun parmi R⁸ et R⁹, qui peuvent être identiques ou différents, est choisi parmi hydrogène et méthyle ;
ou un sel pharmaceutiquement acceptable de celui-ci.

2. Dérivé de la quinazoline de formule Ia tel que défini dans la revendication 1, **caractérisé en ce que** Y est halogéno ou méthyle ; ou un sel pharmaceutiquement acceptable de celui-ci.

3. Dérivé de la quinazoline de formule Ia tel que défini dans la revendication 2, **caractérisé en ce que** Y est chloro ; ou un sel pharmaceutiquement acceptable de celui-ci.

4. Dérivé de la quinazoline de formule Ia tel que défini dans l'une quelconque ou plusieurs des revendications 1 à 3, **caractérisé en ce que** Q² est choisi parmi phényle, 2-pyridyle, 2-pyrazinyle, 1,3-thiazol-4-yle, 1,3-thiazol-5-yle et 3-isoxazolyle ; ou un sel pharmaceutiquement acceptable de celui-ci.

5. Dérivé de la quinazoline de formule Ia tel que défini dans l'une quelconque ou plusieurs des revendications 1 à 4, **caractérisé en ce que** X³ est cyclopropylidène ; ou un sel pharmaceutiquement acceptable de celui-ci.

6. Dérivé de la quinazoline de formule Ia tel que défini dans l'une quelconque ou plusieurs des revendications 1 à 5, **caractérisé en ce que** Z est choisi parmi hydroxy, diméthylamino, méthoxy et éthoxy ; ou un sel pharmaceutiquement acceptable de celui-ci.

7. Dérivé de la quinazoline de formule Ia tel que défini dans la revendication 7, **caractérisé en ce que** Z est hydroxy ; ou un sel pharmaceutiquement acceptable de celui-ci.

8. Dérivé de la quinazoline de formule Ia tel que défini dans la revendication 1, **caractérisé en ce que**
**Q²** est choisi parmi pyridyle, pyrazinyle, 1,3-thiazolyle et isoxazolyle
et où Q² porte éventuellement un ou plusieurs substituants, qui peuvent être identiques ou différents, choisis parmi halogéno, hydroxy, cyano, carboxy, nitro, amino, C₁₋₄-alkyle, C₁₋₄-alcoxy, C₂₋₄-alcényle, C₂₋₄-alcynyle, C₁₋₄-alkylthio, C₁₋₄-alkylsulfinyle, C₁₋₄-alkylsulfonyle, C₂₋₄-alcanoyle, N-C₁₋₄-alkylamino, N,N-di(C₁₋₄-alkyl)amino, C₁₋₄-alcoxycarbonyle, carbamoyle, N-C₁₋₄-alkylcarbamoyle, N,N-di(C₁₋₄-alkyl)carbamoyle, C₂₋₄-alcanoyloxy, C₂₋₄-alcanoylamino, N-C₁₋₄-alkyl-C₂₋₄-alcanoylamino, halogéno-C₁₋₄-alkyle, hydroxy-C₁₋₄-alkyle, C₁₋₄-alcoxy-C₁₋₄-alkyle, cyano-C₁₋₄-alkyle, carboxy-C₁₋₄-alkyle, amino-C₁₋₄-alkyle, N-C₁₋₄-alkylamino-C₁₋₄-alkyle et N,N-di(C₁₋₄-alkyl)amino-C₁₋₄-alkyle ;
**X³** est choisi parmi -CH₂- et -CH₂CH₂- ;
**Z** est choisi parmi hydroxy et di(C₁₋₃-alkyl)amino ; et
**R²** et **Y** sont tels que définis dans la revendication 1 ;
ou un sel pharmaceutiquement acceptable de celui-ci.

9. Dérivé de la quinazoline de formule Ia tel que défini dans la revendication 8, **caractérisé en ce que** Y est choisi parmi halogéno et méthyle ; ou un sel pharmaceutiquement acceptable de celui-ci.

10. Dérivé de la quinazoline de formule Ia tel que défini dans la revendication 9, **caractérisé en ce que** Y est chloro ; ou un sel pharmaceutiquement acceptable de celui-ci.

11. Dérivé de la quinazoline de formule Ia tel que défini dans l'une quelconque ou plusieurs des revendications 8 à 10, **caractérisé en ce que** Q² est choisi parmi 2-pyridyle, 2-pyrazinyle, 1,3-thiazol-4-yle, 1,3-thiazol-5-yle et 3-isoxazolyle ; ou un sel pharmaceutiquement acceptable de celui-ci.

12. Dérivé de la quinazoline de formule Ia tel que défini dans l'une quelconque ou plusieurs des revendications 8 à 11, **caractérisé en ce que** Z-X³ est hydroxyméthyle ; ou un sel pharmaceutiquement acceptable de celui-ci.

13. Dérivé de la quinazoline tel que défini dans la revendication 1, choisi parmi un ou plusieurs des composés suivantes :
le 2-((2R)-2-{[(4-{[3-chloro-4-(pyridin-2-ylméthoxy)phényl]amino}quinazolin-5-yl)oxy]méthyl}pyrrolidin-1-yl)-2-oxoéthanol ;
le 2-((2S)-2-{[(4-{[3-chloro-4-(pyridin-2-ylméthoxy)phényl]amino}quinazolin-5-yl)oxy]méthyl}pyrrolidin-1-yl)-2-oxoéthanol ;
la *N*-[3-chloro-4-(pyridin-2-ylméthoxy)phényl]-5-({(2R)-1-[(diméthylamino)acétyl]pyrrolidin-2-yl}méthoxy)quinazolin-4-amine ;
la *N*-[3-chloro-4-(pyridin-2-ylméthoxy)phényl]-5-({(2S)-1-[(diméthylamino)acétyl]pyrrolidin-2-yl}méthoxy)quinazolin-4-amine ;
le 1-((2R)-2-{[(4-{[3-chloro-4-(pyridin-2-ylméthoxy)phényl]amino}quinazolin-5-yl)oxy]méthyl}pyrrolidin-1-yl)-2-méthyl-1-oxopropan-2-ol ;
le 1-[((2R)-2-{[(4-{[3-chloro-4-(pyridin-2-ylméthoxy)phényl]amino}quinazolin-5-yl)oxy]méthyl}pyrrolidin-1-yl)carbonyl]cyclopropanol ;
le 3-((2R)-2-{[(4-{[3-chloro-4-(pyridin-2-ylméthoxy)phényl]amino}quinazolin-5-yl)oxy]méthyl}pyrrolidin-1-yl)-2,2-diméthyl-3-oxopropan-1-ol ;
le (2S)-1-((2R)-2-{[(4-{[3-chloro-4-(pyridin-2-ylméthoxy)phényl]amino}quinazolin-5-yl)oxy]méthyl}pyrrolidin-1-yl)-1-oxopropan-2-ol ;
la *N*-[3-chloro-4-(pyridin-2-ylméthoxy)phényl]-5-{[(2R)-1-(éthoxyacétyl)pyrrolidin-2-yl]méthoxy}quinazolin-4-amine ;
la *N*-[3-chloro-4-(pyridin-2-ylméthoxy)phényl]-5-{[(2R)-1-(méthoxyacétyl)pyrrolidin-2-yl]méthoxy}quinazolin-4-amine ;
la *N*-[3-chloro-4-(1,3-thiazol-4-ylméthoxy)phényl]-5-({(2R)-1-[(diméthylamino)acétyl]pyrrolidin-2-yl}méthoxy)quinazolin-4-amine ;
le 2-((2R)-2-{[(4-{[3-chloro-4-(1,3-thiazol-4-ylméthoxy)phényl]amino}quinazolin-5-yl)oxy]méthyl}pyrrolidin-1-yl)-2-oxoéthanol ;
la *N*-{3-chloro-4-[(5-méthylisoxazol-3-yl)méthoxy]phényl}-5-({(2R)-1-[(diméthylamino)acétyl]pyrrolidin-2-yl}méthoxy)quinazolin-4-amine ;
le 2-[(2R)-2-({[4-({3-chloro-4-[(5-méthylisoxazol-3-yl)méthoxy]phényl}amino)quinazolin-5-yl]oxy}méthyl)pyrrolidin-1-yl]-2-oxoéthanol ;
la *N*-[3-chloro-4-(1,3-thiazol-5-ylméthoxy)phényl]-5-({(2R)-1-[(diméthylamino)acétyl]pyrrolidin-2-yl}méthoxy)quinazolin-4-amine ;
le 2-((2R)-2-{[(4-{[3-chloro-4-(1,3-thiazol-5-ylméthoxy)phényl]amino}quinazolin-5-yl)oxy]méthyl}pyrrolidin-1-yl)-2-oxoéthanol ;
la *N*-[3-chloro-4-(pyrazin-2-ylméthoxy)phényl]-5-({(2R)-1-[(diméthylamino)acétyl]pyrrolidin-2-yl}méthoxy)quinazolin-4-amine ;
le 2-((2R)-2-{[(4-{[3-chloro-4-(pyrazin-2-ylméthoxy)phényl]amino}quinazolin-5-yl)oxy]méthyl}pyrrolidin-1-yl)-2-oxoéthanol ;
le (2R)-1-((2R)-2-{[(4-{[3-chloro-4-(pyridin-2-ylméthoxy)phényl]amino}quinazolin-5-yl)oxy]méthyl}pyrrolidin-1-yl)-1-oxopropan-2-ol ;
le 2-[(2S)-2-({[4-({3-chloro-4-[(6-méthylpyridin-2-yl)méthoxy]phényl}amino)quinazolin-5-yl]oxy}méthyl)pyrrolidin-1-yl]-2-oxoéthanol ;
le 2-[(2S)-2-({[4-({3-chloro-4-[(2-fluorobenzyl)oxy]phényl}amino)quinazolin-5-yl]oxy}méthyl)pyrrolidin-1-yl]-2-oxoéthanol ;
le 2-[(2S)-2-({[4-({3-chloro-4-[(3-fluorobenzyl)oxy]phényl}amino)quinazolin-5-yl]oxy}méthyl)pyrrolidin-1-yl]-2-oxoéthanol ;
le 2-((2S)-2-{[(4-{[3-chloro-4-(1,3-thiazol-4-ylméthoxy)phényl]amino}quinazolin-5-yl)oxy]méthyl}pyrrolidin-1-yl)-2-oxoéthanol ;
le 2-((2S)-2-{[(4-{[3-chloro-4-(pyrazin-2-ylméthoxy)phényl]amino}quinazolin-5-yl)oxy]méthyl}pyrrolidin-1-yl)-2-oxoéthanol ;
la *N*-{3-chloro-4-[(6-méthylpyridin-2-yl)méthoxy]phényl}-5-({(2S)-1-[(diméthylamino)acétyl]pyrrolidin-2-yl}méthoxy)quinazolin-4-amine ;
la *N*-{3-chloro-4-[(2-fluorobenzyl)oxy]phényl}-5-({(2S)-1-[(diméthylamino)acétyl]pyrrolidin-2-yl}méthoxy)quinazolin-4-amine ;
la *N*-{3-chloro-4-[(3-fluorobenzyl)oxy]phényl}-5-({(2S)-1-[(diméthylamino)acétyl]pyrrolidin-2-yl}méthoxy)quinazolin-4-amine ;
la *N*-[3-chloro-4-(pyrazin-2-ylméthoxy)phényl]-5-({(2S)-1-[(diméthylamino)acétyl]pyrrolidin-2-yl}méthoxy)quinazolin-4-amine ;
la *N*-[3-chloro-4-(1,3-thiazol-4-ylméthoxy)phényl]-5-({(2S)-1-[(diméthylamino)acétyl]pyrrolidin-2-yl}méthoxy)quinazolin-4-amine ;
le 2-((2S)-2-{[(4-{[3-méthyl-4-(pyridin-2-ylméthoxy)phényl]amino}quinazolin-5-yl)oxy]méthyl}pyrrolidin-1-yl)-2-oxoéthanol ;
le 2-((2S)-2-{[(4-{[3-méthyl-4-(pyrazin-2-ylméthoxy)phényl]amino}quinazolin-5-yl)oxy]méthyl}pyrrolidin-1-yl)-2-oxoéthanol ;
le 2-((2R)-2-{[(4-{[3-méthyl-4-(pyridin-2-ylméthoxy)phényl]amino}quinazolin-5-yl)oxy]méthyl}pyrrolidin-1-yl)-2-oxoéthanol ;
le 2-((2R)-2-{[(4-{[3-méthyl-4-(pyrazin-2-ylméthoxy)phényl]amino}quinazolin-5-yl)oxy]méthyl}pyrrolidin-1-yl)-2-oxoéthanol ;
le 2-((2R)-2-{[(4-{[3-méthyl-4-(1,3-thiazol-4-ylméthoxy)phényl]amino}quinazolin-5-yl)oxy]méthyl}pyrrolidin-1-yl)-2-oxoéthanol ;
le 2-[(2R)-2-({[4-({3-méthyl-4-[(5-méthylisoxazol-3-yl)méthoxy]phényl}amino)quinazolin-5-yl]oxy}méthyl)pyrrolidin-1-yl]-2-oxoéthanol ;
la 5-{[(2R)-1-(méthoxyacétyl)pyrrolidin-2-yl]méthoxy}-*N*-{3-méthyl-4-[(5-méthylisoxazol-3-yl)méthoxy]phényl}quinazolin-4-amine ;
la 5-{[(2R)-1-(méthoxyacétyl)pyrrolidin-2-yl]méthoxy}-*N*-[3-méthyl-4-(pyridin-2-ylméthoxy)phényl]quinazolin-4-amine ;
la 5-{[(2R)-1-(méthoxyacétyl)pyrrolidin-2-yl]méthoxy}-*N*-[3-méthyl-4-(1,3-thiazol-4-ylméthoxy)phényl]quinazolin-4-amine ;
ou un sel pharmaceutiquement acceptable de ceux-ci.

14. Composition pharmaceutique, **caractérisée en ce qu'**elle comprend un dérivé de la quinazoline de formule Ia, ou un sel pharmaceutiquement acceptable de celui-ci, tel que défini dans l'une quelconque des revendications 1 à 13, en association avec un diluant ou un support pharmaceutiquement acceptable.

15. Dérivé de la quinazoline de formule Ia, ou un sel pharmaceutiquement acceptable de celui-ci, tel que défini dans l'une quelconque des revendications 1 à 13, destiné à être utilisé comme médicament.

16. Dérivé de la quinazoline de formule Ia, ou un sel pharmaceutiquement acceptable de celui-ci, tel que défini dans l'une quelconque des revendications 1 à 13, destiné à être utilisé dans la production d'un effet anti-prolifératif, lequel effet est produit uniquement ou en partie par l'inhibition du récepteur tyrosine kinase erbB2 chez un animal à sang chaud tel que l'homme.

17. Dérivé de la quinazoline de formule Ia, ou un sel pharmaceutiquement acceptable de celui-ci, tel que défini dans l'une quelconque des revendications 1 à 13, destiné à être utilisé dans la production d'un effet inhibiteur du récepteur tyrosine kinase erbB2 chez un animal à sang chaud tel que l'homme.

18. Dérivé de la quinazoline de formule Ia, ou un sel pharmaceutiquement acceptable de celui-ci, tel que défini dans l'une quelconque des revendications 1 à 13, destiné à être utilisé dans la production d'un effet inhibiteur sélectif du récepteur tyrosine kinase erbB2 chez un animal à sang chaud tel que l'homme.

19. Procédé de préparation d'un dérivé de la quinazoline de formule Ia, ou d'un sel pharmaceutiquement acceptable de celui-ci, tel que défini dans la revendication 1, **caractérisé en ce qu'**il comprend:
(a) le couplage, commodément en présence d'une base convenable, d'une quinazoline de formule **II :** 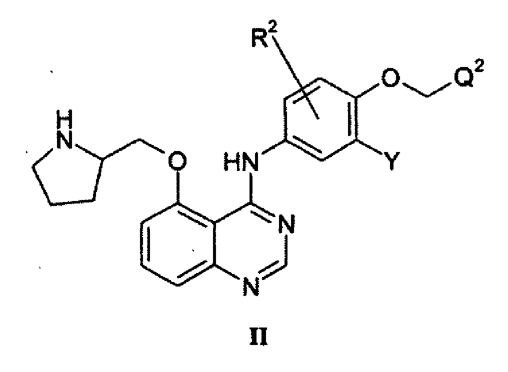 dans laquelle R², Y et Q² ont l'une quelconque des significations définies dans l'une quelconque des revendications 1 à 12, sauf qu'une fonction quelconque est protégée le cas échéant, avec un acide carboxylique de formule **III,** ou un dérivé réactif de celui-ci:
Z-X³COOH **III**
dans laquelle Z et X³ ont l'une quelconque des significations définies dans l'une quelconque des revendications 1 à 12, sauf qu'une fonction quelconque est protégée le cas échéant ; ou
(b) la réaction, commodément en présence d'une base convenable, d'une quinazoline de formule **IV :** 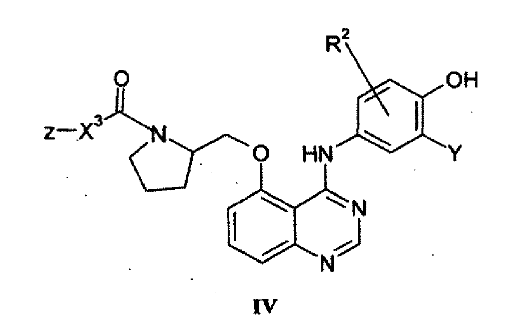 dans laquelle R², X³, Z et Y ont l'une quelconque des significations définies dans l'une quelconque des revendications 1 à 12, sauf qu'une fonction quelconque est protégée le cas échéant, avec un composé de formule **V :**
Q²-CH₂-L¹ **V**
dans laquelle L¹ est un groupement déplaçable convenable et Q² a l'une quelconque des significations définies dans l'une quelconque des revendications 1 à 12, sauf qu'une fonction quelconque est protégée le cas échéant ;
(c) le couplage d'une quinazoline de formule **VI :** 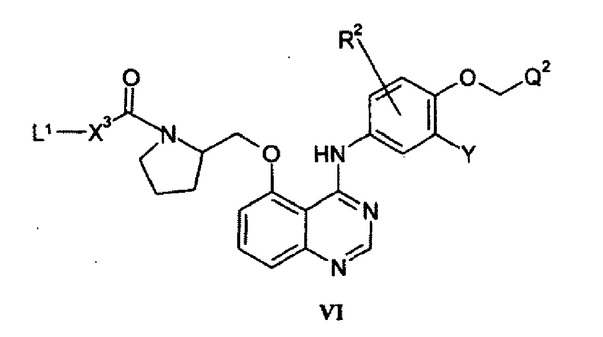
dans laquelle L¹ est un groupement déplaçable convenable et R², X³, Y et Q² ont l'une quelconque des significations définies dans l'une quelconque des revendications 1 à 12, sauf qu'une fonction quelconque est protégée le cas échéant, avec un composé de formule **VII,** ou un dérivé réactif de celui-ci:
Z-H **VII**
dans laquelle Z a l'une quelconque des significations définies dans l'une quelconque des revendications 1 à 12, sauf qu'une fonction quelconque est protégée le cas échéant ; et ensuite, le cas échéant:
(i) la transformation d'un dérivé de la quinazoline de formule I en un autre dérivé de la quinazoline de formule I ;
(ii) l'élimination de tout groupement protecteur qui est présent par des moyens classiques ;
(iii) la formation d'un sel pharmaceutiquement acceptable.
